# EUROPEAN PATENT SPECIFICATION

(11) **EP 4 157 844 B1**
(45) Date of publication and mention of the grant of the patent: **10.07.2024**
(21) Application number: 21728071.8
(22) Date of filing: 25.05.2021
(51) Int. Cl.: C07D 487/04, C07D 491/14, C07D 471/14, A61K 31/519, A61P 35/00, A61P 15/10, A61P 17/06, A61P 25/00, A61P 25/02, A61P 25/04, A61P 27/02, A61P 27/06, A61P 29/00, A61P 31/12

(54) **4-(7H-PYRROLO[2,3-D]PYRIMIDIN-4-YL)-3,6-DIHYDROPYRIDINE-1-(2H)-CARBOXAMIDE DERIVATIVES AS LIMK AND/OR ROCK KINASES INHIBITORS FOR USE IN THE TREATMENT OF CANCER**
4-(7H-PYRROLO[2,3-D]PYRIMIDIN-4-YL)-3,6-DIHYDROPYRIDIN-1-(2H)-CARBOXAMID-DERIVATE ALS LIMK UND/ODER ROCK KINASE-INHIBITOREN ZUR VERWENDUNG IN DER BEHANDLUNG VON KREBS
DÉRIVÉS DE 4-(7H-PYRROLO[2,3-D]PYRIMIDIN-4-YL)-3,6-DIHYDROPYRIDINE-1-(2H)-CARBOXAMIDE EN TANT QU'INHIBITEURS DE LIMK ET/OU ROCK KINASES POUR LE TRAITEMENT DU CANCER

(30) Priority: 26.05.2020 EP 20305546
(43) Date of publication of application: 05.04.2023
(73) Proprietor: Centre National de la Recherche Scientifique, 75016 Paris (FR); Université d'Orléans, 45100 Orléans (FR); Sorbonne Université, 75006 Paris (FR); Institut National de la Santé et de la Recherche Médicale, 75013 Paris (FR); Université de Tours, 37000 Tours (FR); Centre Hospitalier Régional Universitaire de Tours, 37000 Tours (FR)
(72) Inventor: ROUTIER, Sylvain, 45510 Tigy (FR); BENEDETTI, Hélène, 45800 Saint-Jean-de-Braye (FR); VALLÉE-MÉHEUST, Béatrice, 45100 Orléans (FR); BONNET, Pascal, 45160 Olivet (FR); PLÉ, Karen, 41220 Thoury (FR); ACI-SÈCHE, Samia, 45160 Olivet (FR); RUCHAUD, Sandrine, 29250 Saint-Pol-de-Léon (FR); BRAKA, Abdennour, 34090 Montpellier (FR); CHAMPIRÉ, Anthony, 45160 Olivet (FR); ANDRES, Christian, 37390 Cerelles (FR); VOURC'H, Patrick, 37510 Savonnieres (FR)
(74) Representative: Novagraaf Technologies
(86) International application number: PCT/EP2021/063890
(87) International publication number: WO 2021/239727

(56) References cited:
- BRYCE A. HARRISON ET AL: "Discovery and Development of LX7101, a Dual LIM-Kinase and ROCK Inhibitor for the Treatment of Glaucoma", ACS MEDICINAL CHEMISTRY LETTERS, vol. 6, no. 1, 9 December 2014 (2014-12-09), US, pages 84 - 88, XP055737326, ISSN: 1948-5875, DOI: 10.1021/ml500367g

## Description

The present invention concerns new inhibitors of LIMK and/or ROCK kinases, and therapeutic uses thereof. The present invention also concerns these new inhibitors as anticancer agents.

LIM kinases (LIMKs) are serine/threonine and tyrosine kinases involved in regulating cytoskeleton dynamics. They phosphorylate cofilin, resulting in its inhibition (Scott, R. W. and Olson, M. F. (2007) LIM kinases: function, regulation and association with human disease. Journal of molecular medicine (Berlin, Germany). 85, 555-568). Cofilin is a member of the Actin Depolymerizing Factor (ADF) family and regulates actin polymerization dynamics by promoting rapid turnover of actin filaments (Bamburg, J. R. and Bernstein, B. W. (2010) Roles of ADF/cofilin in actin polymerization and beyond. F1000 biology reports. 2, 62; Mizuno, K. (2013) Signaling mechanisms and functional roles of cofilin phosphorylation and dephosphorylation. Cellular signalling. 25, 457-469; Pollard, T. D. and Borisy, G. G. (2003) Cellular motility driven by assembly and disassembly of actin filaments. Cell. 112, 453-465). LIMKs also control microtubule dynamics, independently from their regulation of actin remodeling. The molecular mechanism by which LIMKs regulate microtubule dynamics is still unknown (Prunier, C., Prudent, R., Kapur, R., Sadoul, K. and Lafanechere, L. (2017) LIM kinases: cofilin and beyond. Oncotarget. 8, 41749-41763). LIMKs thus play a crucial role in cytoskeleton remodeling, contributing to many cellular functions, such as cell motility, morphogenesis, division, differentiation, apoptosis, neuronal morphology, neuritogenesis, and oncogenesis.

The LIMK family consists of only two members: LIMK1 and LIMK2. Recently, LlMKs have emerged as new therapeutic targets as they have been shown to be involved in many diseases, for example in numerous cancers, in viral infection, in ocular hypertension and glaucoma formation. They also play a role in Neurofibromatosis type 1 and 2 (NF1 and NF2). LIMKs are also involved in psoriatic lesions, in the inflammatory response, in the development of inflammatory hyperalgesia, central sensitization and chronic pain, in oocyte maturation, and in erectile dysfunction due to old age. More recently, LIMKs have been shown to play key roles in neuronal development and plasticity, they are involved in several neuronal diseases such as Amyotrophic Lateral Sclerosis, Parkinson and Alzheimer diseases, Williams-Beuren syndrome, schizophrenia, intellectual deficiency, ...

Rho-associated coiled-coil-containing kinases (ROCKs) are serine/threonine kinases, members of the AGC kinase family (Hartmann, S.; Ridley, A.J.; Lutz, S. The Function of Rho-Associated Kinases ROCK1 and ROCK2 in the Pathogenesis of Cardiovascular Disease. Front. Pharmacol. 2015, 6, 276). ROCKs phosphorylate several targets : 1/Myosin Light Chain (MLC) together with Myosin Light chain Phosphatase (MLCP) thereby inactivating this later and allowing MLC phosphorylation to persist (Amano, M.; Nakayama, M.; Kaibuchi, K. Rho-kinase/ROCK: A key regulator of the cytoskeleton and cell polarity. Cytoskeleton 2010, 67, 545-554). This results in actomyosin contractility stimulation and cellular contraction. 2/ LIMK2 which becomes activated and phosphorylates cofilin leading to the inactivation of this latter and actin filament stabilisation (Maekawa, M.; Ishizaki, T.; Boku, S.;Watanabe, N.; Fujita, A.; Iwamatsu, A.; Obinata, T.; Ohashi, K.; Mizuno, K.; Narumiya, S. Signaling from Rho to the actin cytoskeleton through protein kinases ROCK and LIM-kinase. Science 1999, 285, 895-898). 3/ Ezrin, radixin and moesin (ERM), which are involved in actin/plasma membrane interactions (Matsui, T.; Maeda, M.; Doi, Y.; Yonemura, S.; Amano, M.; Kaibuchi, K.; Tsukita, S.; Tsukita, S. Rho-kinase phosphorylates COOH-terminal threonines of ezrin/radixin/moesin (ERM) proteins and regulates their head-to-tail association. J. Cell Biol. 1998, 140, 647-657). 4/ Collapsin response mediator protein-2 (CRMP-2), thereby inhibiting its ability to stimulate microtubule assembly and axonal growth (Arimura, N.; Menager, C.; Fukata, Y.; Kaibuchi, K. Role of CRMP-2 in neuronal polarity. J. Neurobiol. 2004, 58, 34-47). 5/ PTEN (phosphatase and tensin homolog), a tumor suppressor thereby preventing it to inhibit cell growth and survival (Li, Z.; Dong, X.;Wang, Z.; Liu,W.; Deng, N.; Ding, Y.; Tang, L.; Hla, T.; Zeng, R.; Li, L.; et al. Regulation of PTEN by Rho small GTPases. Nat. Cell Biol. 2005, 7, 399-404).

Collectively, these actions play a role in actin cytoskeleton remodelling, cell contractility and cell death. Accordingly, ROCKs regulate different cellular functions such as growth, apoptosis, migration and metabolism.

ROCKs are involved in different pathologies: cardiac diseases such as cardiac fibrosis, cardiac hypertrophy, systemic blood pressure disorders, pulmonary hypertension, renal pathologies such as hypertensive nephropathy, Bartter and Gitelman syndrome, diabetic nephropathy, asthma, chronic obstructive pulmonary diseases, traumatic brain injuries, Alzheimer and Parkinson diseases, obesity, diabetis mellitus, cancers, autoimmune diseases (Lupus erythematosus, rhumatoid arthritis).

BRYCE A. HARRISON ET AL: ("Discovery and Development of LX7101, a Dual LIM-Kinase and ROCK Inhibitor for the Treatment of Glaucoma",ACS MEDICINAL CHEMISTRY LETTERS, vol. 6, no. 1, 9 December 2014 (2014-12-09), pages 84-88, ISSN: 1948-5875, DOI: 10.1021/ ml500367g) discloses e.g. 4-(7H-pyrrolo[2,3-d]pyrimidin-4-yl)-piperazin-1-yl-carboxamide derivatives (D1: e.g. page 86, table 3, compounds 1, 11, 13 to 15) as well as a 4-(7H-pyrrolo[2,3-d]pyrimidin-4-yl)-piperidin-1-yl-carboxamide derivative (D1: e.g. page 86, table 3, compound 12) with dual LIMK and ROCK kinase inhibitory activity for use in the treatment of e.g. glaucoma, ocular hypertension and/or intraocular hypertension.

The aim of the present invention is to provide new small molecules with an inhibition activity against LIMK and/or ROCK kinases.

The aim of the present invention is to provide new inhibitors of LIMK and/or ROCK kinases.

Another aim of the present invention is to provide new inhibitors of LlMK and ROCK kinases.

Another aim of the present invention is to provide new potent inhibitors targeting both LIMKs and ROCKs or specific of LIMKs according to the targeted disease.

Another aim of the present invention is to provide new small molecules as inhibitors of LIMK and/or ROCK kinases, said molecules having a wide range of cytotoxicity, from low toxicity to high toxicity according to the potent applications.

Thus, the present invention relates to a compound having the following formula (I): 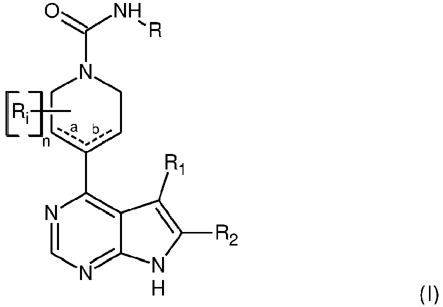 wherein:
- "a" is a single bond and "b" is a double bond, or "a" is a double bond and "b" is a single bond;
- R₁ is selected from the group consisting of: H, (C₁-C₆)alkyl group, halogen, and (C₃-C₇)cycloalkyl group;
- R₂ is selected from the group consisting of: H, and (C₁-C₆)alkyl group;
- or R₁ and R₂ may form together with the carbon atoms carrying them a (C₅-C₇)cycloalkyl group or a (C₅-C₇)heterocycloalkyl group, said heterocycloalkyl group being optionally substituted with a (C₁-C₆)alkyl group;
- n is 0, 1 or 2;
- each Rᵢ, identical or different, is selected from the (C₁-C₆)alkyl groups;
- R is selected from the group consisting of: (C₃-C₇)cycloalkyl group, optionally fused with an aryl group, aryl group, heteroaryl group, heterocycloalkyl group fused with an aryl group, and aryl group fused with a heterocycloalkyl group, said aryl and heteroaryl groups being optionally substituted with at least one substituent R₃ selected from the group consisting of:
   . H,
   . (C₁-C₆)alkyl, said alkyl being optionally interrupted with at least one heteroatom,
   . heterocycloalkyl,
   . aryl, said aryl being optionally substituted with at least one substituent such as:
      . (C₁-C₆)alkoxy;
      . NRₐR_{b}, Rₐ and R_{b}, independently from each other, being H or a (C₁-C₆)alkyl group;
      . -X-R_{c}, X being a bond, a (C₁-C₆)alkylene group, preferably -CH₂-, or -C(=O)-, and R_{c} being a heterocycloalkyl group, optionally substituted with at least one (C₁-C₆)alkyl group, SO₂CH₃ or (C₁-C₆)alkylamino group;
      . halogen;
      . -C(=O)-NRₐR_{b}, Rₐ and R_{b}, independently from each other, being H or a (C₁-C₆)alkyl group;
      . -C(=O)-N(R_{d})-X₁-NRₐR_{b}, R_{d}, Rₐ and R_{b}, independently from each other, being H or a (C₁-C₆)alkyl group, and X₁ being a (C₁-C₆)alkylene group, preferably CH₂; or
      . -O-X₂-NRₑR_{f}, X₂ being a (C₁-C₆)alkylene group, Rₑ and R_{f}, independently from each other, being H or a (C₁-C₆)alkyl group, or at least one of Rₑ and R_{f} is an amino-protecting group such as a carbamate (Boc) or comprises one fluorophore such as difluoroborane derivatives; or
   . heteroaryl, said heteroaryl being optionally substituted with at least one substituent such as:
      . (C₁-C₆)alkoxy;
      . NRₐR_{b}, Rₐ and R_{b}, independently from each other, being H or a (C₁-C₆)alkyl group;
      . -C(=O)-NRₐR_{b}, Rₐ and R_{b}, independently from each other, being H or a (C₁-C₆)alkyl group; or
      . -NH-C(=O)-Rₐ, Rₐ being H or a (C₁-C₆)alkyl group;
   . halogen,
   . halo(C₁-C₆)alkyl,
   . aryloxy, said aryloxy being optionally substituted with at least one substituent such as:
      . (C₁-C₆)alkoxy;
      . NRₐR_{b}, Rₐ and R_{b}, independently from each other, being H or a (C₁-C₆)alkyl group;
      . -X-R_{c}, X being a bond, a (C₁-C₆)alkylene group, preferably -CH₂-, or -C(=O)-, and R_{c} being a heterocycloalkyl group, optionally substituted with at least one (C₁-C₆)alkyl group, SO₂CH₃ or (C₁-C₆)alkylamino group;
      . halogen;
      -C(=O)-NRₐR_{b}, Rₐ and R_{b}, independently from each other, being H or a (C₁-C₆)alkyl group; or
      . -C(=O)-N(R_{d})-X₁-NRₐR_{b}, R_{d}, Rₐ and R_{b}, independently from each other, being H or a (C₁-C₆)alkyl group, and X₁ being a (C₁-C₆)alkylene group, preferably CH₂;
   . -O-halo(C₁-C₆)alkyl,
   . cyano,
   . (C₁-C₆)alkoxy, and
   . -X-NRₐR_{b}, X being selected in the group consisting of: (C₁-C₆)alkylene group, -O-C(=O)-, -C(=O)-, and -NH-C(=O)-, and Rₐ and R_{b}, independently from each other, being H or a (C₁-C₆)alkyl group;
or its pharmaceutically acceptable salts, racemates, diastereomers or enantiomers.

The present invention relates to a compound having the following formula (I): 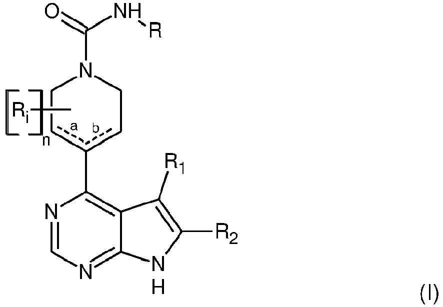 wherein:
- "a" is a single bond and "b" is a double bond, or "a" is a double bond and "b" is a single bond;
- R₁ is selected from the group consisting of: H, (C₁-C₆)alkyl group, halogen, and (C₃-C₇)cycloalkyl group;
- R₂ is selected from the group consisting of: H, and (C₁-C₆)alkyl group;
- or R₁ and R₂ may form together with the carbon atoms carrying them a (C₅-C₇)cycloalkyl group or a (C₅-C₇)heterocycloalkyl group, said heterocycloalkyl group being optionally substituted with a (C₁-C₆)alkyl group;
- n is 0, 1 or 2;
- each Rᵢ, identical or different, is selected from the (C₁-C₆)alkyl groups;
- R is selected from the group consisting of: (C₃-C₇)cycloalkyl group, optionally fused with an aryl group, aryl group, heteroaryl group, heterocycloalkyl group fused with an aryl group, and aryl group fused with a heterocycloalkyl group, said aryl and heteroaryl groups being optionally substituted with at least one substituent R₃ selected from the group consisting of:
   . H,
   . (C₁-C₆)alkyl, said alkyl being optionally interrupted with at least one heteroatom,
   . heterocycloalkyl,
   . aryl, said aryl being optionally substituted with at least one substituent such as:
      . (C₁-C₆)alkoxy;
      . NRₐR_{b}, Rₐ and R_{b}, independently from each other, being H or a (C₁-C₆)alkyl group;
      . -X-R_{c}, X being a bond, a (C₁-C₆)alkylene group, preferably -CH₂-, or
      -C(=O)-, and R_{c} being a heterocycloalkyl group, optionally substituted with at least one (C₁-C₆)alkyl group, SO₂CH₃ or (C₁-C₆)alkylamino group;
      . halogen;
      . -C(=O)-NRₐR_{b}, Rₐ and R_{b}, independently from each other, being H or a (C₁-C₆)alkyl group;
      . -C(=O)-N(R_{d})-X₁-NRₐR_{b}, R_{d}, Rₐ and R_{b}, independently from each other, being H or a (C₁-C₆)alkyl group, and X₁ being a (C₁-C₆)alkylene group, preferably CH₂; or
      . -O-X₂-NRₑR_{f}, X₂ being a (C₁-C₆)alkylene group, Rₑ and R_{f}, independently from each other, being H or a (C₁-C₆)alkyl group, or at least one of Rₑ and R_{f} is an amino-protecting group such as a carbamate (Boc) or comprises one fluorophore or is SO₂CH₃; or
   . heteroaryl, said heteroaryl being optionally substituted with at least one substituent such as:
      . (C₁-C₆)alkoxy;
      . NRₐR_{b}, Rₐ and R_{b}, independently from each other, being H or a (C₁-C₆)alkyl group;
      . -C(=O)-NRₐR_{b}, Rₐ and R_{b}, independently from each other, being H or a (C₁-C₆)alkyl group; or
      . -NH-C(=O)-Rₐ, Rₐ being H or a (C₁-C₆)alkyl group;
   . halogen,
   . halo(C₁-C₆)alkyl,
   . aryloxy, said aryloxy being optionally substituted with at least one substituent such as:
      . (C₁-C₆)alkoxy;
      . OH;
      . NRₐR_{b}, Rₐ and R_{b}, independently from each other, being H or a (C₁-C₆)alkyl group;
      . -X-R_{c}, X being a bond, a (C₁-C₆)alkylene group, preferably -CH₂-, or
      -C(=O)-, and R_{c} being a heterocycloalkyl group, optionally substituted with at least one (C₁-C₆)alkyl group, SO₂CH₃ or (C₁-C₆)alkylamino group;
      . halogen;
      . -O-benzyl,
      . -O-C(=O)-NRₐR_{b}, Rₐ and R_{b}, independently from each other, being H or a (C₁-C₆)alkyl group;
      . -NH-C(=O)-Rₐ, Rₐ being H or a (C₁-C₆)alkyl group;
      . -C(=O)-NRₐR_{b}, Rₐ and R_{b}, independently from each other, being H or a (C₁-C₆)alkyl group; or
      . -C(=O)-N(R_{d})-X₁-NRₐR_{b}, R_{d}, Rₐ and R_{b}, independently from each other, being H or a (C₁-C₆)alkyl group, and X₁ being a (C₁-C₆)alkylene group, preferably CH₂;
   . heteroaryloxy, said heteroaryloxy being optionally substituted with at least one substituent such as:
      -C(=O)-NRₐR_{b}, Rₐ and R_{b}, independently from each other, being H or a (C₁-C₆)alkyl group;
   . -O-halo(C₁-C₆)alkyl,
   . cyano,
   . (C₁-C₆)alkoxy,
   . -X-NRₐR_{b}, X being selected in the group consisting of: (C₁-C₆)alkylene group, -O-C(=O)-, -C(=O)-, and -NH-C(=O)-, and Rₐ and R_{b}, independently from each other, being H or a (C₁-C₆)alkyl group;
   . -X'-R_{c}, X' being -C(=O)- or a -O-(C₁-C₆)alkylene radical, R_{c} being a cycloalkyl group, optionally substituted with at least one halogen, or R_{c} being a heterocycloalkyl group, optionally substituted with SO₂CH₃ or at least one (C₁-C₆)alkyl group; and
   . -O-X₂-NRₑR_{f}, X₂ being a (C₁-C₆)alkylene group, Rₑ and R_{f}, independently from each other, being H or a (C₁-C₆)alkyl group, or at least one of Rₑ and R_{f} is an amino-protecting group such as a carbamate (Boc);
or its pharmaceutically acceptable salts, racemates, diastereomers or enantiomers.

According to an embodiment, in formula (I), R is a (C₃-C₇)cycloalkyl group, preferably an unsubstituted cyclohexyl group.

According to another embodiment, in formula (I), R is an aryl group, optionally substituted with at least one substituent R₃ as defined above, and is preferably an optionally substituted phenyl group as defined above.

According to an embodiment, when R is an aryl group, preferably a phenyl group, substituted with at least one substituted aryl group, preferably a substituted phenyl group, the following R groups may be mentioned:

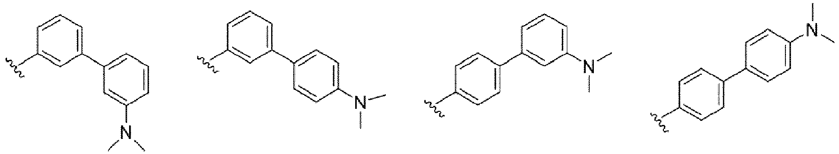

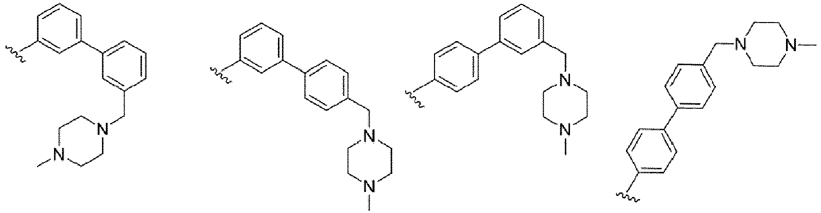

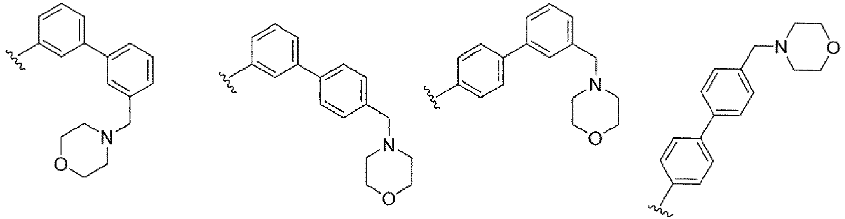

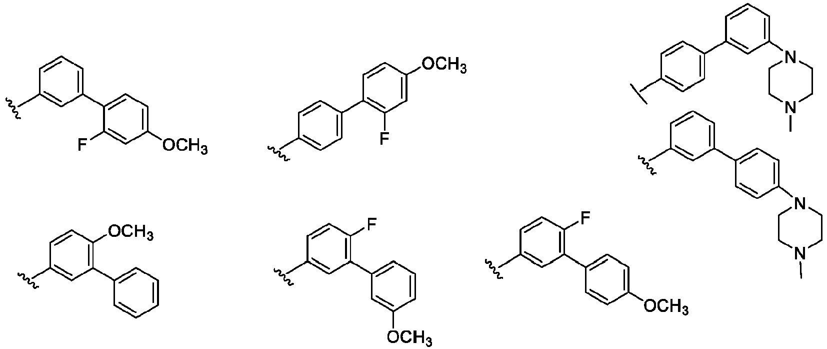

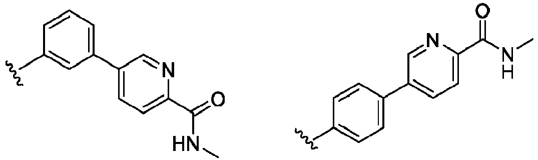

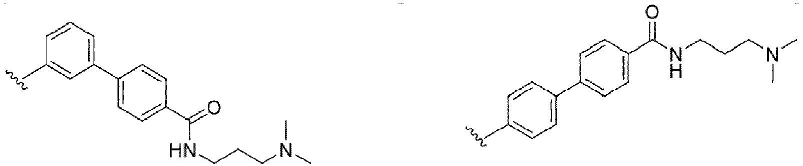

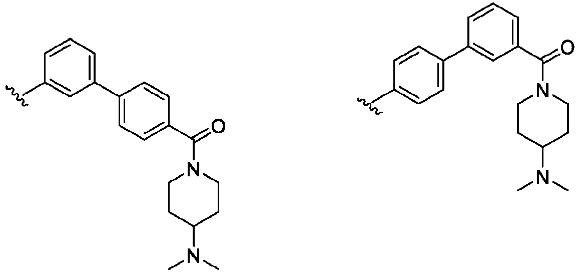

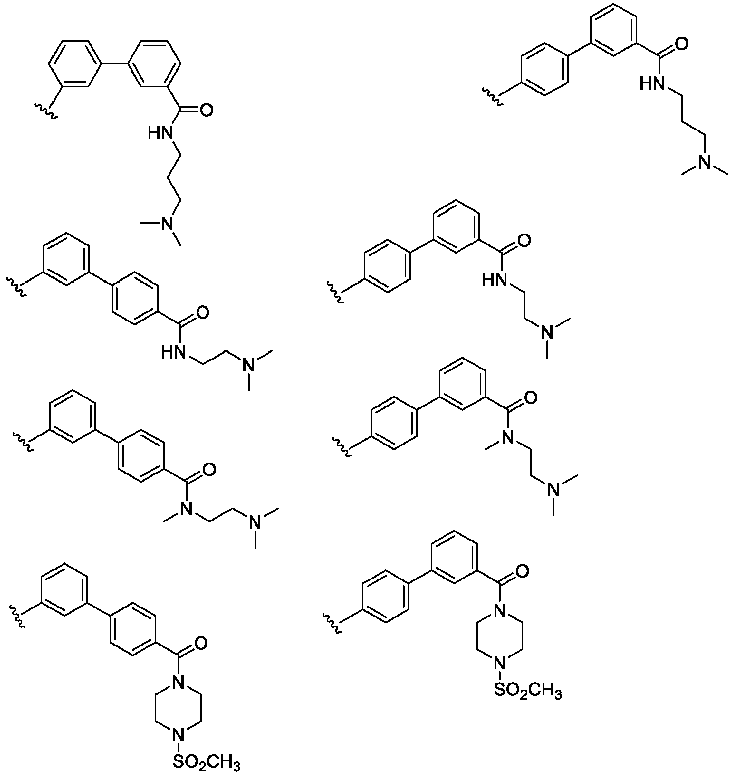

According to an embodiment, in formula (I), R is an aryl group, preferably a phenyl group, substituted with at least one aryl group, preferably a phenyl group, substituted with a -O-X₂-NRₑR_{f} group as defined above.

Preferably, in this embodiment, the -O-X₂-NRₑR_{f} group is selected from the following groups: -O-X₂-NHBoc, preferably -O-(CH₂)₃-NHBoc, -O-X₂-NH-C(=O)Alkyl, preferably -O-(CH₂)₃-NH-C(=O)Me, -O-X₂-NH-SO₂-Alkyl, preferably -O-(CH₂)₃-NH-SO₂-Me, -O-X₂-NRₑR_{f} group wherein Rₑ and R_{f} are alkyl groups, preferably methyl, and the group having the following formula:

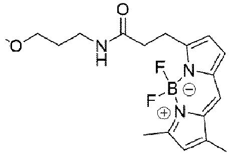

According to an embodiment, at least one of Rₑ and R_{f} is an amino-protecting group such as a carbamate (Boc) or comprises one fluorophore such as difluoroborane derivatives. As difluoroborane derivatives, the fluorophore 5,5-difluoro-1,3-dimethyl-5*H*-dipyrrolo[1,2-*c*:2',1'-*f*][1,3,2]diazaborinine may be mentioned.

According to this embodiment, the following compounds may thus be mentioned:

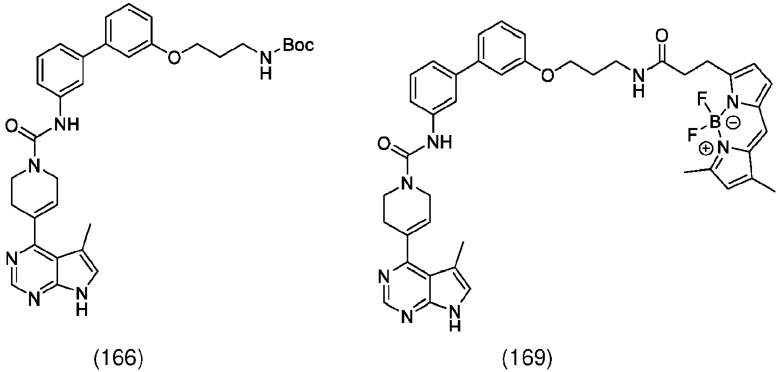

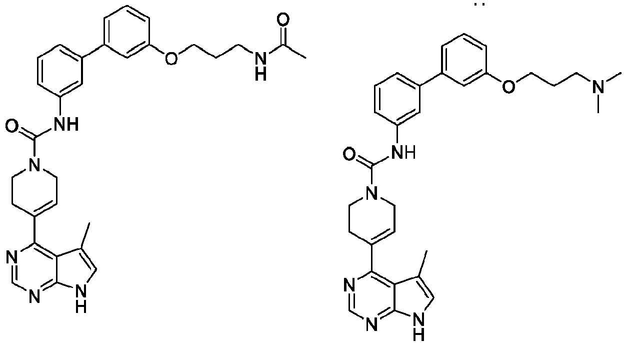

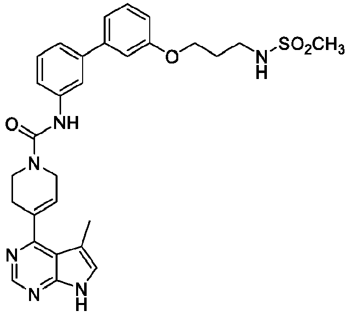

According to an embodiment, when R is an aryl group, preferably a phenyl group, substituted with at least one substituted heteroaryl group, the following R groups may be mentioned:

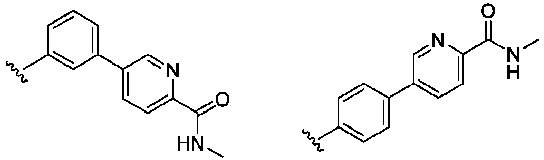

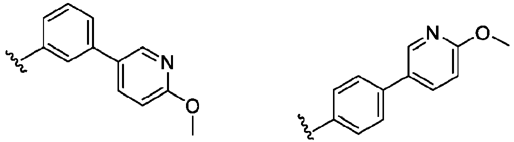

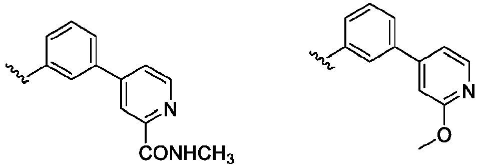

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heteroaryl group" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

The term "heterocycloalkyl group" means: a 4- to 10-membered, saturated or partially unsaturated, monocyclic or bicyclic group comprising from one to three heteroatoms selected from O, S or N; the heterocycloalkyl group may be attached to the rest of the molecule via a carbon atom or via a heteroatom; the term bicyclic heterocycloalkyl includes fused bicycles and spiro-type rings.

By way of saturated heterocycloalkyl comprising from 5 to 6 atoms, mention may be made of oxetanyl, tetrahydrofuranyl, dioxolanyl, pyrrolidinyl, azepinyl, oxazepinyl, pyrazolidinyl, imidazolidinyl, tetrahydrothiophenyl, dithiolanyl, thiazolidinyl, tetrahydropyranyl, tetrahydropyridinyl, dioxanyl, morpholinyl, piperidinyl, piperazinyl, tetrahydrothiopyranyl, dithianyl, thiomorpholinyl or isoxazolidinyl.

Among the heterocycloalkyls, mention may also be made, by way of examples, of bicyclic groups such as (8aR)-hexahydropyrrolo[1,2-a]pyrazin-2(1H)-yl, octahydroindozilinyl, diazepanyl, dihydroimidazopyrazinyl and diazabicycloheptanyl groups, or else diazaspiro rings such as 1,7-diazaspiro[4.4]non-7-yl or 1-ethyl-1,7-diazaspiro[4.4]non-7-yl.

When the heterocycloalkyl is substituted, the substitution(s) may be on one (or more) carbon atom(s) and/or on the heteroatom(s). When the heterocycloalkyl comprises several substituents, they may be borne by one and the same atom or different atoms.

The term "cycloalkyl group" means: a cyclic carbon-based group comprising, unless otherwise mentioned, from 3 to 6 carbon atoms. By way of examples, mention may be made of cyclopropyl, cyclobutyl, cyclopentyl, cyclohexyl, etc. groups.

When an alkyl radical is substituted with an aryl group, the term "arylalkyl" or "aralkyl" radical is used. The "arylalkyl" or "aralkyl" radicals are aryl-alkyl- radicals, the aryl and alkyl groups being as defined above. Among the arylalkyl radicals, mention may in particular be made of the benzyl or phenethyl radicals.

The term "halogen" means: a fluorine, a chlorine, a bromine or an iodine.

The term "alkoxy group" means: an -O-alkyl radical where the alkyl group is as previously defined. By way of examples, mention may be made of -O-(C₁-C₄)alkyl groups, and in particular the -O-methyl group, the -O-ethyl group as -O-Csalkyl group, the -O-propyl group, the -O-isopropyl group, and as -O-C₄alkyl group, the -O-butyl, -O-isobutyl or -O-tert-butyl group.

The abovementioned "alkyl", "cycloalkyl", "aryl", "heteroaryl" and "heterocycloalkyl" radicals can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "haloalkyl group" means: an alkyl group as defined above, in which one or more of the hydrogen atoms is (are) replaced with a halogen atom. By way of example, mention may be made of fluoroalkyls, in particular CF₃ or CHF₂.

The term "carboxyl" means: a COOH group.

The term "oxo" means: "=O".

In some embodiments of the invention, the compounds of the invention can contain one or more asymmetric centers and thus occur as racemates and racemic mixtures, single enantiomers, individual diastereoisomeric mixtures. All such isomeric forms of these compounds are included in the present invention, unless expressly provided otherwise.

In some embodiments, the compounds of the invention can contain one or more double bonds and thus occur as individual or mixtures of Z and/or E isomers. All such isomeric forms of these compounds are included in the present invention, unless expressly provided otherwise.

In the embodiments where the compounds of the invention can contain multiple tautomeric forms, the present invention also includes all tautomeric forms of said compounds unless expressly provided otherwise.

A preferred family of compounds according to the invention are compounds having the following formula (II): 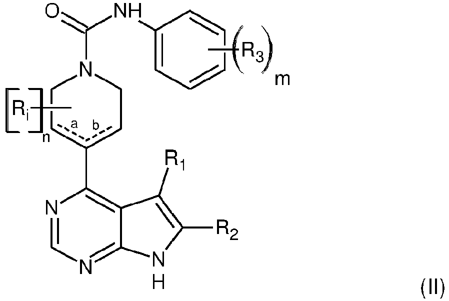 wherein:
- a, b, n, Rᵢ, R₁, and R₂ are as defined above in formula (I), and
- m is 1 or 2, and each R₃, identical or different, is as defined above in formula (I).

Compounds of formula (II) are compounds of formula (I) wherein R is a phenyl group, substituted with at least one R₃ group.

A preferred family of compounds according to the invention are compounds having the following formula (III): 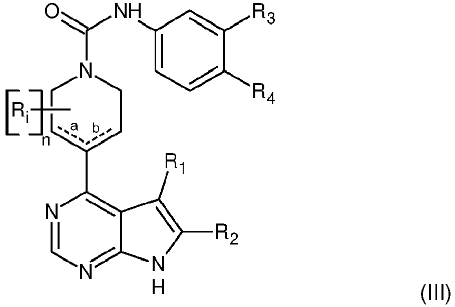 wherein:
- a, b, n, Rᵢ, R₁, R₂, and R₃ are as defined above in formula (I), and
- R₄ is H, halogen or aryloxy.

A preferred family of compounds according to the invention are compounds having the following formula (IV): 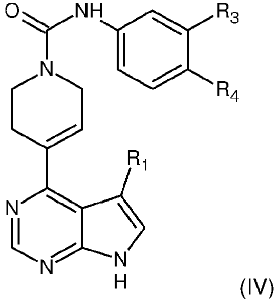 wherein:
- R₁ is H or (C₁-C₆)alkyl group;
- R₃ is selected from the group consisting of:
   - H,
   - (C₁-C₆)alkyl,
   - aryl, preferably phenyl, said aryl being optionally substituted with at least one substituent such as (C₁-C₆)alkoxy, in particular OMe, or halogen such as F,
   - heteroaryl, such as pyridinyl, imidazolyl, pyrrolyl or triazolyl,
   - halogen, such as F, Cl, or Br,
   - halo(C₁-C₆)alkyl, such as CF₃,
   - aryloxy, in particular phenyloxy, said aryloxy being optionally substituted with at least one substituent such as (C₁-C₆)alkoxy, preferably OMe,
   - -O-halo(C₁-C₆)alkyl, such as OCF₃,
   - cyano,
   - (C₁-C₆)alkoxy, such as OMe, and
   - -O-C(=O)-NRₐR_{b}, Rₐ and R_{b}, independently from each other, being H or a (C₁-C₆)alkyl group; and
- R₄ is H, halogen or aryloxy, such as phenyloxy,
or R₃ and R₄ form together with the carbon atoms carrying them a phenyl group or a heterocycloalkyl group comprising preferably 2 oxygen atoms and 3 carbon atoms.

Preferably, in formula (IV), R₁ is methyl.

Preferred compounds of formula (IV) are the followings:

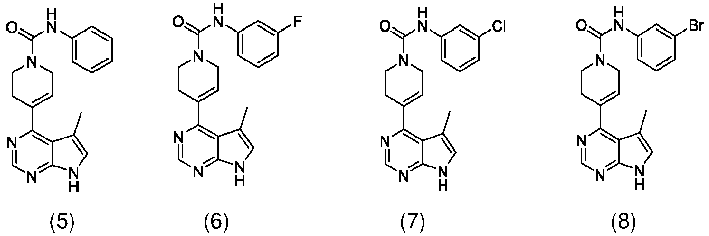

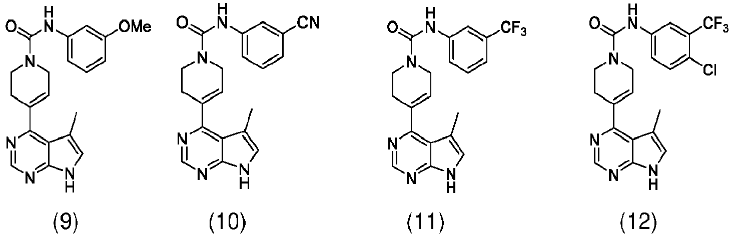

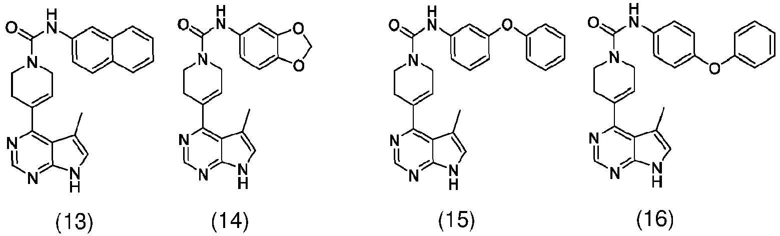

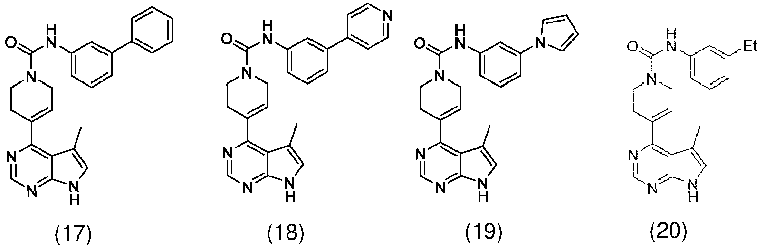

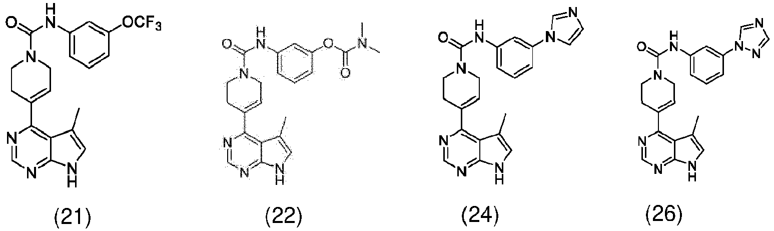

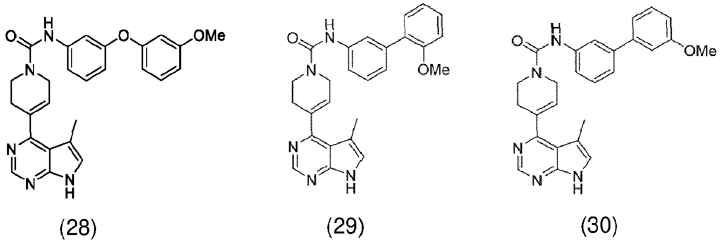

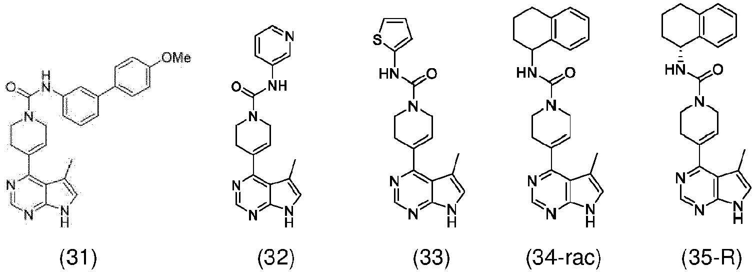

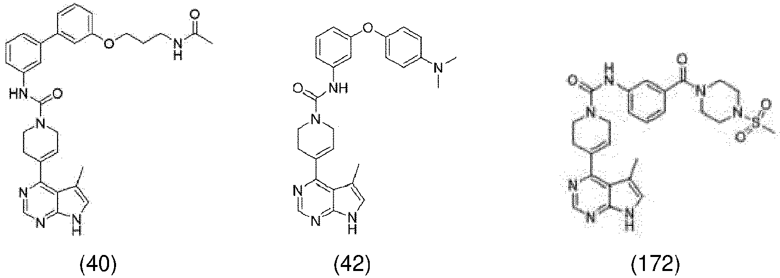

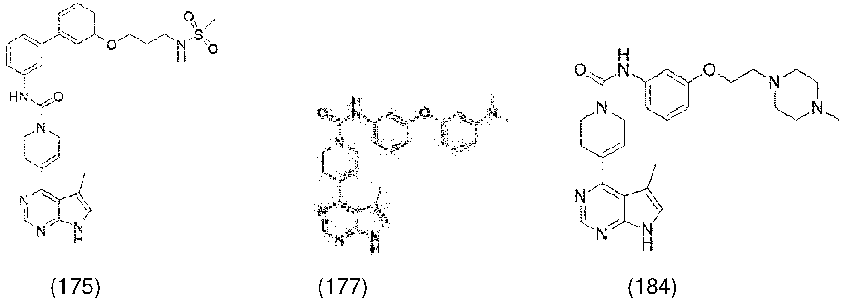

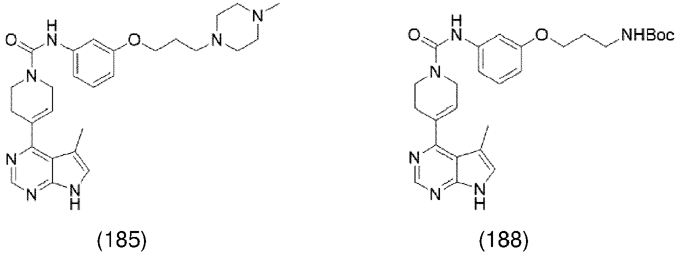

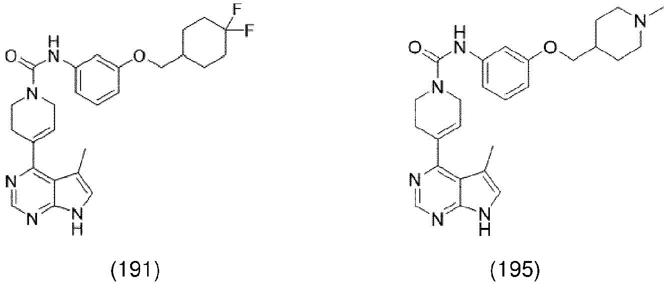

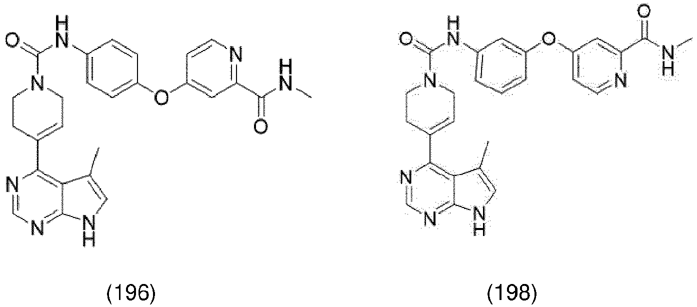

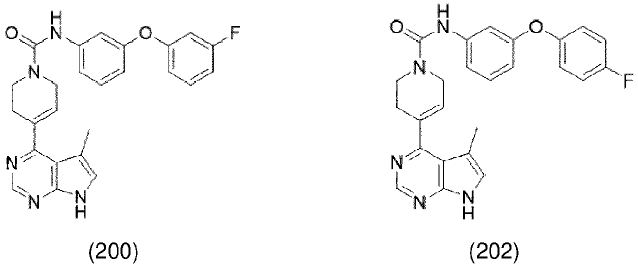

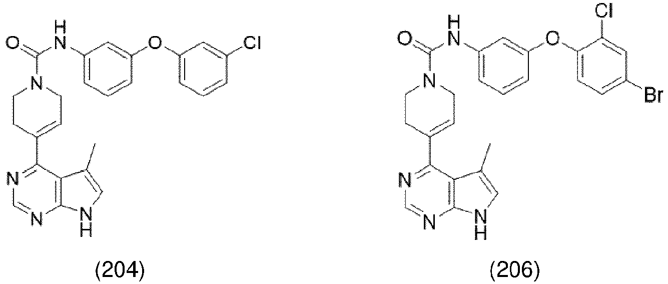

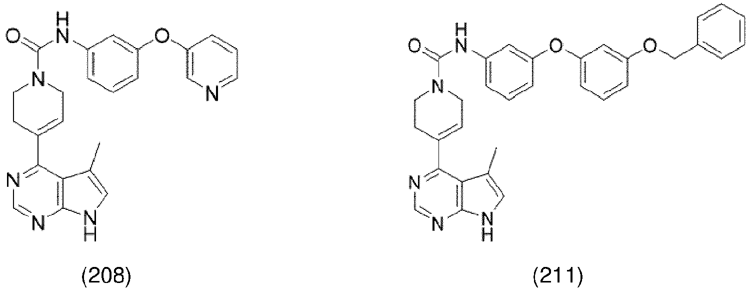

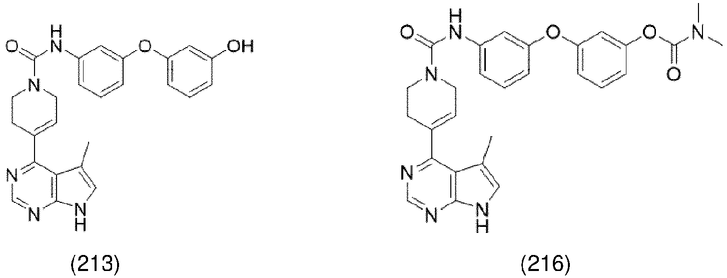

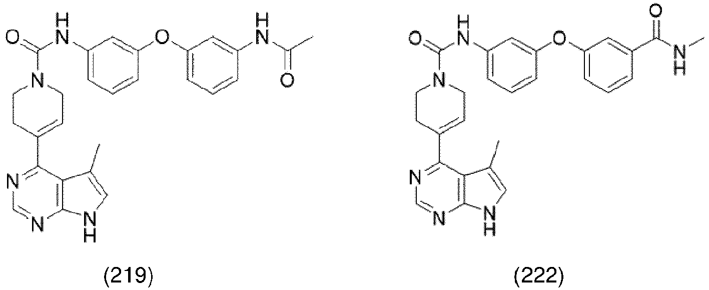

A preferred family of compounds according to the invention are compounds having the following formula (V): 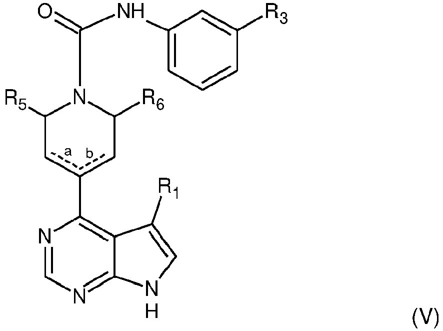 wherein:
- "a" is a single bond and "b" is a double bond, or "a" is a double bond and "b" is a single bond;
- R₁ is (C₁-C₆)alkyl group, preferably methyl;
- R₅ is (C₁-C₆)alkyl group, preferably methyl;
- R₆ is H or (C₁-C₆)alkyl group, preferably methyl;
- R₃ is selected from the group consisting of:
   - halogen, such as F, Cl, or Br, and
   - aryloxy, in particular phenyloxy.

Preferably, in formula (V), R₁ is methyl.

Preferred compounds of formula (V) are the followings:

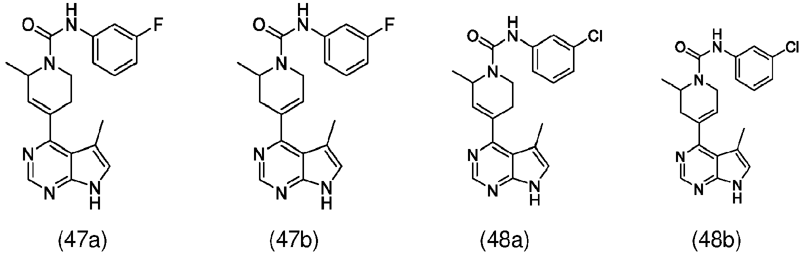

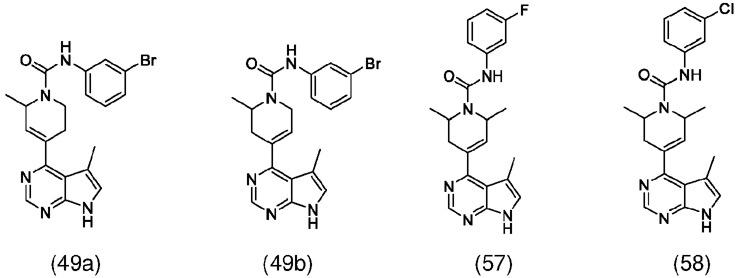

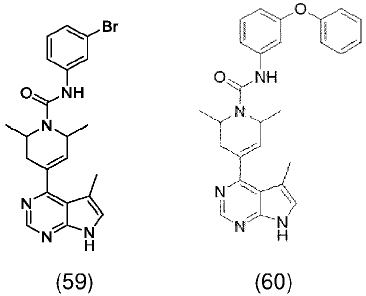

A preferred family of compounds according to the invention are compounds having the following formula (VI): 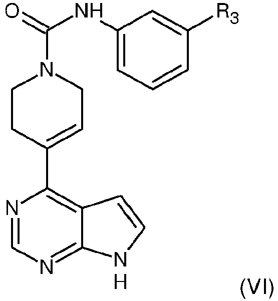 wherein R₃ is halogen.

A preferred compound of formula (VI) is the following:

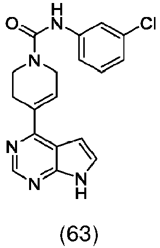

A preferred family of compounds according to the invention are compounds having the following formula (VII): 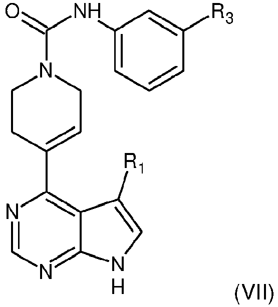 wherein:
- R₁ is halogen, preferably Cl; and
- R₃ is -O-C(=O)-NRₐR_{b}, Rₐ and R_{b}, independently from each other, being H or a (C₁-C₆)alkyl group.

A preferred compound of formula (VII) is the following:

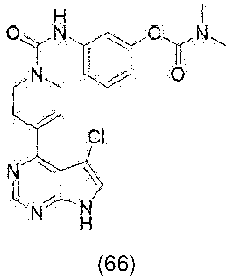

A preferred family of compounds according to the invention are compounds having the following formula (VIII): 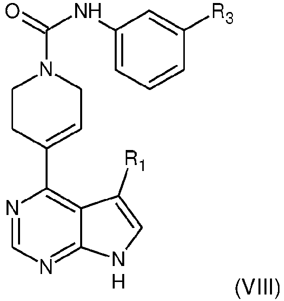 wherein:
- R₁ is (C₃-C₇)cycloalkyl group, preferably cyclopropyl;
- R₃ is selected from the group consisting of:
   - aryl, preferably phenyl, said aryl being optionally substituted with at least one substituent such as (C₁-C₆)alkoxy, in particular OMe,
   - heteroaryl, such as pyrrolyl,
   - halogen, such as F, Cl, or Br,
   - aryloxy, in particular phenyloxy, said aryloxy being optionally substituted with at least one substituent such as (C₁-C₆)alkoxy, preferably OMe, and
   - -O-C(=O)-NRₐR_{b}, Rₐ and R_{b}, independently from each other, being H or a (C₁-C₆)alkyl group.

Preferably, in formula (VIII), R₁ is cyclopropyl.

Preferred compounds of formula (VIII) are the followings:

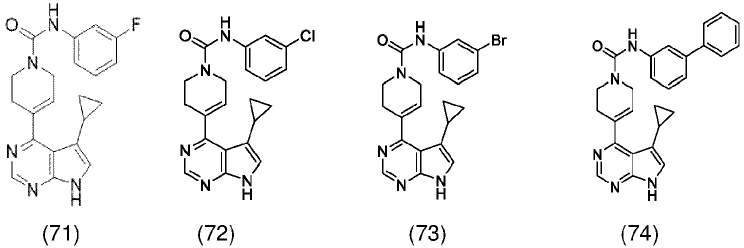

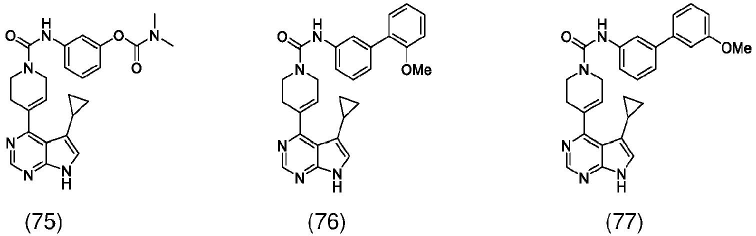

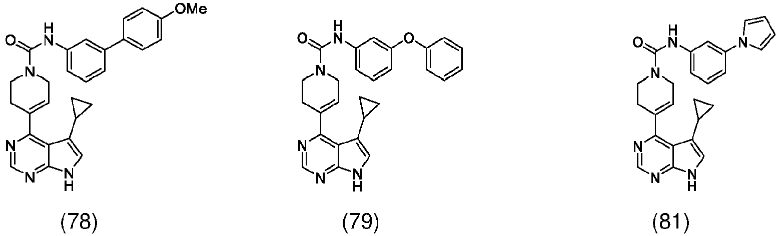

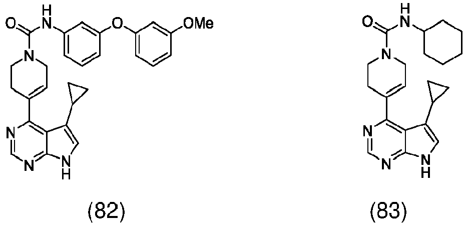

A preferred family of compounds according to the invention are compounds having the following formula (IX): 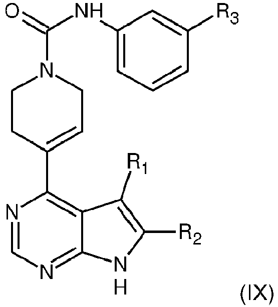 wherein:
- R₁ is (C₁-C₆)alkyl group, preferably methyl;
- R₂ is (C₁-C₆)alkyl group, preferably methyl;
- R₃ is selected from the group consisting of:
   - H,
   - aryl, preferably phenyl, said aryl being optionally substituted with at least one substituent such as (C₁-C₆)alkoxy, in particular OMe,
   - halogen, such as F, Cl, or Br,
   - (C₁-C₆)alkoxy, such as OMe, and
   - -O-C(=O)-NRₐR_{b}, Rₐ and R_{b}, independently from each other, being H or a (C₁-C₆)alkyl group.

Preferably, in formula (IX), R₁ and R₂ are identical. More preferably, they are methyl.

Preferred compounds of formula (IX) are the followings:

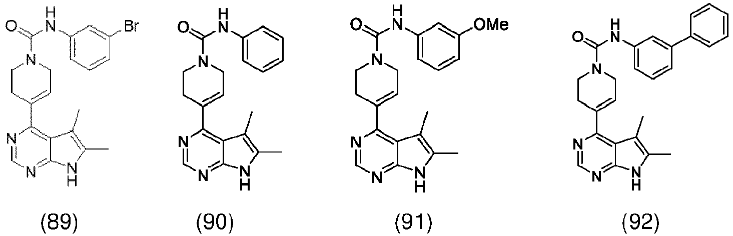

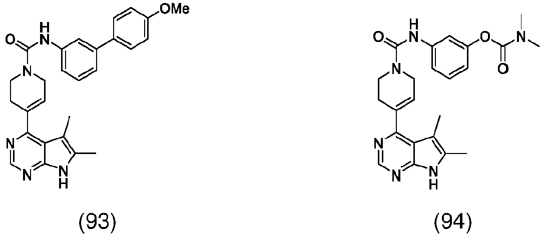

A preferred family of compounds according to the invention are compounds having the following formula (X): 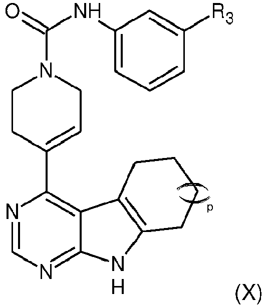 wherein:
- p is 0, 1, or 2;
- R₃ is selected from the group consisting of:
   - H,
   - (C₁-C₆)alkyl, said alkyl being optionally interrupted with at least one heteroatom,
   - heterocycloalkyl, such as morpholinyl,
   - aryl, preferably phenyl, said aryl being optionally substituted with at least one substituent such as (C₁-C₆)alkoxy,
   - heteroaryl, such as pyridinyl,
   - halogen,
   - aryloxy,
   - halo(C₁-C₆)alkyl,
   - cyano,
   - (C₁-C₆)alkoxy, and
   - -X-NRₐR_{b}, X being selected in the group consisting of: (C₁-C₆)alkylene group, -O-C(=O)-, -C(=O)-, and -NH-C(=O)-, and Rₐ and R_{b}, independently from each other, being H or a (C₁-C₆)alkyl group.

Preferred compounds of formula (X) are the followings:

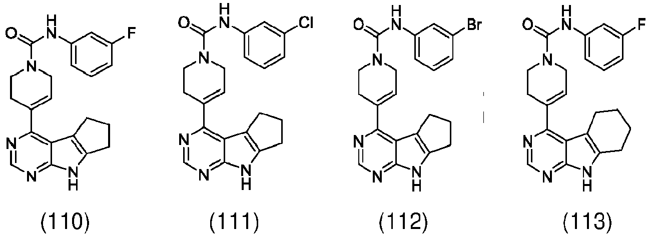

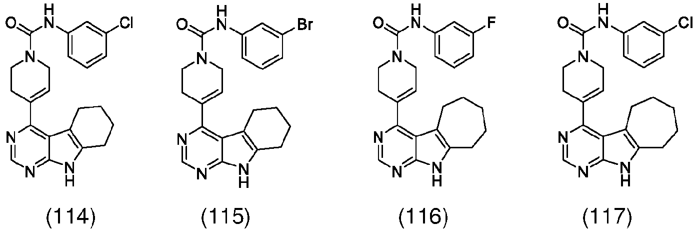

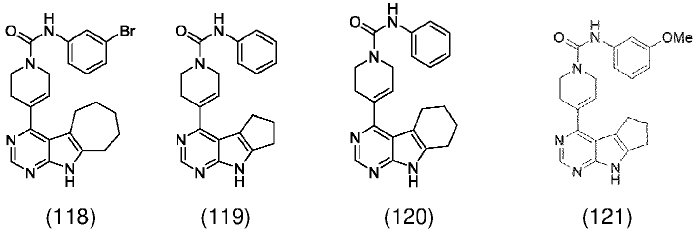

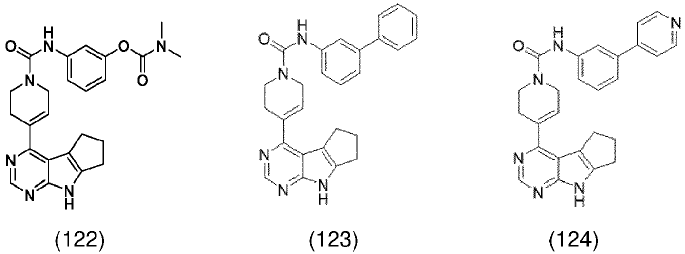

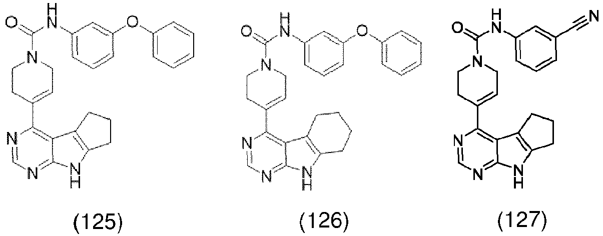

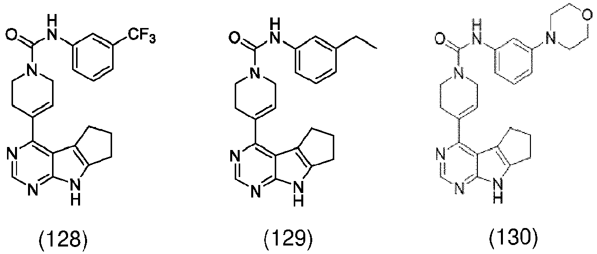

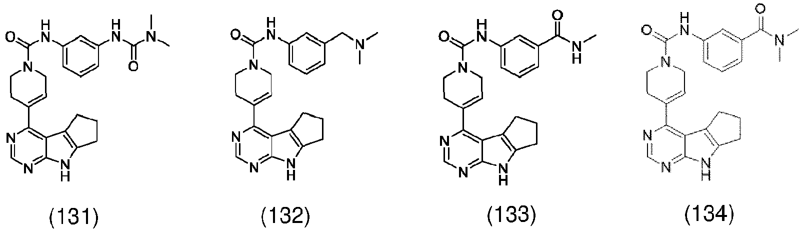

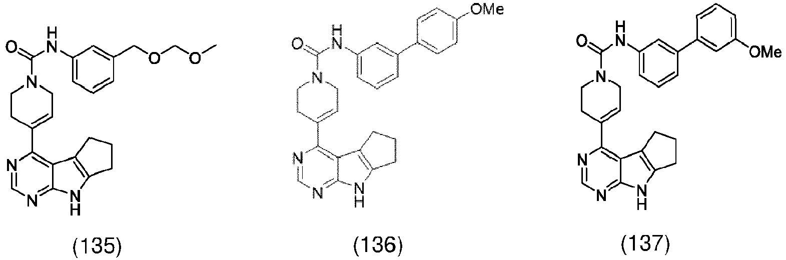

A preferred family of compounds according to the invention are compounds having the following formula (XI): 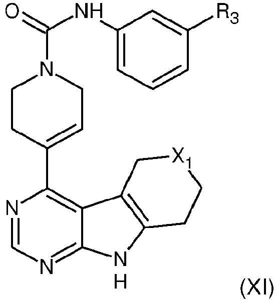 wherein:
- X₁ is -O-, -S-, or -N((C₁-C₆)alkyl)-, such as -N(Me)-; and
- R₃ is halogen.

Preferred compounds of formula (XI) are the followings:

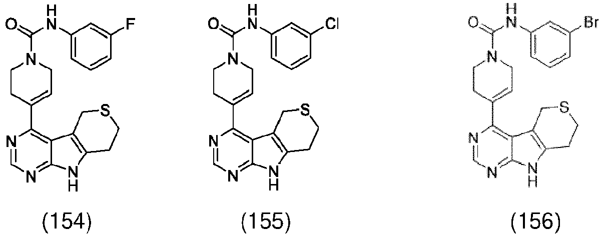

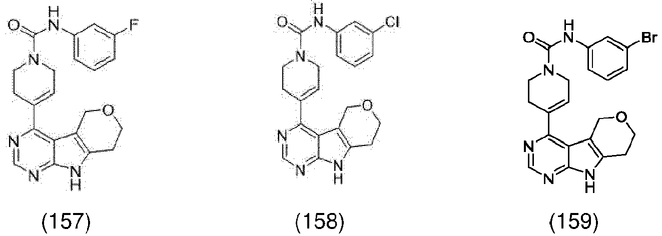

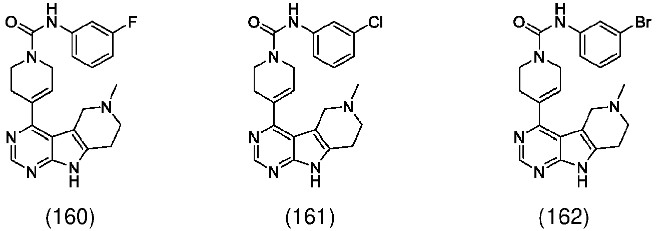

A preferred family of compounds according to the invention are compounds having the following formula (XII): 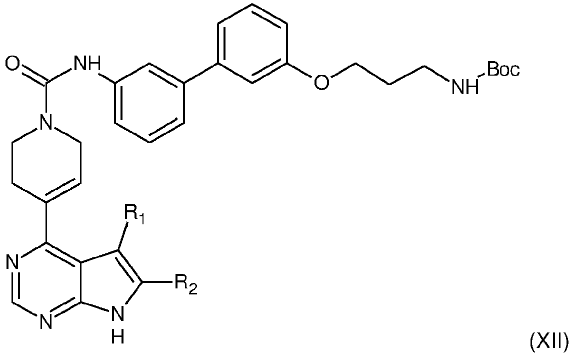 wherein R₁ and R₂ are as defined above in formula (I).

Preferably, in formula (XII), R₁ is a (C₁-C₆)alkyl group, such as methyl or ethyl, or a (C₃-C₇)cycloalkyl group, such as cyclopropyl.

Preferably, in formula (XII), R₂ is H or a (C₁-C₆)alkyl group, such as a ethyl.

Preferred compounds of formula (XII) are the followings:

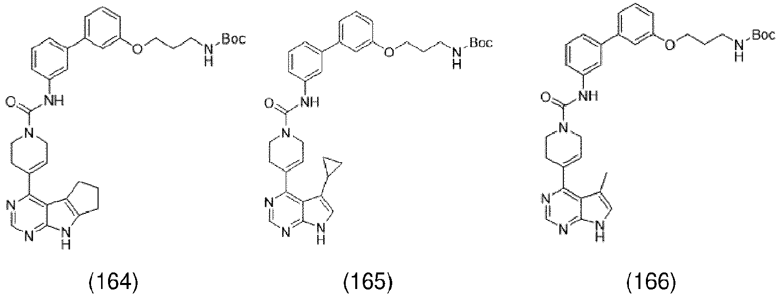

A preferred family of compounds according to the invention are compounds having the following formula (XIII): 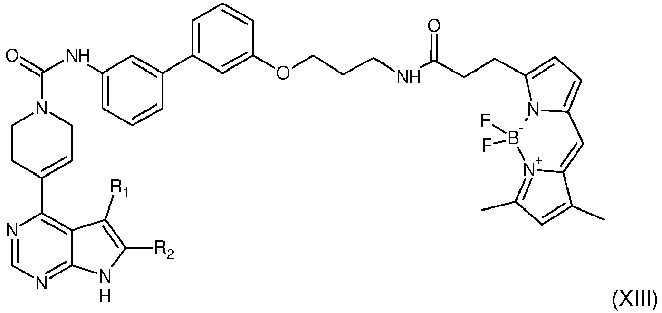 wherein R₁ and R₂ are as defined above in formula (I).

Preferably, in formula (XIII), R₁ is a (C₁-C₆)alkyl group, such as methyl or ethyl, or a (C₃-C₇)cycloalkyl group, such as cyclopropyl.

Preferably, in formula (XIII), R₂ is H or a (C₁-C₆)alkyl group, such as a ethyl.

Preferred compounds of formula (XIII) are the followings:

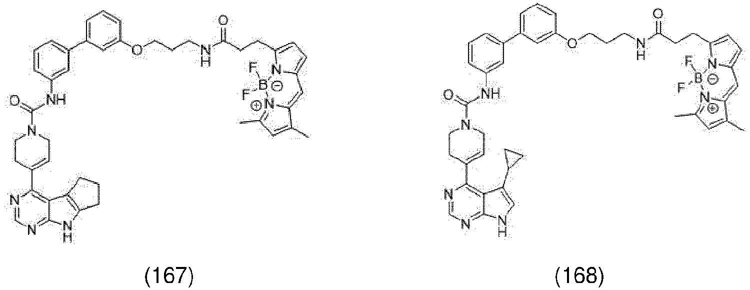

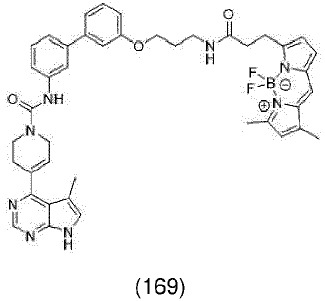

The present invention also relates to a medicament comprising a compound of formula (I) as defined above, or a pharmaceutically acceptable salt thereof.

The present invention also relates to a medicament comprising a compound of formula (II), (III), (IV), (V), (VI), (VII), (VIII), (IX), (X), (XI), (XII) or (XIII) as defined above, or a pharmaceutically acceptable salt thereof.

The present invention also relates to a pharmaceutical composition, comprising a compound of formula (I) as defined above, or a pharmaceutically acceptable salt thereof, and also at least one pharmaceutically acceptable excipient.

The present invention also relates to a pharmaceutical composition, comprising a compound of formula (II), (III), (IV), (V), (VI), (VII), (VIII), (IX), (X), (XI), (XII) or (XIII) as defined above, or a pharmaceutically acceptable salt thereof, and also at least one pharmaceutically acceptable excipient.

Said excipients are selected, according to the pharmaceutical form and the mode of administration desired, from the usual excipients which are known to those skilled in the art.

The present invention relates to a compound of formula (I) as defined above, or an addition salt of this compound with a pharmaceutically acceptable acid, for use as a medicine.

The present invention relates to a compound of formula (I) as defined above, or a pharmaceutically acceptable salt of this compound, for use as a drug.

The present invention also relates to a compound of formula (I) as defined above, or of formula (II), (III), (IV), (V), (VI), (VII), (VIII), (IX), (X), (XI), (XII) or (XIII) as defined above, for use in a method of treating a condition selected in the group consisting of cancers, such as breast, lung, pancreas, prostate, colorectal cancers, leukemias, glioma, melanoma and osteosarcoma, virion infections (HIV, Ebola and Herpes virus...), ocular hypertension and glaucoma formation, Neurofibromatosis type 1 and 2 (NF1 and NF2), psoriatic lesions, inflammatory diseases and hyperalgesia, central sensitization and chronic pain, reproduction (oocyte maturation) erectile dysfunction, and neuronal diseases such as Amyotrophic Lateral Sclerosis, Parkinson and Alzheimer diseases, Williams-Beuren syndrome, schizophrenia, and intellectual deficiency.

The present invention thus also relates to a compound as defined above for use in a method for treating a condition as defined above.

The present invention also relates to a compound of formula (I) as defined above, or of formula (II), (III), (IV), (V), (VI), (VII), (VIII), (IX), (X), (XI), (XII) or (XIII) as defined above, for use in treating cancer.

The present invention also relates to a method for treating the pathological conditions indicated above, which comprises the administration, to a patient, of an effective dose of a compound according to the invention, or a pharmaceutically acceptable salt thereof.

The compounds of the invention may thus be used for treating several pathologies involved in regulating cytoskeleton dynamics cytoskeleton remodeling, cell motility, morphogenesis, division, differentiation, apoptosis, neuronal morphology, neuritogenesis, and oncogenesis.

This includes: cancers, such as breast, lung, pancreas, prostate, colorectal cancers, leukemias, glioma, melanoma and osteosarcoma, virion infections (HIV, Ebola and Herpes virus...), ocular hypertension and glaucoma formation, Neurofibromatosis type 1 and 2 (NF1 and NF2), psoriatic lesions, inflammatory diseases and hyperalgesia, central sensitization and chronic pain, reproduction (oocyte maturation) erectile dysfunction, and neuronal diseases such as Amyotrophic Lateral Sclerosis, Parkinson and Alzheimer diseases, Williams-Beuren syndrome, schizophrenia, intellectual deficiency.

In the pharmaceutical compositions of the present invention for oral, sublingual, subcutaneous, intramuscular, intravenous, topical, local, intratracheal, intranasal, transdermal or rectal administration, the active ingredient of formula (I), above, or the salt thereof, can be administered in unit administration form, as a mixture with conventional pharmaceutical excipients, to animals and to human beings for the treatment of the disorders and diseases as mentioned above.

The suitable unit administration forms include oral forms such as tablets, soft or hard gel capsules, powders, granules and oral solutions or suspensions, sublingual, buccal, intratracheal, intraocular and intranasal administration forms, forms for administration by inhalation, topical, transdermal, subcutaneous, intramuscular or intravenous administration forms, rectal administration forms, and implants. For topical application, the compounds according to the invention can be used in creams, gels, ointments or lotions.

According to the usual practice, the dosage suitable for each patient is determined by the physician according to the mode of administration and the weight and response of said patient.

### EXAMPLES

### CHEMICAL SYNTHESIS

### General Procedure A: Hydrazone formation

To a suspension of 4-hydroxy-6-hydrazinylpyrimidine (1.0 equiv.) in EtOH (0.5 M) was added the corresponding ketone (1.5 equiv.). The reaction was refluxed for 3 h and then cooled to 0°C. The solid was collected by filtration, washed with Et₂O and dried under high vacuum to afford the desired product.

### General Procedure B: Fischer synthesis

A suspension of hydrazone (1.0 equiv.) in tetraline (0.25 M) was heated to 260°C in a sealed tube for 3 h. The reaction mixture was cooled to room temperature. The solid was collected by filtration, washed with petroleum ether and Et₂O several times and dried under high vacuum to afford the desired product.

### General Procedure C: Chlorination

A solution of the tricyclic compound (1.0 equiv.) in POCl₃ (0.15 M) was heated at 100°C for 1 h. Excess POCl₃ was removed under vacuum and the resulting dark slurry was suspended in CH₂Cl₂ and poured into an ice-bath. The mixture was stirred for 1 h and extracted with CH₂Cl₂. The organic layers were dried over MgSO₄, filtered and concentrated under vacuum. The residue was purified by silica gel column chromatography to afford the desired product.

### General Procedure D: Suzuki-Miyaura cross-coupling

To a solution of the chlorinated heterocycle (1.0 equiv.) in THF (0.25 M) were added boronate ester (1.1 equiv.) followed by a 2 M aqueous solution of Na₂CO₃ (3.0 equiv.). The solution was degassed for 20 minutes with Argon and Pd(PPh₃)₄ (0.1 equiv.) was added. The mixture was then heated under microwave irradiation at 120°C for 1.5 h, cooled and extracted with CH₂Cl₂. The organic layers were dried over MgSO₄, filtered and concentrated under vacuum. The residue was purified by silica gel column chromatography to afford the desired product.

### General Procedure E: Hydrogenation

Pd/C (10% wt) (0.05 equiv.) was added to a solution of N-protected compound (1.0 equiv.) in MeOH (0.06 M). The reaction was stirred under H₂ pressure until completion. The mixture was filtered through a pad of celite which was rinsed several times with MeOH. The MeOH was evaporated under reduced pressure and the crude reaction mixture was used in the next step without further purification.

### General Procedure F1: Boc deprotection, free amine

To a solution of the *N*-Boc protected compound (1.0 equiv.) in CH₂Cl₂ (0.04 M) at 0°C was slowly added TFA (35.0 equiv.) (CH₂Cl₂/TFA 10/1). The mixture was allowed to reach room temperature and stirred until completion (TLC monitoring). The solvent was then removed under reduced pressure. The residue was taken up in CH₂Cl₂ and a 2 M aqueous solution of NaOH was added. The mixture was stirred for 30 min and extracted with CH₂Cl₂. The combined organic layers were dried over MgSO₄, filtered and concentrated under vacuum to afford the desired product.

### General Procedure F2: Boc deprotection (amine TFA salt)

To a solution of *N*-Boc protected compound (1.0 equiv.) in CH₂Cl₂ (0.04 M) at 0°C was slowly added TFA (35.0 equiv.). The mixture was allowed to reach room temperature and stirred until completion (TLC monitoring). The solvent was then removed under reduced pressure and the residue was co-evaporated 3 times with Et₂O. The resulting solid was taken up in Et₂O, filtered, washed with Et₂O and pentane and dried at 60°C under vacuum to afford the desired amine as a TFA salt.

### General Procedure G: Urea synthesis using isocyanates

To a solution of the desired amine (1.0 equiv.) in anhydrous CH₂Cl₂ (0.07 M) under argon was added DIPEA (3.0 equiv.). The solution was cooled to 0°C and the corresponding isocyanate (1.1 equiv.) was added. The mixture was stirred at this temperature until completion (TLC monitoring). The solvent was then removed under reduced pressure and the residue was purified by silica gel column chromatography to afford the desired product.

### General Procedure H: Urea synthesis using 4-nitrophenyl chloroformate

To a solution of the desired aniline (1.0 equiv.) in THF (0.07 M) at 0°C was added DIPEA (1.1 to 2.1 equiv.) and 4-nitrophenyl chloroformate (1.2 equiv.). The mixture was stirred at 0°C until total disappearance of the starting material (TLC monitoring). Then, the second amine (free or as a salt derivative) (1.2 equiv.) and DIPEA (2.1 equiv.) were added and the reaction mixture was stirred at 50°C until completion (TLC monitoring). The solvent was removed under reduced pressure and the residue was purified by silica gel column chromatography to afford the desired product.

### General Procedure I: Urea synthesis using Curtius rearrangement

To a solution of acid (1.0 equiv.) was added Et₃N (1.2 equiv.) in THF (0.05 M) at 0°C. Ethyl chloroformate (1.5 equiv.) was added and the reaction was stirred for 1 h at 0°C (TLC monitoring). A solution of NaN₃ (1.5 equiv.) in H₂O was then added at 0°C and stirred for 1.5 h (TLC monitoring). The reaction was quenched with ice and quickly extracted with EtOAc, dried with MgSO₄, filtered and the solvent was removed under reduced pressure without heating. The acylazide was dissolved in toluene (0.05 M) and stirred for 1 h at reflux. Toluene was removed under reduced pressure. To this freshly prepared isocyanate was added a solution of amine (1.0 equiv.) and DIPEA (2.2 equiv.) in anhydrous CH₂Cl₂ at 0°C. The mixture was stirred at this temperature until completion (TLC monitoring). The solvent was then removed under reduced pressure and the residue was purified by silica gel column chromatography to afford the desired product.

### General Procedure J: Boc deprotection (amine HCl salt)

Hydrochloric acid (4M HCI solution in dioxane, 18 equiv.) was slowly added to a solution of the *N*-Boc protected compound (1.0 equiv.) in dioxane (0.11 M) at 0°C. The mixture was allowed to reach room temperature and stirred until completion (TLC monitoring). Diethyl ether was added to precipitate the amine salt which was filtered and washed with Et₂O then dried at 60°C under vacuum to afford the desired amine as an HCI salt.

### General Procedure K: modified Fisher Synthesis

A suspension of hydrazone (1.0 equiv.) in diphenylether (0.22 M) was degassed for 20 minutes with Ar and then heated to reflux for 3 h. After cooling to room temperature the solid was filtered, washed several times with Et₂O then dried at 60°C under vacuum to afford the desired product.

### General Procedure L: Mitsunobu reaction

To a solution of 3-nitrophenol (1.0 equiv.) in dry THF (0.28 M) under argon were added the primary alcohol (2.0 equiv.) and triphenylphosphine (1.7 equiv.). The mixture was stirred at rt for 10 min until full dissolution of the triphenylphosphine. Diisopropyl azodicarboxylate (DIAD) (1.7 equiv.) was then added dropwise at rt over 5 to 10 min. The reaction mixture was stirred at rt for 24h then diluted with H₂O and extracted twice with Et₂O. The combined organic layers were dried over MgSO₄, filtered and concentrated under reduced pressure. The crude reaction mixture was purified by silica gel column chromatography to afford the desired product.

### General Procedure M: Williamson reaction

To a solution of 3-nitrophenol (1.0 equiv.) in dry MeCN (0.80 M) under argon were added K₂CO₃ (1.6 equiv.) and the corresponding alkyl halide (8.0 equiv.). The resulting suspension was stirred under reflux until full conversion (TLC monitoring), then concentrated under reduced pressure. The residue was partitioned between DCM and H₂O. The aqueous layer was separated and extracted twice with DCM then the combined organic layers were dried over MgSO₄, filtered and concentrated under reduced pressure. The crude reaction mixture was purified by silica gel column chromatography to afford the desired product.

### General Procedure N: Nitro group reduction

A 50 mL round bottom flask was loaded with the aromatic nitro compound (1.0 equiv.) then a suspension of 10 wt.% Pd/C (0.1 equiv.) in MeOH (0.18 M) was added. The flask was flushed with hydrogen and stirred at rt under hydrogen gas until full conversion of the starting material. The reaction mixture was then filtered through Celite, washed with methanol and concentrated under reduced pressure to afford the desired amine.

### General Procedure O: N-alkylation

To a solution of alkyl halide (1.0 equiv.) in dry THF (0.18 M) under argon atmosphere was added the amine (3.1 equiv.). The reaction mixture was stirred under reflux until full conversion then cooled to rt and concentrated under reduced pressure. The residue was stirred in an aqueous solution of HCI 1M for 15 min, washed twice with Et₂O and then treated with a 10 % aqueous solution of NaOH to pH - 10-13 . The aqueous layer was extracted with Et₂O (three times) then the combined organic layers were dried over MgSO₄, filtered and concentrated under reduced pressure to afford the desired product.

### General Procedure P: Ullmann coupling with Ar-I

An oven-dried microwave reaction vial was charged with a magnetic stirbar, 3-aminophenol (1.2 equiv.), copper(I) iodide (5 mol %), 2-picolinic acid (10 mol%), aryl iodide (1.0 equiv.) and K₃PO₄ (2.0 equiv.). The tube was then evacuated and back-filled with argon. The evacuation/backfill sequence was repeated two additional times. Under argon, remaining liquid reagents were added, followed by dry DMSO (0.5 M). The vial was sealed then placed in a preheated hot plate at 80 °C and the reaction mixture was stirred vigorously for 18-36 h. The reaction mixture was cooled to room temperature. EtOAc and H₂O were added, the aqueous layer was separated and extracted with EtOAc (2 x). The combined organic layers were washed with brine (3 x), dried over MgSO₄, filtered and concentrated under reduced pressure. The crude residue was purified by silica gel column chromatography to afford the desired aniline.

### FAMILY 1.

### 3-methyl pyrolopyrimidine derivatives

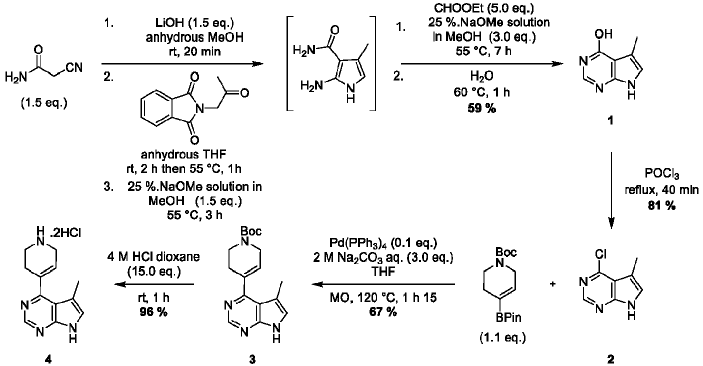

### 5-Methyl-7H-pyrrolo[2,3-d]pyrimidin-4-ol (1)

To a solution of LiOH (3.5 g, 147.7 mmol, 1.5 equiv.) in anhydrous MeOH (120 mL) was added cyanoacetamide (12.4 g, 147.7 mmol, 1.5 equiv.) under argon. This solution was stirred 20 min and addition of a solution of phtalimidoacetone in anhydrous THF (140 mL) was performed over 30 min. The reaction mixture was then stirred 2 h at room temperature and 1 h at 55°C. A sodium methoxide (NaOMe) solution (25% in methanol, 31.6 mL, 147.7 mmol, 1.5 equiv.) was added dropwise at 55°C and the mixture was stirred 3 h. Ethyl formate (HCOOEt, 39.7 mL, 492.2 mmol, 5.0 equiv.) followed by NaOMe solution (25% in methanol, 63.2 mL, 295.3 mmol, 3.0 equiv.) were then added and the reaction mixture was stirred at 55°C overnight. After cooling, the reaction mixture was diluted with water (500 mL), heated at 60°C for 1 h, and then concentrated under reduced pressure to small volume (≈ 500 mL). The resulting solution was slowly acidified to pH = 7 with a 6 N aqueous HCI solution, cooled to about 5°C and stirred at this temperature for 30 min. The solid was filtered, washed with water (100 mL) and dried at 50°C under vacuum overnight to afford compound 1 (8.65 g, 59%) as a light brown solid. ¹H NMR (250 MHz, DMSO-*d₆*) δ 11.50 (br s, 2H, 2 x NH), 7.73 (s, 1H, CH), 6.75 - 6.72 (m, 1H, CH), 2.26 (d, *J* = 1.1 Hz, 3H, CH₃). Org. Process Res. Dev. 2007, 11 (1), 86-89.

### 4-Chloro-5-methyl-7H-pyrrolo[2,3-d]pyrimidine (2)

The hydroxy compound **1** (2.0 g, 13.4 mmol) was dissolved in POCl₃ (50 mL) and stirred at reflux for 1.5 h. After cooling, the mixture was slowly poured into a vigorously stirred ice-water bath and stirred for 30 min. The mixture was carefully neutralized to pH 7 with solid NaOH. Then, the mixture was extracted with CH₂Cl₂ (3 x 150 mL). The combined organic layers were dried over MgSO₄, filtered and concentrated under reduced pressure. The residue was purified by flash silica gel column chromatography using a gradient solvent system of PE/EtOAc from 100/0 up to 70/30. Finally the product was precipitated in a mixture of acetone/PE to 2 (3.6 g, 81 %) as a beige solid. ¹H NMR (250 MHz, CDCl₃) δ 9.49 (br s, 1H, NH), 8.60 (s, 1H, CH), 7.11 (dd, *J* = 2.3 Hz, *J* = 1.2 Hz, 1H, CH), 2.50 (d, *J* = 1.2 Hz, 3H, CH₃). West, R. A. J. Org. Chem. 1961, 26, 4959-4961.

### tert-Butyl 4-(5-methyl-7H-pyrrolo[2,3-d]pyrimidin-4-yl)-3,6-dihydro pyridine-1(2H)-carboxylate (3)

The reaction was carried out as described in general procedure **D** using the chlorinated bicycle **2** (0.50 g, 2.99 mmol), *N*-Boc-1,2,3,6-tetrahydropyridine-4-boronic acid pinacol ester (1.02 g, 3.29 mmol, 1.1 equiv.), Pd(PPh₃)₄ (0.35 g, 0.30 mmol, 0.1 equiv.) in THF (12 mL) and a 2 M solution of Na₂CO₃ (4.5 mL, 8.98 mmol, 3.0 equiv.). The crude reaction mixture was purified by silica gel column chromatography using a gradient solvent system of CH₂Cl₂/acetone from 90/10 up to 60/40 to afford compound **3** (0.63 g, 67%) as a beige solid. R*_{f}* (CH₂Cl₂/acetone 60/40) 0.33. Mp: 190 - 192°C..¹H NMR (250 MHz, CDCl₃) δ 10.40 (br s, 1H, NH), 8.81 (s, 1H, CH), 7.12 (s, 1H, CH), 6.06 - 5.87 (m, 1H, CH), 4.24 - 4.09 (m, 2H, CH₂), 3.72 (t, *J* = 5.6 Hz, 2H, CH₂), 2.77 - 2.65 (m, 2H, CH₂), 2.29 (d, *J* = 1.1 Hz, 3H, CH₃), 1.51 (s, 9H, 3 x CH₃). HRMS (EI-MS) m/z calcd for C₁₇H₂₃N₄O₂ [M+H]⁺: 315.1816, found: 315.1814.

### 5-Methyl-4-(1,2,3,6-tetrahydropyridin-4-yl)-7H-pyrrolo[2,3-d]pyrimidine dihydrochloride (4)

To a solution of the *N*-Boc protected compound **3** (1.68 g, 5.35 mmol) in 1,4-dioxane (5 mL) at 0°C was added a 4 M solution of HCl in 1,4-dioxane (20 mL, 80.0 mmol, 15.0 equiv.). The reaction mixture was stirred for 1 hour at room temperature. After completion, Et₂O (200 mL) was added and the resulting precipitate was filtered and washed with Et₂O (3 x 30 mL) to afford compound **4** (1.47 g, 96 %) as a beige solid. Mp: > 260°C. ¹H NMR (400 MHz, DMSO-*d₆*) δ 13.23 (br s, 1H, NH), 9.94 (br s, 2H, NH + H⁺), 8.98 (s, 1H, CH), 7.73 (s, 1H, CH), 6.39 - 6.28 (m, 1H, CH), 4.48 - 4.11 (m, 1H, H⁺), 3.91 - 3.81 (m, 2H, CH₂), 3.41 - 3.30 (m, 2H, CH₂), 2.98 - 2.88 (m, 2H, CH₂), 2.31 (d, *J* = 1.2 Hz, 3H, CH₃). HRMS (EI-MS) m/z calcd for C₁₂H₁₅N₄ [M+H]⁺: 215.1291, found: 215.1295.

### SUBFAMILY 1.1.

### 3-methyl pyrolopyrimidine derivatives with an unsubstituted hydropyridine

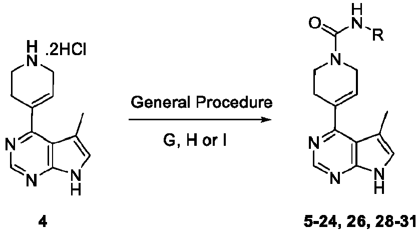

| Compound | General Procedure | Yield (%) | R |
|---|---|---|---|
| **5** | G | 80 | 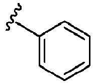 |
| **6** | G | 60 | 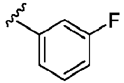 |
| **7** | G | 47 | 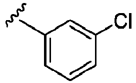 |
| **8** | G | 70 | 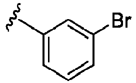 |
| **9** | G | 46 | 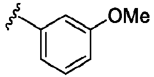 |
| **10** | G | 76 | 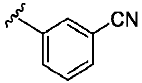 |
| **11** | G | 72 | 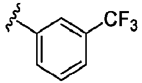 |
| **12** | G | 47 | 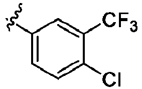 |
| **13** | G | 60 | 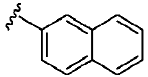 |
| **14** | G | 35 | 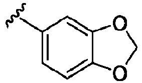 |
| **15** | G | 49 | 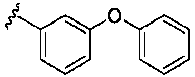 |
| **16** | G | 85 | 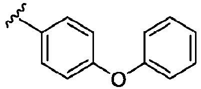 |
| **17** | I | 34 | 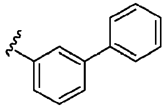 |
| **18** | I | 15 | 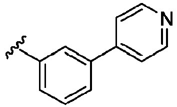 |
| **19** | I | 49 | 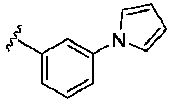 |
| **20** | H | 58 | 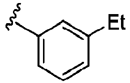 |
| **21** | H | 70 | 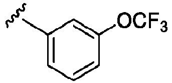 |
| **22** | H | 57 | 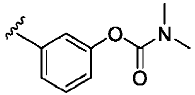 |
| **24** | H | 45 | 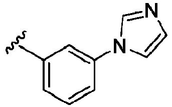 |
| **26** | H | 63 | 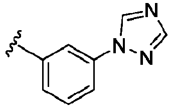 |
| **28** | H | 55 | 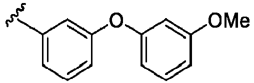 |
| **29** | H | 72 | 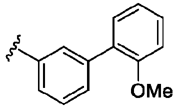 |
| **30** | H | 78 | 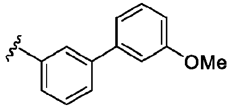 |
| **31** | H | 72 | 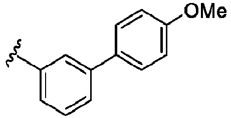 |
| **32** | H | 60 | 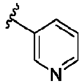 |
| **33** | G | 54 | 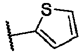 |
| **34-rac** | H | 63 | 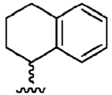 |
| **35-R** | H | 60 | 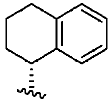 |
| **40** | H | 40 | 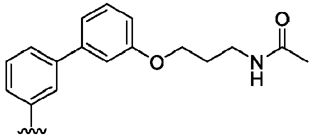 |
| **42** | H | 42 | 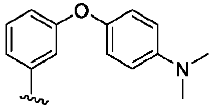 |
| **172** | H | 52 | 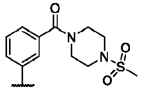 |
| **175** | H | 59 | 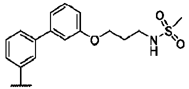 |
| **177** | H | 78 | 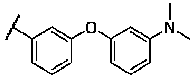 |
| **184** | H | 15 | 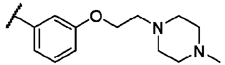 |
| **185** | H | 22 | 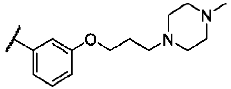 |
| **188** | H | 57 | 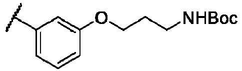 |
| **191** | H | 59 | 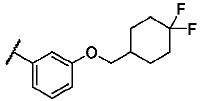 |
| **195** | H | 34 | 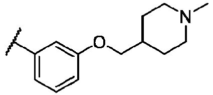 |
| **196** | H | 88 | 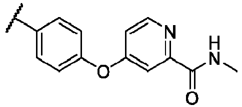 |
| **198** | H | 73 | 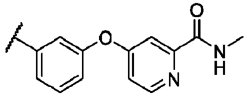 |
| **200** | H | 76 | 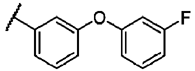 |
| **202** | H | 74 | 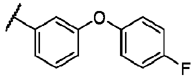 |
| **204** | H | 85 | 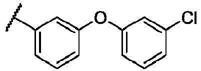 |
| **206** | H | 73 | 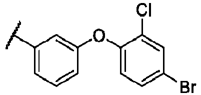 |
| **208** | H | 68 | 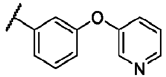 |
| **211** | H | 79 | 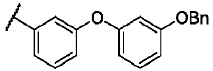 |
| **213** | H | 46 | 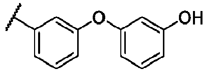 |
| **216** | H | 64 | 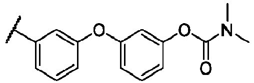 |
| **219** | H | 24 | 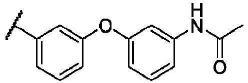 |
| **222** | H | 41 | 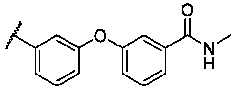 |

### Example 1: 4-(5-Methyl-7H-pyrrolo[2,3-d]pyrimidin-4-yl)-N-phenyl-3,6-dihydropyridine-1(2H)-carboxamide (5)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 100/0 up to 40/60 to afford compound **5** (64 mg, 80 %) as an off-white solid. R*_{f}* (CH₂Cl₂/acetone 30/70) 0.35. Mp: 245 - 247°C. ¹H NMR (250 MHz, MeOD-d₄) δ 8.63 (s, 1H, CH), 7.45 - 7.36 (m, 2H, 2 x CH), 7.33 - 7.24 (m, 2H, 2 x CH), 7.23 (d, *J* = 1.3 Hz, 1H, CH), 7.09 - 6.98 (m, 1H, CH), 6.08 - 6.00 (m, 1H, CH), 4.29 (q, *J* = 2.8 Hz, 2H, CH₂), 3.84 (t, *J* = 5.6 Hz, 2H, CH₂), 2.80 - 2.68 (m, 2H, CH₂), 2.30 (d, *J* = 1.2 Hz, 3H, CH₃). HRMS (EI-MS) m/z calcd for C₁₉H₂₀N₅O [M+H]⁺: 334.1662, found: 334.1661.

### Example 2: N-(3-Fluorophenyl)-4-(5-methyl-7H-pyrrolo[2,3-d]pyrimidin-4-yl)-3,6-dihydropyridine-1(2H)-carboxamide (6)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 90/10 up to 60/40 to afford compound **65 6** (51 mg, 60%) as an off-white solid. R*_{f}* (CH₂Cl₂/acetone 70/30) 0.28. Mp: 241-243°C. ¹H NMR (400 MHz, DMSO-*d₆*) δ 11.83 (br s, 1H, NH), 8.79 (br s, 1H, NH), 8.65 (s, 1H, CH), 7.49 (d, *J* = 12.4 Hz, 1H, CH), 7.38 - 7.23 (m, 3H, 3 x CH), 6.86 - 6.66 (m, 1H, CH), 6.05 (s, 1H, CH), 4.34 - 4.10 (m, 2H, CH₂), 3.81 - 3.69 (m, 2H, CH₂), 2.74 - 2.64 (m, 2H, CH₂), 2.23 (s, 3H, CH₃). ¹⁹F NMR (376 MHz, DMSO-d₆) δ -112.7 - -113.0 (m). HRMS (EI-MS) m/z calcd for C₁₉H₁₉FN₅O [M+H]⁺: 352.1568, found: 352.1570.

### Example 3: N-(3-Chlorophenyl)-4-(5-methyl-7H-pyrrolo[2,3-d]pyrimidin-4-yl)-3,6-dihydropyridine-1(2H)-carboxamide (7)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 90/10 up to 60/40 to afford compound **7** (42 mg, 47 %) as an off-white solid. R*_{f}* (CH₂Cl₂/acetone 70/30) 0.29. Mp: 232 - 234°C. ¹H NMR (400 MHz, DMSO-d₆) δ 11.84 (br s, 1H, NH), 8.77 (br s, 1H, NH), 8.65 (s, 1H, CH), 7.70 (t, *J* = 2.1 Hz, 1H, CH), 7.45 (dd, *J* = 8.2 Hz, *J* = 2.0 Hz, 1H, CH), 7.32 (s, 1H, CH), 7.27 (t, *J* = 8.1 Hz, 1H, CH), 6.99 (dd, *J* = 8.0 Hz, *J* = 2.1 Hz, 1H, CH), 6.09 - 6.01 (m, 1H, CH), 4.26 - 4.19 (m, 2H, CH₂), 3.74 (t, *J* = 5.6 Hz, 2H, CH₂), 2.73 - 2.61 (m, 2H, CH₂), 2.24 (s, 3H, CH₃). HRMS (EI-MS) m/z calcd for C₁₉H₁₉ClN₅O [M+H]⁺: 368.1273, found: 368.1275.

### Example 4: N-(3-Bromophenyl)-4-(5-methyl-7H-pyrrolo[2,3-d]pyrimidin-4-yl)-3,6-dihydropyridine-1(2H)-carboxamide (8)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 90/10 up to 60/40 to afford compound **8** (69 mg, 70 %) as a white solid. R*_{f}* (CH₂Cl₂/acetone 70/30) 0.28. Mp: 223 - 225°C. ¹H NMR (400 MHz, DMSO-d₆) δ 11.82 (br s, 1H, NH), 8.75 (br s, 1H, NH), 8.65 (s, 1H, CH), 7.83 (s, 1H, CH), 7.50 (d, *J* = 8.3 Hz, 1H, CH), 7.31 (s, 1H, CH), 7.21 (t, *J* = 8.0 Hz, 1H, CH), 7.12 (d, *J* = 8.0 Hz, 1H, CH), 6.05 (s, 1H, 4.30 - 4.13 (m, 2H, CH₂), 3.73 (t, *J* = 5.6 Hz, 2H, CH₂), 2.75 - 2.62 (m, 2H, CH₂), 2.23 (s, 3H, CH₃). HRMS (EI-MS) m/z calcd for C₁₉H₁₉BrN₅O [M+H]⁺: 412.0767, found: 412.0766.

### Example 5: N-(3-Methoxyphenyl)-4-(5-methyl-7H-pyrrolo[2,3-d]pyrimidin-4-yl)-3,6-dihydropyridine-1(2H)-carboxamide (9)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 90/10 up to 60/40 to afford compound **9** (40 mg, 46 %) as a white solid. R*_{f}* (CH₂Cl₂/acetone 70/30) 0.30. Mp: 212 - 214°C. ¹H NMR (400 MHz, DMSO-*d₆*) δ 11.82 (br s, 1H, NH), 8.65 (s, 1H, CH), 8.56 (br s, 1H, NH), 7.38 - 7.06 (m, 4H, 4 x CH), 6.67 - 6.37 (m, 1H, CH), 6.15 - 5.95 (m, 1H, CH), 4.22 (s, 2H, CH₂), 3.72 (m, 5H, CH₂ + OCH₃), 2.74 - 2.59 (m, 2H, CH₂), 2.24 (s, 3H, CH₃). HRMS (EI-MS) m/z calcd for C₂₀H₂₂N₅O₂ [M+H]⁺: 364.1768, found: 364.1770.

### Example 6: N-(3-Cyanophenyl)-4-(5-methyl-7H-pyrrolo[2,3-d]pyrimidin-4-yl)-3,6-dihydropyridine-1(2H)-carboxamide (10)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 90/10 up to 40/60 to afford compound **10** (65 mg, 76 %) as an off-white solid. R*_{f}* (CH₂Cl₂/acetone 50/50) 0.19. Mp: 207 - 209°C. ¹H NMR (250 MHz, DMSO-*d₆*) δ 8.63 (s, 1H, CH), 7.89 - 7.86 (m, 1H, CH), 7.71 (ddd, *J* = 8.2 Hz, *J* = 2.3 Hz, *J* = 1.2 Hz, 1H, CH), 7.44 (td, *J* = 7.6 Hz, *J* = 2.4 Hz, 1H, CH), 7.35 (dt, *J* = 7.7 Hz, *J* = 1.4 Hz, 1H, CH), 7.24 (d, *J* = 1.2 Hz, 1H, CH), 6.13 - 5.98 (m, 1H, CH), 4.37 - 4.23 (m, 2H, CH₂), 3.86 (t, *J* = 5.6 Hz, 2H, CH₂), 2.80 - 2.67 (m, 2H, CH₂), 2.31 (d, *J* = 1.2 Hz, 3H, CH₃). ¹HRMS (EI-MS) m/z calcd for C₂₀H₁₉N₆O [M+H]⁺: 359.1615, found: 359.1616.

### Example 7: 4-(5-Methyl-7H-pyrrolo[2,3-d]pyrimidin-4-yl)-N-(3-(trifluoromethyl)phenyl)-3,6-dihydropyridine-1(2H)-carboxamide (11)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **11** (69 mg, 72 %) as an off-white solid. R*_{f}* (CH₂Cl₂/acetone 50/50) 0.33. Mp: degradation 231°C. ¹H NMR (400 MHz, DMSO-d₆) δ 11.83 (br s, 1H, NH), 8.93 (br s, 1H, NH), 8.65 (s, 1H, CH), 7.97 (s, 1H, CH), 7.80 (d, *J* = 8.3 Hz, 1H, CH), 7.48 (t, *J* = 8.0 Hz, 1H, CH), 7.32 (s, 1H, CH), 7.28 (d, *J* = 7.8 Hz, 1H, CH), 6.11 - 5.96 (m, 1H, CH), 4.30 - 4.11 (m, 2H, CH₂), 3.76 (t, *J* = 5.6 Hz, 2H, CH₂), 2.73 - 2.61 (m, 2H, CH₂), 2.24 (s, 3H, CH₃). ¹⁹F NMR (376 MHz, DMSO-d₆) δ -61.2 (s). HRMS (EI-MS) m/z calcd for C₂₀H₁₉F₃N₅O [M+H]⁺: 402.1536, found: 402.1536.

### Example 8: N-(4-Chloro-3-(trifluoromethyl)phenyl)-4-(5-methyl-7H-pyrrolo[2,3-d]pyrimidin-4-yl)-3,6-dihydropyridine-1(2H)-carboxamide (12)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 80/20 up to 40/60 to afford compound **12** (49 mg, 47 %) as an off-white solid. R*_{f}* (CH₂Cl₂/acetone 60/40) 0.22. Mp: 228 - 230°C. ¹H NMR (400 MHz, DMSO-*d₆*) δ 11.83 (br s, 1H, NH), 9.03 (br s, 1H, NH), 8.65 (s, 1H, CH), 8.10 (d, *J* = 2.6 Hz, 1H, CH), 7.85 (dd, *J* = 8.8 Hz, *J* =2.6 Hz, 1H, CH), 7.59 (d, *J* = 8.8 Hz, 1H, CH), 7.31 (s, 1H, CH), 6.10 - 6.02 (m, 1H, CH), 4.29 - 4.18 (m, 2H, CH₂), 3.75 (t, *J* = 5.6 Hz, 2H, CH₂), 2.73 - 2.64 (m, 2H, CH₂), 2.23 (s, 3H, CH₃). ¹⁹F NMR (376 MHz, DMSO-d₆) δ -61.4 (s). HRMS (EI-MS) m/z calcd for C₂₀H₁₈ClF₃N₅O [M+H]⁺: 436.1146, found: 436.1145.

### Example 9: 4-(5-Methyl-7H-pyrrolo[2,3-d]pyrimidin-4-yl)-N-(naphthalen-2-yl)-3,6-dihydropyridine-1(2H)-carboxamide (13)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **13** (55 mg, 60 %) as an off-white solid. R*_{f}* (CH₂Cl₂/acetone 50/50) 0.31. Mp: 233 - 235°C. ¹H NMR (250 MHz, MeOD-d₄) δ 8.64 (s, 1H, CH), 7.91 (d, *J* = 2.1 Hz, 1H, CH), 7.84 - 7.69 (m, 3H, 3xCH), 7.56 (dd, *J* = 8.8 Hz, *J* = 2.2 Hz, 1H, CH), 7.47 - 7.30 (m, 2H, 2 x CH), 7.24 (d, *J* = 1.2 Hz, 1H, CH), 6.13 - 5.99 (m, 1H, CH), 4.39 - 4.31 (m, 2H, CH₂), 3.90 (t, *J* = 5.6 Hz, 2H, CH₂), 2.83 - 2.69 (m, 2H, CH₂), 2.33 (d, *J* = 1.2 Hz, 3H, CH₃). HRMS (EI-MS) m/z calcd for C₂₃H₂₂N₅O [M+H]⁺: 384.1819, found: 384.1815.

### Example 10: N-(Benzo[d][1,3]dioxol-5-yl)-4-(5-methyl-7H-pyrrolo[2,3-d]pyrimidin-4-yl)-3,6-dihydropyridine-1(2H)-carboxamide (14)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 80/20 up to 40/60 to afford compound **14** (32 mg, 35%) as an off-white solid. R*_{f}* (CH₂Cl₂/acetone 60/40) 0.13. Mp: 235 - 237°C. ¹H NMR (400 MHz, DMSO-*d₆*) δ 11.85 (br s, 1H, NH), 8.64 (s, 1H, CH), 8.47 (br s, 1H, NH), 7.38 - 7.24 (m, 1H, CH), 7.22 - 7.11 (m, 1H, CH), 6.93 - 6.69 (m, 2H, 2 x CH), 6.09 - 5.81 (m, 3H, CH + OCH₂O), 4.28 - 4.05 (m, 2H, CH₂), 3.79 - 3.59 (m, 2H, CH₂), 2.73 - 2.58 (m, 2H, CH₂), 2.23 (d, *J* = 17.4 Hz, 3H, CH₃). HRMS (EI-MS) m/z calcd for C₂₀H₂₀N₅O₃ [M+H]⁺: 378.1561, found: 378.1560.

### Example 11: 4-(5-Methyl-7H-pyrrolo[2,3-d]pyrimidin-4-yl)-N-(3-phenoxyphenyl)-3,6-dihydropyridine-1(2H)-carboxamide (15)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 80/20 up to 70/30 to afford compound **15** (50 mg, 49 %) as a white solid. R*_{f}* (CH₂Cl₂/acetone 60/40) 0.21. Mp: 196 - 198°C. ¹H NMR (400 MHz, MeOD-d₄) δ 8.62 (s, 1H, CH), 7.41 - 6.93 (m, 9H, 9xCH), 6.69 - 6.61 (m, 1H, CH), 6.13 - 5.96 (m, 1H, CH), 4.30 - 4.22 (m, 2H, CH₂), 3.82 (t, *J* = 5.6 Hz, 2H, CH₂), 2.77 - 2.64 (m, 2H, CH₂), 2.29 (s, 3H, CH₃). HRMS (EI-MS) m/z calcd for C₂₅H₂₄N₅O₂ [M+H]⁺: 426.1925, found: 426.1923.

### Example 12: 4-(5-Methyl-7H-pyrrolo[2,3-d]pyrimidin-4-yl)-N-(4-phenoxyphenyl)-3,6-dihydropyridine-1(2H)-carboxamide (16)

The reaction was carried out as described in general procedure **G** using amine **4** (70 mg, 0.24 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **16** (87 mg, 85 %) as a white solid. R*_{f}* (CH₂Cl₂/acetone 60/40) 0.18. Mp: 224 - 226°C. ¹H NMR (400 MHz, DMSO-d₆) δ 11.83 (br s, 1H, NH), 8.65 (s, 1H, CH), 8.61 (br s, 1H, NH), 7.52 (d, *J* = 8.9 Hz, 2H, 2 x CH), 7.35 (t, *J* = 7.8 Hz, 2H, 2 x CH), 7.32 (s, 1H, CH), 7.08 (t, *J* = 7.4 Hz, 1H, CH), 6.95 (d, *J* = 8.3 Hz, 4H, 4xCH), 6.05 (s, 1H, CH), 4.28 - 4.17 (m, 2H, CH₂), 3.74 (t, *J* = 5.6 Hz, 2H, CH₂), 2.75 - 2.62 (m, 2H, CH₂), 2.24 (s, 3H, CH₃). HRMS (EI-MS) m/z calcd for C₂₅H₂₄N₅O₂ [M+H]⁺: 426.1925, found: 426.1927.

### Example 13: N-([1,1'-Biphenyl]-3-yl)-4-(5-methyl-7H-pyrrolo[2,3-d] pyrimidin-4-yl)-3,6-dihydropyridine-1(2H)-carboxamide (17)

The reaction was carried out as described in general procedure **I** using biphenyl-3-carboxylic acid (61 mg, 0.31 mmol), and the amine **4** (89 mg, 0.31 mmol). The crude reaction mixture was purified by silica gel column chromatography using a gradient solvent system of CH₂Cl₂/acetone from 100/0 up to 60/40 to afford compound **17** (43 mg, 34 %) as a white solid. R*_{f}* (CH₂Cl₂/acetone 70/30) 0.31. Mp: 235 - 237°C. ¹H NMR (400 MHz, DMSO-*d₆*) δ 11.83 (br s, 1H, NH), 8.68 (br s, 1H, NH), 8.65 (s, 1H, CH), 7.83 (s, 1H, CH), 7.71 - 7.18 (m, 9H, 9xCH), 6.17 - 5.97 (m, 1H, CH), 4.33 - 4.15 (m, 2H, CH₂), 3.83 - 3.67 (m, 2H, CH₂), 2.78 - 2.62 (m, 2H, CH₂), 2.25 (s, 3H, CH₃). HRMS (EI-MS) m/z calcd for C₂₅H₂₄N₅O [M+H]⁺: 410.1975, found: 410.1983.

### Example 14: 4-(5-Methyl-7H-pyrrolo[2,3-d]pyrimidin-4-yl)-N-(3-(pyridin-4-yl)phenyl)-3,6-dihydropyridine-1(2H)-carboxamide (18)

The reaction was carried out as described in general procedure **I** using 3-(pyridin-4-yl)benzoic acid (62 mg, 0.31 mmol), and the amine **4** (89 mg, 0.31 mmol). The crude reaction mixture was purified by silica gel column chromatography using a gradient solvent system of CH₂Cl₂/acetone from 100/0 up to 50/50 to afford compound **18** (19 mg, 15 %) as a beige solid. R*_{f}* (CH₂Cl₂/acetone 50/50) 0.24. Mp: 208 - 210°C. ¹H NMR (400 MHz, DMSO-*d₆*) δ 11.83 (br s, 1H, NH), 8.77 (br s, 1H, NH), 8.71 - 8.59 (m, 3H, 3 x CH), 8.01 - 7.90 (m, 1H, CH), 7.73 - 7.56 (m, 2H, 2 x CH), 7.45 - 7.36 (m, 3H, 3xCH), 7.32 (s, 1H, CH), 6.07 (s, 1H, CH), 4.26 (s, 2H, CH₂), 3.77 (s, 2H, CH₂), 2.70 (s, 2H, CH₂), 2.25 (s, 3H, CH₃). HRMS (EI-MS) m/z calcd for C₂₄H₂₄N₆O [M+H]⁺: 411.1928, found: 411.1933.

### Example 15: N-(3-(1H-Pyrrol-1-yl)phenyl)-4-(5-methyl-7H-pyrrolo[2,3-d] pyrimidin-4-yl)-3,6-dihydropyridine-1(2H)-carboxamide (19)

The reaction was carried out as described in general procedure **I** using 3-(1*H-*pyrrol-1-yl)benzoic acid (58 mg, 0.31 mmol), and amine **4** (89 mg, 0.31 mmol, 1.0 equiv.). The crude reaction mixture was purified by silica gel column chromatography using a gradient solvent system of CH₂Cl₂/acetone from 100/0 up to 50/50 to afford compound **19** (61 mg, 49 %) as an off-white solid. R*_{f}* (CH₂Cl₂/acetone 50/50) 0.28. Mp: 215 - 217°C¹H NMR (400 MHz, DMSO-*d₆*) δ 11.85 (br s, 1H, NH), 8.75 (br s, 1H, NH), 8.66 (s, 1H, CH), 7.76 (s, 1H, CH), 7.52 - 7.06 (m, 6H, 6 x CH), 6.26 (s, 2H, 2 x CH), 6.18 - 5.97 (m, 1H, CH), 4.37 - 4.09 (m, 2H, CH₂), 3.85 - 3.69 (m, 2H, CH₂), 2.77 - 2.63 (m, 2H, CH₂), 2.24 (s, 3H, CH₃). HRMS (EI-MS) m/z calcd for C₂₃H₂₃N₆O [M+H]⁺: 399.1928, found: 399.1928.

### Example 16: N-(3-Ethylphenyl)-4-(5-methyl-7H-pyrrolo[2,3-d]pyrimidin-4-yl)-3,6-dihydropyridine-1(2H)-carboxamide (20)

The reaction was carried out as described in general procedure **H** using 3-ethylaniline (31 µL, 0.25 mmol) and amine **4** (85 mg, 0.30 mmol) in THF (4 mL). The crude reaction mixture was purified by silica gel column chromatography using a gradient solvent system of CH₂Cl₂/acetone from 80/20 up to 30/70 to afford compound **20** (52 mg, 58%) as a white solid. R*_{f}* (CH₂Cl₂/acetone 60/40) 0.20. Mp: 210 - 212°C. ¹H NMR (400 MHz, DMSO-*d₆*) δ 11.82 (br s, 1H, NH), 8.65 (s, 1H, CH), 8.51 (br s, 1H, NH), 7.40 - 7.28 (m, 3H, 3 x CH), 7.14 (t, *J* = 7.7 Hz, 1H, CH), 6.80 (d, *J* = 7.6 Hz, 1H, CH), 6.04 (s, 1H, CH), 4.30 - 4.14 (m, 2H, CH₂), 3.73 (t, *J* = 5.6 Hz, 2H, CH₂), 2.73 - 2.64 (m, 2H, CH₂), 2.55 (q, *J* = 7.7 Hz, 2H, CH₂), 2.24 (s, 3H, CH₃), 1.17 (t, *J* = 7.6 Hz, 3H, CH₃). HRMS (EI-MS) m/z calcd for C₂₁H₂₄N₅O [M+H]⁺: 362.1975, found: 362.1975.

### Example 17: 4-(5-Methyl-7H-pyrrolo[2,3-d]pyrimidin-4-yl)-N-(3-(trifluoromethoxy)phenyl)-3,6-dihydropyridine-1(2H)-carboxamide (21)

The reaction was carried out as described in general procedure **H** using 3-(trifluoromethoxy)aniline (33 µL, 0.25 mmol) and amine **4** (85 mg, 0.30 mmol, 1.2 equiv.) in THF (4 mL). The crude reaction mixture was purified by silica gel column chromatography using a gradient solvent system of CH₂Cl₂/acetone from 80/20 up to 20/80 to afford compound **21** (73 mg, 70%) as a white solid. R*_{f}* (CH₂Cl₂/acetone 60/40) 0.18. Mp: 227 - 229°C. ¹H NMR (400 MHz, DMSO-*d₆*) δ 11.83 (br s, 1H, NH), 8.87 (br s, 1H, NH), 8.65 (s, 1H, CH), 7.67 (s, 1H, CH), 7.52 (d, *J* = 8.3 Hz, 1H, CH), 7.36 (t, *J* = 8.3 Hz, 1H, CH), 7.32 (s, 1H, CH), 6.91 (d, *J* = 8.2 Hz, 1H, CH), 6.05 (s, 1H, CH), 4.33 - 4.13 (m, 2H, CH₂), 3.75 (t, *J* = 5.7 Hz, 2H, CH₂), 2.76 - 2.61 (m, 2H, CH₂), 2.23 (s, 3H, CH₃). ¹⁹F NMR (376 MHz, DMSO-*d₆*) δ -56.6 (s). HRMS (EI-MS) m/z calcd for C₂₀H₁₉F₃N₅O₂ [M+H]⁺: 418.1485, found: 418.1484.

### Example 18: 3-(4-(5-Methyl-7H-pyrrolo[2,3-d]pyrimidin-4-yl)-1,2,3,6-tetrahydropyridine-1-carboxamido)phenyl dimethylcarbamate (22)

The reaction was carried out as described in general procedure **H** using 3-aminophenyldimethyl carbamate (45 mg, 0.25 mmol) and amine **4** (85 mg, 0.30 mmol, 1.2 equiv.) in THF (4 mL). The crude mixture was purified by silica gel column chromatography using a gradient solvent system of CH₂Cl₂/acetone from 90/10 up to 20/80 to afford compound **22** (60 mg, 57%) as a white solid. R*_{f}* (CH₂Cl₂/acetone 60/40) 0.21. Mp: 214 - 216°C. ¹H NMR (400 MHz, DMSO-*d₆*) δ 11.81 (br s, 1H, NH), 8.89 - 8.45 (m, 2H, NH + CH), 7.60 - 7.10 (m, 4H, 4 x CH), 6.87 - 6.49 (m, 1H, CH), 6.07 - 5.92 (m, 1H, CH), 4.29 - 4.09 (m, 2H, CH₂), 3.71 (t, *J* = 5.7 Hz, 2H, CH₂), 3.03 (s, 3H, NCH₃), 2.91 (s, 3H, NCH₃), 2.75 - 2.60 (m, 2H, CH₂), 2.23 (s, 3H, CH₃). HRMS (EI-MS) m/z calcd for C₂₂H₂₅N₆O₃ [M+H]⁺: 421.1983, found: 421.1978.

### 3-(1H-Imidazol-1-yl)aniline (23)

An oven-dried microwave vial was charged with a magnetic stirbar, Cul (28 mg, 0.15 mmol, 0.05 equiv.), K₃PO₄ (1.31 g, 6.17 mmol, 2.1 equiv.), imidazole (0.20 g, 2.94 mmol, 1.0 equiv.) and 1,10-Phenanthroline (52 mg, 0.29 mmol, 0.1 equiv.). The tube was then evacuated and back-filled with argon. The evacuation/backfill sequence was repeated two additional times. Under a counterflow of argon, 3-iodoaniline (0.42 mL, 3.53 mmol, 1.2 equiv.) and degassed 1,4-dioxane (1.5 mL) were added by syringe. The tube was placed in a preheated oil bath at 110 °C and the solution was stirred vigorously for 24 h. The reaction mixture was cooled to room temperature, diluted with EtOAc (2-3 mL), filtered through a plug of Celite and rinsed with EtOAc. The filtrate was concentrated under reduced pressure and the resulting residue was purified by silica gel column chromatography using a gradient solvent system of PE/EtOAc from 90/10 up to 80/20 to afford compound **23** (0.38 g, 82 %) as a beige solid. ¹H NMR (250 MHz, DMSO-*d₆*) δ 7.82 (s, 1H, CH), 7.28 - 7.16 (m, 3H, 3 x CH), 6.79 - 6.73 (m, 1H, CH), 6.70 - 6.63 (m, 2H, 2 x CH), 3.75 (br s, 2H, NH₂). All spectral data corresponded to literature values as found in Suresh, P.; Pitchumani, K. J. Org. Chem. 2008, 73, 9121-9124.

### Example 19: N-(3-(1H-Imidazol-1-yl)phenyl)-4-(5-methyl-7H-pyrrolo[2,3-d]pyrimidin-4-yl)-3,6-dihydropyridine-1(2H)-carboxamide (24)

The reaction was carried out as described in general procedure **H** using aniline **23** (40 mg, 0.25 mmol) and amine **4** (85 mg, 0.30 mmol, 1.2 equiv.) in THF (4 mL). The crude mixture was purified by silica gel column chromatography using a gradient solvent system of CH₂Cl₂/acetone from 90/10 up to 40/60 to afford compound 24 (45 mg, 45 %) as an off-white solid. R*_{f}* (CH₂Cl₂/acetone 50/50) 0.23. Mp: 245 - 247°C¹H NMR (400 MHz, DMSO-*d₆*) δ 11.84 (br s, 1H, NH), 8.83 (br s, 1H, NH), 8.65 (s, 1H, CH), 8.13 (s, 1H, H), 7.80 (t, *J* = 2.2 Hz, 1H, CH), 7.62 (s, 1H, CH), 7.52 (dd, *J* = 8.0 Hz, *J* = 2.0 Hz, 1H, CH), 7.39 (t, *J* = 8.1 Hz, 1H, CH), 7.32 (s, 1H, CH), 7.20 (dd, *J* = 7.9 Hz, *J* = 2.2 Hz, 1H, CH), 7.11 (s, 1H, CH), 6.11 - 6.02 (m, 1H, CH), 4.32 - 4.16 (m, 2H, CH₂), 3.76 (t, *J* = 5.5 Hz, 2H, CH₂), 2.70 (s, 2H, CH₂), 2.24 (s, 3H, CH₃). HRMS (EI-MS) m/z calcd for C₂₂H₂₂N₇O [M+H]⁺: 400.1880, found: 400.1880.

### 3-(1H-1,2,4-Triazol-1-yl)aniline (84) 25 GB 156

An oven-dried microwave vial was charged with a magnetic stirbar, Cul (28 mg, 0.15 mmol, 0.05 equiv.), K₃PO₄ (1.31 g, 6.17 mmol, 2.1 equiv.), 1,2,4-triazole (0.20 g, 2.94 mmol, 1.0 equiv.) and 1,10-phenanthroline (52 mg, 0.29 mmol, 0.1 equiv.). The tube was then evacuated and back-filled with argon. The evacuation/backfill sequence was repeated two additional times. Under a counterflow of argon, 3-iodoaniline (0.42 mL, 3.53 mmol, 1.2 equiv.) and degassed 1,4-dioxane (1.5 mL) were added by syringe. The tube was placed in a preheated oil bath at 110°C and the solution was stirred vigorously for 24 h. The reaction mixture was cooled to room temperature, diluted with EtOAc (2-3 mL), filtered through a plug of Celite and rinsed with EtOAc. The filtrate was concentrated under reduced pressure and the resulting residue was purified by silica gel column chromatography using a gradient solvent system of PE/EtOAc from 90/10 up to 70/30 to afford compound **25** (0.41 g, 87 %) as a brown solid. R*_{f}* (CH₂Cl₂/acetone 60/40) 0.49. Mp: 114 - 116°C. ¹H NMR (400 MHz, DMSO-*d₆*) δ 9.13 (s, 1H, CH), 8.17 (s, 1H, CH), 7.15 (t, *J* = 8.0 Hz, 1H, CH), 7.04 (t, *J* = 2.1 Hz, 1H, CH), 6.94 (ddd, *J* = 7.9 Hz, *J* = 2.1 Hz, *J* = 0.9 Hz, 1H, CH), 6.60 (ddd, *J* = 8.1 Hz, *J* = 2.2 Hz, *J* = 0.9 Hz, 1H, CH), 5.48 (br s, 2H, NH₂). HRMS (EI-MS) m/z calcd for C₈H₉N₄ [M+H]⁺: 161.0822, found: 161.0820.

### Example 20: N-(3-(1H-1,2,4-Triazol-1-yl)phenyl)-4-(5-methyl-7H-pyrrolo[2,3-d]pyrimidin-4-yl)-3,6-dihydropyridine-1(2H)-carboxamide (26)

The reaction was carried out as described in general procedure **H** using aniline **25** (40 mg, 0.25 mmol) and amine **4** (85 mg, 0.30 mmol, 1.2 equiv.) in THF (4 mL). The crude reaction mixture was purified by silica gel column chromatography using a gradient solvent system of CH₂Cl₂/acetone from 90/10 up to 40/60 to afford compound **26** (63 mg, 63 %) as a white solid. R*_{f}* (CH₂Cl₂/acetone 50/50) 0.21. Mp: 228 - 230°C. ¹H NMR (400 MHz, DMSO-*d₆*) δ 11.83 (br s, 1H, NH), 9.22 (s, 1H, CH), 8.90 (br s, 1H, NH), 8.65 (s, 1H, CH), 8.22 (s, 1H, CH), 8.13 - 8.06 (m, 1H, CH), 7.63 - 7.53 (m, 1H, CH), 7.45 - 7.39 (m, 2H, 2 x CH), 7.32 (s, 1H, CH), 6.18 - 5.99 (m, 1H, CH), 4.34 - 4.21 (m, 2H, CH₂), 3.77 (t, *J* = 5.6 Hz, 2H, CH₂), 2.77 - 2.64 (m, 2H, CH₂), 2.28 - 2.18 (m, 3H, CH₃). HRMS (EI-MS) m/z calcd for C₂₁H₂₁N₈O [M+H]⁺: 401.1833, found: 401.1832.

### 3-(3-Methoxyphenoxy)aniline (27)

An oven-dried microwave vial was charged with a magnetic stirring bar, Cul (29 mg, 0.15 mmol, 0.05 equiv.), 2-picolinic acid (123 mg, 0.30 mmol, 0.10 equiv.), 3-aminophenol (0.39 g, 3.60 mmol, 1.2 equiv.) and K₃PO₄ (1.27 g, 3.60 mmol, 2.0 equiv.). The tube was then evacuated and back-filled with argon. The evacuation/backfill sequence was repeated two additional times. Under a counterflow of argon, 3-iodoanisole (0.36 mL, 3.00 mmol, 1.0 equiv.) and DMSO (6 mL) were added by syringe. The tube was placed in a preheated oil bath at 80°C and the reaction mixture was stirred vigorously for 24 h. The reaction mixture was cooled to room temperature. EtOAc (30 mL) and H₂O (5 mL) were added and the mixture was stirred. The organic layer was separated and the aqueous layer was extracted twice with EtOAc (30 mL). The combined organic layers was dried over MgSO₄ and filtered through a pad of silica gel. The filtrate was concentrated and the resulting residue was purified by silica gel column chromatography using a gradient solvent system cyclohexane/EtOAc from 100/0 up to 90/10 to afford compound **27** (87 mg, 85 %) as a brown oil. ¹H NMR (250 MHz, CDCl₃) δ 7.23 (t, *J* = 8.2 Hz, 1H, CH), 7.11 (t, *J* = 8.0 Hz, 1H, CH), 6.70 - 6.60 (m, 3H, 3 x CH), 6.47 - 6.43 (m, 1H, CH), 6.41 (m, 1H, CH), 6.35 (m, 1H, CH), 3.78 (s, 3H, OCH₃), 3.69 (br s, 2H, NH₂). All spectral data corresponded to literature values as found in Maiti, D.; Buchwald, S. L. J. Am. Chem. Soc. 2009, 131, 17423-17429.

### Example 21: 4-(5-Methyl-7H-pyrrolo[2,3-d]pyrimidin-4-yl)-N-(4-phenoxyphenyl)-3,6-dihydropyridine-1(2H)-carboxamide (28)

The reaction was carried out as described in general procedure **H** using aniline **27** (54 mg, 0.25 mmol) and amine **4** (85 mg, 0.30 mmol, 1.2 equiv.) in THF (4 mL). The crude reaction mixture was purified by silica gel column chromatography using a gradient solvent system of CH₂Cl₂/acetone from 80/20 up to 20/80 to afford compound **28** (63 mg, 55 %) as a beige solid. R*_{f}* (CH₂Cl₂/acetone 60/40) 0.21. Mp: degradation 132°C. ¹H NMR (400 MHz, DMSO-*d₆*) δ 11.91 (br s, 1H, NH), 8.68 (br s, 1H, NH), 8.66 (s, 1H, CH), 7.36 - 7.21 (m, 5H, 5 x CH), 6.71 (dd, *J* = 8.3 Hz, *J =* 2.4 Hz, 1H, CH), 6.65 - 6.52 (m, 3H, 3xCH), 6.09 - 6.02 (m, 1H, CH), 4.24 - 4.16 (m, 2H, CH₂), 3.74 (s, 3H, OCH₃), 3.71 (t, *J* = 5.5 Hz, 2H, CH₂), 2.69 - 2.61 (m, 2H, CH₂), 2.22 (s, 3H, CH₃). HRMS (EI-MS) m/z calcd for C₂₆H₂₆N₅O₃ [M+H]⁺: 456.2030, found: 456.2031.

### Example 22: N-[3-(2-Methoxyphenyl)phenyl]-4-(5-methyl-7H-pyrrolo[2,3-d]pyrimidin-4-yl)-3,6-dihydro-2H-pyridine-1-carboxamide (29)

The reaction was carried out as described in general procedure **H** with 2'-methoxy-[1,1'-biphenyl]-3-amine (26 mg, 0.13 mmol) and amine **4** (47 mg, 0.16 mmol, 1.2 equiv.) in THF (4 mL). The crude mixture was purified by silica gel column chromatography using a gradient solvent system CH₂Cl₂/acetone from 100/0 up to 40/60 to afford **29** (43 mg, 72 %) as a white solid. R*_{f}* (CH₂Cl₂/acetone 50/50) 0.18. Mp: degradation 205°C. ¹H NMR (250 MHz, DMSO-*d₆*) δ 11.83 (s, 1H, NH), 8.65 (s, 1H, CH), 8.62 (s, 1H, NH), 7.60 (s 1H, CH), 7.49 (d, *J* = 8.0 Hz, 1H, CH), 7.40 - 7.20 (m, 4H, 4 x CH), 7.18 - 6.95 (m, 3H, 3 x CH), 6.05 (s, 1H, CH), 4.27 - 4.19 (m, 2H, CH₂), 3.86 - 3.63 (m, 5H, OCH₃ + CH₂), 2.68 (s, 2H, CH₂), 2.24 (s, 3H, CH₃). HRMS (EI-MS) m/z calcd for CH₆H₂₆N₅O₂[M+H]⁺: 440.2081, found: 440.2080.

### Example 23: N-[3-(3-Methoxyphenyl)phenyl]-4-(5-methyl-7H-pyrrolo[2,3-d]pyrimidin-4-yl)-3,6-dihydro-2H-pyridine-1-carboxamide (30)

The reaction was carried out as described in general procedure **H** with 3'-methoxy-[1,1'-biphenyl]-3-amine (26 mg, 0.13 mmol) and amine **4** (47 mg, 0.16 mmol, 1.2 equiv.) in THF (4 mL). The crude mixture was purified by silica gel column chromatography using a gradient solvent system CH₂Cl₂/acetone from 100/0 up to 40/60 to afford **30** (47 mg, 78%) as a white solid. R*_{f}* (CH₂Cl₂/acetone 50/50) 0.19. Mp: degradation 224°C. ¹H NMR (250 MHz, DMSO-*d₆*) δ 11.83 (s, 1H, NH), 8.68 (s, 1H, CH), 8.65 (s, 1H, NH), 7.81 (s, 1H, CH), 7.56 (d, *J* = 7.9 Hz, 1H, CH), 7.47-7.04 (m, 6H, 6 x CH), 6.94 (d, *J* = 8.1 Hz, 1H, CH), 6.07 (s, 1H, CH), 4.26 (s, 2H, CH₂), 3.82 (s, 3H, CH₃), 3.76 (t, *J=* 4.6 Hz, 2H, CH₂), 2.70 (s, 2H, CH₂), 2.25 (s, 3H, CH₃). HRMS (EI-MS) m/z calcd for C₂₆H₂₆N₅O₂[M+H]⁺: 440.2081, found: 440.2083.

### Example 24: N-[3-(4-Methoxyphenyl)phenyl]-4-(5-methyl-7H-1-pyrrolo[2,3-d]pyrimidin-4-yl)-3,6-dihydro-2H-pyridine-1-carboxamide (31)

The reaction was carried out as described in general procedure **H** with 4'-methoxy-[1,1'-biphenyl]-3-amine (26 mg, 0.13 mmol) and amine **4** (47 mg, 0.16 mmol, 1.2 equiv.) in THF (4 mL). The crude mixture was purified by silica gel column chromatography using a gradient solvent system of CH₂Cl₂/acetone from 100/0 up to 40/60 to afford **31** (43 mg, 72 %) as a white solid. R*_{f}* (CH₂Cl₂/acetone 50/50) 0.25. Mp: degradation 252°C. ¹H NMR (250 MHz, DMSO-*d*₆) δ 11.83 (s, 1H, NH), 8.65 (s, 2H, CH + NH), 7.77 (s, 1H, CH), 7.60 - 7.45 (m, 3H, 3xCH), 7.37 - 7.15 (m, 3H, 3xCH), 7.03 (d, *J* = 8.8 Hz, 2H, CH), 6.06 (s, 1H, CH), 4.25 (s, 2H, CH₂), 3.85 - 3.71 (m, 5H, OCH₃+ CH₂), 2.69 (s, 2H, CH₂), 2.25 (s, 3H, CH₃). HRMS (EI-MS) m/z calcd for C₂₆H₂₆N₅O₂[M+H]⁺: 440.2081, found: 440.2082.

### Example 25: 4-(5-methyl-7H-pyrrolo[2,3-d]pyrimidin-4-yl)-N-(3-pyridyl)-3,6-dihydro-2H-pyridine-1-carboxamide (32)

The reaction was carried out as described in general procedure **H** with 3-aminopyridine (75 mg, 0.80 mmol) and amine **4** (240 mg, 0.96 mmol, 1.2 eq.) in THF (12 mL). The crude reaction mixture was purified by silica gel column chromatography using a gradient solvent system of CH₂Cl₂/MeOH from 100/0 up to 95/5 to afford compound **32** (162 mg, 60%) as a beige solid. R*_{f}* (CH₂Cl₂/MeOH 90/10) 0.24. Mp: 170°C. ¹H NMR (400 MHz, MeOD-d₄) δ 8.72 (brs, 1H, CH), 8.66 (s, 1H, CH), 8.22 (brs, 1H, CH), 8.07 - 8.00 (m, 1H, CH), 7.44 (dd, *J* = 8.4, 4.9 Hz, 1H, CH), 7.28 (brs, 1H, CH), 6.09 (s, 1H, CH), 4.36 - 4.31 (m, 2H, CH₂), 3.87 (t, *J* = 5.6 Hz, 2H, CH₂), 2.78 - 2.71 (m, 2H, CH₂), 2.32 (s, 3H, CH₃). HRMS (EI-MS): m/z calcd for C₁₈H₁₉N₆O [M+H]⁺: 335.1615; found: 335.1617.

### Example 26: 4-(5-methyl-7H-pyrrolo[2,3-d]pyrimidin-4-yl)-N-(2-thienyl)-3,6-dihydro-2H-pyridine-1-carboxamide (33)

The reaction was carried out as described in general procedure **G** with amine **4** (550 mg, 2.19 mmol) in anhydrous CH₂Cl₂ (30 mL). The crude reaction mixture was purified by silica gel column chromatography using a gradient solvent system of CH₂Cl₂/acetone from 100/0 up to 50/50 to afford compound **33** (278 mg, 54%) as a beige solid. R*_{f}* (CH₂Cl₂/acetone 50/50) 0.57. Mp: 241-143°C. ¹H NMR (400 MHz, MeOD-d₄) δ 8.62 (s, 1H, CH), 7.28 (dd, *J* = 5.1, 3.2 Hz, 1H, CH), 7.25 - 7.21 (m, 2H, CH), 7.13 (dd, *J* = 5.1, 1.4 Hz, 1H, CH), 6.07 - 6.01 (m, 1H, CH), 4.27 (q, *J* = 2.9 Hz, 2H, CH₂), 3.83 (t, *J* = 5.6 Hz, 2H, CH₂), 2.76 - 2.68 (m, 2H, CH₂), 2.30 (d, *J* = 1.2 Hz, 3H, CH₃). HRMS (EI-MS): m/z calcd for C₁₇H₁₈N₅OS [M+H]⁺: 340.1227; found: 340.1227.

### Example 27: 4-(5-methyl-7H-pyrrolo[2,3-d]pyrimidin-4-yl)-N-tetralin-1-yl-3,6-dihydro-2H-pyridine-1-carboxamide (34-rac and 35-R)

The reaction was carried out as described in general procedure **H** with 1,2,3,4-tetrahydro-1-naphthylamine as a racemic mixture or with the pure (*R*) enantiomer (73 mg, 0.50 mmol) and amine **4** (150 mg, 0.60 mmol, 1.2 eq.) in THF (7 mL). The crude reaction mixture was purified by silica gel column chromatography using a gradient solvent system of CH₂Cl₂/MeOH from 100/0 up to 95/5 to afford compound **34-rac** (122 mg, 63%) or **35-R** (116 mg, 60%) as a beige solid in both cases. Trace amounts of DIPEA were removed by washing the solid with water. R*_{f}* (CH₂Cl₂/MeOH 95/5) 0.34. Mp: 170°C. ¹H NMR (400 MHz, MeOD-d₄) δ 8.61 (s, 1H, CH), 7.31 - 7.26 (m, 1H), 7.23 (d, *J* = 1.2 Hz, 1H), 7.16 - 7.03 (m, 3H), 6.00 (dt, *J* = 3.3, 1.7 Hz, 1H, CH), 5.06 (t, *J* = 6.4 Hz, 1H, CH), 4.19 (q, *J* = 3.0 Hz, 2H, CH₂), 3.81 - 3.69 (m, 2H, CH₂), 2.91 - 2.72 (m, 2H, CH₂), 2.70 - 2.62 (m, 2H, CH₂), 2.31 (d, *J* = 1.2 Hz, 3H, CH₃), 2.12 - 1.93 (m, 2H, CH₂), 1.91 - 1.76 (m, 2H, CH₂). HRMS (EI-MS): m/z calcd for C₂₃H₂₆N₅O [M+H]⁺: 388.2132; found: 388.2131.

### (4-methylsulfonylpiperazin-1-yl)-(3-nitrophenyl)methanone (170)

1-Methylsulfonyl-piperazine (350 µL, 4.04 mmol, 1.5 equiv.) and Et₃N (670 µL, 4.84 mmol, 1.8 equiv.) were suspended in DCM (15 mL). The solution was cooled to 0 °C and 3-nitrobenzoyl chloride (500 mg, 2.69 mmol, 1.0 equiv.) in 7.0 mL of DCM was added dropwise. The reaction was stirred at rt for 3 h, and then washed with a saturated NaHCO₃ solution (30 mL) and a 1M HCl solution (30 mL). The organic layer was dried over MgSO₄, filtered and concentrated under reduced pressure to give compound **170** (684 mg, 81%) as a brown amorphous solid which was used without further purification in the next step. R*_{f}* (EP/AcOEt 20/80) 0.41 ¹H NMR (400 MHz, CDCl₃) δ (ppm) 8.35 - 8.31 (m, 1H, CH), 8.30 - 8.27 (m, 1H, CH), 7.79 - 7.74 (m, 1H, CH), 7.66 (t, *J* = 7.9 Hz, 1H, CH), 4.09-3.44 (m, 4H, 2 x CH₂), 3.30 (brs, 4H, 2 x CH₂), 2.83 (s, 3H, CH₃). HRMS (EI-MS) m/z calcd for C₁₂H₁₆N₃O₅S [M+H]⁺: 314.0803, found: 314.0805.

### (3-aminophenyl)-(4-methylsulfonylpiperazin-1-yl)methanone (171)

Tin chloride (1.51 g, 7.95 mmol, 5.0 equiv.) was added to a solution of compound **170** in ethanol (15 mL). The reaction was heated to 80 °C for 6 h. The solvent was then concentrated under reduced pressure, water was added and the pH was corrected to approx. 12 with a solution of 2M NaOH. The resulting solid was filtered over diatomaceous earth and the recovered filtrate was extracted with EtOAc (3 x 30 mL). The combined organic layers were dried over MgSO₄, filtered and concentrated under reduced pressure to give compound **171** (303 mg, 67%) as a brown oil which was used without further purification in the next step. R*_{f}* (CH₂Cl₂/acetone 50/50) 0.27. ¹H NMR (400 MHz, CDCl₃) δ (ppm) 7.18 (t, *J* = 7.7 Hz, 1H, CH), 6.76 - 6.68 (m, 3H, 3 x CH), 3.94 - 3.49 (m, 6H, NH₂ + 2 x CH₂), 3.23 (brs, 4H, 2 x CH₂), 2.80 (s, 3H, CH₃). HRMS (EI-MS) m/z calcd for C₁₂H₁₈N₃O₅S [M+H]⁺: 284.1066, found: 284.1063.

### Example 27-1: 4-(5-methyl-7H-pyrrolo[2,3-d]pyrimidin-4-yl)-N-[3-(4-methylsulfonylpiperazine-1-carbonyl)phenyl]-3,6-dihydro-2H-pyridine-1-carboxamide (172)

The reaction was carried out as described in general procedure **H** with aniline **171** (150 mg, 0.53 mmol), 4-nitrophenyl chloroformate (128 mg, 0.64 mmol, 1.2 equiv.), DIPEA (190 µL, 1.11 mmol, 2.1 equiv.), and amine **4** (159 mg, 0.64 mmol, 1.2 equiv.) in dry THF (7.6 mL). The crude reaction mixture was purified by silica gel column chromatography using a gradient solvent system of DCM/acetone from 100/0 up to 20/80 to afford compound **172** (143 mg, 52 %) as a beige solid. R*_{f}* (CH₂Cl₂/acetone 30/70) 0.29 Mp: 140-142°C. ¹H NMR (400 MHz, MeOD-d₄) 8.63 (s, 1H, CH), 7.59 (t, *J=* 1.9 Hz, 1H, CH), 7.51 (ddd, *J* = 8.2, 2.3, 1.1 Hz, 1H, CH), 7.40 (t, *J=* 7.9 Hz, 1H, CH), 7.24 (d, *J* = 1.3 Hz, 1H, CH), 7.11 (dt, *J* = 7.5, 1.3 Hz, 1H, CH), 6.08 - 6.03 (m, 1H, CH), 4.31 (q, *J* = 2.8 Hz, 2H, CH₂), 3.86 (t, *J* = 5.6 Hz, 2H, CH₂), 3.95 - 3.54 (m, 4H, 2 x CH₂), 3.37 - 3.20 (m, 4H, 2 x CH₂), 2.88 (s, 3H, CH₃), 2.78 - 2.71 (m, 2H, CH₂), 2.31 (d, *J* = 1.2 Hz, 3H, CH₃). HRMS (EI-MS) m/z calcd for C₂₅H₃₀N₇O₄S [M+H]⁺: 524.2068, found: 524.2075.

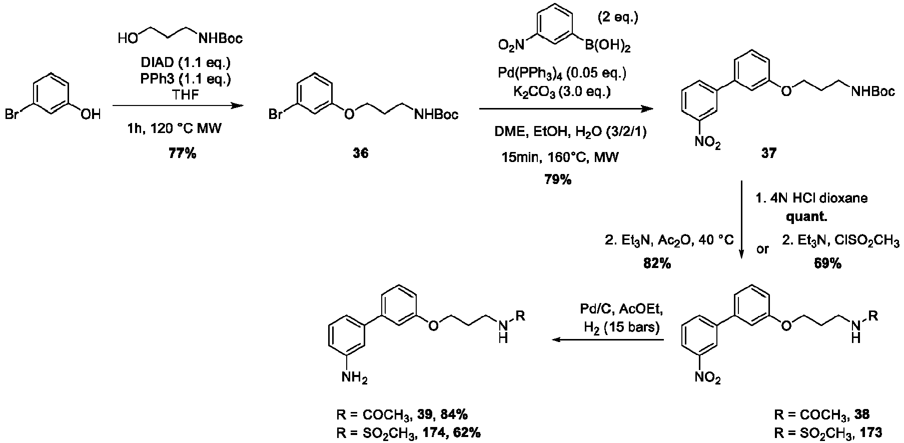

### tert-Butyl N-[3-(3-bromophenoxy)propyl]carbamate (36)

*tert*-Butyl *N*-(3-hydroxypropyl)carbamate (912 mg, 5.20 mmol, 1.5 equiv.) and triphenylphosphine (1.0 g, 3.81 mmol, 1.1 equiv.). was added to a solution of 3-bromophenol (600 mg, 3.47 mmol) in THF (12 mL) in a microwave tube. A solution of DIAD (768 mg, 3.81 mmol, 1.1 equiv.) in THF (5 mL) was added dropwise and the tube was sealed and the mixture heated in the microwave at 120°C for 1 h. After cooling, an additional amount of triphenylphosphine and DIAD were added (1 equiv.) and the tube heated at 120°C for 30 min. The mixture was concentrated, and the residue taken up in EtOAc, washed with a saturated solution of NaCl (30 mL), dried with MgSO₄ and concentrated under reduced pressure. The crude reaction mixture was purified by silica gel column chromatography using a gradient solvent system of EP/Et₂O from 100/0 up to 50/50 to afford compound **36** (874 mg, 77%) as a white solid. R*_{f}*(EP/Et₂O 50/50) 0.51. Mp: 77 - 79°C. ¹H NMR (400 MHz, CDCl₃) δ 7.18 - 7.01 (m, 3H, 3 x CH), 6.85 - 6.79 (m, 1H, CH), 4.72 (s, 1H, NH), 4.00 (t, *J* = 6.2 Hz, 2H, CH₂), 3.31 (q, *J* = 6.3 Hz, 2H, CH₂), 1.97 (p, *J* = 6.2 Hz, 2H, CH₂), 1.44 (s, 9H, 3 x CH₃). HRMS (EI-MS) m/z calcd for C₁₄H₂₁BrNO₃ [M+H]⁺: 330.0699, found: 330.0695.

### tert-Butyl N-[3-[3-(3-nitrophenyl)phenoxy]propyl]carbamate (37)

Compound **36** (300 mg, 0.91 mmol), 3-phenylboronic acid (304 mg, 1.82 mmol, 2.0 equiv.) and K₂CO₃ (377 mg, 2.73 mmol, 3.0 equiv.) were suspended in a mixture of DME/EtOH/H₂O (1.8 mL, 1.2 mL and 0.6 mL). The solution was degassed for 20 minutes under Ar and Pd(PPh₃)₄ (53 mg, 0.045 mmol, 0.05 equiv.) was added. The reaction was heated in the microwave at 160°C for 15 min. After cooling, the mixture was extracted with CH₂Cl₂ (3 x 10 mL). The combined organic layers were dried (MgSO₄), filtered and evaporated under reduced pressure. The crude reaction mixture was purified by silica gel column chromatography using a gradient solvent system of EP/Et₂O from 100/0 up to 70/30 to afford compound **37** (267 mg, 79 %) as a yellow solid. R*_{f}*(EP/Et₂O 80/20) 0.26. Mp: 93 - 95°C. ¹H NMR (400 MHz, MeOD-d₄) δ 8.41 (t, *J* = 1.9 Hz, 1H, CH), 8.20 (ddd, *J* = 8.2, 2.2, 0.8 Hz, 1H, CH), 8.03 - 7.98 (m, 1H, CH), 7.67 (t, *J* = 8.0 Hz, 1H, CH), 7.39 (t, *J* = 7.9 Hz, 1H, CH), 7.24 (d, *J* = 7.7 Hz, 1H, CH), 7.22 - 7.19 (m, 1H, CH), 6.99 (dd, *J* = 8.2, 1.8 Hz, 1H, CH), 4.09 (t, *J* = 6.2 Hz, 2H, CH₂), 3.26 (t, *J* = 6.8 Hz, 2H, CH₂), 1.97 (p, *J* = 6.5 Hz, 2H, CH₂), 1.42 (s, 9H, 3 x CH₃). HRMS (EI-MS) m/z calcd for C₂₀H₂₄NaN₂O₅ [M+Na]⁺: 395.1577, found: 395.1575.

### N-[3-[3-(3-Nitrophenyl)phenoxy]propyl]acetamide (38)

A 4N HCl solution in dioxane (1.10 mL, 4.3 mmol, 8.0 equiv.) was added to the Boc protected amine **37** (200 mg, 0.54 mmol) in CH₂Cl₂ (2 mL) at 0°C under argon. The reaction was stirred for 2 hr at rt or until finished (TLC). The solvent was then evaporated, and the residue was taken up in CH₂Cl₂ (5 mL) and a saturated solution of K₂CO₃ was added until pH 8-9. The mixture was stirred for 30 min and extracted with CH₂Cl₂ (2 x 5 mL). The combined organic layers were dried over MgSO₄, filtered and concentrated under reduced pressure to afford the desired product which was used without further purification in the next step.

The free amine was then placed in round bottom flask under Ar, diluted with anhydrous CH₂Cl₂ (2 mL), and cooled to 0°C. Triethylamine (38 µL, 0.28 mmol, 1.5 equiv.) was added followed by acetic anhydride (0.26 ml, 2.69 mmol, 5 equiv.) and the mixture was heated at 40°C for 2 h. The reaction was then quenched with Na₂CO₃ and the aqueous layer was extracted with AcOEt (3 x 10 mL). The combined organic layers were washed with brine, dried over MgSO₄, and the solvent removed under reduced pressure. The crude reaction mixture was purified by silica gel column chromatography using a gradient solvent system of CH₂Cl₂/MeOH from 100/0 up to 95/5 to afford compound **38** (139 mg, 82%) as a yellow oil. R*_{f}* (CH₂Cl₂/MeOH 95/5) 0.48. ¹H NMR (400 MHz, MeOD-d₄) δ 8.37 (t, *J* = 2.0 Hz, 1H, CH), 8.21 - 8.13 (m, 1H, CH), 8.00 - 7.94 (m, 1H, CH), 7.64 (t, *J* = 8.0 Hz, 1H, CH), 7.37 (t, *J* = 7.9 Hz, 1H, CH), 7.24 - 7.15 (m, 1H, CH), 7.17 (t, *J* = 2.1 Hz, 1H, CH), 6.97 (dd, *J* = 8.3, 2.4 Hz, 1H, CH), 4.08 (t, *J* = 6.1 Hz, 2H, CH₂), 3.37 (t, *J* = 6.9 Hz, 2H, CH₂), 1.99 (p, *J* = 6.6 Hz, 2H, CH₂), 1.94 (s, 3H, CH₃). HRMS (EI-MS): m/z calcd for C₁₇H₁₉N₂O₄ [M+H]⁺: 315.1339; found 315.1334.

### N-(3-((3'-Amino-[1,1'-biphenyl]-3-yl)oxy)propyl)acetamide (39)

Pd/C (10% wt) (5 mg, 0.04 mmol, 0.1 equiv.) was added to a solution of the nitro compound **38** (133 mg, 0.42 mmol) in AcOEt (12 mL) in a small hydrogenation reactor. The reaction was stirred under an H₂ pressure of 15 bar until completion. The mixture was then filtered through a pad of celite which was rinsed several times with MeOH. The solvent was evaporated under reduced pressure to give the desired amine **39** (101 mg, 84 %) as a yellow oil which was used in the next step without further purification. R*_{f}* (CH₂Cl₂/MeOH 90/10) 0.19. ¹H NMR (400 MHz, MeOD-d₄) δ 7.27 (t, *J* = 7.9 Hz, 1H, CH), 7.18 - 7.07 (m, 3H, CH), 6.96 (t, *J* = 2.0 Hz, 1H, CH), 6.93 - 6.88 (m, 1H, CH), 6.88 - 6.83 (m, 1H, CH), 6.72 - 6.67 (m, 1H, CH), 4.03 (t, J = 6.1 Hz, 2H, CH₂-4), 3.35 (t, J = 6.9 Hz, 2H, CH₂-2), 2.00 - 1.94 (m, 2H, CH₂-3), 1.93 (s, 3H CH₃). HRMS (EI-MS): m/z calcd for C₁₇H₂₁N₂O₂ [M+H]⁺: 285.1598; found 285.1595.

### IV-[3-[3-(3-nitrophenyl)phenoxy]propyl]methanesulfonamide (173)

A 4N HCl solution in dioxane (1.10 mL, 4.3 mmol, 8.0 equiv.) was added to the Boc protected amine **37** (200 mg, 0.54 mmol) in CH₂Cl₂ (2 mL) at 0 °C under argon. The reaction was stirred for 2 hr at rt or until finished (tlc). The solvent was then evaporated, and the residue was taken up in CH₂Cl₂ (5 mL) and a saturated solution of K₂CO₃ was added until pH 8-9. The mixture was stirred for 30 min and extracted with CH₂Cl₂ (2 x 5 mL). The combined organic layers were dried over MgSO₄, filtered and concentrated under reduced pressure to afford the desired product which was used without further purification in the next step.

The free amine was then placed in round bottom flask under Ar and diluted with anhydrous CH₂Cl₂ (2 mL). Triethylamine (38 µL, 0.28 mmol, 1.5 equiv.) was added followed by methanesulfonyl chloride (47 µL, 0,61 mmol, 1,1 equiv.) and the reaction was stirred at rt overnight. The reaction was then quenched with 0.1 M HCl (15 mL) and the aqueous layer was extracted with AcOEt (3 x 10 mL). The combined organic layers were washed with brine, dried over MgSO₄, and the solvent removed under reduced pressure. The crude reaction mixture was purified by silica gel column chromatography using a gradient solvent system of CH₂Cl₂/MeOH from 100/0 up to 98/2 to afford compound **173** (132 mg, 69%) as a yellow oil. R*_{f}* (CH₂Cl₂/MeOH 90/10) 0.75. ¹H NMR (400 MHz, MeOD-d₄) δ 8.30 (t, *J* = 2.0 Hz, 1H, CH), 8.11 (ddd, *J* = 8.1, 2.2, 1.0 Hz, 1H, CH), 7.91 (ddd, *J* = 7.8, 1.7, 0.9 Hz, 1H, CH), 7.59 (t, *J* = 8.0 Hz, 1H, CH), 7.33 (t, *J* = 7.9 Hz, 1H, CH), 7.17 - 7.10 (m, 2H, 2 x CH), 6.98 - 6.91 (m, 1H, CH), 4.10 (t, *J* = 6.1 Hz, 2H, CH₂), 3.28 (t, *J* = 6.8 Hz, 2H, CH₂), 2.94 (s, 3H, CH₃), 2.02 (p, *J=* 6.5 Hz, 2H, CH₂). HRMS (EI-MS) m/z calcd for C₁₆H₁₉N₂O₅S [M+H]⁺: 351.1009, found: 351.1007.

### N-[3-[3-(3-aminophenyl)phenoxy]propyl]methanesulfonamide (174)

Hydrazine monohydrate (14 µL, 0.29 mmol, 0.2 equiv.) and Fe(acac)₃ (1.3 mg, 0.25 % mol) were added to a solution of compound **173** (512 mg, 1.46 mmol) in methanol (1.5 mL) in a sealed tube. The reaction was then heated to 130 °C for 15 min. After cooling, the crude product was filter over diatomaceous earth, and the filtrate evaporated under reduced pressure to give the amine **174** (289 mg, 62%) as a brown oil which was used without further purification in the next step. R*_{f}* (CH₂Cl₂/MeOH 80/20) 0.49. ¹H NMR (400 MHz, MeOD-d₄) δ (ppm) 7.29 (t, *J* = 7.9 Hz, 1H, CH), 7.19 - 7.09 (m, 3H, CH), 6.99 - 6.96 (m, 1H, CH), 6.94 - 6.86 (m, 2H, CH), 6.74 - 6.69 (m, 1H, CH), 4.12 (t, *J* = 6.0 Hz, 2H, CH₂), 3.28 (t, *J* = 6.8 Hz, 2H, CH₂), 2.93 (s, 3H, CH₃), 2.03 (p, *J=* 6.5 Hz, 2H, CH₂). HRMS (EI-MS) m/z calcd for C₁₆H₂₁N₂O₅S [M+H]⁺: 321.1269, found: 321.1267.

### Example 28: N-(3'-(3-Acetamidopropoxy)-[1,1'-biphenyl]-3-yl)-4-(5-methyl-7H-pyrrolo[2,3-d]pyrimidin-4-yl)-5,6-dihydropyridine-1(2H)-carboxamide (40)

The reaction was carried out as described in general procedure **H** with aniline **39** (97 mg, 0.34 mmol) and amine **4** (94 mg, 0.38 mmol, 1.1 eq.) in THF (5 mL). The crude reaction mixture was purified by silica gel column chromatography using a gradient solvent system of CH₂Cl₂/MeOH from 100/0 up to 90/10 to afford compound 40 (74 mg, 40 %) as a beige solid. R*_{f}* (CH₂Cl₂/MeOH 90/10) 0.18. ¹H NMR (400 MHz, MeOD-d₄) 8.64 (s, 1H, CH), 7.71 (brs, 1H, CH), 7.46 - 7.23 (m, 5H, 5 x CH), 7.22 - 7.14 (m, 2H, 2 x CH), 6.93 - 6.86 (m, 1H, CH), 6.07 (brs, 1H, CH), 4.32 (d, *J* = 2.0 Hz, 2H, CH₂), 4.08 (t, J = 6.2 Hz, 2H, CH₂), 3.86 (t, J = 5.6 Hz, 2H, CH₂), 3.37 (t, J = 6.9 Hz, 2H, CH₂), 2.74 (brs, 2H, CH₂), 2.31 (s, 3H, CH₃), 2.03 - 1.96 (m, 2H, CH₂), 1.94 (s, 3H, CH₃). HRMS (EI-MS): m/z calcd for C₃₀H₃₃N₆O₃ [M+H]⁺: 525.2609; found: 525.2611.

### Example 28-1: N-[3-[3-[3-(methanesulfonamido)propoxy]phenyl]phenyl]-4-(5-methyl-7H-pyrrolo[2,3-d]pyrimidin-4-yl)-3,6-dihydro-2H-pyridine-1-carboxamide (175)

The reaction was carried out as described in general procedure **H** with aniline **174** (273 mg, 0.85 mmol), 4-nitrophenyl chloroformate (205 mg, 1.02 mmol, 1.2 equiv.), DIPEA (310 µL, 1.79 mmol, 2.1 equiv.), and amine **4** (255 mg, 1.02 mmol, 1.2 equiv.) in dry THF (12.0 mL). The crude reaction mixture was purified by silica gel column chromatography using a gradient solvent system of DCM/acetone from 100/0 up to 20/80 to afford compound **174** (283 mg, 59 %) as a beige solid. R*_{f}* (CH₂Cl₂/acetone 50/50) 0.24. Mp: 114-116°C. ¹H NMR (400 MHz, MeOD-d₄) δ (ppm) 8.63 (s, 1H, CH), 7.89 (s, 0.65H, NH), 7.71 (s, 1H, CH), 7.46 - 7.15 (m, 7H, 7 x CH), 6.97 - 6.88 (m, 1H, CH), 6.05 (brs, 1H, CH), 4.35 - 4.29 (m, 2H, CH₂), 4.14 (t, *J* = 6.1 Hz, 2H, CH₂), 3.87 (t, *J* = 5.6 Hz, 2H, CH₂), 3.28 (t, *J=* 6.9 Hz, 2H, CH₂), 2.93 (s, 3H, CH₃), 2.77 - 2.72 (m, 2H, CH₂), 2.31 (s, 3H, CH₃), 2.04 (p, *J* = 6.5 Hz, 2H, CH₂). HRMS (EI-MS) m/z calcd for C₂₉H₃₃N₆O₄S [M+H]⁺: 561.2286, found: 561.2279.

### 3-[4-(Dimethylamino)phenoxy]aniline (41)

An oven-dried screw cap test tube was charged with a magnetic stir bar, copper(I) iodide (28 mg, 0.15 mmol, 0.1 equiv.), 2-picolinic acid (36 mg, 0.30 mmol, 0.2 equiv.), 4-bromo-*N*,*N*-dimethyl aniline (300 mg, 1.5 mmol), 3-aminophenol (196 mg, 1.80 mmol, 1.2 equiv.) and K₃PO₄ (636 mg, 3.00 mmol, 2 equiv.). The tube was then evacuated and back-filled with argon. The evacuation/back-fill sequence was repeated two additional times. Under a counterflow of argon, dimethyl sulfoxide (5 mL) was added by syringe. The tube was placed in a preheated plate at 90°C and the reaction mixture was stirred vigorously for 24 h. The reaction mixture was then cooled to room temperature. Ethyl acetate and water were added and the mixture was stirred. The organic layer was separated and the aqueous layer was extracted with ethyl acetate (2 x 25 mL). The combined organic layers were dried over MgSO₄ and filtered, and the mixture was concentrated under reduced pressure. The crude reaction mixture was purified by silica gel column chromatography using a gradient solvent system of EP/AcOEt (100/0 to 80/20). The product was then precipitated with pentane to give aniline **41** (0.181 g, 53%) as an amorphous beige solid. ¹H NMR (250 MHz, CDCl₃) δ 7.07 (t, *J* = 8.0 Hz, 1H, CH), 7.02-6.95 (m, 2H, 2 x CH), 6.80-6.72 (m, 2H, 2 x CH), 6.36 (dd, *J* = 8.1, 2.3 Hz, 2H, 2 x CH), 6.27 (t, *J* = 2.0 Hz, 1H, CH) 3.66 (brs, 2H, NH₂), 2.96 (s, 6H, 2 x CH₃)₂). HRMS (EI-MS): m/z calcd for C₁₄H₁₇N₂O [M+H]⁺ 229.1335; found: 229.1338.

### Example 29: N-[3-[4-(Dimethylamino)phenoxy]phenyl]-4-(5-methyl-7H-pyrrolo[2,3-d]pyrimidin-4-yl)-3,6-dihydro-2H-pyridine-1-carboxamide (42)

The reaction was carried out as described in general procedure **H** with the prepared aniline derivative **41** (66 mg, 0.29 mmol) and amine **4** (100 mg, 0.35 mmol, 1.2 equiv.) in THF (4 mL). The crude mixture was purified by silica gel column chromatography using a gradient solvent system of CH₂Cl₂/acetone from 100/0 up to 50/50. The product was then precipitated with pentane to afford **42** (57 mg, 42 %) as an amorphous beige solid. ¹H NMR (250 MHz, DMSO) δ 11.82 (s, 1H, NH), 8.64 (s, 1H, CH), 8.60 (s, 1H, NH), 7.31 (s, 1H, CH), 7.26 - 7.08 (m, 3H, 3 x CH), 7.00 - 6.85 (m, 2H, 2 x CH), 6.82 - 6.69 (m, 2H, 2 x CH), 6.57 - 6.44 (m, 1H, CH), 6.02 (s, 1H, CH), 4.18 (s, 2H, CH₂), 3.69 (s, 2H, CH₂), 2.87 (s, 6H, 2 x CH₃), 2.65 (s, 2H, CH₂), 2.22 (s, 3H, CH₃). HRMS (EI-MS): m/z calcd for C₂₇H₂₈N₆O₂ [M+H]⁺: 469.2347; found: 469.2341.

### 3-(3-aminophenoxy)-N,N-dimethylaniline (176)

An oven dried microwave reaction vial was loaded with 3-aminophenol (196 mg, 1.80 mmol, 1.2 equiv.), Cul (29 mg, 0.15 mmol, 10 mol%.), 2-picolinic acid (37 mg, 0.30 mmol, 20 mol%) and K₃PO₄ (637 mg, 3.00 mmol, 2.0 equiv.). It was then evacuated and back-filled with argon 3 times then 3-bromo-*N*,*N*-dimethylaniline (300 mg, 1.50 mmol, 1.0 equiv.) and dry DMSO (3.0 mL) were added under argon and the reaction mixture was stirred at 90°C in a preheated plate for 24 h. The reaction mixture was diluted with H₂O (25 mL) and EtOAc (25 mL), the aqueous layer was separated and extracted with EtOAc (3 x 20 mL) then the combined organic layers were washed with brine (3 x 15 mL), dried over MgSO₄, filtered and concentrated under reduced pressure. The crude reaction mixture was purified by silica gel column chromatography using a gradient solvent system of PE/EtOAc from 100 up to 80/20 to afford compound **176** (166 mg, 49%) as a brown oil. R*_{f}* (PE/EtOAc 90/10) 0.19. ¹H NMR (250 MHz, CDCl₃) δ 7.17 (t, *J* = 8.1 Hz, 1H, CH), 7.08 (t, *J* = 8.0 Hz, 1H, CH) 6.48 (ddd, *J* = 8.2, 2.5, 0.8 Hz, 1H, CH), 6.45 - 6.30 (m, 5H, 5 x CH), 3.66 (s, 2H, NH₂), 2.93 (s, 6H, 2 x CH₃). HRMS (EI-MS) m/z calcd for C₁₄H₁₇N₂O [M+H]⁺: 229.1335, found: 229.1338.

### Example 94: N-(3-(3-(dimethylamino)phenoxy)phenyl)-4-(5-methyl-7H-pyrrolo[2,3-d]pyrimidin-4-yl)-3,6-dihydropyridine-1(2H)-carboxamide (177)

The reaction was carried out as described in general procedure **H** with aniline **176** (78 mg, 0.34 mmol, 1.0 equiv.), 4-nitrophenyl chloroformate (83 mg, 0.41 mmol, 1.2 equiv.), DIPEA (143 µL, 0.82 mmol, 2.4 equiv.), and amine **4** (103 mg, 0.41 mmol, 1.2 equiv.) in dry THF (5 mL). The crude reaction mixture was purified by silica gel column chromatography using a gradient solvent system of DCM/MeOH from 100 up to 95/5 to afford compound **177** (124 mg, 78%) as a brown solid. R*_{f}* (DCM/MeOH 95/5) 0.38. Mp: 114-116 °C. ¹H NMR (250 MHz, DMSO-d₆) δ 11.82 (s, 1H, NH₇), 8.64 (s, 2H, NH + CH-2), 7.35 - 7.10 (m, 5H, CH-6 + 4 x CH), 6.58 (ddd, *J* = 7.6, 2.4, 1.4 Hz, 1H, CH), 6.49 (ddd, *J* = 8.5, 2.5, 0.8 Hz, 1H, CH), 6.37 (t, *J* = 2.3 Hz, 1H, CH), 6.24 (ddd, *J* = 8.0, 2.2, 0.8 Hz, 1H, CH), 6.03 (br s, 1H, CH), 4.19 (d, *J* = 3.0 Hz, 2H, CH₂), 3.71 (t, *J* = 5.5 Hz, 2H, CH₂), 2.88 (s, 6H, 2 x CH₃), 2.66 (brs, 2H, CH₂), 2.22 (d, *J* = 1.1 Hz, 3H, CH₃). HRMS (EI-MS) m/z calcd for C₂₇H₂₉N₆O₂ [M+H]⁺: 469.2347, found: 469.2348.

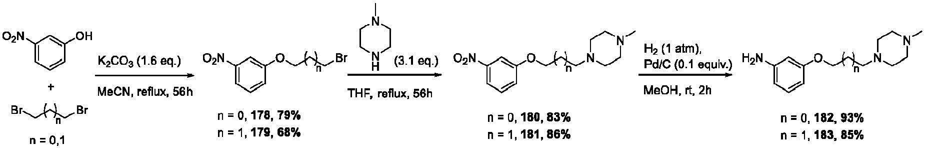

### 1-(2-bromoethoxy)-3-nitrobenzene (178)

The reaction was carried out as described in general procedure **M** using 3-nitrophenol (500 mg, 3.59 mmol, 1.0 equiv.), 1,2-dibromoethane (2.47 mL, 28.70 mmol, 8.0 equiv.), and K₂CO₃ (794 mg, 5.74 mmol, 1.6 equiv.) in dry MeCN (4.5 mL) for 9 h. The crude residue was purified by silica gel column chromatography using a gradient solvent system of PE/EtOAc from 100 up to 95/5 to afford compound **178** (698 mg, 79%) as a yellow solid. R*_{f}* (PE/EtOAc 90/10) 0.50. ¹H NMR (250 MHz, DMSO-d₆) δ 7.84 (ddd, *J* = 8.1, 2.2, 1.0 Hz, 1H, CH), 7.74 (t, *J* = 2.3 Hz, 1H, CH), 7.60 (t, *J* = 8.2 Hz, 1H, CH), 7.45 (ddd, *J* = 8.3, 2.5, 1.0 Hz, 1H, CH), 4.58 - 4.37 (m, 2H, CH₂), 3.92 - 3.77 (m, 2H, CH₂). All spectra data corresponded to the literature values as found in Kawamoto M. et al., Chem. Commun., 2010, 46, 8344-8346.

### 1-methyl-4-(2-(3-nitrophenoxy)ethyl)piperazine (180)

The reaction was carried out as described in general procedure **O** using alkyl halide **178** (406 mg, 1.65 mmol, 1.0 equiv.) and 1-methylpiperazine (568 µL, 5.12 mmol, 3.1 equiv.) in dry THF (9 mL) for 24h to afford compound **180** (362 mg, 83%) as a beige solid. ¹H NMR (250 MHz, CDCl₃) δ 7.81 (ddd, *J* = 8.1, 2.1, 1.0 Hz, 1H, CH), 7.74 (t, *J* = 2.3 Hz, 1H, CH), 7.41 (t, *J* = 8.2 Hz, 1H, CH), 7.22 (ddd, *J* = 8.3, 2.5, 1.0 Hz, 1H, CH), 4.17 (t, *J =* 5.7 Hz, 2H, CH₂), 2.84 (t, *J* = 5.7 Hz, 2H, CH₂), 2.62 (brs, 4H, 2 x CH₂), 2.47 (brs, 4H, 2 x CH₂), 2.29 (s, 3H, CH₃). All spectra data correspond to the literature values as found in Lee S. et al., J. Med Chem., 2020, 63, 3908-3914.

### 3-(2-(4-methylpiperazin-1-yl)ethoxy)aniline (182)

The reaction was carried out as described in general procedure **N** using nitro **180** (315 mg, 1.19 mmol, 1.0 equiv.) and Pd/C 10 wt.% (128 mg, 0.12 mmol, 0.1 equiv.) in MeOH (7 mL) for 2 h to afford compound **182** (261 mg, 93%) as a yellow oil. This product was used without further purification in the next step. ¹H NMR (250 MHz, DMSO-d₆) δ 6.87 (t, *J* = 8.3 Hz, 1H, CH), 6.28 - 6.00 (m, 3H, 3 x CH), 5.00 (brs, 2H, NH₂), 3.94 (t, *J=* 5.9 Hz, 2H, CH₂), 2.63 (t, *J=* 5.9 Hz, 2H, CH₂), 2.46 (brs, 4H, 2 x CH₂), 2.31 (brs, 4H, 2 x CH₂), 2.14 (s, 3H, CH₃). HRMS (EI-MS) m/z calcd for C₁₃H₂₂N₃O [M+H]⁺: 236.1757, found: 236.1760.

### Example 95: 4-(5-methyl-7H-pyrrolo[2,3-d]pyrimidin-4-yl)-N-(3-(2-(4-methylpiperazin-1-yl)ethoxy)phenyl)-3,6-dihydropyridine-1(2H)-carboxamide (184)

The reaction was carried out as described in general procedure **H** with aniline **182** (81 mg, 0.34 mmol, 1.0 equiv.), 4-nitrophenyl chloroformate (83 mg, 0.41 mmol, 1.2 equiv.), DIPEA (144 µL, 0.83 mmol, 2.4 equiv.), and amine **4** (104 mg, 0.41 mmol, 1.2 equiv.) in dry THF (5 mL). The crude reaction mixture was purified by silica gel column chromatography using a gradient solvent system of DCM/MeOH/NH₄OH from 100 up to 80/20/1 following by a preparative TLC using the solvent system DCM/MeOH/NH₄OH 80/20/1 to afford compound **184** (24 mg, 15%) as a pale orange solid.

R*_{f}* (DCM/MeOH/NH₄OH 90/10/1) 0.34. Mp: degradation 111 °C. ¹H NMR (250 MHz, DMSO-d₆) δ 11.83 (s, 1H, NH), 8.64 (s, 1H, NH), 8.54 (s, 1H, CH), 7.33 - 7.30 (m, 1H, CH), 7.25 - 7.01 (m, 3H, 3 x CH), 6.52 (dt, *J* = 7.4, 2.3 Hz, 1H, CH), 6.08 - 6.02 (m, 1H, CH), 4.21 (d, *J=* 2.8 Hz, 2H, CH₂), 4.02 (t, *J* = 5.8 Hz, 2H, CH₂), 3.72 (t, *J* = 5.6 Hz, 2H, CH₂), 2.68 (t, *J* = 5.6 Hz, 4H, 2 x CH₂), 2.52-2.49 (m, 4H, 2 x CH₂), 2.37 (brs, 4H, 2 x CH₂), 2.24 (d, *J* = 1.1 Hz, 3H, CH₃), 2.18 (s, 3H, CH₃). HRMS (EI-MS) m/z calcd for C₂₆H₃₄N₇O₂ [M+H]⁺: 476.2768, found: 476.2768.

### 1-(3-bromopropoxy)-3-nitrobenzene (179)

The reaction was carried out as described in general procedure **M** using 3-nitrophenol (500 mg, 3.59 mmol, 1.0 equiv.), 1,3-dibromopropane (2.91 mL, 28.70 mmol, 8.0 equiv.), and K₂CO₃ (794 mg, 5.74 mmol, 1.6 equiv.) in dry MeCN (4.5 mL) for 56 h. The crude reaction mixture was purified by silica gel column chromatography using PE/DCM 70/30 to afford compound **179** (634 mg, 68%) as a yellow oil. R*_{f}* (PE/EtOAc 90/10) 0.45. ¹H NMR (250 MHz, CDCl₃) δ 7.84 (ddd, *J* = 8.1, 2.2, 1.0 Hz, 1H, CH), 7.74 (t, *J* = 2.3 Hz, 1H, CH), 7.44 (t, *J* = 8.2 Hz, 1H, CH), 7.23 (ddd, *J* = 8.3, 2.6, 1.0 Hz, 1H, CH), 4.20 (t, *J=* 5.8 Hz, 2H, CH₂), 3.62 (t, *J=* 6.3 Hz, 2H, CH₂), 2.36 (p, *J* = 6.1 Hz, 2H, CH₂). HRMS (EI-MS) m/z calcd for C₉H₁₁BrNO₃ [M+H]⁺: 259.9917, found: 259.9915.

### 1-methyl-4-(3-(3-nitrophenoxy)propyl)piperazine (181)

The reaction was carried out as described in general procedure **O** using the alkyl halide **179** (400 mg, 1.54 mmol, 1.0 equiv.), 1-methylpiperazine (530 µL, 4.77 mmol, 3.1 equiv.) in dry THF (9 mL) for 56 h to afford compound **181** (369 mg, 86%) as a yellow oil. ¹H NMR (250 MHz, CDCl₃) δ 7.80 (ddd, *J* = 8.1, 2.1, 1.0 Hz, 1H, CH), 7.72 (t, *J* = 2.3 Hz, 1H, CH), 7.40 (t, *J* = 8.2 Hz, 1H, CH), 7.21 (ddd, *J* = 8.3, 2.5, 1.0 Hz, 1H, CH), 4.09 (t, *J* = 6.3 Hz, 2H, CH₂), 2.60 - 2.38 (m, 10H, 5 x CH₂), 2.29 (s, 3H, CH₃), 2.01 (p, 2H, CH₂). HRMS (EI-MS) m/z calcd for C₁₄H₂₂N₃O₃ [M+H]⁺: 280.1656, found: 280.1655.

### 3-(3-(4-methylpiperazin-1-yl)propoxy)aniline (183)

The reaction was carried out as described in general procedure **N** using the nitro compound **181** (276 mg, 0.99 mmol, 1.0 equiv.) and Pd/C 10 wt.% (105 mg, 0.01 mmol, 0.1 equiv.) in MeOH (5.5 mL) for 2 h to afford compound **183** (208 mg, 85%) as a beige solid. Mp: 54-56 °C. ¹H NMR (400 MHz, CDCl₃) δ 7.04 (t, *J* = 8.0 Hz, 1H, CH), 6.29 (ddd, *J* = 15.3, 8.0, 2.2 Hz, 2H, 2 x CH), 6.24 (t, *J* = 2.3 Hz, 1H, CH), 3.96 (t, *J* = 6.4 Hz, 2H, CH₂), 3.63 (s, 2H, NH₂), 2.76 - 2.33 (m, 10H, 5 x CH₂), 2.29 (s, 3H, CH₃), 1.94 (p, *J* = 6.7 Hz, 1H, CH₂). HRMS (EI-MS) m/z calcd for C₁₄H₂₄N₃O [M+H]⁺: 250.1914, found: 250.1916.

### Example 96: 4-(5-methyl-7H-pyrrolo[2,3-d]pyrimidin-4-yl)-N-(3-(3-(4-methylpiperazin-1-yl)propoxy)phenyl)-3,6-dihydropyridine-1(2H)-carboxamide (185)

The reaction was carried out as described in general procedure **H** with aniline **183** (178 mg, 0.71 mmol, 1.0 equiv.), 4-nitrophenyl chloroformate (173 mg, 0.86 mmol, 1.2 equiv.), DIPEA (448 µL, 2.57 mmol, 2.4 equiv.), and amine **4** (246 mg, 0.86 mmol, 1.2 equiv.) in dry THF (10 mL). The crude reaction mixture was purified by silica gel column chromatography using a gradient solvent system of DCM/MeOH/NH₄OH from 100 up to 90/10/1 to afford compound **185** (77 mg, 22%) as a beige powder. R*_{f}* (DCM/MeOH/NH₄OH 90/10/1) 0.46. Mp: 102-104°C. ¹H NMR (250 MHz, DMSO-d₆) δ 11.83 (s, 1H, NH), 8.64 (s, 1H, CH), 8.54 (s, 1H, NH), 7.36 - 7.27 (m, 1H, 1H, CH), 7.20 (t, *J* = 2.1 Hz, 1H, CH), 7.17 - 7.03 (m, 2H, 2 x CH), 6.50 (dt, *J* = 7.5, 2.0 Hz, 1H, CH), 6.09 - 6.00 (m, 1H, CH), 4.27 - 4.16 (m, 2H, CH₂), 3.94 (t, *J* = 6.4 Hz, 2H, CH₂), 3.72 (t, *J* = 5.6 Hz, 2H, CH₂), 2.72 - 2.63 (m, 2H, CH₂), 2.49 - 2.27 (m, 10H, 5 x CH₂), 2.24 (d, *J* = 1.1 Hz, 3H, CH₃), 2.17 (s, 3H, CH₃), 1.85 (p, *J=* 6.7 Hz, 2H, CH₂). HRMS (EI-MS) m/z calcd for C₂₇H₃₆N₇O₂ [M+H]⁺: 490.2925, found: 490.2922.

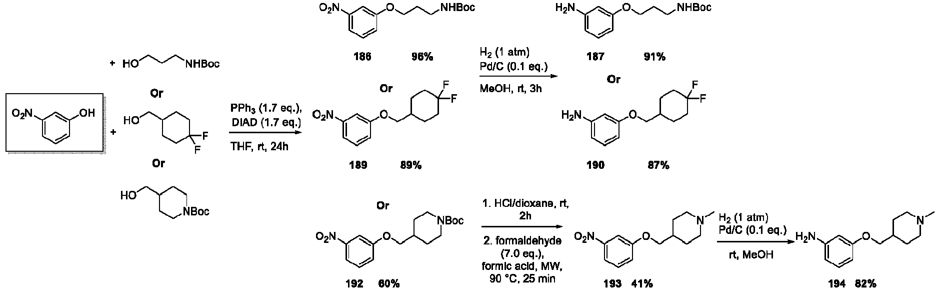

### tert-butyl (3-(3-nitrophenoxy)propyl)carbamate (186)

The reaction was carried out as described in general procedure **L** using 3-nitrophenol (200 mg, 1.44 mmol, 1.0 equiv.), 3-(Boc-amino)-1-propanol (492 µL, 2.88 mmol, 2.0 equiv.), PPh₃ (642 mg, 2.45 mmol, 1.7 equiv.), and DIAD (0.482 mL, 2.45 mmol, 1.7 equiv.) in dry THF (5 mL). The crude reaction mixture was purified by silica gel column chromatography using a gradient solvent system of PE/EtOAc from 95/5 up to 80/20 to afford compound **186** (412 mg, 96%) as a yellow oil. R*_{f}* (PE/EtOAc 80/20) 0.33. ¹H NMR (250 MHz, CDCl₃) δ 7.82 (ddd, *J=* 8.1, 2.1, 1.0 Hz, 1H, CH), 7.72 (t, *J* = 2.3 Hz, 1H, CH), 7.43 (t, *J* = 8.2 Hz, 1H, CH), 7.22 (ddd, *J* = 8.3, 2.6, 1.0 Hz, 1H, CH), 4.69 (s, 1H, NH), 4.10 (t, *J* = 6.0 Hz, 2H, CH₂), 3.34 (q, *J* = 6.6 Hz, 2H, CH₂), 2.02 (p, *J* = 6.4 Hz, 2H, CH₂), 1.44 (s, 9H, 3 x CH₃). HRMS (ElMS) m/z calcd for C₁₄H₂₁N₂O₅ [M+H]⁺: 297.1445, found: 297.1440.

### tert-butyl (3-(3-aminophenoxy)propyl)carbamate (187)

The reaction was carried out as described in general procedure **N** using the nitro compound **186** (239 mg, 0.81 mmol, 1.0 equiv.) and Pd/C 10 wt.% (86 mg, 0.081 mmol, 0.1 equiv.) in MeOH (4.5 mL) for 3 h to afford compound **187** (196 mg, 91%) as a yellow solid. Mp: 68-70 °C. ¹H NMR (250 MHz, CDCl₃) δ 7.05 (t, *J* = 8.0 Hz, 1H, CH), 6.37 - 6.27 (m, 2H, 2 x CH), 6.24 (t, *J* = 2.3 Hz, 1H, CH), 4.75 (brs, 1H, NH), 3.98 (t, *J* = 6.0 Hz, 2H, CH₂), 3.65 (brs, 2H, NH₂), 3.31 (q, *J* = 6.4 Hz, 2H, CH₂), 1.95 (p, *J* = 6.3 Hz, 2H, CH₂), 1.44 (s, 9H, 3 x CH₃). HRMS (EI-MS) m/z calcd for C₁₄H₂₃N₂O₃ [M+H]⁺: 267.1703, found: 267.1702.

### Example 97: tert-Butyl (3-(3-(4-(5-methyl-7H-pyrrolo[2,3-d]pyrimidin-4-yl)-1,2,3,6-tetrahydropyridine-1-carboxamido)phenoxy)propyl)carbamate (188)

The reaction was carried out as described in general procedure **H** with aniline **187** (78 mg, 0.29 mmol, 1.0 equiv.), 4-nitrophenyl chloroformate (71 mg, 0.35 mmol, 1.2 equiv.), DIPEA (122 µL, 0.70 mmol, 2.4 equiv.), and amine **4** (88 mg, 0.35 mmol, 1.2 equiv.) in dry THF (4.0 mL). The crude reaction mixture was purified by silica gel column chromatography using a gradient solvent system of DCM/MeOH from 98/2 up to 95/5 to afford compound **188** (85 mg, 57%) as a white powder. R*_{f}* (DCM/MeOH 95/5) 0.42. Mp: degradation 103 °C ¹H NMR (250 MHz, DMSO-d₆) δ 11.81 (s, 1H, NH), 8.64 (s, 1H, CH), 8.54 (s, 1H, NH), 7.35 - 7.27 (m, 1H, CH), 7.24 - 7.16 (m, 1H, CH), 7.17 - 7.02 (m, 2H, 2 x CH), 6.88 (t, *J* = 5.1 Hz, 1H, NH), 6.50 (dt, *J* = 7.6, 2.0 Hz, 1H, CH), 6.12 - 5.99 (m, 1H, CH), 4.21 (d, *J=* 2.6 Hz, 2H, CH₂), 3.92 (t, *J* = 6.2 Hz, 2H, CH₂), 3.73 (t, *J* = 5.6 Hz, 2H, CH₂), 3.07 (q, *J* = 6.6 Hz, 2H, CH₂), 2.68 (brs, 2H, CH₂), 2.24 (s, 3H, CH₃), 1.82 (p, *J* = 6.6 Hz, 2H, CH₂), 1.37 (s, 9H, 3 x CH₃). HRMS (EI-MS) m/z calcd for C₂₇H₃₅N₆O₄ [M+H]⁺: 507.2714, found: 507.2709.

### 1-((4,4-difluorocyclohexyl)methoxy)-3-nitrobenzene (189)

The reaction was carried out as described in general procedure **L** using 3-nitrophenol (300 mg, 2.16 mmol, 1.0 equiv.), (4,4-difluorocyclohexyl)methanol (562 µL, 4.32 mmol, 2.0 equiv.), PPh₃ (963 mg, 3.67 mmol, 1.7 equiv.), and DIAD (0.723 mL, 3.67 mmol, 1.7 equiv.) in dry THF (8 mL). The crude reaction mixture was purified by silica gel column chromatography using a gradient solvent system of PE/DCM from 100 up to 75/25 to afford compound **189** (524 mg, 89%) as a yellow solid. R*_{f}* (PE/DCM 80/20) 0.28. Mp: 75 □ 77 °C. ¹H NMR (400 MHz, CDCl₃) δ 7.82 (dd, *J* = 8.1, 2.0 Hz, 1H, CH), 7.71 (t, *J* = 2.3 Hz, 1H, CH), 7.43 (t, *J* = 8.2 Hz, 1H, CH), 7.21 (dd, *J* = 8.3, 2.5 Hz, 1H, CH), 3.89 (d, *J=* 6.0 Hz, 2H, CH₂), 2.25-2.10 (m, 2H, CH₂), 2.03 - 1.89 (m, 3H, CH + CH₂), 1.89 - 1.66 (m, 2H, CH₂), 1.53 - 1.39 (m, 2H, CH₂). ¹⁹F NMR (376 MHz, CDCl₃) δ -91.6 (d, *J* = 236.5 Hz, 1F), -102.2 (dtt, *J* = 236.1, 33.5, 10.5 Hz, 1F). HRMS (EI-MS) No MH+ or MH- ionization was detected.

### 3-((4,4-difluorocyclohexyl)methoxy)aniline (190)

The reaction was carried out as described in general procedure **N** using the nitro **189** (308 mg, 1.14 mmol, 1.0 equiv.) and Pd/C 10 wt.% (121 mg, 0.114 mmol, 0.1 equiv.) in MeOH (6 mL) for 3 h to afford compound **190** (239 mg, 87%) as a colorless oil. The product was used in the next step without further purification. ¹H NMR (400 MHz, CDCl₃) δ 7.05 (t, *J* = 8.0 Hz, 1H, CH), 6.34 - 6.27 (m, 2H, 2 x CH), 6.23 (t, *J* = 2.3 Hz, 1H, CH), 3.77 (d, *J* = 6.3 Hz, 2H, CH₂), 3.65 (s, 2H, NH₂), 2.19 - 2.06 (m, 2H, CH₂), 1.99 - 1.90 (m, 2H, CH₂), 1.85 - 1.65 (m, 3H, CH + CH₂), 1.49 - 1.34 (m, 2H, CH₂). ¹⁹F NMR (376 MHz, CDCl₃) δ -91.4 (d, *J* = 235.7 Hz, 1F), -102.0 (dtt, *J* = 236.2, 33.2, 9.5 Hz, 1F). HRMS (EI-MS) m/z calcd for C₁₃H₁₈F₂NO [M+H]⁺: 242.1351, found: 242.1350.

### Example 98: N-(3-((4,4-difluorocyclohexyl)methoxy)phenyl)-4-(5-methyl-7H-pyrrolo[2,3-d]pyrimidin-4-yl)-3,6-dihydropyridine-1(2H)-carboxamide (191)

The reaction was carried out as described in general procedure **H** using aniline **190** (101 mg, 0.42 mmol, 1.0 equiv.), 4-nitrophenyl chloroformate (101 mg, 0.50 mmol, 1.2 equiv.), DIPEA (175 µL, 1.01 mmol, 2.4 equiv.), and amine **4** (126 mg, 0.50 mmol, 1.2 equiv.) in dry THF (6.0 mL). The crude reaction mixture was purified by silica gel column chromatography using a gradient solvent system of DCM/MeOH from 98/2 up to 95/5 to afford compound **191** (119 mg, 59%) as a white powder. R*_{f}* (DCM/MeOH 95/5) 0.31. Mp: degradation 223°C. ¹H NMR (400 MHz, DMSO-d₆) δ 11.82 (s, 1H, NH), 8.65 (s, 1H, CH), 8.54 (s, 1H, NH), 7.31 (s, 1H, CH), 7.21 (s, 1H, CH), 7.17 - 7.05 (m, 2H, 2 x CH), 6.52 (d, *J* = 7.6 Hz, 1H, CH), 6.04 (brs, 1H, CH), 4.21 (d, *J* = 3.0 Hz, 2H,CH₂), 3.80 (d, *J* = 5.8 Hz, 2H, CH₂), 3.72 (t, *J* = 5.6 Hz, 2H, CH₂), 2.67 (brs, 2H, CH₂), 2.23 (s, 3H, CH₃), 2.11 - 1.97 (m, 2H, CH₂), 2.0 - 1.7 (m, 5H, 2 x CH₂ + CH), 1.39 - 1.20 (m, 2H, CH₂). ¹⁹F NMR (376 MHz, DMSO) δ -89.2 (d, *J* = 232.1 Hz, 1F), -99.6 (dt, *J* = 231.0, 35.9 Hz, 1F). HRMS (EI-MS) m/z calcd for C₂₆H₃₀F₂N₅O₂ [M+H]⁺: 482.2362, found: 482.2363.

### 4-((3-nitrophenoxy)methyl)piperidine (192)

The reaction was carried out as described in general procedure **L** using 3-nitrophenol (700 mg, 5.03 mmol, 1.0 equiv.), 1-Boc-4-piperidinemethanol (2.17 g, 10.10 mmol, 2.0 equiv.), PPh₃ (2.24 g, 8.55 mmol, 1.7 equiv.), and DIAD (1.68 mL, 8.55 mmol, 1.7 equiv.) in dry THF (28 mL). The reaction mixture was filtered on silica gel. The crude intermediate product was then dissolved in dry DCM (30 mL), the reaction mixture was cooled to 0 °C then 4N HCl in 1,4-dioxane (6.0 mL, 24.10 mmol, 8.0 equiv.) was added. The solution was stirred at rt for 3 h and concentrated under reduced pressure. The white residue was partitioned between Et₂O (20 mL) and H₂O (20 mL), and the organic layer was removed. The aqueous layer was then washed with Et₂O (15 mL), treated with a saturated aqueous solution of NaHCO₃ until pH 8-9 and extracted with DCM (3 x 40 mL). The combined organic layers were dried over MgSO₄, filtered and concentrated under reduced pressure to afford compound **192** (326 mg, 27%) as a sticky orange solid which was used without further purification in the next step. ¹H NMR (250 MHz, DMSO-d₆) δ 7.80 (ddd, *J* = 8.1, 2.2, 1.0 Hz, 1H, CH), 7.68 (t, *J* = 2.3 Hz, 1H, CH), 7.56 (t, *J* = 8.2 Hz, 1H, CH), 7.40 (ddd, *J* = 8.3, 2.5, 1.0 Hz, 1H, CH), 3.93 (d, *J=* 6.3 Hz, 2H, CH₂), 2.99 (d, *J* = 12.2 Hz, 2H, CH₂), 2.59 - 2.46 (m, 2H, CH₂), 1.97 - 1.78 (m, 1H, CH), 1.72 (d, *J* = 12.8 Hz, 2H, CH₂), 1.22 (qd, *J =* 12.1, 4.1 Hz, 2H, CH₂). HRMS (EI-MS) m/z calcd for C₁₂H₁₇N₂O₃ [M+H]⁺: 237.1234, found: 237.1235.

### 1-methyl-4-((3-nitrophenoxy)methyl)piperidine (193)

To a microwave reaction vial loaded with compound **192** (216 mg, 0.91 mmol, 1.0 equiv.) were added formic acid (0.8 mL) and a formaldehyde solution (37 wt. % in H₂O, 0.48 mL, 6.40 mmol, 7.0 equiv.). The tube was sealed and submitted to microwave irradiation at 90 °C for 2 cycles of 10 min. The reaction mixture was then diluted in H₂O (10 mL) and DCM (35 mL), and a saturated aqueous solution of NaHCO₃ was added to pH ~ 9. The aqueous layer was extracted with DCM (3 x 20 mL) then the combined organic layers were dried over MgSO₄, filtered and concentrated under reduced pressure to afford compound **193** (207 mg, 90%) as a yellow solid. The product was used without further purification in the next step. Mp: 68 - 70 °C. ¹H NMR (250 MHz, DMSO-d₆) δ 7.80 (ddd, *J* = 8.1, 2.2, 1.0 Hz, 1H, CH), 7.69 (t, *J* = 2.3 Hz, 1H, CH), 7.56 (t, *J* = 8.2 Hz, 1H, CH), 7.41 (ddd, *J* = 8.3, 2.5, 1.0 Hz, 1H, CH), 3.95 (d, *J* = 5.9 Hz, 2H, CH₂), 2.88 - 2.70 (m, 2H, CH₂), 2.15 (s, 3H, CH₃), 1.92 - 1.79 (m, 2H, CH₂), 1.78 - 1.61 (m, 3H, CH + CH₂), 1.42 - 1.15 (m, 2H, CH₂). HRMS (EI-MS) m/z calcd for C₁₃H₁₉N₂O₃ [M+H]⁺: 251.1390, found: 251.1387.

### 3-((1-methylpiperidin-4-yl)methoxy)aniline (194)

The reaction was carried out as described in general procedure **N** using the nitro **193** (171 mg, 0.72 mmol, 1.0 equiv.) and Pd/C 10 wt.% (77 mg, 0.07 mmol, 0.1 equiv.) in MeOH (4 mL) for 2 h to afford compound **194** (131 mg, 82%) as a beige solid. The product was used without further purification in the next step. ¹H NMR (250 MHz, DMSO-d₆) δ 6.86 (t, *J* = 8.3 Hz, 1H, CH), 6.16 - 6.09 (m, 2H, 2 x CH), 6.05 (ddd, *J=* 8.1, 2.3, 1.0 Hz, 1H, CH), 4.99 (s, 2H, NH₂), 3.69 (d, *J=* 5.9 Hz, 2H, CH₂), 2.81 - 2.70 (m, 2H, CH₂), 2.14 (s, 3H, CH₃), 1.83 (td, *J* = 11.6, 2.4 Hz, 2H, CH₂), 1.77 - 1.54 (m, 3H, CH + CH₂), 1.37 - 1.15 (m, 2H, CH₂). HRMS (EI-MS) m/z calcd for C₁₃H₂₁N₂O [M+H]⁺: 221.1648, found: 221.1645.

### Example 99: 4-(5-methyl-7H-pyrrolo[2,3-d]pyrimidin-4-yl)-N-(3-((1-methylpiperidin-4-yl)methoxy)phenyl)-3.6-dihydropyridine-1 (2H)-carboxamide (195)

The reaction was carried out as described in general procedure **H** using aniline **194** (113 mg, 0.51 mmol, 1.0 equiv.), 4-nitrophenyl chloroformate (124 mg, 0.62 mmol, 1.2 equiv.), DIPEA (322 µL, 3.60 mmol, 3.6 equiv.), and amine **4** (177 mg, 0.62 mmol, 1.2 equiv.) in dry THF (7.3 mL). The crude reaction mixture was purified by silica gel column chromatography using a gradient solvent system of DCM/MeOH/NH₄OH from 98/2/1 up to 94/6/1 to afford compound **195** (80 mg, 34%) as a white powder. R*_{f}* (DCM/MeOH/NH₄OH 95/5/1) 0.27. Mp: degradation 135 °C. ¹H NMR (250 MHz, DMSO-d₆) δ 11.83 (s, 1H, NH), 8.64 (s, 1H, CH), 8.54 (s, 1H, NH), 7.32 (brs, 1H, CH), 7.20 (brs, 1H, CH), 7.16 - 7.02 (m, 2H, 2 x CH), 6.51 (d, *J* = 7.4 Hz, 1H, CH), 6.04 (s, 1H, CH), 4.21 (brs, 2H, CH₂), 3.87 - 3.66 (m, 4H, 2 x CH₂), 2.83 (d, *J* = 11.0 Hz, 2H, CH₂), 2.67 (brs, 2H, CH₂), 2.23 (s, 3H, CH₃), 2.20 (s, 3H, CH₃), 1.96 (t, *J* = 11.5 Hz, 2H, CH₂), 1.82 - 1.62 (m, 3H, CH₂ + CH), 1.41 - 1.19 (m, 2H, CH₂). HRMS (EI-MS) m/z calcd for C₂₆H₃₃N₆O₂ [M+H]⁺: 461.2660, found: 461.2663.

### Example 100: N-methyl-4-(4-(4-(5-methyl-7H-pyrrolo[2,3-d]pyrimidin-4-yl)-1,2,3,6-tetrahydropyridine-1-carboxamido)phenoxy)picolinamide (196)

The reaction was carried out as described in general procedure **H** using 4-(4-aminophenoxy)-*N*-methylpicolinamide (100 mg, 0.41 mmol, 1.0 equiv.), 4-nitrophenyl chloroformate (99 mg, 0.49 mmol, 1.2 equiv.), DIPEA (258 µL, 1.48 mmol, 3.6 equiv.), and amine **4** (142 mg, 0.49 mmol, 1.2 equiv.) in dry THF (6 mL). The crude reaction mixture was purified by silica gel column chromatography using a gradient solvent system of DCM/MeOH/NH₄OH from 98/2/0.1 up to 93/7/0.1 to afford compound **196** (175 mg, 88%) as a beige powder. R*_{f}* (DCM/MeOH/NH₄OH 95/5/0.1) 0.44. Mp: degradation 149 °C. ¹H NMR (250 MHz, CDCl₃) δ 11.83 (s, 1H, NH), 8.80 - 8.71 (m, 2H, 2 x NH), 8.65 (s, 1H, CH), 8.52 - 8.48 (m, 1H, CH), 7.70 - 7.60 (m, 2H, 2 x CH), 7.38 (d, *J* = 2.8 Hz, 1H, CH), 7.35 - 7.29 (m, 1H, CH), 7.17 - 7.08 (m, 3H, 3 x CH), 6.06 (brs, 1H, CH), 4.30 - 4.21 (m, 2H, CH₂), 3.76 (t, *J* = 5.5 Hz, 2H, CH₂), 2.79 (d, *J* = 4.8 Hz, 3H, CH₃), 2.70 (brs, 2H, CH₂), 2.29 - 2.22 (m, 3H, CH₃). HRMS (EI-MS) m/z calcd for C₂₆H₂₆N₇O₃ [M+H]⁺: 484.2092, found: 484.2086.

### 4-(3-Aminophenoxy)-N-methyl-2-pyridinecarboxamide (197)

To a solution of 3-aminophenol (336 mg, 3.08 mmol, 1.05 equiv.) in dry DMSO (2 mL) under argon was added t-BuOK (361 mg, 3.22 mmol, 1.1 equiv.). The solution was stirred at rt for 30 min then 4-chloro-*N*-methylpicolinamide (500 mg, 2.93 mmol, 1.0 equiv.) was added. The reaction mixture was stirred at 80 °C for 3 h then cooled to rt then diluted with H₂O (40 mL) and EtOAc (50 mL). The aqueous layer was separated and extracted with EtOAc (2 x 30 mL) then the combined organic layers were washed with brine (15 mL), dried over MgSO₄, filtered and concentrated under reduced pressure. The crude reaction mixture was purified by silica gel column chromatography using a gradient solvent system of DCM/MeOH from 100 up to 98/2 to afford compound **197** (516 mg, 72%) as a colorless oil. R*_{f}* (DCM/MeOH 98/2) 0.59. ¹H NMR (250 MHz, DMSO-d₆) δ 8.75 (q, *J* = 4.8 Hz, 1H, NH), 8.49 (dd, *J* = 5.6, 0.5 Hz, 1H, CH), 7.41 (d, *J* = 2.6 Hz, 1H, CH), 7.20 - 7.02 (m, 2H, 2 x CH), 6.51 (ddd, *J* = 8.1, 2.1, 0.9 Hz, 1H, CH), 6.32 (t, *J* = 2.2 Hz, 1H, CH), 6.27 (ddd, *J* = 7.9, 2.4, 0.9 Hz, 1H, CH), 5.39 (s, 2H, NH₂), 2.79 (d, *J=* 4.9 Hz, 3H, CH₃). HRMS (EI-MS) m/z calcd for C₁₃H₁₄N₃O₂ [M+H]⁺: 244.1081, found: 244.1080.

### Example 101: N-methyl-4-(3-(4-(5-methyl-7H-pyrrolo[2,3-d]pyrimidin-4-yl)-1,2,3,6-tetrahydropyridine-1-carboxamido)phenoxy)picolinamide (198)

The reaction was carried out as described in general procedure **H** with aniline **197** (110 mg, 0.45 mmol, 1.0 equiv.), 4-nitrophenyl chloroformate (109 mg, 0.54 mmol, 1.2 equiv.), DIPEA (284 µL, 1.63 mmol, 3.6 equiv.), and amine **4** (156 mg, 0.54 mmol, 1.2 equiv.) in dry THF (7 mL). The crude reaction mixture was purified by silica gel column chromatography using a gradient solvent system of DCM/MeOH from 98/2 up to 95/5 to afford compound **198** (160 mg, 73%) as a beige powder. R*_{f}* (DCM/MeOH 95/5) 0.54. Mp: 211-213 °C. ¹H NMR (250 MHz, DMSO-d₆) δ 11.82 (s, 1H, NH), 8.85 - 8.71 (m, 2H, 2 x NH), 8.64 (s, 1H, CH), 8.52 (d, *J =* 5.6 Hz, 1H, CH), 7.52 - 7.33 (m, 4H, 4 x CH), 7.30 (brs, 1H, CH), 7.18 (dd, *J* = 5.6, 2.6 Hz, 1H, CH), 6.80 (ddd, *J* = 7.8, 2.3, 1.3 Hz, 1H, CH), 6.03 (brs, 1H, CH), 4.21 (d, *J=* 3.1 Hz, 2H, CH₂), 3.73 (t, *J* = 5.6 Hz, 2H, CH₂), 2.79 (d, *J* = 4.8 Hz, 3H, CH₃), 2.72 - 2.67 (m, 2H, CH₂), 2.22 (d, *J* = 1.0 Hz, 3H, CH₃). HRMS (EI-MS) m/z calcd for C₂₆H₂₆N₇O₃ [M+H]⁺: 484.2092, found: 484.2088.

### 3-(3-fluorophenoxy)aniline (199)

The reaction was carried out as described in general procedure **P** using 3-fluoroiodobenzene (235 µL, 2.00 mmol, 1.0 equiv.), 3-aminophenol (262 mg, 2.40 mmol, 1.2 equiv.), copper(I) iodide (19 mg, 0.10 mmol, 5 mol%), 2-picolinic acid (25 mg, 0.20 mmol, 10 mol%), and K₃PO₄ (849 mg, 4.00 mmol, 2.0 equiv.) in dry DMSO (4 mL). The crude reaction mixture was purified by silica gel column chromatography using a gradient solvent system of PE/EtOAc from 90/10 up to 80/20 to afford compound **199** (406 mg, quant.) as an orange liquid. R*_{f}* (PE/EtOAc 80/20) 0.35. ¹H NMR (400 MHz, CDCl₃) δ 7.33 - 7.22 (m, 1H, CH), 7.14 (t, *J* = 8.0 Hz, 1H, CH), 6.85 - 6.70 (m, 3H, 3 x CH), 6.46 (ddd, *J* = 19.6, 8.1, 2.2 Hz, 2H, 2 x CH), 6.38 (t, *J* = 2.3 Hz, 1H, CH), 3.74 (brs, 2H, NH₂). ¹⁹F NMR (376 MHz, CDCl₃) δ -111.2. HRMS (EI-MS) m/z calcd for C₁₂H₁₁FNO [M+H]⁺: 204.0819, found: 204.0824.

### Example 102: N-(3-(3-fluorophenoxy)phenyl)-4-(5-methyl-7H-pyrrolo[2,3-d]pyrimidin-4-yl)-3,6-dihydropyridine-1(2H)-carboxamide (200)

The reaction was carried out as described in general procedure **H** using aniline **199** (77 mg, 0.38 mmol, 1.0 equiv.), 4-nitrophenyl chloroformate (91 mg, 0.45 mmol, 1.2 equiv.), DIPEA (237 µL, 1.36 mmol, 2.4 equiv.) and amine **4** (130 mg, 0.45 mmol, 1.2 equiv.) in dry THF (5.4 mL). The crude reaction mixture was purified by silica gel column chromatography using a gradient solvent system of DCM/MeOH from 98/2 up to 97/3 to afford compound **200** (129 mg, 76%) as a brown solid. R*_{f}* (DCM/MeOH 95/5) 0.33. Mp: degradation 150 °C. ¹H NMR (400 MHz, DMSO-d₆) δ 11.83 (s, 1H, NH), 8.70 (s, 1H, NH), 8.64 (s, 1H, CH), 7.47 - 7.38 (m, 1H, CH), 7.38 - 7.23 (m, 4H, 4 x CH), 6.96 (td, *J* = 8.5, 2.5 Hz, 1H, CH), 6.90 - 6.80 (m, 2H, 2 x CH), 6.67 (d, *J=* 8.0 Hz, 1H, CH), 6.03 (brs, 1H, CH), 4.20 (d, *J=* 3.0 Hz, 2H, CH₂), 3.71 (t, *J* = 5.5 Hz, 2H, CH₂), 2.66 (brs, 2H, CH₂), 2.22 (s, 3H, CH₃). ¹⁹F NMR (376 MHz, DMSO-d₆) δ -111.0 (s, 1F). HRMS (EI-MS) m/z calcd for C₂₅H₂₃FN₅O₂ [M+H]⁺: 444.1830, found: 444.1835.

### 3-(4-fluorophenoxy)aniline (201)

The reaction was carried out as described in general procedure **P** using 4-fluoroiodobenzene (231 µL, 2.00 mmol, 1.0 equiv.), 3-aminophenol (262 mg, 2.40 mmol, 1.2 equiv.), copper(I) iodide (19 mg, 0.10 mmol, 5 mol%), 2-picolinic acid (25 mg, 0.20 mmol, 10 mol%), and K₃PO₄ (849 mg, 4.00 mmol, 2.0 equiv.) and dry DMSO (4 mL). The crude reaction mixture was purified by silica gel column chromatography using a gradient solvent system of PE/EtOAc from 100 up to 90/10 to afford compound **201** (332 mg, 82%) as an orange oil. R*_{f}* (PE/EtOAc 90/10) 0.32. ¹H NMR (250 MHz, CDCl₃) δ 7.12 - 6.94 (m, 5H, 5 x CH), 6.41 (ddd, *J* = 8.0, 2.2, 0.9 Hz, 1H, CH), 6.34 (ddd, *J* = 8.1, 2.3, 0.9 Hz, 1H, CH), 6.28 (td, *J* = 2.3, 0.4 Hz, 1H, CH), 3.69 (brs, 2H, NH₂). HRMS (EI-MS) m/z calcd for C₁₂H₁₁FNO [M+H]⁺: 204.0819, found: 204.0823.

All spectra data corresponded to the literature values as found in Maiti D., Buchwald S. L., J. Am. Chem. Soc. 2009, 131, 17423-17429.

### Example 103: N-(3-(4-fluorophenoxy)phenyl)-4-(5-methyl-7H-pyrrolo[2,3-d]pyrimidin-4-yl)-3,6-dihydropyridine-1 (2H)-carboxamide (202)

The reaction was carried out as described in general procedure **H** using aniline **201** (79 mg, 0.39 mmol, 1.0 equiv.), 4-nitrophenyl chloroformate (94 mg, 0.47 mmol, 1.2 equiv.), DIPEA (244 µL, 1.40 mmol, 2.4 equiv.) and amine **4** (134 mg, 0.47 mmol, 1.2 equiv.) in dry THF (5.6 mL). The crude reaction mixture was purified by silica gel column chromatography using a gradient solvent system of DCM/MeOH from 98/2 up to 97/3 to afford compound **202** (129 mg, 74%) as a white powder. R*_{f}* (DCM/MeOH 95/5) 0.25. Mp: 213-215 °C. ¹H NMR (400 MHz, DMSO-d₆) δ 11.82 (s, 1H, NH), 8.65 (s, 1H, NH), 8.64 (s, 1H, CH), 7.34 - 7.28 (m, 2H, 2 x CH), 7.27 - 7.19 (m, 4H, 4 x CH), 7.12 - 7.00 (m, 2H, 2 x CH), 6.59 (dd, *J =* 7.6, 2.3 Hz, 1H, CH), 6.03 (brs, 1H, CH), 4.26 - 4.15 (m, 2H, CH₂), 3.71 (t, *J =* 5.6 Hz, 2H, CH₂), 2.66 (brs, 2H, CH₂), 2.22 (s, 3H, CH₃). ¹⁹F NMR (376 MHz, DMSO-d₆) δ -119.99 - - 120.08 (m, 1F). HRMS (EI-MS) m/z calcd for C₂₅H₂₃FN₅O₂ [M+H]⁺: 444.1830, found: 444.1836.

### 3-(3-chlorophenoxy)aniline (203)

The reaction was carried out as described in general procedure **P** using 3-chloroiodobenzene (248 µL, 2.00 mmol, 1.0 equiv.), 3-aminophenol (262 mg, 2.40 mmol, 1.2 equiv.), copper(I) iodide (19 mg, 0.10 mmol, 5 mol%), 2-picolinic acid (25 mg, 0.20 mmol, 10 mol%), and K₃PO₄ (849 mg, 4.00 mmol, 2.0 equiv.) in dry DMSO (4 mL). The crude reaction mixture was purified by silica gel column chromatography using a gradient solvent system of PE/EtOAc from 95/5 up to 80/20 to afford compound **203** (427 mg, 97%) as an orange liquid. R*_{f}* (PE/EtOAc 80/20) 0.39. ¹H NMR (250 MHz, CDCl₃) δ 7.23 (t, *J* = 8.1 Hz, 1H, CH), 7.12 (t, *J* = 8.0 Hz, 1H, CH), 7.05 (ddd, *J* = 7.9, 2.0, 1.0 Hz, 1H, CH), 6.99 (t, *J* = 2.1 Hz, 1H, CH), 6.90 (ddd, *J* = 8.2, 2.4, 1.0 Hz, 1H, CH), 6.46 (ddd, *J =* 8.0, 2.2, 0.9 Hz, 1H, CH), 6.40 (ddd, *J* = 8.0, 2.3, 0.9 Hz, 1H, CH), 6.34 (t, *J* = 2.2 Hz, 1H, CH), 3.72 (brs, 2H, NH₂). HRMS (EI-MS) m/z calcd for C₁₂H₁₁ClNO [M+H]⁺: 220.0524, found: 220.0525.

### Example 104: N-(3-(3-chlorophenoxy)phenyl)-4-(5-methyl-7H-pyrrolo[2,3-d]pyrimidin-4-yl)-3,6-dihydropyridine-1 (2H)-carboxamide (204)

The reaction was carried out as described in general procedure **H** using aniline **203** (90 mg, 0.41 mmol, 1.0 equiv.), 4-nitrophenyl chloroformate (99 mg, 0.49 mmol, 1.2 equiv.), DIPEA (258 µL, 1.48 mmol, 2.4 equiv.) and amine **4** (141 mg, 0.49 mmol, 1.2 equiv.) in dry THF (5.9 mL). The crude reaction mixture was purified by silica gel column chromatography using a gradient solvent system of DCM/MeOH from 98/2 up to 95/5 to afford compound **204** (161 mg, 85%) as a white solid. R*_{f}* (DCM/MeOH 95/5) 0.39. Mp: degradation 124°C. ¹H NMR (400 MHz, DMSO-d₆) δ 11.82 (s, 1H, NH), 8.70 (s, 1H, NH), 8.64 (s, 1H, CH), 7.45 - 7.25 (m, 5H, 5 x CH), 7.22 - 7.16 (m, 1H, CH), 7.08 - 7.05 (m, 1H, CH), 6.98 (dd, *J* = 8.3, 2.4 Hz, 1H, CH), 6.66 (d, *J* = 8.0 Hz, 1H, CH), 6.09 - 5.99 (m, 1H, CH), 4.20 (d, *J* = 3.1 Hz, 2H, CH₂), 3.72 (t, *J* = 5.6 Hz, 2H, CH₂), 2.67 (brs, 2H, CH₂), 2.22 (s, 3H, CH₃). HRMS (EI-MS) m/z calcd for C₂₅H₂₃ClN₅O₂ [M+H]⁺: 460.1535, found: 460.1539.

### 3-(4-bromo-2-chlorophenoxy)aniline (205)

To a solution of 3-aminophenol (274 mg, 2.51 mmol, 1.05 equiv.) in dry DMSO (2 mL) under argon was added t-BuOK (295 mg, 2.63 mmol, 1.1 equiv.). The solution was stirred at rt for 30 min then 4-bromo-2-chloro-1-fluorobenzene (290 µL, 2.39 mmol, 1.0 equiv.) was added. The reaction mixture was stirred at 80 °C for 18 h then cooled to rt, diluted with H₂O (25 mL) and EtOAc (50 mL). The aqueous layer was separated and extracted with EtOAc (2 x 50 mL) then the combined organic layers were washed with brine (3 x 20 mL), dried over MgSO₄, filtered and concentrated under reduced pressure. The crude reaction mixture was purified by silica gel column chromatography using a gradient solvent system of PE/EtOAc from 100 up to 90/10 to afford compound **205** (612 mg, 86%) as an orange oil. R*_{f}* (PE/EtOAc 90/10) 0.21. ¹H NMR (250 MHz, CDCl₃) δ 7.59 (d, *J* = 2.4 Hz, 1H, CH), 7.31 (dd, *J* = 8.7, 2.4 Hz, 1H, CH), 7.10 (t, *J* = 8.0 Hz, 1H, CH), 6.87 (d, *J* = 8.7 Hz, 1H, CH), 6.44 (ddd, *J* = 8.0, 2.2, 0.9 Hz, 1H, CH), 6.33 (ddd, *J* = 8.0, 2.4, 0.9 Hz, 1H, CH), 6.28 (t, *J* = 2.2 Hz, 1H, CH), 3.72 (brs, 2H, NH₂). HRMS (EI-MS) m/z calcd for C₁₂H₁₀BrClNO [M+H]⁺: 299.9607, found: 299.9606.

### Example 105: N-(3-(4-bromo-2-chlorophenoxy)phenyl)-4-(5-methyl-7H-pyrrolo[2,3-d]pyrimidin-4-yl)-3,6-dihydropyridine-1(2H)-carboxamide (206)

The reaction was carried out as described in general procedure **H** using aniline **205** (65 mg, 0.22 mmol, 1.0 equiv.), 4-nitrophenyl chloroformate (53 mg, 0.26 mmol, 1.2 equiv.), DIPEA (137 µL, 0.79 mmol, 2.4 equiv.) and amine **4** (75 mg, 0.26 mmol, 1.2 equiv.) in dry THF (3.1 mL). The crude reaction mixture was purified by silica gel column chromatography using a gradient solvent system of DCM/MeOH from 98/2 up to 96/4 to afford compound **206** (87 mg, 73%) as a white solid. R*_{f}* (DCM/MeOH 95/5) 0.30. Mp: degradation 148°C. ¹H NMR (250 MHz, DMSO-d₆) δ 11.85 (s, 1H, NH), 8.70 (s, 1H, NH), 8.64 (s, 1H, CH), 7.89 (d, *J* = 2.4 Hz, 1H, CH), 7.57 (dd, *J* = 8.7, 2.4 Hz, 1H, CH), 7.40 - 7.19 (m, 4H, 4 x CH), 7.06 (d, *J* = 8.8 Hz, 1H, CH), 6.61 (ddd, *J* = 7.8, 2.5, 1.2 Hz, 1H, CH), 6.03 (brs, 1H, CH), 4.19 (d, *J* = 3.1 Hz, 2H, CH₂), 3.70 (t, *J* = 5.6 Hz, 2H, CH₂), 2.66 (brs, 2H, CH₂), 2.22 (d, *J* = 1.1 Hz, 3H, CH₃). HRMS (EI-MS) m/z calcd for C₂₅H₂₂BrClN₅O₂ [M+H]⁺: 540.0620, found: 540.0621.

### 3-(pyridin-3-yloxy)aniline (207)

The reaction was carried out as described in general procedure **P** using 3-iodopyridine (410 mg, 2.00 mmol, 1.0 equiv.), 3-aminophenol (262 mg, 2.40 mmol, 1.2 equiv.), copper(I) iodide (19 mg, 0.10 mmol, 5 mol%), 2-picolinic acid (25 mg, 0.20 mmol, 10 mol%), and K₃PO₄ (849 mg, 4.00 mmol, 2.0 equiv.) in dry DMSO (4 mL). The crude reaction mixture was purified by silica gel column chromatography using a gradient solvent system of PE/EtOAc from 80/20 up to 50/50 to afford compound **207** (247 mg, 55%) as a brown oil. R*_{f}* (PE/EtOAc 50/50) 0.30. ¹H NMR (250 MHz, CDCl₃) δ 8.41 (dd, *J=* 2.8, 0.8 Hz, 1H, CH), 8.35 (dd, *J=* 4.5, 1.6 Hz, 1H, CH), 7.34 - 7.21 (m, 2H, 2 x CH), 7.12 (t, *J* = 8.0 Hz, 1H, CH), 6.46 (ddd, *J* = 8.0, 2.2, 0.9 Hz, 1H, CH), 6.39 (ddd, *J* = 8.0, 2.3, 0.9 Hz, 1H, CH), 6.34 (t, *J* = 2.2 Hz, 1H, CH), 3.73 (brs, 2H, NH₂). HRMS (EI-MS) m/z calcd for C₁₁H₁₁N₂O [M+H]⁺: 187.0866, found: 187.0871.

All spectra data corresponded to the literature values as found in Maiti D., Buchwald S. L., J. Am. Chem. Soc. 2009, 131, 17423-17429.

### Example 106: 4-(5-methyl-7H-pyrrolo[2,3-d]pyrimidin-4-yl)-N-(3-(pyridin-3-yloxy)phenyl)-3,6-dihydropyridine-1(2H)-carboxamide (208)

The reaction was carried out as described in general procedure **H** using aniline **207** (70 mg, 0.38 mmol, 1.0 equiv.), 4-nitrophenyl chloroformate (91 mg, 0.45 mmol, 1.2 equiv.), DIPEA (236 µL, 1.35 mmol, 2.4 equiv.) and amine **4** (130 mg, 0.45 mmol, 1.2 equiv.) in dry THF (5.4 mL). The crude reaction mixture was purified by silica gel column chromatography using a gradient solvent system of DCM/MeOH from 98/2 up to 95/5 to afford compound **208** (112 mg, 68%) as a white powder. R*_{f}* (DCM/MeOH 95/5) 0.24. Mp: degradation 132°C. ¹H NMR (250 MHz, DMSO-d₆) δ 11.83 (s, 1H, NH), 8.71 (s, 1H, NH), 8.64 (s, 1H, CH), 8.43 - 8.32 (m, 2H, 2 x CH), 7.49 - 7.40 (m, 2H, 2 x CH), 7.38 - 7.24 (m, 4H, 4 x CH), 6.72 - 6.61 (m, 1H, CH), 6.03 (brs, 1H, CH), 4.19 (d, *J* = 2.4 Hz, 2H, CH₂), 3.71 (t, *J* = 5.5 Hz, 2H, CH₂), 2.66 (brs, 2H, CH₂), 2.22 (s, 3H, CH₃). HRMS (EI-MS) m/z calcd for C₂₄H₂₃N₆O₂ [M+H]⁺: 427.1877, found: 427.1885.

### 1-(benzyloxy)-3-iodobenzene (209)

To a solution of 3-iodophenol (500 mg, 2.27 mmol, 1.0 equiv.) in acetone (3 mL) were added K₂CO₃ (471 mg, 3.41 mmol, 1.5 equiv.), and benzyl bromide (406 µL, 3.41 mmol, 1.5 equiv.). The resulting suspension was stirred at rt for 18 h, diluted with a saturated solution of NH₄Cl (15 mL) then the aqueous layer was separated and extracted with Et₂O (2 x 30 mL). The combined organic layers were dried over MgSO₄, filtered and concentrated under reduced pressure. The crude product was precipitated in PE at 0 °C, after filtration of the solid the filtrate was evaporated and precipitated again to afford compound **209** (366 mg, 52%) as a white powder. ¹H NMR (250 MHz, CDCl₃) δ 7.48 - 7.27 (m, 7H, 7 x CH), 7.05 - 6.89 (m, 2H, CH), 5.03 (s, 2H, CH₂).

All spectra data corresponded to the literature values as found Kutz S. K. et al., Synthesis, 2017, 49, 275-292.

### 3-(3-(benzyloxy)phenoxy)aniline (210)

The reaction was carried out as described in general procedure **P** using the iodoaryl **209** (310 mg, 1.00 mmol, 1.0 equiv.), 3-aminophenol (131 mg, 1.20 mmol, 1.2 equiv.), copper(I) iodide (10 mg, 0.05 mmol, 5 mol%), 2-picolinic acid (12 mg, 0.10 mmol, 10 mol%), and K₃PO₄ (425 mg, 2.00 mmol, 2.0 equiv.) in dry DMSO (2 mL). The crude reaction mixture was purified by silica gel column chromatography using a gradient solvent system of PE/EtOAc from 90/10 up to 80/20 to afford compound **210** (221 mg, 76%) as a brown oil. R*_{f}* (PE/EtOAc 80/20) 0.38. ¹H NMR (250 MHz, CDCl₃) δ 7.46 - 7.30 (m, 5H, 5 x CH), 7.22 (t, *J* = 8.1 Hz, 1H, CH), 7.09 (t, *J* = 8.0 Hz, 1H, CH), 6.72 (dd, *J* = 8.2, 2.4 Hz, 1H, CH), 6.68 - 6.58 (m, 2H, 2 x CH), 6.48 - 6.37 (m, 2H, 2 x CH), 6.33 (t, *J =* 2.2 Hz, 1H, CH), 5.02 (s, 2H, CH₂), 3.68 (s, 2H, NH₂). HRMS (EI-MS) m/z calcd for C₁₉H₁₈NO₂ [M+H]⁺: 292.1332, found: 292.1336.

### Example 107: N-(3-(3-(benzyloxy)phenoxy)phenyl)-4-(5-methyl-7H-pyrrolo[2,3-d]pyrimidin-4-yl)-3,6-dihydropyridine-1(2H)-carboxamide (211)

The reaction was carried out as described in general procedure **H** using aniline **210** (54 mg, 0.19 mmol, 1.0 equiv.), 4-nitrophenyl chloroformate (45 mg, 0.22 mmol, 1.2 equiv.), DIPEA (116 µL, 0.67 mmol, 2.4 equiv.) and amine **4** (64 mg, 0.22 mmol, 1.2 equiv.) in dry THF (2.6 mL). The crude reaction mixture was purified by silica gel column chromatography using a gradient solvent system of DCM/MeOH from 100 up to 97/3 to afford compound **211** (79 mg, 79%) as a white powder. R*_{f}* (DCM/MeOH 95/5) 0.29. Mp: degradation 110 °C. ¹H NMR (250 MHz, DMSO-d₆) δ 11.82 (s, 1H, NH), 8.67 (brs, 1H, NH), 8.64 (s, 1H, CH), 7.48 - 7.19 (m, 10H, 10 x CH), 6.78 (ddd, *J* = 8.3, 2.5, 0.9 Hz, 1H, CH), 6.66 (t, *J =* 2.3 Hz, 1H, CH), 6.64 - 6.53 (m, 2H, 2 x CH), 6.05 - 5.99 (m, 1H, CH), 5.08 (s, 2H, CH₂), 4.26 - 4.14 (m, 2H, CH₂), 3.71 (t, *J* = 5.5 Hz, 2H, CH₂), 2.66 (brs, 2H, CH₂), 2.22 (d, *J=* 1.0 Hz, 3H, CH₃). HRMS (EI-MS) m/z calcd for C₃₂H₃₀N₅O₃ [M+H]⁺: 532.2343, found: 532.2351.

### 3-(3-aminophenoxy)phenol (212)

The reaction was carried out as described in general procedure **N** using compound **210** (139 mg, 0.48 mmol, 1.0 equiv.) and Pd/C 10 wt.% (51 mg, 0.05 mmol, 0.1 equiv.) in MeOH (2.7 mL) for 3 h to afford compound **212** (90 mg, 94%) as an amorphous beige solid. ¹H NMR (250 MHz, DMSO-d₆) δ 9.51 (s, 1H, OH), 7.12 (t, *J* = 8.1 Hz, 1H, CH), 6.98 (t, *J* = 8.0 Hz, 1H, CH), 6.48 (ddd, *J* = 8.1, 2.3, 1.0 Hz, 1H, CH), 6.39 (ddd, J = 8.1, 2.4, 1.0 Hz, 1H, CH), 6.35 - 6.27 (m, 2H, 2 x CH), 6.17 (t, *J* = 2.2 Hz, 1H, CH), 6.12 (ddd, *J* = 7.9, 2.4, 1.0 Hz, 1H, CH), 5.20 (brs, 2H, NH₂). HRMS (EI-MS) m/z calcd for C₁₂H₁₂NO₂ [M+H]⁺: 202.0863, found: 202.0867.

### Example 108: N-(3-(3-hydroxyphenoxy)phenyl)-4-(5-methyl-7H-pyrrolo[2,3-d]pyrimidin-4-yl)-3,6-dihydropyridine-1(2H)-carboxamide (213)

The reaction was carried out as described in general procedure **H** using aniline **212** (79 mg, 0.39 mmol, 1.0 equiv.), 4-nitrophenyl chloroformate (95 mg, 0.47 mmol, 1.2 equiv.), DIPEA (246 µL, 1.41 mmol, 2.4 equiv.) and amine **4** (136 mg, 0.47 mmol, 1.2 equiv.) in dry THF (5.6 mL). The crude reaction mixture was purified by silica gel column chromatography using a gradient solvent system of DCM/MeOH from 98/2 up to 96/4 to afford compound **213** (80 mg, 46%) as a white powder. R*_{f}* (DCM/MeOH 95/5) 0.22. Mp: degradation 144 °C. ¹H NMR (250 MHz, DMSO-d₆) δ 11.81 (s, 1H, NH), 9.55 (s, 1H, OH), 8.65 (s, 1H, NH), 8.64 (s, 1H, CH), 7.37 - 7.20 (m, 4H, 4 x CH), 7.15 (t, *J =* 8.1 Hz, 1H, CH), 6.61 (ddd, *J* = 7.7, 2.4, 1.3 Hz, 1H, CH), 6.52 (ddd, *J* = 8.1, 2.3, 0.9 Hz, 1H, CH), 6.43 (ddd, *J* = 8.1, 2.4, 0.9 Hz, 1H, CH), 6.38 (t, *J* = 2.3 Hz, 1H, CH), 6.08 - 5.98 (m, 1H, CH), 4.20 (d, *J* = 3.1 Hz, 2H, CH₂), 3.71 (t, *J =* 5.5 Hz, 2H, CH₂), 2.66 (brs, 2H, CH₂), 2.22 (d, *J* = 1.1 Hz, 3H, CH₃). HRMS (EI-MS) m/z calcd for C₂₅H₂₄N₅O₃ [M+H]⁺: 442.1874, found: 442.1883.

### 3-iodophenyl dimethylcarbamate (214)

To a solution of 3-iodophenol (600 mg, 2.73 mmol, 1.0 equiv.) in dry MeCN (14 mL) under argon atmosphere were added K₂CO₃ (567 mg, 4.10 mmol, 1.5 equiv.), and dimethylcarbamyl chloride (377 µL, 4.10 mmol, 1.5 equiv.). The reaction mixture was stirred under reflux for 18 h then cooled to rt and concentrated under reduced pressure. The residue was dissolved in EtOAc (50 mL) and H₂O (20 mL), then the aqueous layer was separated and extracted with EtOAc (2 x 30 mL). The combined organic layers were washed with KOH 1M (15 mL), dried over MgSO₄, filtered and concentrated under reduced pressure. The crude reaction mixture was purified by silica gel column chromatography using a gradient solvent system of PE/EtOAc from 100 up to 90/10 to afford compound **214** (730 mg, 92%) as a colorless oil. R*_{f}* (PE/EtOAc 90/10) 0.40. ¹H NMR (250 MHz, CDCl₃) δ 7.57 - 7.47 (m, 2H, 2 x CH), 7.14 - 7.01 (m, 2H, 2 x CH), 3.04 (d, *J* = 18.5 Hz, 6H, 2 x CH₃). HRMS (EI-MS) m/z calcd for C₉H₁₁INO₂ [M+H]⁺: 291.9829, found: 291.9834.

### 3-(3-aminophenoxy)phenyl dimethylcarbamate (215)

The reaction was carried out as described in general procedure **P** using iodoaryl **214** (582 mg, 2.00 mmol, 1.0 equiv.), 3-aminophenol (262 mg, 2.40 mmol, 1.2 equiv.), copper(I) iodide (19 mg, 0.10 mmol, 5 mol%), 2-picolinic acid (25 mg, 0.20 mmol, 10 mol%) and K₃PO₄ (849 mg, 4.00 mmol, 2.0 equiv.) in dry DMSO (4 mL). The crude reaction mixture was purified by silica gel column chromatography using a gradient solvent system of PE/EtOAc from 80/20 up to 70/30 to afford compound **215** (166 mg, 30%) as a brown oil. R*_{f}* (PE/EtOAc 70/30) 0.18. ¹H NMR (250 MHz, CDCl₃) δ 7.28 (t, *J* = 8.2 Hz, 1H, CH), 7.10 (t, *J* = 8.0 Hz, 1H, CH), 6.85 (dd, *J* = 8.2, 2.3 Hz, 2H, 2 x CH), 6.76 (t, *J* = 2.3 Hz, 1H, CH), 6.43 (ddd, *J* = 8.0, 2.2, 1.2 Hz, 2H, 2 x CH), 6.35 (t, *J* = 2.2 Hz, 1H, CH), 3.69 (s, 2H, NH₂), 3.03 (d, *J* = 19.1 Hz, 6H, 2 x CH₃). HRMS (EI-MS) m/z calcd for C₁₅H₁₇N₂O₃ [M+H]⁺: 273.1234, found: 273.1235.

### Example 109: 3-(3-(4-(5-methyl-7H-pyrrolo[2,3-d]pyrimidin-4-yl)-1,2,3,6-tetrahydropyridine-1-carboxamido)phenoxy)phenyl dimethylcarbamate (216)

The reaction was carried out as described in general procedure **H** using aniline **215** (69 mg, 0.25 mmol, 1.0 equiv.), 4-nitrophenyl chloroformate (61 mg, 0.30 mmol, 1.2 equiv.), DIPEA (157 µL, 0.91 mmol, 2.4 equiv.) and amine **4** (87 mg, 0.30 mmol, 1.2 equiv.) in dry THF (4 mL). The crude reaction mixture was purified by silica gel column chromatography using a gradient solvent system of DCM/MeOH from 98/2 up to 95/5 to afford compound **216** (81 mg, 64%) as a white powder after co-evaporation with ethanol. R*_{f}* (DCM/MeOH 95/5) 0.27. Mp: degradation 132 °C. ¹H NMR (250 MHz, DMSO-d₆) δ 11.84 (s, 1H, NH), 8.72 (s, 1H, NH), 8.64 (s, 1H, CH), 7.45 - 7.19 (m, 5H, 5 x CH), 6.94 - 6.81 (m, 2H, 2 x CH), 6.74 (t, *J* = 2.3 Hz, 1H, CH), 6.64 (ddd, *J* = 7.8, 2.4, 1.2 Hz, 1H, CH), 6.12 - 5.97 (m, 1H, CH), 4.28 - 4.13 (m, 2H, CH₂), 3.72 (t, *J* = 5.6 Hz, 2H, CH₂), 3.00 (s, 3H, CH₃), 2.88 (s, 3H, CH₃), 2.67 (brs, 2H, CH₂), 2.22 (s, 3H, CH₃). HRMS (EI-MS) m/z calcd for C₂₈H₂₉N₆O₄ [M+H]⁺: 513.2245, found: 513.2235.

### N-(3-iodophenyl)acetamide (217)

A solution of 3-iodoaniline (329 µL, 2.74 mmol, 1.0 equiv.) in acetic anhydride (5.5 mL) was stirred at rt for 4 h. The reaction mixture was poured into cold water, neutralized with KOH 1M and extracted with DCM (4 x 50 mL). The combined organic layers were dried over MgSO₄, filtered and concentrated under reduced pressure to afford compound **217** (714 mg, quant.) as a brown solid. The obtained product was used in the next step without further purification. ¹H NMR (250 MHz, CDCl₃) δ 7.90 (s, 1H, CH), 7.45 (t, *J* = 8.9 Hz, 2H, 2 x CH), 7.27 (brs, 1H, NH), 7.03 (t, *J=* 8.0 Hz, 1H, CH), 2.17 (s, 3H, CH₃).

All spectra data corresponded to the literature values as found in Linstad E. J. et al., Org. Biomol. Chem., 2017, 15, 2246-2252.

### N-(3-(3-aminophenoxy)phenyl)acetamide (218)

The reaction was carried out as described in general procedure **P** using iodoaryl **217** (522 mg, 2.00 mmol, 1.0 equiv.), 3-aminophenol (262 mg, 2.40 mmol, 1.2 equiv.), copper(I) iodide (19 mg, 0.10 mmol, 5 mol%), 2-picolinic acid (25 mg, 0.20 mmol, 10 mol%) and K₃PO₄ (849 mg, 4.00 mmol, 2.0 equiv.) in dry DMSO (4 mL). The crude reaction mixture was purified by silica gel column chromatography using a gradient solvent system of PE/EtOAc from 80/20 up to 50/50 to afford compound **218** (320 mg, 66%) as a brown oil. R*_{f}* (PE/EtOAc 50/50) 0.16. ¹H NMR (250 MHz, DMSO-d₆) δ 9.98 (s, 1H, NH), 7.36 - 7.19 (m, 3H, 3 x CH), 6.99 (t, *J* = 8.0 Hz, 1H, CH), 6.65 (ddd, *J* = 7.4, 2.3, 1.6 Hz, 1H, CH), 6.31 (ddd, *J* = 8.0, 2.1, 1.0 Hz, 1H, CH), 6.22 - 6.08 (m, 2H, 2 x CH), 5.24 (s, 2H, NH₂), 2.00 (s, 3H, CH₃). HRMS (EI-MS) m/z calcd for C₁₄H₁₅N₂O₂ [M+H]⁺: 243.1128, found: 243.1134.

### Example 110: N-(3-(3-acetamidophenoxy)phenyl)-4-(5-methyl-7H-pyrrolo[2,3-d]pyrimidin-4-yl)-3,6-dihydropyridine-1(2H)-carboxamide (219)

The reaction was carried out as described in general procedure **H** using aniline **218** (100 mg, 0.41 mmol, 1.0 equiv.), 4-nitrophenyl chloroformate (100 mg, 0.50 mmol, 1.2 equiv.), DIPEA (260 µL, 1.49 mmol, 2.4 equiv.) and amine **4** (143 mg, 0.50 mmol, 1.2 equiv.) in dry THF (6.0 mL). The crude reaction mixture was purified by silica gel column chromatography using a gradient solvent system of DCM/MeOH from 98/2 up to 94/6 to afford compound **219** (46 mg, 24%) as an white powder after co-evaporation with ethanol. R*_{f}* (DCM/MeOH 95/5) 0.30. Mp: degradation 156 °C. ¹H NMR (250 MHz, DMSO-d₆) δ 11.84 (s, 1H, NH), 10.00 (s, 1H, NH), 8.68 (s, 1H, NH), 8.64 (s, 1H, CH), 7.50 - 7.17 (m, 7H, 7 x CH), 6.77 - 6.56 (m, 2H, 2 x CH), 6.03 (brs, 1H, CH), 4.20 (s, 2H, CH₂), 3.71 (t, *J* = 5.6 Hz, 2H, CH₂), 2.66 (brs, 2H, CH₂), 2.22 (s, 3H, CH₃), 2.01 (s, 3H, CH₃). HRMS (EI-MS) m/z calcd for C₂₇H₂₇N₆O₃ [M+H]⁺: 483.2139, found: 483.2135.

### 3-iodo-N-methylbenzamide (220)

Under argon atmosphere, 3-iodobenzoic acid (400 mg, 1.61 mmol, 1.0 equiv.), DMAP (236 mg, 1.93 mmol, 1.2 equiv.) and HATU (734 mg, 1.93 mmol, 1.2 equiv.) were suspended in dry THF (16 mL) followed by the addition of DIPEA (336 µL, 1.93 mmol, 1.2 equiv.). The resulting suspension was stirred at rt for 20 min then methylamine hydrochloride (130 mg, 1.93 mmol, 1.2 equiv.) was added, and the reaction mixture was stirred at rt for 30 min and concentrated to dryness. The residue was dissolved in DCM (50 mL), the organic layer was washed with 1M HCl (15 mL), H₂O (15 mL), a saturated solution of NaHCO₃ (15 mL), H₂O (4 x 30 mL) and brine (30 mL). The organic layer was dried over MgSO₄, filtered and concentrated under reduced pressure to afford compound **220** (328 mg, 78%) as a white solid. The obtained product was used in the next step without further purification. ¹H NMR (250 MHz, DMSO-d₆) δ 8.52 (brs, 1H, NH), 8.17 (t, *J=* 1.6 Hz, 1H, CH), 7.85 (dddd, *J* = 10.8, 7.8, 1.7, 1.1 Hz, 2H, 2 x CH), 7.27 (td, *J* = 7.8, 0.4 Hz, 1H, CH), 2.77 (d, *J* = 4.6 Hz, 3H, CH₃). All spectra data corresponded to the literature values as found Sun X. et al., Chem. Commun., 2020, 56, 1298-1301.

### 3-(3-aminophenoxy)-N-methylbenzamide (221)

The reaction was carried out as described in general procedure **P** using iodoaryl **220** (274 mg, 1.05 mmol, 1.0 equiv.), 3-aminophenol (138 mg, 1.26 mmol, 1.2 equiv.), copper(I) iodide (10 mg, 0.05 mmol, 5 mol%), 2-picolinic acid (14 mg, 0.11 mmol, 10 mol%) and K₃PO₄ (446 mg, 2.10 mmol, 2.0 equiv.) in dry DMSO (2.1 mL). The crude reaction mixture was purified by silica gel column chromatography using a gradient solvent system of DCM/MeOH from 100 up to 98/2 to afford compound **221** (184 mg, 72%) as an orange oil. R*_{f}* (DCM/MeOH 95/5) 0.29. ¹H NMR (250 MHz, CDCl₃) δ 7.44 (dt, *J* = 7.7, 1.4 Hz, 1H, CH), 7.38 - 7.35 (m, 1H, CH), 7.30 (t, *J* = 7.9 Hz, 1H, CH), 7.12 - 7.01 (m, 2H, 2 x CH), 6.57 (brs, 1H, NH), 6.40 (ddd, *J* = 8.0, 2.2, 0.9 Hz, 1H, CH), 6.34 (ddd, *J* = 8.0, 2.3, 0.9 Hz, 1H, CH), 6.26 (t, *J* = 2.2 Hz, 1H, CH), 3.73 (brs, 2H, NH₂), 2.91 (d, *J=* 4.8 Hz, 3H, CH₃). HRMS (EI-MS) m/z calcd for C₁₄H₁₅N₂O₂ [M+H]⁺: 243.1128, found: 243.1132.

### Example 111: 4-(5-methyl-7H-pyrrolo[2,3-d]pyrimidin-4-yl)-N-(3-(3-(methylcarbamoyl)phenoxy)phenyl)-3,6-dihydropyridine-1(2H)-carboxamide (222)

The reaction was carried out as described in general procedure **H** using aniline **221** (76 mg, 0.29 mmol, 1.0 equiv.), 4-nitrophenyl chloroformate (70 mg, 0.35 mmol, 1.2 equiv.), DIPEA (182 µL, 1.05 mmol, 2.4 equiv.) and amine **4** (100 mg, 0.35 mmol, 1.2 equiv.) in dry THF (4.0 mL). The crude reaction mixture was purified by silica gel column chromatography using a gradient solvent system of DCM/MeOH from 98/2 up to 95/5 to afford compound **222** (57 mg, 41%) as an white powder. R*_{f}* (DCM/MeOH 95/5) 0.31. Mp: degradation 190 °C. ¹H NMR (250 MHz, DMSO-d₆) δ 11.84 (s, 1H, NH), 8.70 (brs, 1H, NH), 8.64 (s, 1H, CH), 8.47 (d, *J* = 4.7 Hz, 1H, NH), 7.60 (dt, *J* = 7.8, 1.3 Hz, 1H, CH), 7.54 - 7.42 (m, 2H, 2 x CH), 7.37 - 7.22 (m, 5H, 4 x CH), 7.17 (ddd, *J* = 8.0, 2.6, 1.1 Hz, 1H, CH), 6.67 - 6.61 (m, 1H, CH), 6.07- 5.99 (m, 1H, CH), 4.20 (d, *J=* 3.1 Hz, 2H, CH₂), 3.71 (t, *J=* 5.6 Hz, 2H, CH₂), 2.76 (d, *J=* 4.4 Hz, 3H, CH₃), 2.66 (brs, 2H, CH₂), 2.21 (s, 3H, CH₃). HRMS (EI-MS) m/z calcd for C₂₇H₂₇N₆O₃ [M+H]⁺: 483.2139, found: 483.2148.

### SUBFAMILY 1.2.

### 3-methyl pyrolopyrimidine derivatives with a substituted hydropyridine

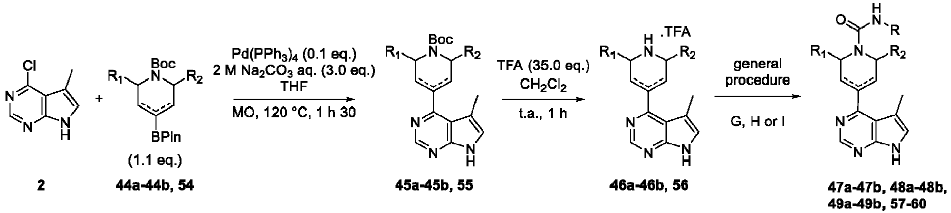

| compound | General procedure | Yield (%) | R₁ | R₂ | R |
|---|---|---|---|---|---|
| **47a-47b** | G | 84 | CH₃ | H | 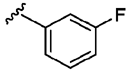 |
| **48a-48b** | G | 56 | CH₃ | H | 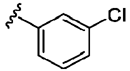 |
| **49a-49b** | G | 71 | CH₃ | H | 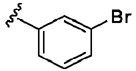 |
| **57** | G | 54 | CH₃ | CH₃ | 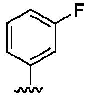 |
| **58** | G | 73 | CH₃ | CH₃ | 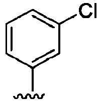 |
| **59** | G | 71 | CH₃ | CH₃ | 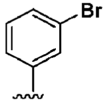 |
| **60** | G | 49 | CH₃ | CH₃ | 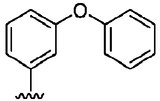 |

### tert-Butyl 6-methyl-4-(1,1,2,2,3,3,4,4,4-nonafluorobutylsulfonyloxy)-3,6-dihydro-2H-pyridine-1-carboxylate (43a) and tert-Butyl 2-methyl-4-(1,1,2,2,3,3,4,4,4-nonafluorobutylsulfonyl oxy)-3,6-dihydro-2H-pyridine-1-carboxylate (43b)

DBU (1.96 mL, 13.1 mmol, 1.4 equiv.) and perfluoro-1-butanesulfonyl fluoride (1.68 mL, 9.4 mmol) was added dropwise to a solution of *N*-Boc-2-methyl-piperidone (2.0 g, 9.4 mmol) in anhydrous THF (60 mL) at 0°C under argon atmosphere. The mixture was allowed to reach room temperature and stirred overnight. TLC monitoring the next day was performed to ensure complete conversion (if not complete DBU (0.25 equiv.) and perfluoro-1-butanesulfonyl fluoride (0.5 equiv.) can be added every 1.5 h). The reaction mixture was then treated with a saturated aqueous solution of NaHCO₃ (50 mL) and extracted with Et₂O (2 x 100 mL). The combined organic layers were dried over MgSO₄, filtered and concentrated under reduced pressure. The residue was purified by flash silica gel column chromatography using a gradient solvent system of PE/EtOAc from 100/0 up to 95/5 to afford compounds **43a-43b** (4.03 g, 87%) as a colorless oil containing a mixture of diastereoisomeres (7/3). R*_{f}* (PE/EtOAc 70/30) 0.37. Product **43a** (main product) ¹H NMR (250 MHz, CDCl₃) δ 5.72 (m, 1H, CH), 4.78 - 4.57 (m, 1H, CH), 4.32 - 4.15 (m, 1H, CH), 3.08 - 2.89 (m, 1H, CH), 2.67 - 2.48 (m, 1H, CH), 2.26 - 2.13 (m, 1H, CH), 1.47 (s, 9H, 3 xC H₃), 1.23 (d, *J=* 6.8 Hz, 3H, CH₃). Product **43b** ¹H NMR (250 MHz, CDCl₃) δ 5.76 (m, 1H, CH), 4.78 - 4.57 (m, 1H, CH), 4.51 - 4.34 (m, 1H, CH), 3.63 (ddt, *J* = 18.8 Hz, *J* = 4.7 Hz, *J* = 2.5 Hz, 1H, CH), 2.87 - 2.72 (m, 1H, CH), 2.11 - 2.00 (m, 1H, CH), 1.46 (s, 9H, 3 x CH₃), 1.17 (d, *J* = 6.9 Hz, 3H, CH₃). HRMS (EI-MS) m/z calcd for C₁₅H₁₉F₉NO₅S [M+H]⁺: 496.0835, found: 496.0835.

### tert-Butyl 6-methyl-4-(4,4,5,5-tetramethyl-1,3,2-dioxaborolan-2-yl)-3,6-dihydropyridine-1(2H)-carboxylate (44a) and tert-Butyl 2-methyl-4-(4,4,5,5-tetramethyl-1,3,2-dioxaborolan-2-yl)-3,6-dihydropyridine-1(2H)-carboxylate (44b)

To a solution of **43a-43b** (5.0 g, 10.0 mmol) in 1,4-dioxane (50 mL) were added bis(pinacolato)diboron (2.9 g, 11.5 mmol, 1.15 equiv.), potassium acetate (3.5 g, 35.2 mmol, 3.5 equiv.) and dppf (170 mg, 0.3 mmol, 0.03 equiv.). The solution was degassed for 15 min and Pd(dppf)Cl₂·DCM (245 mg, 0.3 mmol, 0.03 equiv.) was added. The mixture was then heated at 80°C overnight, cooled and extracted with EtOAc (2 x 200 mL). The combined organic layers were washed with brine (100 mL), dried over MgSO₄, filtered and concentrated under vacuum. The crude residue was purified by silica gel column chromatography using a solvent system of PE/EtOAc 95/5 to afford compounds **44b-44b** (3.77 g, 83 %) as a colorless oil containing a mixture of diastereoisomeres (7/3). R*_{f}* (PE/EtOAc 90/10) 0.19. Product **44a** (main) ¹H NMR (250 MHz, CDCl₃) δ 6.41 - 6.33 (m, 1H, CH), 4.53 - 4.34 (m, 1H, CH), 4.12 - 3.94 (m, 1H, CH), 2.82 - 2.63 (m, 1H, CH), 2.26 - 2.09 (m, 2H, 2 x CH), 1.44 (s, 9H, 3 x CH₃), 1.25 (s, 12H, 4 x CH₃), 1.17 (d, *J* = 6.9 Hz, 3H, CH₃). Product **44b** ¹H NMR (250 MHz, CDCl₃) δ 6.48 - 6.42 (m, 1H, CH), 4.53 - 4.34 (m, 1H, CH), 4.20 (dt, *J* = 20.2 Hz, *J* = 3.8 Hz, 1H, CH), 3.66 - 3.51 (m, 1H, CH), 2.50 - 2.34 (m, 1H, CH), 2.09 - 1.98 (m, 1H, CH), 1.44 (s, 9H, 3 x CH₃), 1.25 (s, 12H, 4 x CH₃), 1.04 (d, *J* = 6.8 Hz, 3H, CH₃). HRMS (EI-MS) m/z calcd for C₁₇H₃₁BNO₄ [M+H]⁺: 324.2341, found: 324.2342.

### tert-Butyl 6-methyl-4-(5-methyl-7H-pyrrolo[2,3-d]pyrimidin-4-yl)-3,6-dihydropyridine-1(2H)-carboxylate (45a) and tert-Butyl 2-methyl-4-(5-methyl-7H-pyrrolo[2,3-d]pyrimidin-4-yl)-3,6-dihydropyridine-1(2H)-carboxylate (45b)

The reaction was carried out as described in general procedure **D** using the chlorinated bicycle **2** (0.4 g, 2.4 mmol) and the boronate ester **44b-44b** (850 mg, 2.6 mmol, 1.1 equiv.) in THF (12 mL). The crude mixture was purified by silica gel column chromatography using a gradient solvent system of CH₂Cl₂/acetone from 90/10 up to 70/30 to afford compounds **45a-45b** (1.25 g, 80%) as a white solid containing a mixture of diastereoisomeres (7/3). R*_{f}* (PE/EtOAc 70/30) 0.18. Mp: 161 - 163°C. Product **35a** (main) ¹H NMR (250 MHz, MeOD-*d₄*) δ 8.59 (s, 1H, CH), 7.18 (d, *J* = 1.0 Hz, 1H, CH), 5.90 - 5.86 (m, 1H, CH), 4.76 - 4.57 (m, 1H, CH), 4.22 (dd, *J* = 13.3 Hz, *J* = 4.8 Hz, 1H, CH), 3.21 - 3.00 (m, 1H, CH), 2.70 - 2.52 (m, 1H, CH), 2.51 - 2.37 (m, 1H, CH), 2.24 (d, *J* = 1.1 Hz, 3H, CH₃), 1.49 (s, 9H, 3 x CH₃), 1.31 (d, *J=* 6.8 Hz, 3H, CH₃). Product **35b** ¹H NMR (250 MHz, MeOD-*d₄*) δ 8.59 (s, 1H, CH), 7.18 (d, *J=* 1.0 Hz, 1H, CH), 5.95 - 5.90 (m, 1H, CH), 4.76-4.57 (m, 1H, CH), 4.42 (dt, *J* = 19.5 Hz, *J* = 3.3 Hz, 1H, CH), 3.87 - 3.69 (m, 1H, CH), 2.94 - 2.77 (m, 1H, CH), 2.51 - 2.37 (m, 1H, CH), 2.24 (d, *J* = 1.1 Hz, 3H, CH₃), 1.49 (s, 9H, 3xCH₃), 1.25 (d, *J* = 6.8 Hz, 1H, CH₃). HRMS (EI-MS) m/z calcd for C₁₈H₂₅N₄O₂ [M+H]⁺: 329.1972, found: 329.1972.

### 5-Methyl-4-(6-methyl-1,2,3,6-tetrahydropyridin-4-yl)-7H-pyrrolo[2,3-d]pyrimidine trifluoroacetate (46a) and 5-Methyl-4-(2-methyl-1,2,3,6-tetrahydropyridin-4-yl)-7H-pyrrolo[2,3-d]pyrimidine trifluoroacetate (46b)

The reaction was carried out as described in general procedure **F2** using *N*-boc protected compound **45b-45b** (0.6 g, 1.8 mmol) and TFA (4.9 mL, 63.9 mmol, 35.0 equiv.) in CH₂Cl₂ (50 mL). The reaction was complete after 1 h. Workup gave compounds **46a-46b** (0.44 g, 72 %) as an offwhite solid containing a mixture of diastereoisomeres (7/3). Mp: 199 - 201°C. Product **46a** (main) ¹H NMR (250 MHz, MeOD-*d₄*) δ 8.60 (s, 1H, CH), 7.21 (d, *J* = 1.2 Hz, 1H, CH), 5.92 - 5.85 (m, 1H, CH), 3.70 - 3.58 (m, 1H, CH), 3.34 - 3.21 (m, 1H, CH), 3.10 - 2.95 (m, 1H, CH), 2.61 - 2.50 (m, 2H, 2 x CH), 2.32 (d, *J=* 1.2 Hz, 3H, CH₃), 1.31 (d, *J=* 7.0 Hz, 3H, CH₃). ¹⁹F NMR (235 MHz, MeOD-*d₄*) δ -76.9 (s). Product **46b** ¹H NMR (250 MHz, MeOD-*d₄*) δ 8.60 (s, 1H, CH), 7.21 (d, *J* = 1.2 Hz, 1H, CH), 6.03 - 5.95 (m, 1H, CH), 3.70 - 3.58 (m, 1H, CH), 3.33 - 3.29 (m, 1H, CH), 3.10 - 2.95 (m, 1H, CH), 2.71 - 2.59 (m, 1H, CH), 2.30 (d, *J* = 1.2 Hz, 3H, CH₃), 2.28 - 2.14 (m, 1H, CH), 1.26 (d, *J* = 7.0 Hz, 3H, CH₃). ¹⁹F NMR (235 MHz, MeOD-*d₄*) δ -76.9 (s). HRMS (EI-MS) m/z calcd for C₁₃H₁₈N₄[M+H]⁺: 229.1448, found: 229.1447.

### Example 30: N-(3-fluorophenyl)-6-methyl-4-(5-methyl-7H-pyrrolo[2,3-d]pyrimidin-4-yl)-3,6-dihydropyridine-1(2H)-carboxamide (47a) and N-(3-fluorophenyl)-2-methyl-4-(5-methyl-7H-pyrrolo[2,3-d]pyrimidin-4-yl)-3,6-dihydropyridine-1(2H)-carboxamide (47b)

The reaction was carried out as described in general procedure **G** using amine **46a-46b** (70 mg, 0.20 mmol) in anhydrous CH₂Cl₂ (5 mL). The crude reaction mixture was purified by silica gel column chromatography using a gradient solvent system CH₂Cl₂/acetone from 90/10 up to 70/30 to afford compounds **47a-47b** (61 mg, 84%) as a white solid containing a mixture of diastereoisomers (7/3). R*_{f}* (CH₂Cl₂/acetone 50/50) 0.28. Mp: 154 - 156°C. Product **47a** (main) ¹H NMR (400 MHz, MeOD-*d₄*) δ 8.62 (s, 1H, CH), 7.32 (dt, *J* = 11.6 Hz, *J* = 2.2 Hz, 1H, CH), 7.29 - 7.22 (m, 2H, 2 x CH), 7.19 (ddd, *J* = 8.2 Hz, *J* = 2.1 Hz, *J* = 1.1 Hz, 1H, CH), 6.73 (tdd, *J* = 8.4 Hz, *J* = 2.5 Hz, J = 1.1 Hz, 1H, CH), 6.03 - 5.99 (m, 1H, CH), 4.96 - 4.88 (m, 1H, CH), 4.32 (dd, *J* = 13.7 Hz, *J* = 5.4 Hz, 1H, CH), 3.38 - 3.27 (m, 1H, CH), 2.83 - 2.69 (m, 1H, CH), 2.63 - 2.56 (m, 1H, CH), 2.32 (d, *J =* 1.2 Hz, 3H, CH₃), 1.42 (d, *J* = 6.8 Hz, 3H, CH₃). ¹⁹F NMR (376 MHz, MeOD-*d₄*) δ -115.0 (s). Product **47b** **¹**H NMR (400 MHz, MeOD-*d₄*) δ 8.63 (s, 1H, CH), 7.32 (dt, *J* = 11.6 Hz, *J* = 2.2 Hz, 1H, CH), 7.29 - 7.22 (m, 2H, 2 x CH), 7.19 (ddd, *J* = 8.2 Hz, *J* = 2.1 Hz, *J* = 1.1 Hz, 1H, CH), 6.73 (tdd, *J* = 8.4 Hz, *J* = 2.5 Hz, *J* = 1.1 Hz, 1H, CH), 6.07 - 6.04 (m, 1H, CH), 4.87 - 4.79 (m, 1H, CH), 4.54 (dt, *J* = 19.0 Hz, *J* = 3.7 Hz, 1H, CH), 4.07 - 3.89 (m, 1H, CH), 3.07 - 2.92 (m, 1H, CH), 2.58 - 2.51 (m, 1H, CH), 2.32 (d, *J =* 1.2 Hz, 3H, CH₃), 1.36 (d, *J* = 6.8 Hz, 3H, CH₃). ¹⁹F NMR (376 MHz, MeOD-*d₄*) δ -76.9 (s). HRMS (EI-MS) m/z calcd for C₂₀H₂₁FN₅O [M+H]⁺: 366.1725, found: 366.1723.

### Example 31: N-(3-Chlorophenyl)-6-methyl-4-(5-methyl-7H-pyrrolo[2,3-d]pyrimidin-4-yl)-3,6-dihydropyridine-1(2H)-carboxamide (48a) and N-(3-Chlorophenyl)-2-methyl-4-(5-methyl-7H-pyrrolo[2,3-d]pyrimidin-4-yl)-3,6-dihydropyridine-1(2H)-carboxamide (48 b)

The reaction was carried out as described in general procedure **G** using amine **46a-46b** (70 mg, 0.20 mmol) in anhydrous CH₂Cl₂ (5 mL). The crude reaction mixture was purified by silica gel column chromatography using a gradient solvent system CH₂Cl₂/acetone from 90/10 up to 70/30 to afford compounds **48a-48b** (43 mg, 56 %) as a white solid containing a mixture of diastereoisomers (7/3). R*_{f}* (CH₂Cl₂/acetone 50/50) 0.31. Mp: 159 - 161°C. Product **48a** (main) ¹H NMR (400 MHz, MeOD-*d₄*) δ 8.62 (s, 1H, CH), 7.56 (t, *J* = 2.0 Hz, 1H, CH), 7.36 - 7.30 (m, 1H, CH), 7.27 - 7.19 (m, 2H, 2 x CH), 7.04 - 6.93 (m, 1H, CH), 6.02 - 5.97 (m, 1H, CH), 4.97 - 4.86 (m, 1H, CH), 4.31 (dd, *J* = 13.7 Hz, *J* = 5.3 Hz, 1H, CH), 3.38 - 3.26 (d, *J* = 3.8 Hz, 1H, CH), 2.83 - 2.69 (m, 1H, CH), 2.63 - 2.57 (m, 1H, CH), 2.34 - 2.29 (m, 3H, CH₃), 1.42 (d, *J=* 6.7 Hz, 3H, CH₃). Product **48b** ¹H NMR (400 MHz, MeOD-*d₄*) δ 8.63 (s, 1H, CH), 7.56 (t, *J* = 2.0 Hz, 1H, CH), 7.36 - 7.30 (m, 1H, CH), 7.27 - 7.19 (m, 2H, 2 x CH), 7.04 - 6.93 (m, 1H, CH), 6.08 - 6.03 (m, 1H, CH), 4.85 - 4.77 (m, 1H, CH), 4.54 (dt, *J* = 18.9 Hz, *J=* 3.6 Hz, 1H, CH), 4.03 - 3.91 (m, 1H, CH), 3.05 - 2.94 (m, 1H, CH), 2.57 - 2.50 (m, 1H, CH), 2.34 - 2.29 (m, 3H, CH₃), 1.36 (d, *J* = 6.7 Hz, 3H, CH₃). HRMS (EI-MS) m/z calcd for C₂₀H₂₁ClN₅O [M+H]⁺: 382.1429, found: 382.1426.

### Example 32: N-(3-bromophenyl)-6-methyl-4-(5-methyl-7H-pyrrolo[2,3-d]pyrimidin-4-yl)-3,6-dihydropyridine-1(2H)-carboxamide (49a) and N-(3-bromophenyl)-2-methyl-4-(5-methyl-7H-pyrrolo[2,3-d]pyrimidin-4-yl)-3,6-dihydropyridine-1(2H)-carboxamide (49b)

The reaction was carried out as described in general procedure **G** using amine **46a-46b** (70 mg, 0.20 mmol) in anhydrous CH₂Cl₂ (5 mL). The crude reaction mixture was purified by silica gel column chromatography using a gradient solvent system CH₂Cl₂/acetone from 90/10 up to 70/30 to afford compounds **49a-49b** (61 mg, 71%) as a white solid containing a mixture of diastereoisomers (7/3). R*_{f}* (CH₂Cl₂/acetone 50/50) 0.31. Mp: 159 - 161°C. Product **49a** (main) ¹H NMR (400 MHz, MeOD-*d₄*) δ 8.62 (s, 1H, CH), 7.72 - 7.70 (m, 1H, CH), 7.37 (dt, *J* = 7.3 Hz, *J=* 2.1 Hz, 1H, CH), 7.25 - 7.22 (m, 1H, CH), 7.21 - 7.13 (m, 2H, 2 x CH), 6.03 - 5.99 (m, 1H, CH), 4.96 - 4.88 (m, 1H, CH), 4.31 (dd, *J* = 13.7 Hz, *J=* 5.3 Hz, 1H, CH), 3.38 - 3.26 (m, 1H, CH), 2.82 - 2.69 (m, 1H, CH), 2.64 - 2.51 (m, 1H, CH), 2.32 (d, *J =* 1.3 Hz, 3H, CH₃), 1.42 (d, *J =* 6.7 Hz, 3H, CH₃). Product **49b** ¹H NMR (400 MHz, MeOD-*d₄*) δ 8.63 (s, 1H, CH), 7.72 - 7.70 (m, 1H, CH), 7.37 (dt, *J* = 7.3 Hz, *J=* 2.1 Hz, 1H, CH), 7.25 - 7.22 (m, 1H, CH), 7.21 - 7.13 (m, 2H, 2 x CH), 6.08 - 6.03 (m, 1H, CH), 4.87 - 4.74 (m, 1H, CH), 4.54 (dt, *J* = 18.9 Hz, *J* = 3.6 Hz, 1H, CH), 4.04 - 3.92 (m, 1H, CH), 3.06 - 2.93 (m, 1H, CH), 2.64 - 2.51 (m, 1H, CH), 2.32 (m, 3H, CH₃), 1.36 (d, *J* = 6.8 Hz, 3H, CH₃). HRMS (EI-MS) m/z calcd for C₂₀H₂₁BrN₅O [M+H]⁺: 426.0924, found: 426.0921.

### Dimethyl 2,6-dimethyl-4-oxopiperidine-3,5-dicarboxylate (50)

Ammonia gas was bubbled through a mixture of dimethyl-1,3-acetonedicarboxylate (19.0 g, 109 mmol) and acetaldehyde (12.5 mL, 284 mmol, 2.6 equiv.) at -30°C until the liquid was saturated. The solution was stored in the freezer (4°C) for 20 h. After solvent evaporation under reduced pressure the yellow residue was purified by silica gel column chromatography using a gradient solvent system of PE/EtOAc from 100/0 up to 70/30 to afford compound **50** (14.0 g, 53%) as an orange oil. ¹H NMR (250 MHz, CDCl₃) δ 3.74 - 3.65 (m, 8H, 2 x CH + 2 x OCH₃), 2.97 - 2.91 (m, 2H, 2 x CH), 1.26 - 1.23 (d, *J* = 6.6 Hz, 3H, CH₃), 1.15 - 1.09 (d, *J* = 6.6 Hz, 3H, CH₃)(see WO2015158283, 2015, pp 172).

### 2,6-Dimethylpiperidin-4-one hydrochloride (51)

A solution of **50** (14.0 g, 65 mmol) in a 10% aqueous HCl solution (100 mL) was heated to 110°C for 24 h. The solution was cooled and the solvent was removed under reduced pressure to afford compound **51** (8.5 g, 80%) as an orange solid which was used in the next step without further purification. ¹H NMR (250 MHz, DMSO-*d₆*) δ 10.11 - 9.79 (m, 2H, NH + H⁺), 3.91 - 3.74 (m, 1H, CH), 3.61 - 3.43 (m, 1H, CH), 2.77 - 2.60 (m, 2H, CH₂), 2.48 - 2.35 (m, 2H, CH₂), 1.35 (d, *J* = 6.4 Hz, 3H, CH₃), 1.30 (d, *J=* 6.7 Hz, 3H, CH₃). WO2015158283, 2015, pp 172.

### tert-Butyl 2,6-dimethyl-4-oxopiperidine-1-carboxylate (52)

Na₂CO₃ (11.0 g, 104 mmol, 2.0 equiv.) was added portionwise to a solution of **51** (8.5 g, 52 mmol) in 1,4-dioxane (70 mL) and H₂O (70 mL). Boc₂O (22.7 g, 104 mmol, 2.0 equiv.) was then added and the resulting reaction mixture was stirred at room temperature for 24 h. The solvent was removed under reduced pressure and the resulting aqueous solution was extracted with Et₂O (3 x 200 mL). The combined organic layers were washed with brine (100 mL), dried over MgSO₄, filtered and concentrated under vacuum. The crude mixture was purified by silica gel column chromatography using a gradient solvent system of PE/EtOAc from 97/3 up to 80/20 to afford compound **122 52** (2.5 g, 21 %) as an off-white solid containing a mixture of diastereoisomers (Trans/Cis, 9/1). Main isomer (trans) **52:** ¹H NMR (250 MHz, CDCl₃) δ 4.44 - 4.23 (m, 2H, 2 x CH), 2.83 (dd, *J* = 17.8 Hz, *J* = 6.4 Hz, 2H, 2 x CH), 2.35 (dd, *J* = 17.8 Hz, *J* = 1.9 Hz, 2H, 2 x CH), 1.48 (s, 9H, 3 x CH₃), 1.23 (dd, *J=* 6.8 Hz, *J=* 0.7 Hz, 6H, 2 x CH₃). WO2015158283, 2015, pp 172.

### tert-Butyl 2,6-dimethyl-4-(((trifluoromethyl)sulfonyl)oxy)-3,6-dihydropyridine-1(2H)-carboxylate (53)

A 1 M solution of NaHMDS in THF (5.7 mL, 5.72 mmol, 1.3 equiv.) was added dropwise to a solution of **52** (1.0 g, 4.40 mmol) in anhydrous THF (25 mL) at -78°C under argon. The solution was stirred at this temperature for 1 h and a solution of *N-*phenyl-bis(trifluoromethanesulfonimide) (1.89 g, 5.28 mmol, 1.2 equiv.) in THF (12 mL) was then slowly added. The reaction mixture was allowed to reach room temperature and stirred for 2.5 h. The solvent was removed under reduced pressure and the residue was taken up in Et₂O (40 mL) and washed with cold water (2 x 10 mL). The ether layers were dried with MgSO₄, filtered and concentrated under vacuum. The residue was purified by silica gel column chromatography using a solvent system of PE/EtOAc 99/1 to afford compound **53** (0.98 g, 62%) as an off-white solid containing the trans product as an enantiomeric mixture. ¹H NMR (250 MHz, CDCl₃) δ 5.82 - 5.77 (m, 1H, CH), 4.42 - 4.27 (m, 2H, 2 x CH), 2.91 - 2.77 (m, 1H, CH), 2.17 (dd, *J* = 16.5 Hz, *J* = 2.8 Hz, 1H, CH), 1.48 (s, 9H, 3 x CH₃), 1.36 (d, *J* = 6.3 Hz, 3H, CH₃), 1.25 - 1.21 (d, *J* = 6.3 Hz, 3H, CH₃). Heterocycles, 1996, 43, 10, 2131-2138.

### tert-Butyl 2,6-dimethyl-4-(4,4,5,5-tetramethyl-1,3,2-dioxaborolan-2-yl)-3,6-dihydropyridine-1(2H)-carboxylate (54)

To a solution of **53** (1.0 g, 2.78 mmol) in 1,4-dioxane (20 mL) were added bis(pinacolato)diboron (0.78 g, 3.06 mmol, 1.1 equiv.), potassium acetate (0.82 g, 8.34 mmol, 3.0 equiv.) and dppf (46 mg, 0.08 mmol, 0.03 equiv.). The solution was degassed for 15 min and Pd(dppf)Cl₂·CH₂Cl₂ (68 mg, 0.08 mmol, 0.03 equiv.) was added. The mixture was then heated to 80°C for 12 h, cooled and extracted with EtOAc (2 x 100 mL). The organic layers were washed with brine (100 mL), dried over MgSO₄, filtered and concentrated under vacuum. The residue was purified by silica gel column chromatography using a gradient solvent system of PE/EtOAc from 95/5 up to 70/30 to afford compound **54** (0.72 g, 77 %) as a white solid containing the trans product as an enantiomeric mixture. ¹H NMR (250 MHz, CDCl₃) δ 6.55 (dd, *J* = 5.0 Hz, *J* = 2.9 Hz, 1H CH), 4.25 - 4.03 (m, 2H, 2 x CH), 2.41 - 2.27 (m, 1H, CH), 2.14 (ddd, J= 16.0 Hz, *J* = 2.4 Hz, *J* = 0.9 Hz, 1H, CH), 1.44 (s, 9H, 3 x CH₃), 1.26 - 1.21 (m, 15H, 5xCH₃), 1.02 (dd, *J* = 6.5 Hz, *J* = 0.6 Hz, 3H, CH₃). WO2008016534, 2008, 133 pp.

### tert-Butyl 2,6-dimethyl-4-(5-methyl-7H-pyrrolo[2,3-d]pyrimidin-4-yl)-3,6-dihydropyridine-1(2H)-carboxylate (55)

The reaction was carried out as described in general procedure **D** using the chlorinated bicycle **2** (0.30 g, 1.80 mmol) and boronic acid pinacol ester **54** (0.67 g, 1.98 mmol, 1.1 equiv.) in THF (9 mL). The crude mixture was purified by silica gel column chromatography using a gradient solvent system of CH₂Cl₂/acetone from 100/0 up to 70/30 to afford compound **55** (0.43 g, 70 %) as a beige solid containing the trans product as an enantiomeric mixture. R*_{f}* (CH₂Cl₂/acetone 80/20) 0.26. Mp: degradation 119 °C. ¹H NMR (400 MHz, CDCl₃) δ 9.81 (br s, 1H, NH), 8.81 (s, 1H, CH), 7.19 - 6.97 (m, 1H, CH), 6.17 (dd, *J* = 5.2 Hz, *J=* 2.8 Hz, 1H, CH), 4.51 - 4.40 (m, 2H, 2 x CH), 3.06 - 2.95 (m, 1H, CH), 2.67 (dd, *J* = 15.9 Hz, *J* = 2.4 Hz, 1H, CH), 2.35 (d, *J =* 1.2 Hz, 3H, CH₃), 1.52 (s, 9H, 3xCH₃), 1.43 (d, *J =* 6.4 Hz, 3H, CH₃), 1.28 (d, *J* = 6.2 Hz, 3H, CH₃). HRMS (EI-MS) m/z calcd for C₁₉H₂₇N₄O₂ [M+H]⁺: 343.2129, found: 343.2128.

### 4-(2,6-Dimethyl-1,2,3,6-tetrahydropyridin-4-yl)-5-methyl-7H-pyrrolo[2,3-d]pyrimidine trifluoroacetate (56)

The reaction was carried out as described in general procedure **F1** using the *N*-Boc protected compound **55** (0.4 g, 1.17 mmol) in CH₂Cl₂ (32 mL). The reaction was complete after 1 h. Workup gave compound **56** (0.31 g, 74 %) as a light yellow solid containing the trans product as an enantiomeric mixture. Mp: degradation 136°C. ¹H NMR (400 MHz, DMSO-*d₆*) δ 11.80 (br s, 1H, NH), 8.60 (s, 1H, CH), 7.29 (s, 1H, CH), 5.93 (s, 1H, CH), 3.74 (s, 1H, CH), 3.63 (s, 2H, NH + H⁺), 3.22 (s, 1H, CH), 2.60 - 2.53 (m, 1H, CH), 2.29 - 2.19 (m, 4H, CH + CH₃), 1.23 (d, *J* = 6.9 Hz, 3H, CH₃), 1.16 (d, *J* = 5.6 Hz, 3H, CH₃). ¹⁹F NMR (376 MHz, DMSO-*d₆*) δ -73.5 (s). HRMS (EI-MS) m/z calcd for C₁₄H₁₉N₄ [M+H]⁺: 243.1604, found: 243.1603.

### Example 33: N-(3-Fluorophenyl)-2,6-dimethyl-4-(5-methyl-7H-pyrrolo[2,3-d]pyrimidin-4-yl)-3,6-dihydropyridine-1(2H)-carboxamide (57)

The reaction was carried out as described in general procedure **G** using amine **56** (60 mg, 0.25 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **57** (51 mg, 54 %) as a white solid containing the trans product as an enantiomeric mixture. R*_{f}* (CH₂Cl₂/acetone 80/20) 0.11. Mp: 210 - 212 °C. ¹H NMR (400 MHz, DMSO-*d₆*) δ 11.84 (br s, 1H, NH), 8.87 (br s, 1H, NH), 8.67 (s, 1H, CH), 7.48 (d, *J* = 12.0 Hz, 1H, CH), 7.34 (s, 1H, CH), 7.32 - 7.24 (m, 2H, 2 x CH), 6.75 (t, *J =* 8.1 Hz, 1H, CH), 6.24 - 6.12 (m, 1H, CH), 4.60 - 4.55 (m, 1H, CH), 4.55 - 4.48 (m, 1H, CH), 2.95 (d, *J* = 14.8 Hz, 1H, CH), 2.72 (d, *J* = 16.0 Hz, 1H, CH), 2.29 (s, 3H, CH₃), 1.37 (d, *J =* 6.3 Hz, 3H, CH₃), 1.19 (d, *J* = 6.3 Hz, 3H, CH₃). ¹⁹F NMR (376 MHz, DMSO-*d₆*) δ - 112.9 (s). HRMS (EI-MS) m/z calcd for C₂₁H₂₃FN₅O [M+H]⁺: 380.1881, found: 380.1880.

### Example 34: N-(3-Chlorophenyl)-2,6-dimethyl-4-(5-methyl-7H-pyrrolo[2,3-d]pyrimidin-4-yl)-3,6-dihydropyridine -1(2H)-carboxamide (58)

The reaction was carried out as described in general procedure **G** using amine **56** (60 mg, 0.25 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **58** (72 mg, 73%) as a white solid containing the trans product as an enantiomeric mixture. R*_{f}* (CH₂Cl₂/acetone 80/20) 0.10. Mp: 240 - 242°C. ¹H NMR (400 MHz, DMSO-*d₆*) δ 11.84 (br s, 1H, NH), 8.84 (br s, 1H, NH), 8.67 (s, 1H, CH), 7.70 (s, 1H, CH), 7.44 (d, *J* = 8.3 Hz, 1H, CH), 7.34 (s, 1H, CH), 7.27 (t, *J* = 8.1 Hz, 1H, CH), 6.99 (d, *J* = 7.9 Hz, 1H, CH), 6.23 - 6.10 (m, 1H, CH), 4.61 - 4.55 (m, 1H, CH), 4.55 - 4.49 (m, 1H, CH), 2.95 (d, *J* = 17.5 Hz, 1H, CH), 2.72 (d, *J* = 16.0 Hz, 1H, CH), 2.29 (s, 3H, CH₃), 1.37 (d, *J* = 6.2 Hz, 3H, CH₃), 1.19 (d, *J* = 6.3 Hz, 3H, CH₃). HRMS (EI-MS) m/z calcd for C₂₁H₂₃ClN₅O [M+H]⁺: 396.1586, found: 396.1585.

### Example 35: N-(3-Bromophenyl)-2,6-dimethyl-4-(5-methyl-7H-pyrrolo[2,3-d]pyrimidin-4-yl)-3,6-dihydropyridine-1(2H)-carboxamide (59)

The reaction was carried out as described in general procedure **G** using amine **56** (60 mg, 0.25 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **59** (77 mg, 71%) as a white solid containing the trans product as an enantiomeric mixture. R*_{f}* (CH₂Cl₂/acetone 80/20) 0.11. Mp: 238 - 240°C. ¹H NMR (400 MHz, DMSO-*d₆*) δ 11.84 (br s, 1H, NH), 8.83 (br s, 1H, NH), 8.67 (s, 1H, CH), 7.84 (t, *J* = 2.0 Hz, 1H, CH), 7.49 (d, *J* = 8.2 Hz, 1H, CH), 7.33 (s, 1H, CH), 7.21 (t, *J =* 8.0 Hz, 1H, CH), 7.12 (d, *J* = 8.0 Hz, 1H, CH), 6.22 - 6.11 (m, 1H, CH), 4.64 - 4.47 (m, 2H, 2 x CH), 3.00 - 2.88 (m, 1H, CH), 2.72 (d, *J* = 16.0 Hz, 1H, CH), 2.29 (s, 3H, CH₃), 1.36 (d, *J* = 6.3 Hz, 3H, CH₃), 1.19 (d, *J* = 6.3 Hz, 3H, CH₃). HRMS (EI-MS) m/z calcd for C₂₁H₂₃BrN₅O[M+H]⁺: 440.1080, found: 440.1081.

### Example 36: 2,6-Dimethyl-4-(5-methyl-7H-pyrrolo[2,3-d]pyrimidin-4-yl)-N-(3-phenoxyphenyl)-3,6-dihydro pyridine-1(2H)-carboxamide (60)

The reaction was carried out as described in general procedure **G** using amine **56** (60 mg, 0.25 mmol) in anhydrous CH₂Cl₂ (4 mL). The crude reaction mixture was purified by silica gel column chromatography using a gradient solvent system of CH₂Cl₂/acetone from 80/20 up to 30/70 to afford compound **60** (56 mg, 49%) as a white solid containing the trans product as an enantiomeric mixture. R*_{f}* (CH₂Cl₂/acetone 80/20) 0.15. Mp: 212 - 214°C. ¹H NMR (400 MHz, DMSO-*d₆*) δ 11.84 (br s, 1H, NH), 8.76 (br s, 1H, NH), 8.66 (s, 1H, CH), 7.47 - 7.22 (m, 6H, 6xCH), 7.13 (t, *J=* 7.2 Hz, 1H, CH), 7.02 (d, *J=* 7.9 Hz, 2H, 2 x CH), 6.60 (d, *J=* 8.0 Hz, 1H, CH), 6.15 (s, 1H, CH), 4.62 - 4.52 (m, 1H, CH), 4.52 - 4.44 (m, 1H, CH), 2.92 (d, *J=* 15.9 Hz, 1H, CH), 2.69 (d, *J=* 16.1 Hz, 1H, CH), 2.28 (s, 3H, CH₃), 1.34 (d, *J* = 6.2 Hz, 3H, CH₃), 1.17 (d, *J* = 6.3 Hz, 3H, CH₃). HRMS (EI-MS) m/z calcd for C₂₇H₂₈N₅O₂ [M+H]⁺: 454.2238, found: 454.2238.

### FAMILY 2.

**Unsubstituted pyrolopyrimidine derivatives with a hydropyridine.**

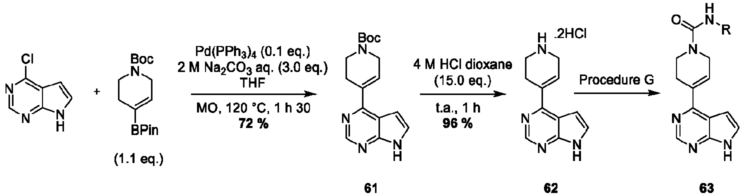

| Compound | General procedure | Yield (%) | R |
|---|---|---|---|
| **63** | G | 71 | 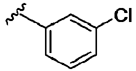 |

### tert-Butyl 4-(7H-pyrrolo[2,3-d]pyrimidin-4-yl)-3,6-dihydropyridine-1(2H)-carboxylate (61)

The reaction was carried out as described in general procedure **D** using 4-chloro-7*H*-pyrrolo[2,3-*d*]pyrimidine (0.60 g, 3.91 mmol) and *N*-Boc-1,2,3,6-tetrahydropyridine-4-boronic acid pinacol ester (1.3 g, 4.30 mmol, 1.1 equiv.) in THF (10 mL). The crude reaction mixture was purified by silica gel column chromatography using a gradient solvent system of CH₂Cl₂/acetone from 90/10 up to 50/50. To remove final impurities, the product was precipitated in a mixture of acetone/pentane to afford compound **61** (0.85 g, 72 %) as a pale yellow solid. R*_{f}* (CH₂Cl₂/acetone 70/30) 0.43. Mp: 159 - 161°C. ¹H NMR (400 MHz, MeOD-*d₄*) δ 8.64 (s, 1H, CH), 7.42 (d, *J* = 3.7 Hz, 1H, CH), 6.77 - 6.73 (m, 1H, CH), 6.72 (d, *J* = 3.6 Hz, 1H, CH), 4.21 - 4.15 (m, 2H, CH₂), 3.66 (t, *J* = 5.7 Hz, 2H, CH₂), 2.78 - 2.71 (m, 2H, CH₂), 1.49 (s, 9H, 3 x CH₃). HRMS (EI-MS) m/z calcd for C₁₆H₂₁N₄O₂ [M+H]⁺: 301.1659, found: 301.1659.

### 4-(1,2,3,6-Tetrahydropyridin-4-yl)-7H-pyrrolo[2,3-d]pyrimidine dihydrochloride (62)

The reaction was carried out as described in general procedure **J** with *N*-Boc protected compound **61** (100 mg, 0.28 mmol). The reaction mixture was stirred for 1 hour at room temperature. After completion, Et₂O (20 mL) was added and the resulting precipitate was filtered and washed with Et₂O (3 x 10 mL) to afford compound **62** (87 mg, 96 %) as a light brown solid. Mp: degradation > 260°C. ¹H NMR (400 MHz, DMSO-*d₆*) δ 12.91 (br s, 1H, NH), 9.63 (br s, 2H, NH + H⁺), 8.98 - 8.82 (m, 1H, CH), 7.93 - 7.76 (s, 1H, CH), 7.07 - 6.91 (m, 2H, 2 x CH), 4.57 - 4.08 (m, 1H, H⁺), 3.96 - 3.86 (s, 2H, CH₂), 3.44 - 3.26 (s, 2H, CH₂), 3.12 - 2.87 (s, 2H, CH₂). HRMS (EI-MS) m/z calcd for C₁₁H₁₃N₄[M+H]⁺: 201.1135, found: 201.1135.

### Example 37: N-(3-Chlorophenyl)-4-(7H-pyrrolo[2,3-d]pyrimidin-4-yl)-3,6-dihydro-pyridine-1(2H)-carboxamide (63)

The reaction was carried out as described in general procedure **G** using amine **62** (70 mg, 0.26 mmol). The crude reaction mixture was purified by silica gel column chromatography using a gradient solvent system of CH₂Cl₂/acetone from 100/0 up to 50/50 to afford compound **63** (64 mg, 71%) as a white solid. R*_{f}* (CH₂Cl₂/acetone 50/50) 0.29. Mp: degradation 122°C. ¹H NMR (250 MHz, MeOD-*d₄*) δ 8.68 (s, 1H, CH), 7.57 (t, *J* = 2.0 Hz, 1H, CH), 7.48 (d, *J* = 3.6 Hz, 1H, CH), 7.33 (ddd, *J* = 8.2 Hz, *J* = 2.0 Hz, *J* = 1.2 Hz, 1H, CH), 7.24 (t, *J=* 8.0 Hz, 1H, CH), 7.01 (ddd, *J* = 7.7 Hz, *J* = 2.1 Hz, *J* = 1.2 Hz, 1H, CH), 6.90 - 6.83 (m, 1H, CH), 6.82 (d, *J* = 3.6 Hz, 1H, CH), 4.41 - 4.28 (m, 2H, CH₂), 3.82 (t, *J* = 5.6 Hz, 2H, CH₂), 2.96 - 2.82 (m, 2H, CH₂). HRMS (EI-MS) m/z calcd for C₁₈H₁₇ClN₅O [M+H]⁺: 354.1116, found: 354.1114.

### FAMILY 3.

### 3-chloropyrolopyrimidine derivatives with a hydropyridine.

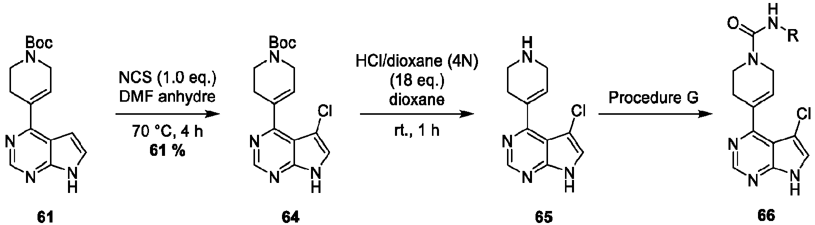

| Compound | General procedure | Yield (%) | R |
|---|---|---|---|
| **66** | H | 70 | 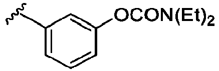 |

### tert-Butyl 4-(5-chloro-7H-pyrrolo[2,3-d]pyrimidin-4-yl)-3,6-dihydro-2H-pyridine-1-carboxylate (64)

N-Chlorosuccinimide (222 mg, 1.66 mmol, 1.0 equiv.) was added to a solution of **61** (500 mg, 1.66 mmol) in anhydrous DMF (0.047 M). The reaction mixture was heated to 70°C and stirred for 4h until completion (TLC monitoring). After cooling, it was pour into water and the aqueous layer was extracted with EtOAc (2 x 100 mL). The combined organic layers were washed with brine (4 x 200 mL) and a saturated sodium thiosulfate solution, dried over MgSO₄, filtered, and concentrated. The residue was purified by silica gel column chromatography using a gradient solvent system of CH₂Cl₂/acetone from 100/0 up to 60/40 to afford compound **64** (340 mg, 61%) as a white solid. R*_{f}* (CH₂Cl₂/acetone 70/30) 0.41. Mp: degradation 189°C. ¹H NMR (250 MHz, MeOD-*d₄*) δ 8.69 (s, 1H, CH), 7.51 (s, 1H, CH), 6.11 (s, 1H, CH), 4.17 (q, *J=* 2.8 Hz, 2H, CH₂), 3.71 (t, *J=* 5.6 Hz, 2H, CH₂), 2.66 (m, 2H, CH₂), 1.50 (s, 9H, CH₃). HRMS (EI-MS) m/z calcd for C₁₆H₂₀ClN₄O₂[M+H]⁺: 335.1269, found: 335.1272.

### 5-Chloro-4-(1,2,3,6-tetrahydropyridin-4-yl)-7H-pyrrolo[2,3-d]pyrimidine (65)

The reaction was carried out as described in general procedure **J** using **64** (250 mg, 0.75 mmol). The desired product **65** (195 mg, 85 %) was obtained as a white solid. R*_{f}* (CH₂Cl₂/MeOH 80/20) 0.03. Mp: degradation >266°C. ¹H NMR (400 MHz, DMSO-*d₆*) δ 13.07 (s, 1H, NH), 9.70 (s, 2H, NH₂ (sel)), 8.88 (s, 1H, CH), 7.95 (s, 1H, CH), 6.27 (s, 1H, CH), 3.86 (s, 2H, CH₂), 3.42 - 3.21 (s, 2H, CH₂), 2.88 (s, 2H, CH₂). HRMS (EI-MS) m/z calcd for C₁₁H₁₂ClN₄[M+H]⁺: 235.0745, found: 235.0743.

### Example 38: [3-[[4-(5-Chloro-7H-pyrrolo[2,3-d]pyrimidin-4-yl)-3,6-dihydro-2H-pyridine-1-carbonyl]amino] phenyl] N,N-dimethylcarbamate (66)

The reaction was carried out as described in general procedure **H** using 3-aminophenyl dimethylcarbamate (24 mg, 0.14 mmol) and **65** (50 mg, 0.16 mmol, 1.2 equiv.). The crude reaction mixture was purified by silica gel column chromatography using a gradient solvent system CH₂Cl₂/acetone from 10/0 up to 60/40 to afford compound **66** (42 mg, 70%) as a white solid. R*_{f}* (CH₂Cl₂/acetone 50/50) 0.25. Mp: degradation 132 °C. ¹H NMR (250 MHz, DMSO-*d₆*) δ 12.53 (s, 1H, NH), 8.77 (s, 1H, CH), 8.69 (s, 1H, NH), 7.79 (t, *J=* 2.1 Hz, 1H, CH), 7.42 - 7.28 (m, 2H, 2 x CH), 7.22 (t, *J* = 8.0 Hz, 1H, CH), 6.69 (d, *J* = 8.3 Hz, 1H, CH), 6.22 (s, 1H, CH), 4.25 (s, 2H, CH₂), 3.73 (t, *J* = 5.5 Hz, 2H, CH₂), 3.03 (s, 3H, NCH₃), 2.90 (s, 3H, NCH₃), 2.71 (s, 2H, CH₂). HRMS (EI-MS) m/z calcd for C₂₁H₂₂ClN₆O₃[M+H]⁺: 441.1436, found: 441.1436.

### FAMILY 4.

### 3-cyclopropylpyrrolopyrimidine derivatives with an unsubstituted hydropyridine.

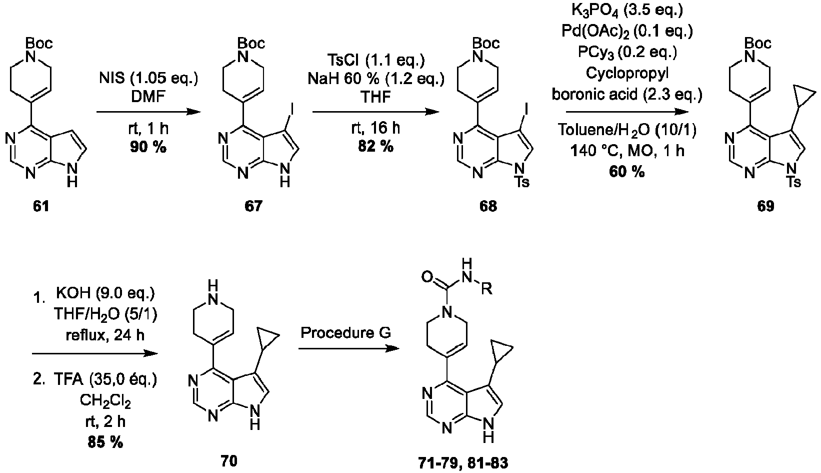

| Compound | General procedure | Yield (%) | R |
|---|---|---|---|
| **71** | G | 56 | 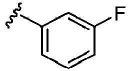 |
| **72** | G | 67 | 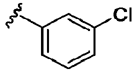 |
| **73** | G | 50 | 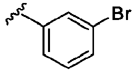 |
| **74** | H | 48 | 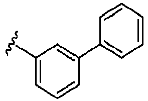 |
| **75** | H | 64 | 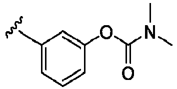 |
| **76** | H | 58 | 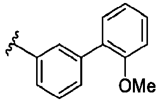 |
| **77** | H | 67 | 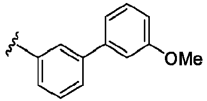 |
| **78** | H | 67 | 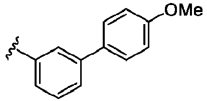 |
| **79** | G | 58 | 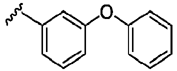 |
| **81** | H | 70 | 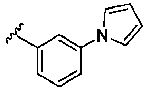 |
| **82** | H | 53 | 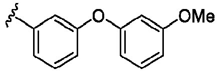 |
| **83** | H | 60 | 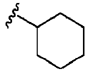 |

### tert-Butyl 4-(5-iodo-7H-pyrrolo[2,3-d]pyrimidin-4-yl)-5,6-dihydropyridine-1(2H)-carboxylate (67)

To a solution of **14** 61 (1.40 g, 4.66 mmol) in anhydrous DMF (25 mL) under an argon atmosphere was added NIS (1.10 g, 4.89 mmol, 1.05 equiv.) at 0 °C. The mixture was stirred at this temperature for 20 minutes and was allowed to warm to room temperature. The solution was poured into ice (50 g) and the aqueous phase was extracted with EtOAc (3 x 100 mL). The combined organic layers were washed with a saturated solution of NaCl (4 x 50 mL), dried over MgSO₄, filtered and concentrated under reduced pressure. The crude residue was dissolved in a minimum of acetone, precipitated with pentane and filtered to afford compound **67** (1.79 g, 90 %) as a light brown solid. R*_{f}* (CH₂Cl₂/acetone 70/30) 0.16. Mp: 151 - 153 °C. ¹H NMR (400 MHz, MeOD-*d₄*) δ 8.67 (s, 1H, CH), 7.61 (s, 1H, CH), 6.02 - 5.92 (m, 1H, CH), 4.25 - 4.14 (m, 2H, CH₂), 3.73 (t, *J=* 5.6 Hz, 2H, CH₂), 2.62 - 2.52 (m, 2H, CH₂), 1.49 (s, 9H, 3 x CH₃). HRMS (EI-MS) m/z calcd for C₁₆H₂₀IN₄O₂ [M+H]⁺: 427.0625, found: 427.0626.

### tert-Butyl 4-(5-iodo-7-tosyl-7H-pyrrolo[2,3-d]pyrimidin-4-yl)-5,6-dihydropyridine-1(2H)-carboxylate (68)

NaH (60 % dispersion in mineral oil, 0.18 g, 4.50 mmol, 1.2 equiv.) was added to a solution of the iodide derivative **67** (1.60 g, 3.75 mmol) in anhydrous THF (35 mL) under an argon atmosphere at room temperature. The solution was stirred for 30 minutes before the addition of *p*-toluenesulfonyl chloride (0.79 g, 4.13 mmol, 1.1 equiv.). Stirring was continued overnight. After completion, the reaction was quenched with H₂O (20 mL) and the aqueous layer was extracted with EtOAc (3 x 100 mL). The combined organic layers were washed with a saturated solution of NaCl (100 mL), dried over MgSO₄, filtered and concentrated under reduced pressure. The crude reaction mixture was purified by silica gel column chromatography using a gradient solvent system of PE/EtOAc from 100/0 up to 50/50 to afford compound **68** (1.78 g, 82 %) as a white solid. R*_{f}* (CH₂Cl₂/acetone 70/30) 0.91. Mp: 183 - 185°C. ¹H NMR (400 MHz, DMSO-*d₆*) δ 8.91 (s, 1H, CH), 8.21 (s, 1H, CH), 8.07 (d, *J* = 8.4 Hz, 2H, 2 x CH), 7.47 (d, *J* = 8.2 Hz, 2H, 2 x CH), 6.11 - 5.96 (m, 1H, CH), 4.16 - 4.02 (m, 2H, CH₂), 3.58 (t, *J* = 5.7 Hz, 2H, CH₂), 2.55 - 2.48 (m, 2H, CH₂), 2.36 (s, 3H, CH₃), 1.43 (s, 9H, 3 x CH₃). HRMS (EI-MS) m/z calcd for C₂₃H₂₆IN₄O₄S [M+H]⁺: 581.0714, found: 581.0712.

### tert-Butyl 4-(5-cyclopropyl-7-tosyl-7H-pyrrolo[2,3-d]pyrimidin-4-yl)-5,6-dihydropyridine-1(2H)-carboxylate (69)

A microwave vial was charged with a magnetic stirring bar, compound **68** (0.70 g, 1.21 mmol), cyclopropylboronic acid (0.26 g, 3.05 mmol, 2.5 equiv.), K₃PO₄ (0.90 g, 4.24 mmol, 3.5 equiv.), P(Cy)₃ (68 mg, 0.24 mmol, 0.2 equiv.), toluene (15 mL) and H₂O (1.5 mL). The solution was degassed with argon for 15 minutes under stirring. Pd(OAc)₂ (27 mg, 0.12 mmol, 0.1 equiv.) was added to the solution, the tube was sealed and the reaction mixture was heated under microwave irradiation at 140°C for 1 h. After cooling, cyclopropylboronic acid (0.10 g, 1.21 mmol, 1.0 equiv.) was again added and the reaction mixture was stirred for 30 minutes under microwave irradiation at 140°C. After completion, H₂O (30 mL) was added and the aqueous phase was extracted with CH₂Cl₂ (2 x 100 mL). The combined organic layers were washed with a saturated solution of NaCl (50 mL), dried over MgSO₄, filtered and concentrated under reduced pressure. The crude reaction mixture was purified by silica gel column chromatography using a gradient solvent system of CH₂Cl₂/acetone from 100/0 up to 60/40 to afford compound **69** (0.36 g, 60 %) as a beige solid. R*_{f}* (CH₂Cl₂/acetone 70/30) 0.90. Mp: degradation 90°C. ¹H NMR (400 MHz, MeOD-*d₄*) δ 8.77 (s, 1H, CH), 8.03 (d, *J* = 8.4 Hz, 2H, 2 x CH), 7.55 (d, *J=* 1.3 Hz, 1H, CH), 7.37 (d, *J=* 8.1 Hz, 2H, 2 x CH), 6.20 - 6.05 (m, 1H, CH), 4.18 - 4.06 (m, 2H, CH₂), 3.68 (t, *J* = 5.6 Hz, 2H, CH₂), 2.65 - 2.57 (m, 2H, CH₂), 2.37 (s, 3H, CH₃), 1.85 - 1.75 (m, 1H, CH), 1.48 (s, 9H, 3 x CH₃), 0.99 - 0.83 (m, 2H, CH₂), 0.72 - 0.61 (m, 2H, CH₂). HRMS (EI-MS) m/z calcd for C₂₆H₃₁N₄O₄S [M+H]⁺: 495.2061, found: 495.2058.

### 5-Cycloropyl-4-(1,2,3,6-tetrahydropyridin-4-yl)-7H-pyrrolo[2,3-d]pyrimidine (70)

To a solution of **69** (0.30 g, 0.61 mmol) in THF/H₂O (12 mL, 5:1) was added KOH (0.31 g, 5.46 mmol, 9.0 equiv.). The reaction mixture was heated to reflux for 24 h. After cooling, the solution was extracted with CH₂Cl₂ (2 x 50 mL) and the combined organic layers were washed with a saturated solution of NaCl (30 mL), dried over MgSO₄, filtered and concentrated under reduced pressure. The residue was taken up in CH₂Cl₂ (20 mL), the solution was cooled to 0°C and TFA (1.6 mL, 21.35 mmol, 35.0 equiv.) was added. The mixture was stirred for 2 h at room temperature. The excess of TFA was removed under reduced pressure. The residue was taken up in CH₂Cl₂ (10 mL) and a 2 M aqueous solution of NaOH (3 mL) was added. The mixture was stirred for 30 min and extracted with CH₂Cl₂ (30 mL). The organic layer was dried over MgSO₄, filtered and concentrated under vacuum to afford **70** (125 mg, 85 %) as a white solid. Mp: 184 - 186°C. ¹H NMR (400 MHz, MeOD-*d₄*) δ 8.60 (s, 1H, CH), 7.13 (d, *J=* 1.1 Hz, 1H, CH), 6.25 - 6.15 (m, 1H, CH), 3.58 - 3.51 (m, 2H, CH₂), 3.14 (t, *J =* 5.7 Hz, 2H, CH₂), 2.68 - 2.58 (m, 2H, CH₂), 1.99 - 1.86 (m, 1H, CH), 0.94 - 0.84 (m, 2H, CH₂), 0.66 - 0.57 (m, 2H, CH₂). HRMS (EI-MS) m/z calcd for C₁₄H₁₇N₄ [M+H]⁺: 241.1448, found: 241.1447.

### Example 39: 4-(5-Cyclopropyl-7H-pyrrolo[2,3-d]pyrimidin-4-yl)-N-(3-fluoro-phenyl)-5,6-dihydropyridine-1(2H)-carboxamide (71)

The reaction was carried out as described in general procedure **G** using amine **70** (50 mg, 0.21 mmol) and 3-fluorophenyl isocyanate (26 µL, 0.23 mmol, 1.1 equiv.) in anhydrous CH₂Cl₂ (5 mL). The crude reaction mixture was purified by silica gel column chromatography using a gradient solvent system of CH₂Cl₂/acetone from 90/10 up to 30/70 to afford compound **71** (44 mg, 56 %) as a white solid. R*_{f}* (CH₂Cl₂/acetone 50/50) 0.28. Mp: 149 - 151°C. ¹H NMR (250 MHz, MeOD-*d₄*) δ 8.63 (s, 1H, CH), 7.41 - 7.12 (m, 4H, 4 x CH), 6.81 - 6.67 (m, 1H, CH), 6.29 - 6.17 (m, 1H, CH), 4.36 - 4.21 (m, 2H, CH₂), 3.85 (t, *J=* 5.6 Hz, 2H, CH₂), 2.86 - 2.70 (m, 2H, CH₂), 1.95 - 1.77 (m, 1H, CH), 0.99 - 0.77 (m, 2H, CH₂), 0.67 - 0.51 (m, 2H, CH₂). ¹⁹F NMR (235 MHz, MeOD-*d₄*) δ -114.87 - -115.04 (m). HRMS (EI-MS) m/z calcd for C₂₁H₂₁FN₅O [M+H]⁺: 378.1725, found: 378.1724.

### Example 40: N-(3-Chlorophenyl)-4-(5-cyclopropyl-7H-pyrrolo[2,3-d] pyrimidin-4-yl)-5,6-dihydropyridine-1(2H)-carboxamide (72)

The reaction was carried out as described in general procedure **G** using amine **70** (50 mg, 0.21 mmol) and 3-chlorophenyl isocyanate (28 µL, 0.23 mmol, 1.1 equiv.) in anhydrous CH₂Cl₂ (5 mL). The crude reaction mixture was purified by silica gel column chromatography using a gradient solvent system of CH₂Cl₂/acetone from 90/10 up to 30/70 to afford compound **72** (55 mg, 67%) as a white solid. R*_{f}* (CH₂Cl₂/acetone 50/50) 0.28. Mp: 174 - 176°C. ¹H NMR (400 MHz, MeOD-*d₄*) δ 8.63 (s, 1H, CH), 7.55 (t, *J* = 2.1 Hz, 1H, CH), 7.31 (d, *J* = 8.2 Hz, 1H, CH), 7.24 (t, *J* = 8.0 Hz, 1H, CH), 7.16 (s, 1H, CH), 7.01 (d, *J* = 7.2 Hz, 1H, CH), 6.25 - 6.19 (m, 1H, CH), 4.36 - 4.23 (m, 2H, CH₂), 3.85 (t, *J* = 5.6 Hz, 2H, CH₂), 2.84 - 2.71 (m, 2H, CH₂), 1.93 - 1.77 (m, 1H, CH), 0.90 - 0.83 (m, 2H, CH₂), 0.64 - 0.57 (m, 2H, CH₂). HRMS (EI-MS) m/z calcd for C₂₁H₂₁ClN₅O [M+H]⁺: 394.1429, found: 394.1426.

### Example 41: N-(3-Bromophenyl)-4-(5-cyclopropyl-7H-pyrrolo[2,3-d]pyrimidin-4-yl)-5,6-dihydropyridine-1(2H)-carboxamide (73)

The reaction was carried out as described in general procedure **G** using amine **70** (50 mg, 0.21 mmol) and 3-bromophenyl isocyanate (29 µL, 0.23 mmol, 1.1 equiv.) in anhydrous CH₂Cl₂ (5 mL). The crude reaction mixture was purified by silica gel column chromatography using a gradient solvent system of CH₂Cl₂/acetone from 90/10 up to 30/70 to afford compound **73** (46 mg, 50 %) as a white solid. R*_{f}* (CH₂Cl₂/acetone 50/50) 0.28. Mp: 179 - 181°C. ¹H NMR (250 MHz, MeOD-*d₄*) δ 8.63 (s, 1H, CH), 7.74 - 7.66 (m, 1H, CH), 7.40 - 7.32 (m, 1H, CH), 7.23 - 7.11 (m, 3H, CH + 2 x CH), 6.25 - 6.19 (m, 1H, CH), 4.34 - 4.23 (m, 2H, CH₂), 3.85 (t, *J* = 5.6 Hz, 2H, CH₂), 2.85 - 2.70 (m, 2H, CH₂), 1.95 - 1.78 (m, 1H, CH), 0.94 - 0.78 (m, 2H, CH₂), 0.67 - 0.54 (m, 2H, CH₂). HRMS (EI-MS) m/z calcd for C₂₁H₂₁BrN₅O [M+H]⁺: 438.0924, found: 438.0922.

### Example 42: 4-(5-Cyclopropyl-7H-pyrrolo[2,3-d]pyrimidin-4-yl)-N-(3-phenylphenyl)-3,6-dihydropyridine-1(2H)-carboxamide (74)

The reaction was carried out as described in general procedure **H** with 1,1'-biphenyl]-3-amine (31 µL, 0.25 mmol) and amine **70.HCl** (37 mg, 0.12 mmol, 1.2 equiv.) in anhydrous THF (4 mL). The crude reaction mixture was purified by silica gel column chromatography using a gradient solvent system of CH₂Cl₂/acetone from 100/0 up to 40/60 to give the desired compound **74** (25 mg, 48 %) as a yellow solid. R*_{f}* (CH₂Cl₂/ acetone 40/60) 0.3. Mp: degradation 145°C. ¹H NMR (400 MHz, DMSO-*d₆*) δ 11.86 (s, 1H, NH), 8.73 - 8.57 (m, 2H, NH + CH), 7.82 (s, 1H, CH), 7.62 (d, *J* = 7.6 Hz, 2H, 2 x CH), 7.54 (d, *J* = 8.0 Hz, 1H, CH), 7.47 (t, *J* = 7.5 Hz, 2H, 2 x CH), 7.35 (q, *J* = 7.8 Hz, 2H, 2 x CH), 7.27 - 7.22 (m, 2H, 2 x CH), 6.29 (s, 1H, CH), 4.25 (s, 2H, CH₂), 3.77 (t, *J* = 5.5 Hz, 2H, CH₂), 2.72 (s, 2H, CH₂), 1.90 - 1.78 (m, 1H, CH), 0.85 - 0.78 (m, 2H, CH₂), 0.64 - 0.57 (m, 2H, CH₂). HRMS (EI-MS) m/z calcd for C₂₇H₂₆N₅O[M+H]⁺: 436.2132, found: 436.2130.

### Example 43: [3-[[4-(5-Cyclopropyl-7H-pyrrolo[2,3-d]pyrimidin-4-yl)-3,6-dihydropyridine-1(2H)-carbonyl]amino] phenyl] N,N-dimethylcarbamate (75)

The reaction was carried out as described in general procedure **H** with 3-aminophenyl dimethylcarbamate (18 mg, 0.10 mmol) and amine **70.HCl** (38 mg, 0.12 mmol, 1.2 equiv.) in anhydrous THF (4 mL). The crude reaction mixture was purified by silica gel column chromatography using a gradient solvent system of CH₂Cl₂/acetone from 100/0 up to 25/75 to give the desired compound **75** (35 mg, 64 %) as a white solid. R*_{f}* (CH₂Cl₂/acetone 20/80) 0.28. Mp: degradation 132°C. ¹H NMR (400 MHz, DMSO-*d₆*) δ 11.83 (s, 1H, NH), 8.67 (s, 1H, CH), 8.64 (s, 1H, NH), 7.35 (t, *J* = 2.1 Hz, 1H, CH), 7.31 (d, *J* = 2.1, Hz, 1H, CH), 7.28 - 7.19 (m, 2H, 2 × CH), 6.71 - 6.66 (m, 1H, CH), 6.27 (s, 1H, CH), 4.24 - 4.19 (m, 2H, CH₂), 3.74 (t, *J* = 5.6 Hz, 2H, CH₂), 3.03 (s, 3H, NCH₃), 2.90 (s, 3H, NCH₃), 2.73 - 2.64 (m, 2H, CH₂), 1.88 - 1.77 (m, 1H, CH), 0.84 - 0.76 (m, 2H, CH₂), 0.63 - 0.52 (m, 2H, CH₂). HRMS (EI-MS) m/z calcd for C₂₄H₂₇N₆O₃[M+H]⁺: 447.2139, found: 447.2137.

### Example 44: 4-(5-Cyclopropyl-7H-pyrrolo[2,3-d]pyrimidin-4-yl)-N-[3-(2-methoxyphenyl)phenyl]-3,6 dihydropyridine-1(2H)-carboxamide (76)

The reaction was carried out as described in general procedure **H** with 2'-methoxy-[1,1'-biphenyl]-3-amine (25 mg, 0.12 mmol) and amine **70.HCl** (48 mg, 0.15 mmol, 1.2 equiv.) in anhydrous THF (4 mL). The crude reaction mixture was purified by silica gel column chromatography using a gradient solvent system of CH₂Cl₂/acetone from 100/0 up to 30/70 to give the desired compound **76** (35 mg, 58%) as a white solid. R*_{f}* (CH₂Cl₂/acetone 30/70) 0.43. Mp: degradation 140°C. ¹H NMR (400 MHz, DMSO-*d₆*) δ 11.95 (s, 1H, NH), 8.68 (s, 1H, CH), 8.61 (s, 1H, NH), 7.59 (s, 1H, CH), 7.48 (d, *J* = 8.2 Hz, 1H, CH), 7.33 (t, *J* = 8.2 Hz, 1H, CH), 7.31 - 7.21 (m, 3H, 3xCH), 7.10 (d, *J* = 8.3 Hz, 1H, CH), 7.08 - 6.98 (m, 2H, 2 x CH), 6.30 (s, 1H, CH), 4.24 (s, 2H, CH₂), 3.76 (s, 5H, OCH₃ + CH₂), 2.71 (s, 2H, CH₂), 1.84 (m, 1H, CH), 0.85 - 0.78 (m, 2H, CH₂), 0.64 - 0.57 (m, 2H, CH₂). HRMS (EI-MS) m/z calcd for C₂₈H₂₈N₅O₂[M+H]⁺: 466.2238, found: 466.2234.

### Example 45: 4-(5-Cyclopropyl-7H+pyrrolo[2,3-d]pyrimidin-4-yl)-N-[3-(3-methoxyphenyl)phenyl]-3,6-dihydropyridine-1(2H)-carboxamide (77)

The reaction was carried out as described in general procedure **H** with 3'-methoxy-[1,1'-biphenyl]-3-amine (25 mg, 0.12 mmol) and amine **70.HCl** (48 mg, 0.15 mmol, 1.2 equiv.) in anhydrous THF (4 mL). The crude reaction mixture was purified by silica gel column chromatography using a gradient solvent system of CH₂Cl₂/acetone from 100/0 up to 40/60 to give the desired compound **77** (40 mg, 67 %) as a white solid. R*_{f}* (CH₂Cl₂/acetone 30/70) 0.67. Mp: degradation 199°C. ¹H NMR (400 MHz, DMSO-*d₆*) δ 11.86 (s, 1H, NH), 8.66 (s, 2H, NH + CH), 7.80 (s, 1H, CH), 7.55 (d, *J* = 8.1 Hz, 1H, CH), 7.38 (t, *J* = 7.9 Hz, 1H, CH), 7.33 (t, *J* = 7.9 Hz, 1H, CH), 7.29 - 7.22 (m, 2H, 2 x CH), 7.18 (d, *J* = 7.7 Hz, 1H, CH), 7.16 - 7.11 (m, 1H, CH), 6.94 (dd, *J* = 8.2, 2.6 Hz, 1H, CH), 6.29 (s, 1H, CH), 4.30 - 4.20 (m, 2H, CH₂), 3.82 (s, 3H, OCH₃), 3.77 (t, *J* = 5.7 Hz, 2H, CH₂), 2.72 (s, 2H, CH₂), 1.91 - 1.78 (m, 1H, CH), 0.86 - 0.76 (m, 2H, CH₂), 0.65 - 0.52 (m, 2H, CH₂). HRMS (ElMS) m/z calcd for C₂₈H₂₈N₅O₂[M+H]⁺: 466.2238, found: 466.2235.

### Example 46: 4-(5-Cyclopropyl-7H-pyrrolo[2,3-d]pyrimidin-4-yl)-N-[3-(4-methoxyphenyl)phenyl]-3,6-dihydropyridine-1(2H)-carboxamide (78)

The reaction was carried out as described in general procedure **H** with 4'-methoxy-[1,1'-biphenyl]-3-amine (25 mg, 0.12 mmol) and amine **70.HCl** (48 mg, 0.15 mmol, 1.2 equiv.) in anhydrous THF (4 mL). The crude reaction mixture was purified by silica gel column chromatography using a gradient solvent system of CH₂Cl₂/acetone from 100/0 up to 40/60 to give the desired compound **78** (40 mg, 67 %) as a light beige solid. R*_{f}* (CH₂Cl₂/ acetone 30/70) 0.4. Mp: degradation 149°C. ¹H NMR (400 MHz, DMSO-*d₆*) δ 11.89 (s, 1H, NH), 8.67 (s, 1H, CH), 8.64 (s, 1H, NH), 7.77 (s, 1H, CH), 7.55 (d, *J* = 8.6 Hz, 2H, 2 x CH), 7.48 (d, *J* = 8.1 Hz, 1H, CH), 7.33 - 7.24 (m, 2H, 2 x CH), 7.19 (d, *J* = 7.6 Hz, 1H, CH), 7.03 (d, *J* = 8.5 Hz, 2H, 2 x CH), 6.30 (s, 1H, CH), 4.31 - 4.19 (m, 2H, CH₂), 3.84 - 3.71 (m, 5H, OCH₃ + CH₂), 2.72 (s, 2H, CH₂), 1.90 - 1.78 (m, 1H, CH), 0.82 (d, *J* = 7.0 Hz, 2H, CH₂), 0.61 (d, *J* = 4.2 Hz, 2H, CH₂). HRMS (EI-MS) m/z calcd for C₂₈H₂₈N₅O₂[M+H]⁺: 466.2238, found: 466.2234.

### Example 47: 4-(5-Cyclopropyl-7H-pyrrolo[2,3-d]pyrimidin-4-yl)-N-(3-phenoxyphenyl)-3,6-dihydropyridine-1(2H)-carboxamide (79)

The reaction was carried out as described in general procedure **G** with amine **70.HCl** (48 mg, 0.15 mmol) in CH₂Cl₂ (4 mL). The crude reaction mixture was purified by silica gel column chromatography using a gradient solvent system of CH₂Cl₂/acetone from 80/20 up to 70/30 to give the desired compound **79** (35 mg, 58 %) as a white solid. R*_{f}* (CH₂Cl₂/ acetone 30/70) 0.52. Mp: degradation 124°C. ¹H NMR (400 MHz, DMSO-*d₆*) δ 11.89 (s, 1H, NH), 8.66 (s, 2H, CH + NH), 7.39 (t, *J* = 8.0 Hz, 2H, 2 x CH), 7.33 - 7.20 (m, 4H, 4xCH), 7.13 (t, *J* = 7.4 Hz, 1H, CH), 7.01 (d, *J* = 7.7 Hz, 2H, 2 x CH), 6.64 - 6.57 (m, 1H, CH), 6.26 (s, 1H, CH), 4.22 - 4.17 (m, 2H, CH₂), 3.72 (t, *J* = 5.5 Hz, 2H, CH₂), 2.68 (m, 2H, CH₂), 1.87 - 1.74 (m, 1H, CH), 0.85 - 0.72 (m, 2H, CH₂), 0.64 - 0.53 (m, 2H, CH₂). HRMS (EI-MS) m/z calcd for C₂₇H₂₆N₅O₂ [M+H]⁺: 452.2081, found: 452.2076.

### 3-(1H-Pyrrol-1-yl)aniline (80)

An oven-dried microwave vial was charged with a magnetic stirring bar, Cul (29 mg, 0.15 mmol, 0.05 equiv.), K₃PO₄ (1.33 g, 6.26 mmol, 2.1 equiv.) and 1,10-Phenanthroline (107 mg, 0.60 mmol, 0.2 equiv.). The tube was then evacuated and back-filled with argon. The evacuation/backfill sequence was repeated two additional times. Under a counterflow of argon, 3-iodoaniline (0.43 mL, 3.58 mmol, 1.2 equiv.), pyrrole (0.21 mL, 2.98 mmol) and degassed 1,4-dioxane (1.5 mL) were added by syringe. The tube was placed in a preheated oil bath at 110 °C and the solution was stirred vigorously for 24 h. The reaction mixture was cooled to room temperature, diluted with EtOAc (2-3 mL), filtered through a plug of Celite and eluted with EtOAc. The filtrate was concentrated under reduced pressure and the resulting residue was purified by silica gel column chromatography using a gradient solvent system PE/EtOAc from 90/10 up to 80/20 to afford compound **80** (0.41 g, 87 %) as a brown solid. ¹H NMR (250 MHz, CDCl₃) δ 7.22 (t, *J* = 8.0 Hz, 1H, H), 7.11 (t, *J* = 2.2 Hz, 2H, 2 x CH), 6.83 (ddd, *J* = 8.0 Hz, *J* = 2.1 Hz, *J* = 0.9 Hz, 1H, CH), 6.71 (t, *J* = 2.2 Hz, 1H, CH), 6.57 (ddd, *J* = 8.0 Hz, *J* = 2.3 Hz, *J* =0.9 Hz, 1H, CH), 6.40 (t, *J* = 2.2 Hz, 2H, 2 x CH), 3.76 (br s, 2H, NH₂). Tetrahedron 2011, 67, 898-903.

### Example 48: 4-(5-Cyclopropyl-7H-pyrrolo[2,3-d]pyrimidin-4-yl)-N-(3-pyrrol-1-ylphenyl)-3,6-dihydropyridine-1(2H)-carboxamide (81)

The reaction was carried out as described in general procedure **H** with 3-(1*H*-pyrrol-1-yl)aniline **80** (22 mg, 0.14 mmol) and amine **70.HCl** (53 mg, 0.16 mmol, 1.2 equiv.) in anhydrous THF (4 mL). The crude reaction mixture was purified by silica gel column chromatography using a gradient solvent system of CH₂Cl₂/acetone from 100/0 up to 50/50 to give the desired compound **81** (42 mg, 70 %) as a white solid. R*_{f}* (CH₂Cl₂/ acetone 70/30) 0.25. Mp: degradation 140°C. ¹H NMR (400 MHz, DMSO-*d₆*) δ 11.84 (s, 1H, NH), 8.72 (s, 1H, NH), 8.65 (s, 1H, CH), 7.74 (s, 1H, CH), 7.41 (d, *J* = 7.9 Hz, 1H, CH), 7.32 (t, *J* = 8.0 Hz, 1H, CH), 7.27 - 7.21 (m, 3H, 3 x CH), 7.13 (d, J = 7.7 Hz, 1H, CH), 6.43 - 6.07 (m, 3H, 3 x CH), 4.31 - 4.16 (m, 2H, CH₂), 3.77 (t, *J* = 5.4 Hz, 2H, CH₂), 2.77 - 2.68 (m, 2H, CH₂), 1.91 - 1.77 (m, 1H, CH), 0.86 - 0.75 (m, 2H, CH₂), 0.66 - 0.53 (m, 2H, CH₂). HRMS (EI-MS) m/z calcd for C₂₅H₂₄N₆O[M+H]⁺: 425.2084, found: 425.2084.

### Example 49: 4-(5-Cyclopropyl-7H-pyrrolo[2,3-d]pyrimidin-4-yl)-N-[3-(3-methoxyphenoxy)phenyl]-3,6-dihydropyridine-1(2H)-carboxamide (82)

The reaction was carried out as described in general procedure **H** with 3-(3-methoxyphenoxy)aniline (22 mg, 0.11 mmol) and amine **70.HCl** (47 mg, 0.15 mmol, 1.2 equiv.) in anhydrous THF (4 mL). The crude reaction mixture was purified by silica gel column chromatography using a gradient solvent system of CH₂Cl₂/acetone from 100/0 up to 40/60 to give the desired compound **82** (32 mg, 53 %) as a white solid. R*_{f}* (CH₂Cl₂/ acetone 40/60) 0.35. Mp: degradation 186°C. ¹H NMR (400 MHz, DMSO-*d₆*) δ 11.83 (s, 1H, NH), 8.69 - 8.61 (m, 2H, NH + CH), 7.39 - 7.07 (m, 5H, 5xCH), 6.71 (dd, *J* = 8.2, *J* = 2.4 Hz, 1H, CH), 6.62 (d, *J* = 7.9 Hz, 1H, CH), 6.58 (t, *J* = 2.3 Hz, 1H, CH), 6.57 - 6.53 (m, 1H, CH), 6.25 (s, 1H, CH), 4.22 - 4.17 (m, 2H, CH₂), 3.80 - 3.64 (m, 5H, OCH₃ + CH₂), 2.68 (s, 2H, CH₂), 1.88 - 1.76 (m, 1H, CH), 0.86 - 0.73 (m, 2H, CH₂), 0.65 - 0.52 (m, 2H, CH₂HRMS (ElMS) m/z calcd for C₂₈H₂₈N₅O₃[M+H]⁺: 482.2187, found: 482.2186.

### Example 50: N-Cyclohexyl-4-(5-cyclopropyl-7H+pyrrolo[2,3-d]pyrimidin-4-yl)-3,6-dihydropyridine-1(2H)-carboxamide (83)

The reaction was carried out as described in general procedure **H** with cyclohexylamine (13 mg, 0.11 mmol) and amine **70.HCl** (51 mg, 0.16 mmol, 1.2 equiv.) in anhydrous THF (4 mL). The crude reaction mixture was purified by silica gel column chromatography using a gradient solvent system of CH₂Cl₂/acetone from 100/0 up to 40/60 to give the desired compound **83** (35 mg, 60%) as a white solid. R*_{f}* (CH₂Cl₂/ acetone 30/70) 0.27. Mp: 201 °C. ¹H NMR (400 MHz, DMSO-*d₆*) δ 11.82 (s, 1H, NH), 8.62 (s, 1H, CH), 7.23 (s, 1H, CH), 6.20 (m, 2H, NH + CH), 4.03 (s, 2H, CH₂), 3.57 (t, *J* = 5.5 Hz, 2H, CH₂), 3.51 - 3.41 (m, 1H, CH), 2.59 (s, 2H, CH₂), 1.84 - 1.63 (m, 5H, CH + CH₂), 1.57 (d, *J* = 11.9 Hz, 1H, CH₂), 1.33 - 0.99 (m, 5H, 2 x CH₂, CH), 0.85 - 0.74 (m, 2H, CH₂), 0.63 - 0.53 (m, 2H, CH₂). HRMS (EI-MS) m/z calcd for C₂₁H₂₈N₅O[M+H]⁺: 366.2290, found: 366.2288.

### FAMILY 5.

### 2,3-dimethylpyrrolopyrimidine derivatives with an unsubstituted hydropyridine.

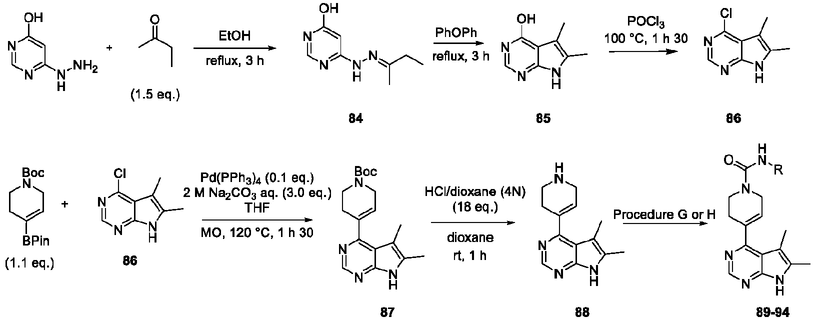

| Compound | General procedure | Yield (%) | R |
|---|---|---|---|
| **89** | G | 75 | 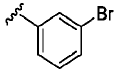 |
| **90** | G | 72 | 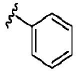 |
| **91** | G | 55 | 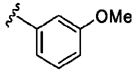 |
| **92** | H | 48 | 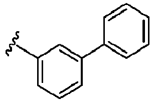 |
| **93** | H | 50 | 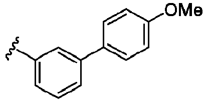 |
| **94** | H | 58 | 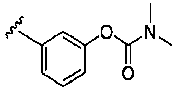 |

### 6-[(2E)-2-(1-Methylpropylidene)hydrazino]pyrimidin-4-ol (84)

The reaction was carried out as described in general procedure **A** with 4-hydroxy-6-hydrazinyl pyrimidine (2.0 g, 15.9 mmol). The desired product **84** (2.7 g, 95%) was obtained as a white solid. R*_{f}* (acetone) 0.31. Mp: degradation 225°C. ¹H NMR (400 MHz, DMSO-*d₆*) δ 11.64 (s, 1H, NH), 9.40 (s, 1H, OH), 7.90 (s, 1H, CH), 5.55 (s, 1H, CH), 2.25 (q, *J* = 7.4 Hz, 2H, CH₂), 1.88 (s, 3H, CH₃), 1.04 (t, *J* = 7.4 Hz, 3H, CH₃). HRMS (EI-MS) m/z calcd for C₈H₁₃N₄O[M+H]⁺: 181.1084, found: 181.1083.

### 5,6-Dimethyl-7H-pyrrolo[2,3-d]pyrimidin-4-ol (85)

The reaction was carried out as described in general procedure **K** using **84** (400 mg, 2.21 mmol). The desired product **85** (300 mg, 92 %) was obtained as a light brown solid. R*_{f}* (CH₂Cl₂/acetone 20/80) 0.35. Mp: degradation >266°C. ¹H NMR (400 MHz, DMSO-*d₆*) δ 11.54 (s, 1H, OH), 11.38 (s, 1H, NH), 7.67 (s, 1H, CH), 2.18 (s, 3H, CH₃), 2.15 (s, 3H, CH₃). HRMS (EI-MS) m/z calcd for C₈H₁₀N₃O[M+H]⁺: 164.0818, found: 164.0814.

### 4-Chloro-5,6-dimethyl-7H-pyrrolo[2,3-d]pyrimidine (86)

The reaction was carried out as described in general procedure C using **85** (400 mg, 2.45 mmol). The crude reaction mixture was purified by silica gel column chromatography using a gradient solvent system of CH₂Cl₂/acetone from 100/0 up to 70/30 to afford compound **86** (267 mg, 60 %) as a white solid. R*_{f}* (CH₂Cl₂/acetone 70/30) 0.49. Mp: degradation 232 °C. ¹H NMR (400 MHz, DMSO-*d₆*) δ 12.13 (s, 1H, NH), 8.40 (s, 1H, CH), 2.32 (s, 3H, CH₃), 2.30 (s, 3H, CH₃). HRMS (EI-MS) m/z calcd for C₈H₉ClN₃[M+H]⁺: 182.0480, found: 182.0475.

### tert-Butyl 4-(5,6-dimethyl-7H-pyrrolo[2,3-d]pyrimidin-4-yl)-3,6-dihydro-2H-pyridine-1-carboxylate (87)

The reaction was carried out as described in general procedure **D** using **86** (109 mg, 0.6 mmol). The crude reaction mixture was purified by silica gel column chromatography using a gradient solvent system of CH₂Cl₂/acetone from 100/0 up to 50/50 to afford compound **87** (58 mg, 30 %) as a white solid. R*_{f}* (CH₂Cl₂/acetone 60/40): 0.26. Mp: degradation 162°C. ¹H NMR (250 MHz, CDCl₃) δ 11.07 (s, 1H, NH), 8.72 (s, 1H, CH), 5.92 (s, 1H, CH), 4.15 (s, 2H, CH₂), 3.71 (t, *J* = 5.6 Hz, 2H, CH₂), 2.76 - 2.65 (m, 2H, CH₂), 2.44 (s, 3H, CH₃), 2.18 (s, 3H, CH₃), 1.50 (s, 9H, CH₃). HRMS (EI-MS) m/z calcd for C₁₈H₂₅N₄O₂[M+H]⁺: 329.1972, found: 329.1973.

### 5,6-Dimethyl-4-(1,2,3,6-tetrahydropyridin-4-yl)-7H-pyrrolo[2,3-d]pyrimidine hydrochloride (88)

The reaction was carried out as described in general procedure **J** using **87** (350 mg, 1.07 mmol). The desired product **88** (345 mg, quant.) was obtained as a yellow solid (HCl salt). Mp: degradation >266°C. ¹H NMR (400 MHz, DMSO-*d₆*) δ 13.27 (s, 1H, NH), 9.95 (s, 2H, NH₂ (salt)), 8.92 (s, 1H, CH), 6.28 (s, 1H, CH), 3.86 (s, 2H, CH₂), 3.35 (s, 2H, CH₂), 2.92 (s, 2H, CH₂), 2.43 (s, 3H, CH₃), 2.21 (s, 3H, CH₃). HRMS (EI-MS) m/z calcd for C₁₃H₁₇N₄[M+H]⁺: 229.1448, found: 229.1446.

### Example 51: N-(3-Bromophenyl)-4-(5,6-dimethyl-7H-pyrrolo[2,3-d]pyrimidin-4-yl)-3,6-dihydro-2H-pyridine-1-carboxamide (89)

The reaction was carried out as described in general procedure **G** using **88** (42 mg, 0.14 mmol). The crude mixture was purified by silica gel column chromatography using a gradient solvent system of CH₂Cl₂/acetone from 100/0 up to 50/50 to afford compound **89** (45 mg, 75%) as a white solid. R*_{f}* (CH₂Cl₂/acetone 50/50): 0.25. Mp: degradation >266°C. ¹H NMR (400 MHz, DMSO-*d₆*) δ 11.76 (s, 1H, NH), 8.75 (s, 1H, NH), 8.55 (s, 1H, CH), 7.83 (s, 1H, CH), 7.50 (d, *J* = 8.6 Hz, 1H, CH), 7.21 (t, *J* = 8.1 Hz, 1H, CH), 7.12 (d, *J* = 8.0 Hz, 1H, CH), 5.97 (s, 1H, CH), 4.21 (s, 2H, CH₂), 3.73 (t, *J* = 5.6 Hz, 2H, CH₂), 2.66 (s, 2H, CH₂), 2.33 (s, 3H, CH₃), 2.13 (s, 3H, CH₃). HRMS (EI-MS) m/z calcd for C₂₀H₂₁BrN₅O[M+H]⁺: 426.0924, found: 426.0919.

### Example 52: 4-(5,6-dimethyl-7H-pyrrolo[2,3-d]pyrimidin-4-yl)-N-phenyl-3,6-dihydro-2H-pyridine-1-carboxamide (90)

The reaction was carried out as described in general procedure **G** using **88** (52 mg, 0.17 mmol). The crude reaction mixture was purified by silica gel column chromatography using a gradient solvent system of CH₂Cl₂/acetone from 100/0 up to 50/50 to afford compound **90** (43 mg, 72%) as a white solid. R*_{f}* (CH₂Cl₂/acetone 70/30) 0.48. Mp: degradation >266°C. ¹H NMR (400 MHz, DMSO-*d₆*) δ 11.77 (s, 1H, NH), 8.59 - 8.53 (m, 2H, NH + CH), 7.50 (d, *J* = 8.0 Hz, 2H, CH), 7.24 (t, *J* = 7.7 Hz, 2H, CH), 6.94 (t, *J* = 7.3 Hz, 1H, CH), 5.98 (s, 1H, CH), 4.21 (s, 2H, CH₂), 3.73 (t, *J* = 5.5 Hz, 2H, CH₂), 2.66 (s, 2H, CH₂), 2.33 (s, 3H, CH₃), 2.14 (s, 3H, CH₃). HRMS (EI-MS) m/z calcd for C₂₀H₂₂N₅O[M+H]⁺: 348.1819, found: 348.1821.

### Example 53: 4-(5,6-dimethyl-7H-pyrrolo[2,3-d]pyrimidin-4-yl)-N-(3-methoxyphenyl)-3,6-dihydro-2H-pyridine-1-carboxamide (91)

The reaction was carried out as described in general procedure **G** using **88** (48 mg, 0.16 mmol). The crude reaction mixture was purified by silica gel column chromatography using a gradient solvent system of CH₂Cl₂/acetone from 100/0 up to 40/60 to afford compound **91** (33 mg, 55%) as a white solid. R*_{f}* (CH₂Cl₂/acetone 30/70) 0.36. Mp: degradation >266°C. ¹H NMR (400 MHz, DMSO-*d₆*) δ 11.80 (s, 1H, NH), 8.60 - 8.53 (m, 2H, NH + CH), 7.20 (s, 1H, CH), 7.17 - 7.06 (m, 2H, CH), 6.52 (d, *J* = 7.5 Hz, 1H, CH), 5.98 (s, 1H, CH), 4.20 (s, 2H, CH₂), 3.72 (s, 5H, CH₃ + CH₂), 2.66 (s, 2H, CH₂), 2.33 (s, 3H, CH₃), 2.13 (s, 3H, CH₃). HRMS (EI-MS) m/z calcd for C₂₁H₂₄N₅O₂[M+H]⁺: 378.1925, found: 378.1922.

### Example 54: 4-(5,6-dimethyl-7H-pyrrolo[2,3-d]pyrimidin-4-yl)-N-(3-phenyl-phenyl)-3,6-dihydro-2H-pyridine-1-carboxamide (92)

The reaction was carried out as described in general procedure **H** with 1,1'-biphenyl]-3-amine (16 mg, 0.14 mmol) and **88** (51 mg, 0.17 mmol, 1.2 equiv.) in THF (4 mL). The crude reaction mixture was purified by silica gel column chromatography using a solvent system of CH₂Cl₂/acetone 70/30 to afford compound **92** (29 mg, 48%) as a white solid. R*_{f}* (CH₂Cl₂/acetone 50/50) 0.35. Mp: degradation 144°C. ¹H NMR (400 MHz, DMSO-*d₆*) δ 11.78 (s, 1H, NH), 8.68 (s, 1H, NH), 8.57 (s, 1H, CH), 7.83 (s, 1H, CH), 7.62 (d, *J* = 7.6 Hz, 2H, CH), 7.55 (d, *J* = 8.0 Hz, 1H, CH), 7.47 (t, *J* = 7.6 Hz, 2H, CH), 7.35 (q, *J* = 7.8 Hz, 2H, CH), 7.24 (d, *J* = 7.7 Hz, 1H, CH), 5.99 (s, 1H, CH), 4.24 (s, 2H, CH₂), 3.76 (t, *J* = 4.9 Hz, 2H, CH₂), 2.68 (s, 2H, CH₂), 2.33 (s, 3H, CH₃), 2.14 (s, 3H, CH₃). HRMS (EI-MS) m/z calcd for C₂₆H₂₆N₅O [M+H]⁺: 424.2132, found: 424.2128.

### Example 55: 4-(5,6-Dimethyl-7H-pyrrolo[2,3-d]pyrimidin-4-yl)-N-[3-(4-methoxyphenyl)phenyl]-3,6-dihydropyridine-1(2H)-carboxamide (93)

The reaction was carried out as described in general procedure **H** using 4'-methoxy-[1,1'-biphenyl]-3-amine (26 mg, 0.13 mmol) and the amine salt **88** (48 mg, 0.16 mmol, 1.2 equiv.) in THF (4 mL). The crude mixture was purified by silica gel column chromatography using a gradient solvent system CH₂Cl₂/acetone from 100/0 up to 40/60 to afford **93** (30 mg, 50%) as a white solid. R*_{f}* (CH₂Cl₂/ acetone 30/70) 0.53. Mp: degradation 248°C. ¹H NMR (400 MHz, DMSO-*d₆*) δ 11.77 (s, 1H, NH), 8.64 (s, 1H, NH), 8.56 (s, 1H, CH), 7.77 (s, 1H, CH), 7.55 (d, *J* = 8.4 Hz, 2H, CH), 7.49 (d, *J* = 7.9 Hz, 1H, CH), 7.30 (t, *J* = 7.8 Hz, 1H, CH), 7.19 (d, *J* = 7.7 Hz, 1H, CH), 7.03 (d, *J* = 8.3 Hz, 2H, CH), 5.99 (s, 1H, CH), 4.23 (s, 2H, CH₂), 3.80 (s, 3H, CH₃), 3.79 - 3.71 (m, 2H, CH₂), 2.67 (s, 2H, CH₂), 2.33 (s, 3H, CH₃), 2.14 (s, 3H, CH₃). HRMS (EI-MS) m/z calcd for C₂₇H₂₈N₅O₂[M+H]⁺: 454.2238, found: 454.2238.

### Example 56: [3-[[4-(5,6-Dimethyl-7H-pyrrolo[2,3-d]pyrimidin-4-yl)-3,6-dihydro-2H-pyridine-1-carbonyl]amino] phenyl] N,N-dimethylcarbamate (94)

The reaction was carried out as described in general procedure **H** using 3-aminophenyldimethyl carbamate (45 mg, 0.25 mmol) and the amine salt **88** (50 mg, 0.17 mmol, 1.2 equiv.) in THF (4 mL). The crude mixture was purified by silica gel column chromatography using a gradient solvent system CH₂Cl₂/acetone from 100/0 up to 50/50 to afford **94** (35 mg, 58%) as a pale yellow solid. R*_{f}* (CH₂Cl₂/acetone 30/70): 0.41. Mp: degradation 142°C. ¹H NMR (400 MHz, DMSO-d₆) δ 11.81 (s, 1H, NH), 8.69 (s, 1H, NH), 8.57 (s, 1H, CH), 7.38 (s, 1H, CH), 7.32 (d, *J* = 8.2 Hz, 1H, CH), 7.22 (t, *J* = 8.1 Hz, 1H, CH), 6.69 (d, *J* = 7.4 Hz, 1H, CH), 5.99 (s, 1H, CH), 4.21 (s, 2H, CH₂), 3.73 (t, *J* = 5.4 Hz, 2H, CH₂), 3.04 (s, 3H, CH₃), 2.91 (s, 3H, CH₃), 2.65 (s, 2H, CH₂), 2.33 (s, 3H, CH₃), 2.13 (s, 3H, CH₃). HRMS (EI-MS) m/z calcd for C₂₃H₂₇N₆O₃ [M+H]⁺: 435.2136, found: 435.2139.

### FAMILY 6.

### Tricyclic derivatives with an alkyl ring in positions 2 and 3 of the pyrrolopyrimidine and an unsubstituted hydropyridine.

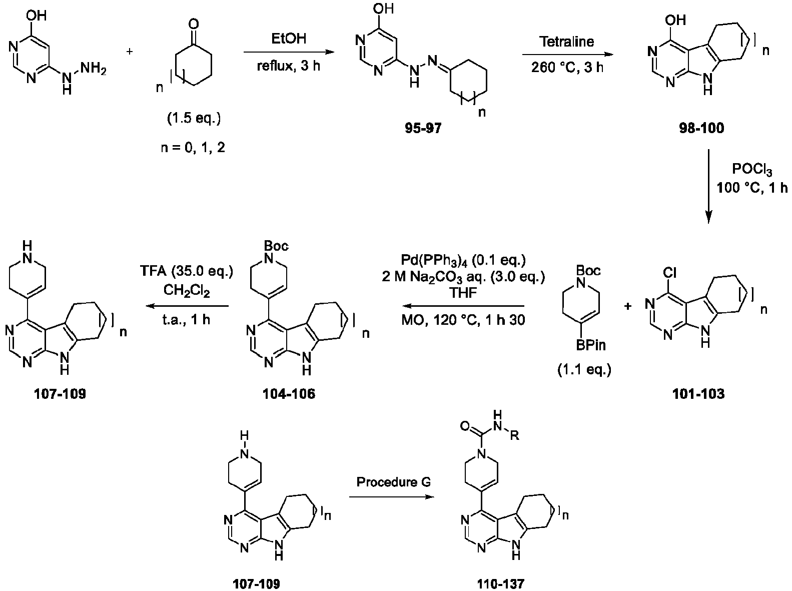

| Compound | General procedure | Yield (%) | n | R |
|---|---|---|---|---|
| **110** | G | 60 | 0 | 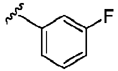 |
| **111** | G | 83 | 0 | 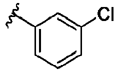 |
| **112** | G | 42 | 0 | 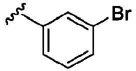 |
| **113** | G | 91 | 1 | 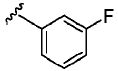 |
| **114** | G | 86 | 1 | 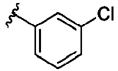 |
| **115** | G | 87 | 1 | 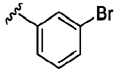 |
| **116** | G | 84 | 2 | 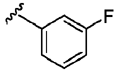 |
| **117** | G | 83 | 2 | 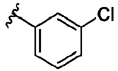 |
| **118** | G | 82 | 2 | 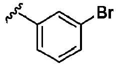 |
| **119** | G | 75 | 0 | 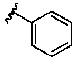 |
| **120** | G | 78 | 1 | 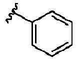 |
| **121** | G | 89 | 0 | 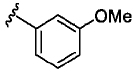 |
| **122** | H | 89 | 0 | 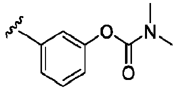 |
| **123** | H | 58 | 0 | 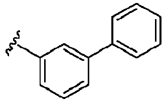 |
| **124** | H | 77 | 0 | 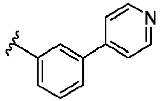 |
| **125** | G | 59 | 0 | 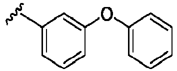 |
| **126** | G | 81 | 1 | 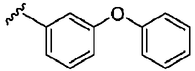 |
| **127** | G | 69 | 0 | 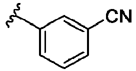 |
| **128** | G | 80 | 0 | 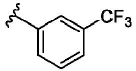 |
| **129** | H | 75 | 0 | 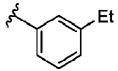 |
| **130** | H | 67 | 0 | 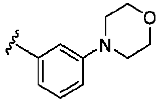 |
| **131** | H | 62 | 0 | 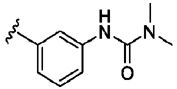 |
| **132** | H | 42 | 0 | 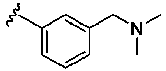 |
| **133** | H | 94 | 0 | 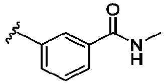 |
| **134** | H | 83 | 0 | 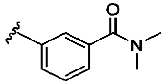 |
| **135** | H | 92 | 0 | 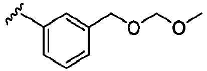 |
| **136** | H | 75 | 0 | 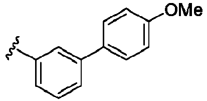 |
| **137** | H | 57 | 0 | 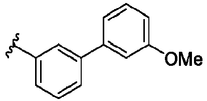 |

### 6-(2-Cyclopentylidenehydrazinyl)pyrimidin-4-ol (95)

The reaction was carried out as described in general procedure **A** using 4-hydroxy-6-hydrazinylpyrimidine (1.0 g, 7.93 mmol) and cyclopentanone (1.05 mL, 11.89 mmol, 1.5 equiv.) in EtOH (16 mL). The precipitate was filtered and washed with Et₂O (2 x 20 mL) to afford compound **95** (1.18 g, 77%) as a white solid. R*_{f}* (CH₂Cl₂/acetone 50/50) 0.10. Mp: > 260°C. ¹H NMR (250 MHz, MeOD-*d₄*) δ 7.91 (s, 1H, CH), 5.84 (s, 1H, CH), 2.52 - 2.27 (m, 4H, CH₂ + CH₂), 1.97 - 1.65 (m, 4H, CH₂ + CH₂). HRMS (EI-MS) m/z calcd for C₉H₁₃N₄O [M+H]⁺: 193.1084, found: 193.1085.

### 6-(2-Cyclohexylidenehydrazinyl)pyrimidin-4-ol (96)

The reaction was carried out as described in general procedure **A** using 4-hydroxy-6-hydrazinylpyrimidine (1.0 g, 7.93 mmol) and cyclohexanone (1.16 mL, 11.89 mmol, 1.5 equiv.) in EtOH (16 mL). The precipitate was filtered and washed with Et₂O (2 x 20 mL) to afford **96** (1.17 g, 72 %) as a white solid. R*_{f}* (CH₂Cl₂/acetone 50/50) 0.11. Mp: > 260 °C. ¹H NMR (250 MHz, MeOD-*d₄*) δ 7.90 (d, *J* = 0.8 Hz, 1H, CH), 5.86 (d, *J* = 0.8 Hz, 1H, CH), 2.49 - 2.39 (m, 2H, CH₂), 2.39 - 2.31 (m, 2H, CH₂), 1.80 - 1.59 (m, 6H, 3 x CH₂). HRMS (EI-MS) m/z calcd for C₁₀H₁₅N₄O [M+H]⁺: 207.1240, found: 207.1239.

### 6-(2-Cycloheptylidenehydrazinyl)pyrimidin-4-ol (97)

The reaction was carried out as described in general procedure **A** using 4-hydroxy-6-hydrazinylpyrimidine (1.0 g, 7.93 mmol) and cycloheptanone (1.4 mL, 11.89 mmol, 1.5 equiv.) in EtOH (16 mL). The precipitate was filtered and washed with Et₂O (2 x 20 mL) to afford compound **97** (1.3 g, 76%) as a beige solid. R*_{f}* (CH₂Cl₂/acetone 50/50) 0.11. Mp: > 260°C. ¹H NMR (400 MHz, DMSO-*d₆*) δ 11.65 (br s, 1H, OH), 9.22 (s, 1H, NH), 7.89 (s, 1H, CH), 5.55 (s, 1H, CH), 2.48 - 2.44 (m, 2H, CH₂), 2.44 - 2.40 (m, 2H, CH₂), 1.68 - 1.60 (m, 2H, CH₂), 1.59 - 1.45 (m, 6H, 3 x CH₂). HRMS (EI-MS) m/z calcd for C₁₁H₁₇N₄O[M+H]⁺: 221.1397, found: 221.1396.

### 5,6,7,8-Tetrahydrocyclopenta[4,5]pyrrolo[2,3-d]pyrimidin-4-ol (98)

The reaction was carried out as described in general procedure **B** using hydrazone **95** (0.5 g, 2.60 mmol) in tetraline (10 mL). The precipitate was filtered and washed several times with PE (3 x 10 mL) and Et₂O (10 mL) to afford compound **98** (0.39 g, 85 %) as a grey solid. R*_{f}* (CH₂Cl₂/MeOH/NH₄OH 90/9/1) 0.33. Mp: > 260°C. ¹H NMR (250 MHz, DMSO-*d₆*) δ 11.66 (br s, 1H, OH), 11.59 (s, 1H, NH), 7.69 (s, 1H, CH), 2.79 - 2.64 (m, 4H, 2 x CH₂), 2.42 - 2.26 (m, 2H, CH₂). HRMS (EI-MS) m/z calcd for C₉H₁₀N₃O[M+H]⁺: 176.0818, found: 176.0821.

### 6,7,8,9-Tetrahydro-5H-pyrimido[4,5-b]indol-4-ol (99)

The reaction was carried out as described in general procedure **B** using hydrazone **96** (0.5 g, 2.42 mmol) in tetraline (10 mL). The precipitate was filtered and washed several times with PE (3 x 10 mL) and Et₂O (10 mL) to afford compound **99** (0.41 g, 89 %) as a beige solid. R*_{f}* (CH₂Cl₂/MeOH/NH₄OH 90/9/1) 0.35. Mp: > 260°C. ¹H NMR (400 MHz, DMSO-*d₆*) δ 11.56 (br s, 1H, OH), 11.37 (s, 1H, NH), 7.68 (s, 1H, CH), 2.66 (t, *J* = 5.9 Hz, 2H, CH₂), 2.55 (t, *J* = 6.0 Hz, 2H, CH₂), 1.79 - 1.65 (m, 4H, 2 x CH₂). HRMS (EI-MS) m/z calcd for C₁₀H₁₂N₃O [M+H]⁺: 190.0975, found: 190.0975.

### 5,6,7,8,9,10-Hexahydrocyclohepta[4,5]pyrrolo[2,3-d]pyrimidin-4-ol (100)

The reaction was carried out as described in general procedure **B** using hydrazone **97** (0.5 g, 2.27 mmol) in tetraline (10 mL). The precipitate was filtered and washed several times with PE (3 x 10 mL) and Et₂O (10 mL) to afford compound **100** (0.41 g, 88 %) as a beige solid. R*_{f}* (CH₂Cl₂/MeOH/NH₄OH 90/9/1) 0.34. Mp: > 260°C. ¹H NMR (250 MHz, DMSO-*d₆*) δ 11.53 (br s, 1H, OH), 11.40 (s, 1H, NH), 7.66 (s, 1H, CH), 3.01 - 2.83 (m, 2H, CH₂), 2.74 - 2.61 (m, 2H, CH₂), 1.83 - 1.72 (m, 2H, CH₂), 1.66 - 1.52 (m, 4H, 2 x CH₂). HRMS (EI-MS) m/z calcd for C₁₁H₁₄N₃O [M+H]⁺: 204.1131, found: 204.1132.

### 4-Chloro-5,6,7,8-tetrahydrocyclopenta[4,5]pyrrolo[2,3-d]pyrimidine (101)

The reaction was carried out as described in general procedure **C** using tricycle **98** (1.0 g, 5.71 mmol) in POCl₃ (40 mL). The crude reaction mixture was purified by silica gel column chromatography using a gradient solvent system of CH₂Cl₂/acetone from 100/0 up to 70/30 to afford **101** (0.83 g, 75%) as a white solid. R*_{f}* (CH₂Cl₂/acetone 80/20) 0.31. Mp: degradation 239°C. ¹H NMR (250 MHz, DMSO-*d₆*) δ 12.31 (br s, 1H, NH), 8.42 (s, 1H, CH), 2.95 - 2.81 (m, 4H, 2 x CH₂), 2.48 - 2.38 (m, 2H, CH₂). HRMS (EI-MS) m/z calcd for C₉H₉ClN₃ [M+H]⁺: 194.0480, found: 194.0480.

### 4-Chloro-6,7,8,9-tetrahydro-5H-pyrimido[4,5-b]indole (102)

The reaction was carried out as described in general procedure **C** using tricycle **99** (1.0 g, 5.28 mmol) in POCl₃ (40 mL). The crude reaction mixture was purified by silica gel column chromatography using a gradient solvent system of CH₂Cl₂/acetone from 100/0 up to 70/30 to afford compound **102** (0.86 g, 78%) as a white solid.R*_{f}* (CH₂Cl₂/acetone 80/20) 0.34. Mp: degradation 240°C. ¹H NMR (250 MHz, DMSO-*d₆*) δ 12.13 (s, 1H, NH), 8.41 (s, 1H, CH), 2.84 - 2.76 (m, 2H, CH₂), 2.76 - 2.67 (m, 2H, CH₂), 1.87 - 1.73 (m, 4H, 2 x CH₂). HRMS (EI-MS) m/z calcd for C₁₀H₁₁ClN₃[M+H]⁺: 208.0636, found: 208.0637.

### 4-Chloro-5,6,7,8,9,10-hexahydrocyclohepta[4,5]pyrrolo[2,3-d]pyrimidine (103)

The reaction was carried out as described in general procedure **C** using tricycle **100** (1.0 g, 4.92 mmol) in POCl₃ (35 mL). The crude reaction mixture was purified by silica gel column chromatography using a gradient solvent system of CH₂Cl₂/acetone from 100/0 up to 70/30 to afford compound **103** (0.81 g, 74%) as an off-white solid. R*_{f}* (CH₂Cl₂/acetone 80/20) 0.36. Mp: 249 - 251 °C. ¹H NMR (250 MHz, DMSO-*d₆*) δ 12.19 (s, 1H, NH), 8.39 (s, 1H, CH), 3.12 - 2.94 (m, 2H, CH₂), 2.89 - 2.77 (m, 2H, CH₂), 1.90 - 1.74 (m, 2H, CH₂), 1.74 - 1.59 (m, 4H, 2 x CH₂). HRMS (EI-MS) m/z calcd for C₁₁H₁₃ClN₃ [M+H]⁺: 222.0793, found: 222.0792.

### tert-Butyl 4-(5,6,7,8-tetrahydrocyclopenta[4,5]pyrrolo[2,3-d]pyrimidin-4-yl)-3,6-dihydropyridine-1(2H)-carboxylate (104)

The reaction was carried out as described in general procedure **D** using the chlorinated tricycle **101** (0.35 g, 1.81 mmol) and *N*-Boc-1,2,3,6-tetrahydropyridine-4-boronic acid pinacol ester (0.62 g, 1.99 mmol, 1.1 equiv.) in THF (9 mL). The crude reaction mixture was purified by silica gel column chromatography using a gradient solvent system of CH₂Cl₂/acetone from 90/10 up to 50/50 to afford compound **104** (0.54 g, 88%) as a white solid. R*_{f}* (CH₂Cl₂/acetone 70/30) 0.23. Mp: 213 - 215°C. ¹H NMR (250 MHz, DMSO-*d₆*) δ 11.95 (s, 1H, NH), 8.55 (s, 1H, CH), 6.56 - 6.46 (m, 1H, CH), 4.15 - 4.04 (m, 2H, CH₂), 3.56 (t, *J* = 5.7 Hz, 2H, CH₂), 2.94 - 2.78 (m, 4H, 2 x CH₂), 2.72 - 2.60 (m, 2H, CH₂), 2.41 (p, *J* = 7.7 Hz, 2H, CH₂), 1.44 (s, 9H, 3 x CH₃). HRMS (EI-MS) m/z calcd for C₁₉H₂₅N₄O₂ [M+H]⁺: 341.1972, found: 341.1970.

### tert-Butyl 4-(6,7,8,9-tetrahydro-5H-pyrimido[4,5-b]indol-4-yl)-3,6-dihydropyridine-1(2H)-carboxylate (105)

The reaction was carried out as described in general procedure **D** using the chlorinated tricycle **102** (0.45 g, 2.17 mmol) and *N*-Boc-1,2,3,6-tetrahydropyridine-4-boronic acid pinacol ester (0.74 g, 2.38 mmol, 1.1 equiv.) in THF (9 mL). The crude reaction mixture was purified by silica gel column chromatography using a gradient solvent system of CH₂Cl₂/acetone from 90/10 up to 50/50 to afford compound **105** (0.67 g, 87%) as a white solid. R*_{f}* (CH₂Cl₂/acetone 70/30) 0.26. Mp: 214 - 216°C. ¹H NMR (250 MHz, DMSO-*d₆*) δ 11.73 (s, 1H, NH), 8.54 (s, 1H, CH), 6.07 - 5.95 (m, 1H, CH), 4.13 - 3.97 (m, 2H, CH₂), 3.64 - 3.49 (m, 2H, CH₂), 2.78 - 2.64 (m, 2H, CH₂), 2.65 - 2.53 (m, 4H, 2 x CH₂), 1.90 - 1.67 (m, 4H, 2 x CH₂), 1.43 (s, 9H, 3 x CH₃). HRMS (EI-MS) m/z calcd for C₂₀H₂₇N₄O₂ [M+H]⁺: 355.2129, found: 355.2129.

### tert-Butyl 4-(5,6,7,8,9,10-hexahydrocyclohepta[4,5]pyrrolo[2,3-d]pyrimidin-4-yl)-3,6-dihydropyridine-1(2H)-carboxylate (106)

The reaction was carried out as described in general procedure **D** using the chlorinated tricycle **103** (0.45 g, 2.03 mmol) and *N*-Boc-1,2,3,6-tetrahydropyridine-4-boronic acid pinacol ester (0.69 g, 2.23 mmol, 1.1 equiv.) in THF (9 mL). The crude reaction mixture was purified by silica gel column chromatography using a gradient solvent system of CH₂Cl₂/acetone from 90/10 up to 50/50 to afford compound **106** (0.50 g, 67%) as a white solid. R*_{f}* (CH₂Cl₂/acetone 70/30) 0.27. Mp: 180 - 182°C. ¹H NMR (250 MHz, DMSO-*d₆*) δ 11.78 (s, 1H, NH), 8.53 (s, 1H, CH), 5.86 (s, 1H, CH), 4.11 - 3.98 (m, 2H, CH₂), 3.65 - 3.51 (m, 2H, CH₂), 2.89 - 2.79 (m, 2H, CH₂), 2.79 - 2.69 (m, 2H, CH₂), 2.63 - 2.53 (m, 2H, CH₂), 1.89 - 1.75 (m, 2H, CH₂), 1.76 - 1.55 (m, 4H, 2 x CH₂), 1.44 (s, 9H, 3 x CH₃). HRMS (EI-MS) m/z calcd for C₂₁H₂₉N₄O₂ [M+H]⁺: 369.2285, found: 369.2286.

### 4-(1,2,3,6-Tetrahydropyridin-4-yl)-5,6,7,8-tetrahydrocyclopenta[4,5] pyrrolo[2,3-d]pyrimidine (107)

The reaction was carried out as described in general procedure **F1** using the *N*-Boc protected compound **104** (1.0 g, 2.94 mmol) in CH₂Cl₂ (80 mL). The reaction was complete after 1 h. Workup gave compound **107** (0.65 g, 92 %) as a beige solid. Mp: > 260°C. ¹H NMR (250 MHz, DMSO-*d₆*) δ 11.91 (br s, 1H, NH), 8.53 (s, 1H, CH), 6.53 (s, 1H, CH), 3.50 - 3.38 (m, 2H, CH₂), 2.98 - 2.75 (m, 6H, 3 x CH₂), 2.59 - 2.45 (m, 2H, CH₂), 2.45 - 2.30 (m, 2H, CH₂). HRMS (EI-MS) m/z calcd for C₁₄H₁₇N₄ [M+H]⁺: 241.1448, found: 241.1446.

### 4-(1,2,3,6-Tetrahydropyridin-4-yl)-6,7,8,9-tetrahydro-5H-pyrimido[4,5-b]indole (108)

The reaction was carried out as described in general procedure **F1** using the *N*-Boc protected compound **105** (1.0 g, 2.82 mmol) in CH₂Cl₂ (80 mL). The reaction was complete after 1 h. Workup gave compound **108** (0.68 g, 95 %) as a light yellow solid. Mp: 187 - 189°C. ¹H NMR (250 MHz, MeOD-*d₄*) δ 8.51 (s, 1H, CH), 6.03 (tt, *J* = 3.3, 1.7 Hz, 1H, CH), 3.54 (dt, *J* = 2.9 Hz, *J* = 2.9 Hz, 2H, CH₂), 3.10 (t, *J* = 5.7 Hz, 2H, CH₂), 2.82 - 2.74 (m, 2H, CH₂), 2.71 - 2.63 (m, 2H, CH₂), 2.65 - 2.51 (m, 2H, CH₂), 2.00 - 1.77 (m, 4H, 2 x CH₂). HRMS (EI-MS) m/z calcd for C₁₅H₁₉N₄ [M+H]⁺: 255.1604, found: 255.1603.

### 4-(1,2,3,6-Tetrahydropyridin-4-yl)-5,6,7,8,9,10-hexahydrocyclohepta[4,5] pyrrolo[2,3-d]pyrimidine ditrifluoroacetate (109)

The reaction was carried out as described in general procedure **F2** using the *N*-Boc protected compound **106** (1.0 g, 2.71 mmol) in CH₂Cl₂ (80 mL). The reaction was complete after 1 h. Workup gave compound **109** (1.17 g, 87 %) as a yellow solid. Mp: degradation 176°C. ¹H NMR (250 MHz, DMSO-*d₆*) δ 12.32 (br s, 1H, NH), 9.21 (s, 2H, NH + H⁺), 8.70 (s, 1H, CH), 6.08 - 5.95 (m, 1H, CH), 3.92 - 3.79 (m, 2H, CH₂), 3.47 - 3.30 (m, 2H, CH₂), 2.94 - 2.85 (m, 2H, CH₂), 2.82 - 2.71 (m, 4H, 2 x CH₂), 1.89 - 1.77 (m, 2H, CH₂), 1.76 - 1.58 (m, 4H, 2 x CH₂). ¹⁹F NMR (235 MHz, DMSO-*d₆*) δ -74.2 (s). HRMS (EI-MS) m/z calcd for C₁₆H₂₁N₄ [M+H]⁺: 269.1761, found: 269.1760.

### Example 57: N-(3-Fluorophenyl)-4-(5,6,7,8-tetrahydrocyclopenta[4,5] pyrrolo[2,3-d]pyrimidin-4-yl)-3,6-dihydropyridine-1(2H)-carboxamide (110)

The reaction was carried out as described in general procedure **G** using amine **107** (60 mg, 0.25 mmol) in anhydrous CH₂Cl₂ (3 mL). The crude reaction mixture was purified by silica gel column chromatography using a gradient solvent system of CH₂Cl₂/acetone from 80/20 up to 30/70 to afford compound **110** (57 mg, 60%) as a light yellow solid. R*_{f}* (CH₂Cl₂/acetone 70/30) 0.26. Mp: degradation 217°C. ¹H NMR (400 MHz, DMSO-*d₆*) δ 11.96 (br s, 1H, NH), 8.77 (br s, 1H, NH), 8.57 (s, 1H, CH), 7.52 - 7.43 (m, 1H, CH), 7.33 - 7.20 (m, 2H, CH), 6.79 - 6.71 (m, 1H, CH), 6.61 - 6.53 (m, 1H, CH), 4.29 - 4.20 (m, 2H, CH₂), 3.71 (t, *J* = 5.6 Hz, 2H, CH₂), 2.92 - 2.82 (m, 4H, 2 x CH₂), 2.78 - 2.71 (m, 2H, CH₂), 2.46 - 2.37 (m, 2H, CH₂). ¹⁹F NMR (376 MHz, DMSO-*d₆*) δ -112.9 (s). HRMS (EI-MS) m/z calcd for C₂₁H₂₁FN₅O [M+H]⁺: 378.1725, found: 378.1724.

### Example 58: N-(3-Chlorophenyl)-4-(5,6,7,8-tetrahydrocyclopenta[4,5] pyrrolo[2,3-d]pyrimidin-4-yl)-3,6-dihydropyridine-1(2H)-carboxamide (111)

The reaction was carried out as described in general procedure **G** using amine **107** (60 mg, 0.25 mmol) in anhydrous CH₂Cl₂ (3 mL). The crude reaction mixture was purified by silica gel column chromatography using a gradient solvent system of CH₂Cl₂/acetone from 80/20 up to 30/70 to afford compound **111** (82 mg, 83%) as a white solid. R*_{f}* (CH₂Cl₂/acetone 70/30) 0.26. Mp: degradation 208°C. ¹H NMR (400 MHz, DMSO-*d₆*) δ 11.96 (br s, 1H, NH), 8.75 (br s, 1H, NH), 8.57 (s, 1H, CH), 7.69 (s, 1H, CH), 7.45 (d, *J* = 8.3 Hz, 1H, CH), 7.26 (t, *J* = 8.1 Hz, 1H, CH), 6.98 (d, *J* = 8.0 Hz, 1H, CH), 6.60 - 6.53 (m, 1H, CH), 4.30 - 4.21 (m, 2H, CH₂), 3.71 (t, *J* = 5.7 Hz, 2H, CH₂), 2.91 - 2.81 (m, 4H, 2 x CH₂), 2.77 - 2.69 (m, 2H, CH₂), 2.46 - 2.37 (m, 2H, CH₂). HRMS (EI-MS) m/z calcd for C₂₁H₂₁ClN₅O [M+H]⁺: 394.1429, found: 394.1430.

### Example 59: N-(3-Bromophenyl)-4-(5,6,7,8-tetrahydrocyclopenta[4,5] pyrrolo[2,3-d]pyrimidin-4-yl)-3,6-dihydropyridine-1(2H)-carboxamide (112)

The reaction was carried out as described in general procedure **G** using amine **107** (60 mg, 0.25 mmol) in anhydrous CH₂Cl₂ (3 mL). The crude reaction mixture was purified by silica gel column chromatography using a gradient solvent system of CH₂Cl₂/acetone from 80/20 up to 30/70 to afford compound **112** (46 mg, 42%) as a beige solid. R*_{f}* (CH₂Cl₂/acetone 70/30) 0.25. Mp: degradation 216°C. ¹H NMR (400 MHz, DMSO-*d₆*) δ 11.96 (br s, 1H, NH), 8.73 (br s, 1H, NH), 8.57 (s, 1H, CH), 7.83 (s, 1H, CH), 7.50 (d, *J* = 8.3 Hz, 1H, CH), 7.20 (t, *J* = 8.0 Hz, 1H, CH), 7.11 (d, *J* = 8.0 Hz, 1H, CH), 6.57 (s, 1H, CH), 4.26 - 4.21 (m, 2H, CH₂), 3.70 (t, *J* = 5.5 Hz, 2H, CH₂), 2.93 - 2.81 (m, 4H, 2 x CH₂), 2.78 - 2.69 (m, 2H, CH₂), 2.45 - 2.37 (m, 2H, CH₂). HRMS (EI-MS) m/z calcd for C₂₁H₂₁BrN₅O [M+H]⁺: 438.0924, found: 438.0925.

### Example 60: N-(3-Fluorophenyl)-4-(6,7,8,9-tetrahydro-5H-pyrimido[4,5-b]indol-4-yl)-3,6-dihydropyridine-1(2H)-carboxamide (113)

The reaction was carried out as described in general procedure **G** using amine **108** (60 mg, 0.24 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound 113 (86 mg, 91 %) as a light yellow solid. R*_{f}* (CH₂Cl₂/acetone 20/80) 0.27. Mp: degradation 246°C. ¹H NMR (400 MHz, DMSO-*d₆*) δ 11.75 (br s, 1H, NH), 8.77 (br s, 1H, NH), 8.56 (s, 1H, CH), 7.49 (dt, *J* = 12.6 Hz, *J* = 2.1 Hz, 1H, CH), 7.34 - 7.21 (m, 2H, 2 x CH), 6.80 - 6.70 (m, 1H, CH), 6.12 - 6.03 (m, 1H, CH), 4.26 - 4.16 (m, 2H, CH₂), 3.72 (t, *J* = 5.6 Hz, 2H, CH₂), 2.75 - 2.65 (m, 4H, 2 x CH₂), 2.58 (t, *J* = 5.8 Hz, 2H, CH₂), 1.87 - 1.78 (m, 2H, CH₂), 1.77 - 1.69 (m, 2H, CH₂). ¹⁹F NMR (376 MHz, DMSO-*d₆*) δ -112.9 (s). HRMS (EI-MS) m/z calcd for C₂₂H₂₃FN₅O[M+H]⁺: 392.1881, found: 392.1880.

### Example 61: N-(3-Chlorophenyl)-4-(6,7,8,9-tetrahydro-5H-pyrimido[4,5-b]indol-4-yl)-3,6-dihydropyridine-1(2H)-carboxamide (114)

The reaction was carried out as described in general procedure **G** using amine **108** (60 mg, 0.24 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound **114** (84 mg, 86 %) as a beige solid. R*_{f}* (CH₂Cl₂/acetone 20/80) 0.28. Mp: degradation 177°C. ¹H NMR (400 MHz, DMSO-*d₆*) δ 11.77 (br s, 1H, NH), 8.76 (br s, 1H, NH), 8.57 (s, 1H, CH), 7.70 (s, 1H, CH), 7.45 (d, *J* = 8.4 Hz, 1H, CH), 7.26 (t, *J* = 8.1 Hz, 1H, CH), 6.99 (d, *J* = 8.8 Hz, 1H, CH), 6.08 (s, 1H, CH), 4.26 - 4.18 (m, 2H, CH₂), 3.72 (t, *J* = 5.6 Hz, 2H, CH₂), 2.76 - 2.64 (m, 4H, 2 x CH₂), 2.61 - 2.55 (m, 2H, CH₂), 1.87 - 1.78 (m, 2H, CH₂), 1.78 - 1.68 (m, 2H, CH₂). HRMS (EI-MS) m/z calcd for C₂₂H₂₃ClN₅O [M+H]⁺: 408.1586, found: 408.1584.

### Example 62: N-(3-Bromophenyl)-4-(6,7,8,9-tetrahydro-5H-pyrimido[4,5-b]indol-4-yl)-3,6-dihydropyridine-1(2H)-carboxamide (115)

The reaction was carried out as described in general procedure **G** using amine **108** (60 mg, 0.24 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound **115** (95 mg, 87%) as a beige solid. R*_{f}* (CH₂Cl₂/acetone 20/80) 0.28. Mp: degradation 198°C. ¹H NMR (400 MHz, DMSO-*d₆*) δ 11.75 (brs, 1H, NH), 8.74 (brs, 1H, NH), 8.56 (s, 1H, CH), 7.86 - 7.80 (m, 1H, CH), 7.50 (d, *J* = 8.1 Hz, 1H, CH), 7.21 (t, *J* = 8.0 Hz, 1H, CH), 7.12 (d, *J* = 8.7 Hz, 1H, CH), 6.07 (s, 1H, CH), 4.26 - 4.16 (m, 2H, CH₂), 3.71 (t, *J* = 5.6 Hz, 2H, CH₂), 2.73 (t, *J* = 5.9 Hz, 2H, CH₂), 2.69 - 2.66 (m, 2H, CH₂), 2.57 (t, *J* = 5.7 Hz, 2H, CH₂), 1.88 - 1.79 (m, 2H, CH₂), 1.78 - 1.68 (m, 2H, CH₂). HRMS (EI-MS) m/z calcd for C₂₂H₂₃BrN₅O [M+H]⁺: 452.1080, found: 452.1079.

### Example 63: N-(3-Fluorophenyl)-4-(5,6,7,8,9,10-hexahydrocyclohepta[4,5] pyrrolo[2,3-d]pyrimidin-4-yl)-3,6-dihydropyridine-1(2H)-carboxamide (116)

The reaction was carried out as described in general procedure **G** using amine **109** (60 mg, 0.16 mmol) in anhydrous CH₂Cl₂ (3 mL). The crude reaction mixture was purified by silica gel column chromatography using a gradient solvent system of CH₂Cl₂/acetone from 100/0 up to 30/70 to afford compound **116** (54 mg, 84%) as a beige solid. R*_{f}* (CH₂Cl₂/acetone 60/40) 0.27. Mp: degradation 151°C. ¹H NMR (400 MHz, DMSO-*d₆*) δ 11.80 (br s, 1H, NH), 8.79 (br s, 1H, NH), 8.55 (s, 1H, H₂ CH), 7.49 (dt, *J* = 12.3 Hz, *J* = 2.2 Hz, 1H, CH), 7.33 - 7.22 (m, 2H, 2 x CH), 6.81 - 6.69 (m, 1H, CH), 5.97 - 5.85 (m, 1H, CH), 4.23 - 4.15 (m, 2H, CH₂), 3.73 (t, *J* = 5.6 Hz, 2H, CH₂), 2.88 - 2.80 (m, 2H, CH₂), 2.80 - 2.73 (m, 2H, CH₂), 2.70 - 2.64 (m, 2H, CH₂), 1.85 - 1.77 (m, 2H, CH₂), 1.73 - 1.66 (m, 2H, CH₂), 1.65 (s, 2H, CH₂). ¹⁹F NMR (376 MHz, DMSO-*d₆*) δ -112.9 (s). HRMS (EI-MS) m/z calcd for C₂₃H₂₅FN₅O [M+H]⁺: 406.2038, found: 406.2035.

### Example 64: N-(3-Chlorophenyl)-4-(5,6,7,8,9,10-hexahydrocyclohepta[4,5] pyrrolo[2,3-d]pyrimidin-4-yl)-3,6-dihydropyridine-1(2H)-carboxamide (117)

The reaction was carried out as described in general procedure **G** using amine **109** (60 mg, 0.16 mmol) in anhydrous CH₂Cl₂ (3 mL). The crude reaction mixture was purified by silica gel column chromatography using a gradient solvent system of CH₂Cl₂/acetone from 100/0 up to 30/70 to afford compound **117** (63 mg, 94%) as a beige solid. R*_{f}* (CH₂Cl₂/acetone 60/40) 0.27. Mp: degradation 163°C. ¹H NMR (400 MHz, DMSO-*d₆*) δ 11.80 (br s, 1H, NH), 8.77 (br s, 1H, NH), 8.55 (s, 1H, CH), 7.70 (s, 1H, CH), 7.45 (d, *J* = 8.4 Hz, 1H, CH), 7.27 (t, *J* = 8.1 Hz, 1H, CH), 6.99 (d, *J* = 8.2 Hz, 1H, CH), 5.96 - 5.87 (m, 1H, CH), 4.27 - 4.10 (m, 2H, CH₂), 3.73 (t, *J* = 5.6 Hz, 2H, CH₂), 2.88 - 2.81 (m, 2H, CH₂), 2.80 - 2.72 (m, 2H, CH₂), 2.71 - 2.62 (m, 2H, CH₂), 1.84 - 1.77 (m, 2H, CH₂), 1.72 - 1.66 (m, 2H, CH₂), 1.66 - 1.60 (m, 2H, CH₂). HRMS (EI-MS) m/z calcd for C₂₃H₂₅ClN₅O [M+H]⁺: 422.1742, found: 422.1740.

### Example 65: N-(3-Bromopheny))-4-(5,6,7,8,9,10-hexahydrocydohepta[4,5] pyrrolo[2,3-d]pyrimidin-4-yl)-3,6-dihydropyridine-1(2H)-carboxamide (118)

The reaction was carried out as described in general procedure **G** using amine **109** (60 mg, 0.16 mmol) in anhydrous CH₂Cl₂ (3 mL). The crude reaction mixture was purified by silica gel column chromatography using a gradient solvent system of CH₂Cl₂/acetone from 100/0 up to 30/70 to afford compound **118** (61 mg, 82%) as a beige solid. R*_{f}* (CH₂Cl₂/acetone 60/40) 0.28. Mp: degradation 178°C. ¹H NMR (400 MHz, DMSO-*d₆*) δ 11.80 (br s, 1H, NH), 8.75 (br s, 1H, NH), 8.55 (s, 1H, CH), 7.83 (s, 1H, CH), 7.50 (d, *J* = 8.2 Hz, 1H, CH), 7.21 (t, *J* = 7.9 Hz, 1H, CH), 7.12 (d, *J* = 8.0 Hz, 1H, CH), 5.98 - 5.86 (m, 1H, CH), 4.26 - 4.13 (m, 2H, CH₂), 3.72 (t, *J* = 5.5 Hz, 2H, CH₂), 2.88 - 2.80 (m, 2H, CH₂), 2.80 - 2.73 (m, 2H, CH₂), 2.70 - 2.62 (m, 2H, CH₂), 1.85 - 1.76 (m, 2H, CH₂), 1.73 - 1.66 (m, 2H, CH₂), 1.66 - 1.60 (m, 2H, CH₂). HRMS (EI-MS) m/z calcd for C₂₃H₂₅BrN₅O [M+H]⁺: 466.1237, found: 466.1234.

### Example 66: N-Phenyl-4-(5,6,7,8-tetrahydrocyclopenta[4,5]pyrrolo[2,3-d]pyrimidin-4-yl)-3,6-dihydropyridine-1(2H)-carboxamide (119)

The reaction was carried out as described in general procedure **G** using amine **107** (60 mg, 0.25 mmol) in anhydrous CH₂Cl₂ (3 mL). The crude reaction mixture was purified by silica gel column chromatography using a gradient solvent system of CH₂Cl₂/acetone from 100/0 up to 40/60 to afford compound **119** (47 mg, 75%) as a light yellow solid. R*_{f}* (CH₂Cl₂/acetone 50/50) 0.24. Mp: degradation 215°C. ¹H NMR (250 MHz, MeOD-*d₄*) δ 8.54 (s, 1H, CH), 7.47 - 7.35 (m, 2H, 2 x CH), 7.35 - 7.21 (m, 2H, 2 x CH), 7.09 - 6.97 (m, 1H, CH), 6.57 - 6.43 (m, 1H, CH), 4.36 - 4.27 (m, 2H, CH₂), 3.81 (t, *J* = 5.6 Hz, 2H, CH₂), 3.03 - 2.86 (m, 4H, 2 x CH₂), 2.86 - 2.73 (m, 2H, CH₂), 2.61 - 2.40 (m, 2H, CH₂). HRMS (EI-MS) m/z calcd for C₂₁H₂₂N₅O [M+H]⁺: 360.1819, found: 360.1819.

### Example 67: N-Phenyl-4-(6,7,8,9-tetrahydro-5H-pyrimido[4,5-b]indol-4-yl)-3,6-dihydropyridine-1(2H)-carboxamide (120)

The reaction was carried out as described in general procedure **G** using amine **108** (60 mg, 0.25 mmol) in anhydrous CH₂Cl₂ (3 mL). The crude reaction mixture was purified by silica gel column chromatography using a gradient solvent system of CH₂Cl₂/acetone from 80/20 up to 80/20 to afford compound **207 120** (69 mg, 78%) as a white solid. R*_{f}* (CH₂Cl₂/acetone 20/80) 0.20. Mp: 255 - 257°C. ¹H NMR (400 MHz, DMSO-*d₆*) δ 11.74 (br s, 1H, NH), 8.56 (s, 2H, NH + CH), 7.50 (d, *J* = 8.0 Hz, 2H, 2 x CH), 7.24 (t, *J* = 7.7 Hz, 2H, 2 x CH), 6.94 (t, *J* = 7.3 Hz, 1H, CH), 6.07 (s, 1H, CH), 4.27 - 4.12 (m, 2H, CH₂), 3.71 (t, *J* = 5.6 Hz, 2H, CH₂), 2.78 - 2.63 (m, 4H, 2 x CH₂), 2.63 - 2.54 (m, 2H, CH₂), 1.87 - 1.77 (m, 2H, CH₂), 1.77 - 1.69 (m, 2H, CH₂). HRMS (EI-MS) m/z calcd for C₂₂H₂₄N₅O [M+H]⁺: 374.1975, found: 374.1972.

### Example 68: N-(3-Methoxyphenyl)-4-(5,6,7,8-tetrahydrocyclopenta[4,5] pyrrolo[2,3-d]pyrimidin-4-yl)-3,6-dihydropyridine-1(2H)-carboxamide (121)

The reaction was carried out as described in general procedure **G** using amine **107** (60 mg, 0.25 mmol) in anhydrous CH₂Cl₂ (3 mL). The crude reaction mixture was purified by silica gel column chromatography using a gradient solvent system of CH₂Cl₂/acetone from 100/0 up to 40/60 to afford compound **121** (55 mg, 89%) as a white solid. R*_{f}* (CH₂Cl₂/acetone 50/50) 0.21. Mp: degradation 206°C. ¹H NMR (250 MHz, MeOD-*d₄*) δ 8.54 (s, 1H, CH), 7.16 (t, *J* = 8.1 Hz, 1H, CH), 7.09 (t, *J* = 2.2 Hz, 1H, CH), 6.97 (ddd, *J* = 8.1 Hz, *J* = 2.0 Hz, *J* = 1.0 Hz, 1H, CH), 6.60 (ddd, *J* = 8.2 Hz, *J* = 2.5 Hz, *J* = 1.0 Hz, 1H, CH), 6.48 (m, 1H, CH), 4.34 - 4.25 (m, 2H, CH₂), 3.80 (t, *J* = 5.6 Hz, 2H, CH₂), 3.78 (s, 3H, OCH₃), 3.05 - 2.85 (m, 4H, 2 x CH₂), 2.85 - 2.73 (m, 2H, CH₂), 2.62 - 2.42 (m, 2H, CH₂). HRMS (EI-MS) m/z calcd for C₂₂H₂₄N₅O₂[M+H]⁺: 390.1925, found: 390.1923.

### Example 69: 3-(4-(5,6,7,8-Tetrahydrocyclopenta[4,5]pyrrolo[2,3-d] pyrimidin-4-yl)-1,2,3,6-tetrahydropyridine-1-carboxamido)phenyl dimethyl carbamate (122)

The reaction was carried out as described in general procedure **H** using 3-aminophenyl dimethylcarbamate (45 mg, 0.25 mmol) and amine **107** (72 mg, 0.30 mmol, 1.2 equiv.) in THF (4 mL). The crude reaction mixture was purified by silica gel column chromatography using a gradient solvent system of CH₂Cl₂/acetone from 80/20 up to 10/90 to afford compound **122** (99 mg, 89%) as a light yellow solid. R*_{f}* (CH₂Cl₂/acetone 20/80) 0.24. Mp: 227 - 229°C. ¹H NMR (250 MHz, DMSO-*d₆*) δ 11.96 (s, 1H, NH), 8.68 (br s, 1H, NH), 8.58 (s, 1H, H₂), 7.38 (d, *J* = 2.2 Hz, 1H, CH), 7.32 (d, *J* = 8.1 Hz, 1H, CH), 7.22 (t, *J* = 8.0 Hz, 1H, CH), 6.69 (d, *J* = 7.9 Hz, 1H, CH), 6.62 - 6.53 (m, 1H, CH), 4.36 - 4.18 (m, 2H, CH₂), 3.70 (t, *J* = 5.3 Hz, 2H, CH₂), 3.03 (s, 3H, NCH₃), 2.97 - 2.81 (m, 7H, 2 x CH₂ + NCH₃), 2.80 - 2.69 (m, 2H, CH₂), 2.46 - 2.35 (m, 2H, CH₂). HRMS (EI-MS) m/z calcd for C₂₄H₂₇N₆O₃ [M+H]⁺: 447.2139, found: 447.2141.

### Example 70: N-([1,1'-Biphenyl]-3-yl)-4-(5,6,7,8-tetrahydrocyclopenta[4,5] pyrrolo[2,3-d]pyrimidin-4-yl)-3,6-dihydropyridine-1(2H)-carboxamide (123)

The reaction was carried out as described in general procedure **H** using 3-aminobiphenyl (42 mg, 0.25 mmol) and amine **107** (72 mg, 0.30 mmol, 1.2 equiv.) in THF (4 mL). The crude reaction mixture was purified by silica gel column chromatography using a gradient solvent system of CH₂Cl₂/acetone from 80/20 up to 10/90 to afford compound **123** (63 mg, 58%) as a white solid. R*_{f}* (CH₂Cl₂/acetone 50/50) 0.26. Mp: 245 - 247°C. ¹H NMR (250 MHz, DMSO-*d₆*) δ 11.96 (br s, 1H, NH), 8.66 (br s, 1H, NH), 8.58 (s, 1H, CH), 7.87 - 7.78 (m, 1H, CH), 7.66 - 7.58 (m, 2H, 2 x CH), 7.57 - 7.41 (m, 3H, 3 x CH), 7.40 - 7.28 (m, 2H, 2 x CH), 7.28 - 7.17 (m, 1H, CH), 6.66 - 6.52 (m, 1H, CH), 4.32 - 4.23 (m, 2H, CH₂), 3.73 (t, *J* = 5.5 Hz, 2H, CH₂), 2.94 - 2.81 (m, 4H, 2 x CH₂), 2.79 - 2.70 (m, 2H, CH₂), 2.48 - 2.35 (m, 2H, CH₂). HRMS (EI-MS) m/z calcd for C₂₇H₂₆N₅O [M+H]⁺: 436.2132, found: 436.2131.

### Example 71: N-(3-(Pyridin-4-yl)phenyl)-4-(5,6,7,8-tetrahydrocyclopenta [4,5]pyrrolo[2,3-d]pyrimidin-4-yl)-3,6-dihydropyridine-1(2H)-carboxamide (124)

The reaction was carried out as described in general procedure **H** using 3-(pyridin-4-yl)aniline (43 mg, 0.25 mmol) and amine **107** (72 mg, 0.30 mmol, 1.2 equiv.) in THF (4 mL). The crude reaction mixture was purified by silica gel column chromatography using a gradient solvent system of CH₂Cl₂/acetone from 60/40 up to 0/100 to afford compound **124** (84 mg, 77%) as an offwhite solid. R*_{f}* (Acetone) 0.16. Mp: > 260°C. ¹H NMR (400 MHz, DMSO-*d₆*) δ 11.97 (br s, 1H, NH), 8.74 (br s, 1H, NH), 8.64 (d, *J* = 5.7 Hz, 2H, 2 x CH), 8.58 (s, 1H, CH), 7.98 - 7.93 (m, 1H, CH), 7.66 - 7.61 (m, 3H, 3 x CH), 7.44 - 7.34 (m, 2H, 2 x CH), 6.64 - 6.56 (m, 1H, CH), 4.32 - 4.24 (m, 2H, CH₂), 3.74 (t, *J* = 5.6 Hz, 2H, CH₂), 2.92 - 2.82 (m, 4H, 2 x CH₂), 2.80 - 2.72 (m, 2H, CH₂), 2.46 - 2.37 (m, 2H, CH₂). HRMS (EI-MS) m/z calcd for C₂₆H₂₅N₆O [M+H]⁺: 437.2084, found: 437.2080.

### Example 72: N-(3-Phenoxyphenyl)-4-(5,6,7,8-tetrahydrocyclopenta[4,5] pyrrolo[2,3-d]pyrimidin-4-yl)-3,6-dihydropyridine-1(2H)-carboxamide (125)

The reaction was carried out as described in general procedure **G** using amine **107** (60 mg, 0.25 mmol) in anhydrous CH₂Cl₂ (3 mL). The crude reaction mixture was purified by silica gel column chromatography using a gradient solvent system of CH₂Cl₂/acetone from 80/20 up to 30/70 to afford compound **125** (67 mg, 59%) as a yellow solid. R*_{f}* (CH₂Cl₂/acetone 50/50) 0.26. Mp: degradation 154°C. ¹H NMR (400 MHz, DMSO-*d₆*) δ 11.96 (br s, 1H, NH), 8.64 (br s, 1H, NH), 8.57 (s, 1H, CH), 7.39 (t, *J* = 7.8 Hz, 2H, 2 x CH), 7.33 - 7.20 (m, 3H, 3 x CH), 7.13 (t, *J=* 7.4 Hz, 1H, CH), 7.01 (d, *J* = 8.0 Hz, 2H, 2 x CH), 6.61 (d, *J* = 8.0 Hz, 1H, CH), 6.58 - 6.53 (m, 1H, CH), 4.25 - 4.16 (m, 2H, CH₂), 3.68 (t, *J* = 5.6 Hz, 2H, CH₂), 2.91 - 2.81 (m, 4H, 2 x CH₂), 2.76 - 2.67 (m, 2H, CH₂), 2.45 - 2.37 (m, 2H, CH₂). HRMS (EI-MS) m/z calcd for C₂₇H₂₆N₅O₂ [M+H]⁺: 452.2081, found: 452.2079.

### Example 73: N-(3-Phenoxyphenyl)-4-(6,7,8,9-tetrahydro-5H-pyrimido[4,5-b]indol-4-yl)-3,6-dihydropyridine-1(2H)-carboxamide (126)

The reaction was carried out as described in general procedure G using amine 108 (60 mg, 0.24 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound **126** (87 mg, 81%) as an off-white solid. R*_{f}* (CH₂Cl₂/acetone 20/80) 0.31. Mp: degradation 171°C. ¹H NMR (400 MHz, DMSO-*d₆*) δ 11.74 (br s, 1H, NH), 8.65 (br s, 1H, NH), 8.56 (s, 1H, CH), 7.39 (t, *J* = 7.8 Hz, 2H, 2 x CH), 7.34 - 7.20 (m, 3H, 3 x CH), 7.13 (t, *J* = 7.4 Hz, 1H, CH), 7.01 (d, *J* = 8.0 Hz, 2H, 2 x CH), 6.61 (d, *J* = 8.0 Hz, 1H, CH), 6.08 - 6.01 (m, 1H, CH), 4.23 - 4.11 (m, 2H, CH₂), 3.69 (t, *J* = 5.6 Hz, 2H, CH₂), 2.75 - 2.69 (m, 2H, CH₂), 2.69 - 2.63 (m, 2H, CH₂), 2.60 - 2.54 (m, 2H, CH₂), 1.86 - 1.78 (m, 2H, CH₂), 1.78 - 1.68 (m, 2H, CH₂). HRMS (EI-MS) m/z calcd for C₂₆H₂₆N₅O₂ [M+H]⁺: 466.2238, found: 466.2236.

### Example 74: N-(3-Cyanophenyl)-4-(7,9,11-triazatricyclo[6.4.0.02,6] dodeca-1(8),2(6),9,11-tetraen-12-yl)-3,6-dihydropyridine-1(2H)-carboxamide (127)

The reaction was carried out as described in general procedure **G** using **107.HCl** (50 mg, 0. 16 mmol) in anhydrous CH₂Cl₂ (3 mL). The crude reaction mixture was purified by silica gel column chromatography using a gradient solvent system CH₂Cl₂/acetone from 100/0 up to 40/60 to afford compound **127** (42 mg, 69%) as a white solid. R*_{f}* (CH₂Cl₂/acetone 50/50) 0.4. Mp: degradation >266°C. ¹H NMR (250 MHz, DMSO-*d₆*) δ 11.97 (s, 1H), 8.92 (s, 1H), 8.58 (s, 1H), 7.98 (s, 1H), 7.80 (d, J = 8.1 Hz, 1H), 7.57 - 7.30 (m, 2H), 6.58 (s, 1H), 4.34 - 4.13 (m, 2H), 3.72 (t, *J* = 5.8 Hz, 2H), 2.94 - 2.81 (m, 4H), 2.76 (brs, 2H), 2.47 - 2.33 (m, 2H). HRMS (EI-MS) m/z calcd for C₂₂H₂₁N₆O[M+H]⁺: 385.1771, found: 385.1768.

### Example 75: 4-(7,9,11-Triazatricyclo[6.4.0.02,6]dodeca-1(8),2(6),9,11-tetraen-12-yl)-N[3(trifluoromethyl)phenyl]-3,6-dihydropyridine-1(2H)-carboxamide (128)

The reaction was carried out as described in general procedure **G** using the amine salt **107.HCl** (50 mg, 0.15 mmol) in anhydrous CH₂Cl₂ (3 mL). The crude reaction mixture was purified by silica gel column chromatography using a gradient solvent system CH₂Cl₂/acetone from 100/0 up to 50/50 to afford compound **128** (55 mg, 80%) as a white solid. R*_{f}* (CH₂Cl₂/acetone 50/50) 0.22. Mp: degradation 220°C. ¹H NMR (250 MHz, DMSO-*d₆*) δ 11.90 (s, 1H, NH), 8.86 (s, 1H, NH), 8.57 (s, 1H, CH), 7.96 (s, 1H, CH), 7.80 (d, *J* = 8.4 Hz, 1H, CH), 7.48 (t, *J* = 8.0 Hz, 1H, CH), 7.27 (d, *J* = 8.2 Hz, 1H, CH), 6.73 - 6.46 (m, 1H, CH), 4.31 - 4.23 (m, 2H, CH₂), 3.73 (t, *J* = 5.6 Hz, 2H, CH₂), 2.95 - 2.81 (m, 4H, CH₂), 2.75 (brs, 2H, CH₂), 2.47 - 2.32 (m, 2H, CH₂). ¹⁹F NMR (235 MHz, DMSO-*d₆*) δ -61.26 (CF₃). HRMS (EI-MS) m/z calcd for C₂₂H₂₁F₃N₅O[M+H]⁺: 428.1693, found: 428.1690.

### Example 76: N-(3-Ethylphenyl)-4-(7,9,11-triazatricyclo[6.4.0.02,6]dodeca-1(8),2(6),9,11-tetraen-12-yl)-3,6-dihydropyridine-1(2H)-carboxamide (129)

The reaction was carried out as described in general procedure **H** using the amine salt **107.HCl** (58 mg, 0.19 mmol) in THF (4 mL). The crude reaction mixture was purified by silica gel column chromatography using a gradient solvent system CH₂Cl₂/acetone from 100/0 up to 60/40 to afford compound **129** (45 mg, 75%) as a light yellow solid. R*_{f}* (CH₂Cl₂/acetone 70/30) 0.24. Mp: degradation 192°C. ¹H NMR (250 MHz, MeOD-*d₄*) δ 8.54 (s, 1H, CH), 7.35 - 7.10 (m, 3H, CH), 6.97 - 6.81 (m, 1H, CH), 6.48 (s, 1H, CH), 4.36 - 4.20 (m, 2H, CH₂), 3.79 (t, J = 5.6 Hz, 2H, CH₂), 2.99 - 2.84 (m, 4H, CH₂), 2.80 (s, 2H, CH₂), 2.69 - 2.41 (m, 4H, CH₂), 1.23 (t, J = 7.6 Hz, 3H, CH₃). HRMS (EI-MS) m/z calcd for C₂₃H₂₆N₅O[M+H]⁺: 388.2132, found: 388.2130.

### Example 77: N-(3-morpholinophenyl)-4-(7,9,11-triazatricyclo[6.4.0.02,6] dodeca-1(8),2(6),9,11-tetraen-12-yl)-3,6-dihydropyridine-1(2H)-carboxamide (130)

The reaction was carried out as described in general procedure **H** using the amine salt **107.HCl** (51 mg, 0.16 mmol) in THF (4 mL). The crude reaction mixture was purified by silica gel column chromatography using a gradient solvent system CH₂Cl₂/acetone from 100/0 up to 40/60 to afford **130** (40 mg, 67%) as a light yellow solid. R*_{f}* (CH₂Cl₂/acetone 30/70) 0.32. Mp: degradation 250°C. ¹H NMR (250 MHz, DMSO-*d₆*) δ 11.96 (s, 1H, NH), 8.57 (s, 1H, CH), 8.42 (s, 1H, NH), 7.30 - 6.90 (m, 3H, CH), 6.57 (brs, 2H, CH + CH), 4.24 (s, 2H, CH₂), 3.86 - 3.58 (m, 6H, CH₂), 3.05 (brs, 4H, CH₂), 2.87 (brs, 4H, CH₂), 2.73 (s, 2H, CH₂), 2.45 - 2.29 (m, 2H, CH₂). HRMS (EI-MS) m/z calcd for C₂₅H₂₉N₆O_{2[}M+H]⁺: 445.2347, found: 445.2347.

### Example 78: N-[3-(Dimethylcarbamoylamino)phenyl]-4-(7,9,11-triazatricyclo[6.4.0.02,6]dodeca-1(8),2(6),9,11-tetraen-12-yl)-3,6-dihydropyridine-1(2H)-carboxamide (131)

The reaction was carried out as described in general procedure **H** using the amine salt **107.HCl** (51 mg, 0.16 mmol) in THF (4 mL). The crude reaction mixture was purified by silica gel column chromatography using a gradient solvent system of CH₂Cl₂/acetone/MeOH from 100/0/0 up to 0/95/5 to afford compound **131** (37 mg, 62%) as a white solid. R*_{f}* (acetone) 0.42. Mp: degradation 208°C. ¹H NMR (250 MHz, DMSO-*d₆*) δ 11.99 (s, 1H, NH), 8.58 (s, 1H, CH), 8.52 (s, 1H, NH), 8.20 (s, 1H, NH), 7.66 (s, 1H, CH), 7.06 (s, 3H, CH), 6.57 (s, 1H, CH), 4.24 (s, 2H, CH₂), 3.69 (t, J = 5.4 Hz, 2H, CH₂), 2.98 - 2.80 (m, 10H, 2x CH₃ + 2x CH₂), 2.73 (s, 2H, CH₂), 2.46 - 2.30 (m, 2H, CH₂). HRMS (EI-MS) m/z calcd for C₂₄H₂₈N₇O₂ [M+H]⁺: 446.2299, found: 446.2300.

### Example 79: N-[3-[(dimethylamino)methyl]phenyl]-4-(7,9.11-triaza-tricyclo[6.4.0.02,6]dodeca-1(8),2(6),9,11-tetraen-12-yl)-3,6-dihydropyridine-1(2H)-carboxamide (132)

The reaction was carried out as described in general procedure H using the amine salt **107.HCl** (54 mg, 0.17 mmol) in THF (4 mL). The crude reaction mixture was purified by silica gel column chromatography using a gradient solvent system of CH₂Cl₂/acetone (0.1% aq. NH₃) from 100/0 up to 86/14 to afford compound 132 (25 mg, 42%) as a light yellow solid. R*_{f}* (CH₂Cl₂/ MeOH/ NH_{3 aq} 91/9/0.1) 0.21. Mp: degradation 180°C. ¹H NMR (250 MHz, DMSO-*d₆*) δ 11.97 (s, 1H, NH), 8.70 - 8.43 (m, 2H, NH + CHₐᵣ), 7.50 (s, 1H, CH), 7.44 (d, *J* = 8.3 Hz, 1H, CH), 7.21 (t, *J* = 7.8 Hz, 1H, CH), 6.91 (d, *J* = 7.5 Hz, 1H, CH), 6.57 (s, 1H, CH), 4.25 (s, 2H, CH₂), 3.70 (t, *J* = 4.4 Hz, 2H, CH₂), 3.50 (s, 2H, CH₂), 3.08 - 2.80 (m, 4H, CH₂), 2.73 (s, 2H, CH₂), 2.45 - 2.32 (m, 2H, CH₂), 2.25 (s, 6H, CH₃). HRMS (EI-MS) m/z calcd for C₂₄H₂₉N₆O[M+H]⁺: 417.2397, found: 417.2397.

### Example 80: N-[3-(Methylcarbamoyl)phenyl]-4-(7,9,11-triazatricyclo [6.4.0.02,6]dodeca-1(8),2(6),9,11-tetraen-12-yl)-3,6-dihydropyridine-1(2H)-carboxamide (133)

The reaction was carried out as described in general procedure **H** using the amine salt **107.HCl** (53 mg, 0.17 mmol) in THF (4 mL). The crude reaction mixture was purified by silica gel column chromatography using a gradient solvent system of CH₂Cl₂/acetone from 100/0 up to 10/90 to afford compound **133** (55 mg, 94 %) as a white solid. R*_{f}* (CH₂Cl₂/ acetone 10/90) 0.27. Mp: degradation 228°C. ¹H NMR (250 MHz, DMSO-*d₆*) δ 11.96 (s, 1H, NH), 8.74 (s, 1H, NH), 8.58 (s, 1H, CH), 8.39 - 8.28 (m, 1H, CH), 7.95 (s, 1H, CH), 7.68 (d, *J* = 8.1 Hz, 1H, CH), 7.47 - 7.22 (m, 2H, CH), 6.58 (s, 1H, CH), 4.27 (s, 2H, CH₂), 3.72 (t, *J* = 5.5 Hz, 2H, CH₂), 2.94 - 2.81 (m, 4H, 2 x CH₂), 2.81 - 2.67 (m, 5H, CH₂, CH₃), 2.46 - 2.37 (m, 2H, CH₂). HRMS (EI-MS) m/z calcd for C₂₃H₂₅N₆O₂[M+H]⁺: 417.2033, found: 417.2034.

### Example 81: N-[3-(Dimethylcarbamoyl)phenyl]-4-(7,9,11-triazatricyclo [6.4.0.02,6]dodeca-1(8),2(6),9,11-tetraen-12-yl)-3,6-dihydropyridine-1(2H)-carboxamide (134)

The reaction was carried out as described in general procedure **H** using the amine salt **107.HCl** (52 mg, 0.17 mmol) in THF (4 mL). The crude reaction mixture was purified by silica gel column chromatography using a gradient solvent system of CH₂Cl₂/acetone from 100/0 up to 05/95 to afford compound **134** (50 mg, 83%) as a white solid. R*_{f}* (acetone) 0.21. Mp: degradation 245°C ¹H NMR (400 MHz, DMSO-*d₆*) δ 11.96 (s, 1H, NH), 8.70 (s, 1H, NH), 8.57 (s, 1H, CH), 7.59 - 7.53 (m, 2H, CH), 7.30 (t, *J* = 7.8 Hz, 1H, CH), 6.95 (d, *J* = 7.6, Hz, 1H, CH), 6.62 - 6.52 (m, 1H, CH), 4.28 - 4.24 (m, 2H, CH₂), 3.71 (t, *J* = 5.5 Hz, 2H, CH₂), 3.03 - 2.81 (m, 10H, CH₃ + CH₂), 2.74 (m, 2H, CH₂), 2.48 - 2.34 (m, 2H, CH₂). HRMS (EI-MS) m/z calcd for C₂₄H₂₆N₆O₂[M+H]⁺: 431.2190, found: 431.2189.

### Example 82: N-[3-(Methoxymethoxymethyl)phenyl]-4-(7,9,11-triazatricyclo[6.4.0.02,6]dodeca-1(8),2(6),9,11-tetraen-12-yl)-3,6-dihydropyridine-1(2H)-carboxamide (135)

The reaction was carried out as described in general procedure **H** using the amine salt **107.HCl** (113 mg, 0.36 mmol) in THF (8 mL). The crude reaction mixture was purified by silica gel column chromatography using a gradient solvent system of CH₂Cl₂/acetone from 100/0 up to 30/70 to afford compound **135** (120 mg, 92%) as a white solid. R*_{f}* (CH₂Cl₂/ acetone 30/70) 0.35. Mp: degradation 196°C. ¹H NMR (250 MHz, DMSO-*d₆*) δ 11.95 (s, 1H, NH), 8.67 - 8.49 (m, 2H, NH + CH), 7.58 - 7.35 (m, 2H, CH), 7.21 (t, *J* = 7.7 Hz, 1H, CH), 6.90 (d, J = 7.4 Hz, 1H, CH), 6.65 - 6.43 (m, 1H, CH), 4.64 (s, 2H, CH₂), 4.47 (s, 2H, CH₂), 4.2 (s, 2H, CH₂), 3.70 (t, *J* = 5.3 Hz, 2H, CH₂), 3.30 (s, 3H, CH₃), 2.99 - 2.80 (m, 4H, CH₂), 2.73 (s, 2H, CH₂), 2.47 - 2.33 (m, 2H, CH₂). HRMS (EI-MS) m/z calcd for C₂₄H₂₈N₅O₃[M+H]⁺: 434.2187, found: 434.2186.

### Example 83: N-[3-(4-Methoxyphenyl)phenyl]-4-(7,9,11-triaza-tricyclo[6.4.0.02,6]dodeca-1(8),2(6),9,11-tetraen-12-yl)-3,6-dihydropyridine-1(2H)-carboxamide (136)

The reaction was carried out as described in general procedure **H** using **107.HCl** (48 mg, 0.15 mmol) in THF (3 mL). The crude reaction mixture was purified by silica gel column chromatography using a gradient solvent system CH₂Cl₂/acetone from 100/0 up to 10/90 to afford compound **136** (45 mg, 75%) as a pale yellow solid. R*_{f}* (CH₂Cl₂/ acetone 20/80) 0.56. Mp: degradation 240°C. ¹H NMR (400 MHz, DMSO-*d₆*) δ 11.96 (s, 1H, NH), 8.63 (s, 1H, NH), 8.58 (s, 1H, CH), 7.77 (s, 1H, CH), 7.55 (d, J = 8.3 Hz, 2H, CH), 7.48 (d, J = 8.1 Hz, 1H, CH), 7.30 (t, J = 7.9 Hz, 1H, CH), 7.19 (d, J = 7.7 Hz, 1H, CH), 7.02 (d, J = 8.3 Hz, 2H, CH), 6.59 (s, 1H, CH), 4.27 (s, 2H, CH₂), 3.79 (s, 3H, CH₃), 3.73 (t, *J* = 4.8 Hz, 2H, CH₂), 2.88 (s, 4H, CH₂), 2.75 (s, 2H, CH₂), 2.47 - 2.36 (m, 2H, CH₂). HRMS (EI-MS) m/z calcd for C₂₈H₂₈N₅O₂[M+H]⁺: 466.2238, found: 466.2240.

### Example 84: N-[3-(3-Methoxypheny))pheny]-4-(7,9,11-triazatricyclo [6.4.0.02,6]dodeca-1(8),2(6),9,11-tetraen-12-yl)-3,6-dihydropyridine-1(2H)-carboxamide (137)

The reaction was carried out as described in general procedure **H** using the amine salt **107.HCl** (48 mg, 0.15 mmol) in THF (3 mL). The crude reaction mixture was purified by silica gel column chromatography using a gradient solvent system CH₂Cl₂/acetone from 100/0 up to 40/60 to afford compound 137 (34 mg, 57%) as a white solid. R*_{f}* (CH₂Cl₂/ acetone 40/60) 0.53. Mp: degradation 208°C. ¹H NMR (400 MHz, DMSO-*d₆*) δ 11.96 (s, 1H, NH), 8.65 (s, 1H, NH), 8.58 (s, 1H, CH), 7.80 (s, 1H, CH), 7.55 (d, J = 8.0 Hz, 1H, CH), 7.38 (t, J = 8.0 Hz, 1H, CH), 7.33 (t, J = 7.9 Hz, 1H, CH), 7.24 (d, J = 8.3 Hz, 1H, CH), 7.18 (d, J = 7.7 Hz, 1H, CH), 7.13 (s, 1H, CH), 6.94 (dd, J = 8.2, 2.5 Hz, 1H, CH), 6.59 (s, 1H, CH), 4.31 - 4.23 (m, 2H, CH₂), 3.82 (s, 3H, CH₃), 3.73 (t, J = 5.6 Hz, 2H, CH₂), 2.97 - 2.84 (m, 4H, CH₂), 2.76 (s, 2H, CH₂), 2.47 - 2.37 (m, 2H, CH₂). HRMS (EI-MS) m/z calcd for C₂₈H₂₈N₅O₂[M+H]⁺: 466.2238, found: 466.2232.

### FAMILY 7.

### Tricyclic derivatives with a heteroalkyl ring in positions 2 and 3 of the pyrrolopyridine and an unsubstituted hydropyridine

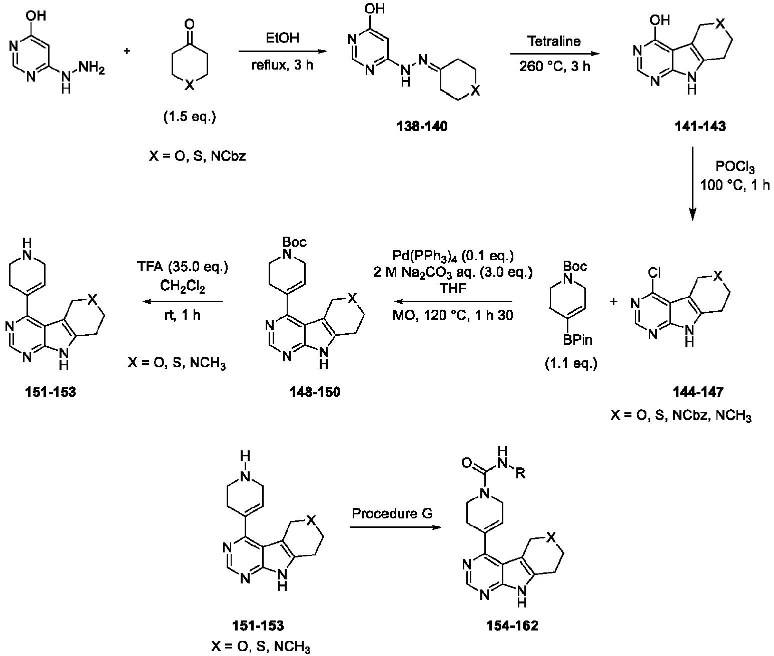

| Compound | General procedure | Yield (%) | X | R |
|---|---|---|---|---|
| **154** | G | 83 | S | 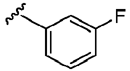 |
| **155** | G | 81 | S | 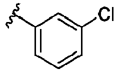 |
| **156** | G | 82 | S | 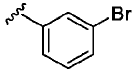 |
| **157** | G | 67 | O | 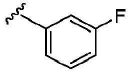 |
| **158** | G | 72 | O | 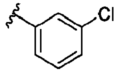 |
| **159** | G | 71 | O | 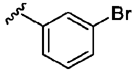 |
| **160** | G | 68 | NCH₃ | 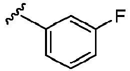 |
| **161** | G | 53 | NCH₃ | 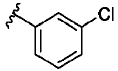 |
| **162** | G | 50 | NCH₃ | 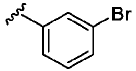 |

### 6-(2-(Tetrahydro-4H-thiopyran-4-ylidene)hydrazinyl)pyrimidin-4-ol (138)

The reaction was carried out as described in general procedure **A** using 4-hydroxy-6-hydrazinyl pyrimidine (1.0 g, 7.93 mmol) and tetrahydro-4*H*-thiopyran-4-one (1.38 mL, 11.89 mmol, 1.5 equiv.) in EtOH (16 mL). The precipitate was filtered and washed with Et₂O (2 x 20 mL) to afford compound **138** (1.51 g, 85%) as a white solid. R*_{f}* (CH₂Cl₂/acetone 50/50) 0.14. Mp: > 260°C. ¹H NMR (250 MHz, DMSO-*d₆*) δ 11.67 (br s, 1H, OH), 9.77 (s, 1H, NH), 7.90 (d, *J=* 0.8 Hz, 1H, CH), 5.56 (d, *J=* 0.8 Hz, 1H, CH), 2.81 - 2.67 (m, 6H, 3 x CH₂), 2.61 - 2.53 (m, 2H, CH₂). HRMS (EI-MS) m/z calcd for C₉H₁₃N₄OS [M+H]⁺: 225.0805, found: 225.0804.

### 6-(2-(Tetrahydro-4H-pyran-4-ylidene)hydrazinyl)pyrimidin-4-ol (139)

The reaction was carried out as described in general procedure **A** using 4-hydroxy-6-hydrazinyl pyrimidine (1.0 g, 7.93 mmol) and tetrahydro-4*H*-pyran-4-one (1.11 mL, 11.89 mmol, 1.5 equiv.) in EtOH (16 mL). The precipitate was filtered and washed with Et₂O (2 x 20 mL) to afford compound **139** (1.28 g, 77 %) as a beige solid. R*_{f}* (CH₂Cl₂/acetone 50/50) 0.06. Mp: > 260°C. ¹H NMR (250 MHz, DMSO-*d₆*) δ 11.65 (br s, 1H, OH), 9.74 (s, 1H, NH), 7.89 (d, *J* = 0.8 Hz, 1H, CH), 5.56 (d, *J* = 0.8 Hz, 1H, CH), 3.73 (t, *J* = 5.7 Hz, 2H, CH₂), 3.65 (t, *J* = 5.8 Hz, 2H, CH₂), 2.55 (t, *J* = 5.8 Hz, 2H, CH₂), 2.36 (t, *J* = 5.6 Hz, 2H, CH₂ HRMS (EI-MS) m/z calcd for C₉H₁₃N₄O₂ [M+H]⁺: 209.1033, found: 209.1032.

### Benzyl 4-(2-(6-hydroxypyrimidin-4-yl)hydrazono)piperidine-1-carboxylate (140)

The reaction was carried out as described in general procedure **A** using 4-hydroxy-6-hydrazinyl pyrimidine (1.0 g, 7.93 mmol) and 1-Cbz-4-piperidone (2.77 g, 11.89 mmol, 1.5 equiv.) in EtOH (16 mL). The precipitate was filtered and washed with Et₂O (2 x 20 mL) to afford compound **140** (2.41 g, 89%) as a white solid. R*_{f}* (CH₂Cl₂/acetone 50/50) 0.11. Mp: 242 - 244°C.. ¹H NMR (250 MHz, DMSO-*d₆*) δ 11.67 (br s, 1H, OH), 9.72 (s, 1H, NH), 7.90 (d, *J* = 0.9 Hz, 1H, CH), 7.50 - 7.19 (m, 5H, 5 x CH), 5.56 (d, *J* = 0.8 Hz, 1H, CH), 5.10 (s, 2H, OCH₂), 3.60 - 3.47 (m, 4H, 2 x CH₂), 2.55 (t, *J* = 6.3 Hz, 2H, CH₂), 2.41 (t, *J=* 6.3 Hz, 2H, CH₂). HRMS (EI-MS) m/z calcd for C₁₇H₂₀N₅O₃ [M+H]⁺: 342.1561, found: 342.1560.

### 5,7,8,9-Tetrahydrothiopyrano[3',4':4,5]pyrrolo[2,3-d]pyrimidin-4-ol (141)

The reaction was carried out as described in general procedure **B** using hydrazone 138 (0.5 g, 2.23 mmol) in tetraline (10 mL). The precipitate was filtered and washed several times with PE (3 x 10 mL) and Et₂O (10 mL) to afford compound **141** (0.40 g, 86%) as a brown solid. R*_{f}* (CH₂Cl₂/MeOH/NH₄OH 90/9/1) 0.39. Mp: > 260°C. ¹H NMR (250 MHz, DMSO-*d₆*) δ 11.70 (br s, 1H, OH), 11.60 (s, 1H, NH), 7.73 (s, 1H, CH), 3.85 - 3.76 (m, 2H, CH₂), 2.96 - 2.75 (m, 4H, 2 x CH₂). HRMS (EI-MS) m/z calcd for C₉H₁₀N₃OS [M+H]+: 208.0539, found: 208.0538.

### 5,7,8,9-Tetrahydropyrano[3',4':4,5]pyrrolo[2,3-d]pyrimidin-4-ol (142)

The reaction was carried out as described in general procedure B using hydrazone **139** (0.5 g, 2.40 mmol) in tetraline (10 mL). The precipitate was filtered and washed several times with PE (3 x 10 mL) and Et₂O (10 mL) to afford compound **142** (0.44 g, 95%) as a brown solid. R*_{f}* (CH₂Cl₂/MeOH/NH₄OH 90/9/1) 0.26. Mp: > 260°C. ¹H NMR (250 MHz, DMSO-*d₆*) δ 11.73 (br s, 1H, OH), 11.67 (s, 1H, NH), 7.74 (s, 1H, CH), 4.79 - 4.65 (m, 2H, CH₂), 3.87 (t, *J* = 5.5 Hz, 2H, CH₂), 2.66 (t, *J* = 5.5 Hz, 2H, CH₂). HRMS (EI-MS) m/z calcd for C₉H₁₀N₃O₂ [M+H]⁺: 192.0768, found: 192.0769.

### Benzyl 4-hydroxy-5,7,8,9-tetrahydro-6H-pyrido[3',4':4,5]pyrrolo[2,3-d]pyrimidine-6-carboxylate (143)

The reaction was carried out as described in general procedure **B** using hydrazone **140** (0.5 g, 1.46 mmol) in tetraline (10 mL). The precipitate was filtered and washed several times with PE (3 x 10 mL) and Et₂O (10 mL) to afford compound **143** (0.45 g, 94%) as a beige solid. R*_{f}* (CH₂Cl₂/MeOH/NH₄OH 90/9/1) 0.42. Mp: > 260°C. ¹H NMR (250 MHz, DMSO-*d₆*) δ 11.76 (br s, 1H, OH), 11.70 (s, 1H, NH), 7.75 (s, 1H, H₂), 7.45 - 7.26 (m, 5H, 5xH), 5.12 (s, 2H, OCH₂), 4.61 (s, 2H, CH₂₍₅₎), 3.73 (t, *J* = 5.8 Hz, 2H, CH₂₍₇₎), 2.68 (t, *J* = 5.7 Hz, 2H, CH₂₍₈₎). HRMS (EI-MS) m/z calcd for C₁₇H₁₇N₄O₃ [M+H]⁺: 325.1295, found: 325.1294.

### 4-Chloro-5,7,8,9-tetrahydrothiopyrano[3',4':4,5]pyrrolo[2,3-d]pyrimidine (144)

The reaction was carried out as described in general procedure **C** using tricycle **141** (1.0 g, 4.83 mmol) in POCl₃ (35 mL). The crude reaction mixture was purified by silica gel column chromatography using a gradient solvent system of CH₂Cl₂/acetone from 100/0 up to 70/30 to afford compound **144** (0.74 g, 68%) as a beige solid. R*_{f}* (CH₂Cl₂/acetone 60/40) 0.28. Mp: > 260°C. ¹H NMR (250 MHz, DMSO-*d₆*) δ 12.34 (br s, 1H, NH), 8.46 (s, 1H, CH), 3.95 (s, 2H, CH₂), 3.03 - 2.92 (m, 4H, 2 x CH₂). HRMS (EI-MS) m/z calcd for C₉H₉ClN₃S [M+H]⁺: 226.0200, found: 226.0200.

### 4-Chloro-5,7,8,9-tetrahydropyrano[3',4':4,5]pyrrolo[2,3-d]pyrimidine (145)

In a round bottom flask, **142** (0.2 g, 1.05 mmol) was suspended in CHCl₃ (4 mL) and treated with 2 drops of *N,N*-dimethylformamide and oxalyl chloride (0.4 mL, 4.73 mmol, 4.5 equiv.). The reaction mixture was heated under reflux for 5 h. The solvent was removed under reduced pressure, the residue was dissolved in CH₂Cl₂ (100 mL) and shaken with a saturated aqueous Na₂CO₃ solution (50 mL). The organic layer was dried over MgSO₄, filtered and concentrated under reduced pressure. The crude reaction mixture was purified by silica gel column chromatography using a gradient solvent system of CH₂Cl₂/acetone from 100/0 up to 50/50 to afford compound **145** (65 mg, 30 %) as a beige solid. R*_{f}* (CH₂Cl₂/acetone 70/30) 0.34. Mp: > 260°C. ¹H NMR (250 MHz, DMSO-*d₆*) δ 12.43 (s, 1H, NH), 8.79 - 8.13 (m, 1H, CH), 5.07 - 4.76 (m, 2H, CH₂), 3.96 (t, *J* = 5.4 Hz, 2H, CH₂), 2.90 - 2.75 (m, 2H, CH₂). HRMS (EI-MS) m/z calcd for C₉H₉ClN₃O [M+H]⁺: 210.0429, found: 210.0428.

### Benzyl 4-chloro-5,7,8,9-tetrahydro-6H-pyrido[3',4':4,5]pyrrolo[2,3-d]pyrimidine-6-carboxylate hydrochloride (146)

The reaction was carried out as described in general procedure **C** using tricycle **143** (1.0 g, 3.08 mmol) in POCl₃ (25 mL). The crude reaction mixture was purified by silica gel column chromatography using a gradient solvent system of CH₂Cl₂/acetone from 80/20 up to 20/80 to afford compound **146** (0.62 g, 53%) as a white solid. R*_{f}* (CH₂Cl₂/acetone 60/40) 0.31. Mp: 180 - 182°C. ¹H NMR (250 MHz, DMSO-*d₆*) δ 12.45 (s, 1H, NH), 8.48 (s, 1H, CH), 7.52 - 7.22 (m, 5H, 5 x CH), 5.14 (s, 2H, OCH₂), 5.00 (br s, 1H, H⁺), 4.87 - 4.64 (m, 2H, CH₂), 3.93 - 3.69 (m, 2H, CH₂), 2.91 - 2.78 (m, 2H, CH₂). HRMS (EI-MS) m/z calcd for C₁₇H₁₇ClN₄O₂ [M+H]⁺: 343.0956, found: 343.0954.

### 4-Chloro-6-methyl-6,7,8,9-tetrahydro-5H-pyrido[3',4':4,5]pyrrolo[2,3-d]pyrimidine (147)

In a round bottom flask, **146** (2.3 g, 6.71 mmol) was dissolved in anhydrous THF (90 mL) under argon and the solution was cooled to 0°C for 10 minutes. Then, LiAlH₄ (1.27 g, 33.55 mmol, 5.0 equiv.) was slowly added, the mixture was stirred at 0 °C for an additional 10 minutes and 2 h at room temperature. Water (2 mL), a 2 M aqueous solution of NaOH (2 mL) and H₂O (2 mL) were successively added dropwise to the mixture. The precipitate was filtered on a celite pad and washed with MeOH (50 mL). The solvent was removed under reduced pressure and the crude reaction mixture was purified by silica gel column chromatography using a gradient solvent system of CH₂Cl₂/MeOH from 95/5 up to 85/15 to afford compound **147** (1.18 g, 79%) as a white solid. R*_{f}* (CH₂Cl₂/MeOH 90/10) 0.17. Mp: 250 - 252°C. ¹H NMR (250 MHz, MeOD-*d₄*) δ 8.42 (s, 1H, CH), 3.86 - 3.82 (m, 2H, CH₂), 2.99 - 2.85 (m, 4H, 2 x CH₂), 2.57 (s, 3H, NCH₃). HRMS (EI-MS) m/z calcd for C₁₀H₁₂ClN₄ [M+H]⁺: 223.0745, found: 223.0745.

### tert-Butyl 4-(5,7,8,9-tetrahydrothiopyrano[3',4':4,5]pyrrolo[2,3-d] pyrimidin-4-yl)-3,6-dihydropyridine-1(2H)-carboxylate (148)

The reaction was carried out as described in general procedure **D** using the chlorinated tricycle **144** (0.35 g, 1.55 mmol) and *N-*Boc-1,2,3,6-tetrahydropyridine-4-boronic acid pinacol ester (0.52 g, 1.71 mmol, 1.1 equiv.) in THF (9 mL). The crude reaction mixture was purified by silica gel column chromatography using a gradient solvent system of CH₂Cl₂/acetone from 90/10 up to 40/60 to afford compound 148 (0.49 g, 85%) as a white solid. R*_{f}* (CH₂Cl₂/acetone 80/20) 0.21. Mp: 211 - 213°C ¹H NMR (250 MHz, MeOD-*d₄*) δ 8.57 (s, 1H, CH), 6.06 - 5.95 (m, 1H, CH), 4.21 - 4.10 (m, 2H, CH₂), 3.76 - 3.66 (m, 4H, 2 x CH₂), 3.10 - 2.93 (m, 4H, 2 x CH₂), 2.69 - 2.56 (m, 2H, CH₂), 1.51 (s, 9H, 3 x CH₃). HRMS (EI-MS) m/z calcd for C₁₉H₂₅N₄O₂S [M+H]⁺: 373.1693, found: 373.1693.

### tert-Butyl 4-(5,7,8,9-tetrahydropyrano[3',4':4,5]pyrrolo[2,3-d]pyrimidin-4-yl)-3,6-dihydropyridine-1(2H)-carboxylate (149)

The reaction was carried out as described in general procedure **D** using the chlorinated tricycle **145** (0.35 g, 1.67 mmol) and *N-*Boc-1,2,3,6-tetrahydropyridine-4-boronic acid pinacol ester (0.57 g, 1.84 mmol, 1.1 equiv.) in THF (9 mL). The crude reaction mixture was purified by silica gel column chromatography using a gradient solvent system of CH₂Cl₂/acetone from 90/10 up to 60/40 to afford compound **149** (0.53 g, 89%) as a white solid. R*_{f}* (CH₂Cl₂/acetone 80/20) 0.27. Mp: 236 - 238°C. ¹H NMR (250 MHz, DMSO-*d₆*) δ 12.02 (s, 1H, NH), 8.61 (s, 1H, CH), 6.11 - 5.94 (m, 1H, CH), 4.67 (s, 2H, CH₂), 4.11 - 4.00 (m, 2H, CH₂), 4.01 - 3.90 (m, 2H, CH₂), 3.63 - 3.50 (m, 2H, CH₂), 2.89 - 2.79 (m, 2H, CH₂), 2.64 - 2.56 (m, 2H, CH₂), 1.45 (s, 9H, 3 x CH₃). HRMS (EI-MS) m/z calcd for C₁₉H₂₅N₄O₃ [M+H]⁺: 357.1921, found: 357.1921.

### tert-Butyl 4-(6-methyl-6,7,8,9-tetrahydro-5H-pyrido[3',4':4,5]pyrrolo[2,3-d]pyrimidin-4-yl)-3,6-dihydropyridine-1(2H)-carboxylate (150)

The reaction was carried out as described in general procedure **D** using the chlorinated tricycle **147** (0.35 g, 1.58 mmol) and *N-*Boc-1,2,3,6-tetrahydropyridine-4-boronic acid pinacol ester (0.54 g, 1.73 mmol, 1.1 equiv.) in THF (9 mL). The crude reaction mixture was purified by silica gel column chromatography using a gradient solvent system CH₂Cl₂/acetone/NH₄OH from 70/28/2 up to 0/98/2. After evaporation, the product was dissolved in CH₂Cl₂ (20 mL), the pH was adjusted to 7 by addition of a 1 M aqueous solution of HCl. The organic layer was separated, dried over MgSO₄, filtered and concentrated under vacuum to afford compound **150** (0.46 g, 79%) as a pink solid. R*_{f}* (CH₂Cl₂/acetone/NH₄OH 70/28/2) 0.18. Mp: 210 - 212°C. ¹H NMR (250 MHz, DMSO-*d₆*) δ 11.90 (s, 1H, NH), 8.57 (s, 1H, CH), 6.09 - 5.96 (m, 1H, CH), 4.05 (s, 2H, CH₂), 3.57 (t, *J* = 5.4 Hz, 2H, CH₂), 3.40 (s, 2H, CH₂), 2.86 - 2.77 (m, 2H, CH₂), 2.75 - 2.65 (m, 2H, CH₂), 2.63 - 2.54 (m, 2H, CH₂), 2.39 (s, 3H, NCH₃), 1.44 (s, 9H, 3 x CH₃). HRMS (EI-MS) m/z calcd for C₂₀H₂₈N₅O₂ [M+H]⁺: 370.2238, found: 370.2236.

### 4-(1,2,3,6-Tetrahydropyridin-4-yl)-5,7,8,9-tetrahydrothiopyrano [3',4':4,5]pyrrolo[2,3-d]pyrimidine (151)

The reaction was carried out as described in general procedure **F1** using the *N*-Boc protected compound **148** (1.0 g, 2.68 mmol) in CH₂Cl₂ (80 mL). The reaction was complete after 1 h. Workup gave compound **151** (0.56 g, 98%) as a beige solid. Mp: 241 - 243°C. ¹H NMR (250 MHz, MeOD-*d₄*) δ 8.56 (s, 1H, CH), 6.01 (tt, *J* = 3.2, 1.8 Hz, 1H, CH), 3.83 - 3.74 (m, 2H, CH₂), 3.53 (dt, *J* = 2.9 Hz, *J* = 2.9 Hz, 2H, CH₂), 3.10 (t, *J* = 5.7 Hz, 2H, CH₂), 3.08 - 2.95 (m, 4H, 2 x CH₂), 2.63 - 2.51 (m, 2H, CH₂). HRMS (EI-MS) m/z calcd for C₁₄H₁₇N₄S [M+H]⁺: 273.1168, found: 273.1168.

### 4-(1,2,3,6-Tetrahydropyridin-4-yl)-5,7,8,9-tetrahydropyrano [3',4':4,5]pyrrolo[2,3 d]pyrimidine (152)

The reaction was carried out as described in general procedure **F1** using the *N*-Boc protected compound **149** (1.0 g, 2.81 mmol) in CH₂Cl₂ (80 mL). The reaction was complete after 1 h. Workup gave compound **152** (0.60 g, 83%) as an off-white solid. Mp: 231 - 233°C. ¹H NMR (250 MHz, DMSO-*d₆*) δ 12.01 (br s, 1H, NH), 8.59 (s, 1H, CH), 6.07 - 5.97 (m, 1H, CH), 4.72 - 4.62 (m, 2H, CH₂), 3.95 (t, *J* = 5.6 Hz, 2H, CH₂), 3.52 - 3.45 (m, 2H, CH₂), 3.02 (t, *J* = 5.7 Hz, 2H, CH₂), 2.88 - 2.76 (m, 2H, CH₂), 2.58 - 2.47 (m, 2H, CH₂). HRMS (EI-MS) m/z calcd for C₁₄H₁₇N₄O [M+H]⁺: 257.1397, found: 257.1396.

### 6-Methyl-4-(1,2,3,6-tetrahydropyridin-4-yl)-6,7,8,9-tetrahydro-5H-pyrido[3',4':4,5]pyrrolo[2,3-d]pyrimidine (153)

The reaction was carried out as described in general procedure **F1** using the *N*-Boc protected compound **150** (1.0 g, 2.71 mmol) in CH₂Cl₂ (80 mL). The reaction was complete after 1 h. Workup gave compound **153** (0.61 g, 84%) as a beige solid. Mp: degradation 226°C. ¹H NMR (250 MHz, MeOD-*d₄*) δ 8.58 (s, 1H, CH), 6.11 - 6.04 (m, 1H, CH), 3.69 - 3.57 (m, 4H, 2 x CH₂), 3.21 (t, *J* = 5.8 Hz, 2H, CH₂), 3.04 - 2.86 (m, 4H, 2 x CH₂), 2.72 - 2.63 (m, 2H, CH₂), 2.54 (s, 3H, NCH₃). HRMS (EI-MS) m/z calcd for C₁₅H₂₀N₅ [M+H]⁺: 270.1713, found: 270.1713.

### Example 85: N-(3-Fluorophenyl)-4-(5,7,8,9-tetrahydrothiopyrano[3',4':4,5] pyrrolo[2,3-d]pyrimidin-4-yl)-3,6-dihydropyridine-1(2H)-carboxamide (154)

The reaction was carried out as described in general procedure **G** using amine **151** (70 mg, 0.26 mmol) in anhydrous CH₂Cl₂ (3 mL). The crude reaction mixture was purified by silica gel column chromatography using a gradient solvent system of CH₂Cl₂/acetone from 90/10 up to 20/80 to afford compound **154** (86 mg, 83%) as a beige solid. R*_{f}* (CH₂Cl₂/acetone 40/60) 0.27. Mp: degradation 200°C. ¹H NMR (400 MHz, DMSO-*d₆*) δ 11.96 (br s, 1H, NH), 8.77 (br s, 1H, NH), 8.61 (s, 1H, CH), 7.48 (d, *J* = 12.3 Hz, 1H, CH), 7.35 - 7.22 (m, 2H, 2 x CH), 6.75 (t, *J* = 8.3 Hz, 1H, CH), 6.13 - 5.99 (m, 1H, CH), 4.27 - 4.17 (m, 2H, CH₂), 3.84 - 3.65 (m, 4H, 2 x CH₂), 3.05 - 2.91 (m, 4H, 2 x CH₂), 2.74 - 2.63 (m, 2H, CH₂). ¹⁹F NMR (376 MHz, DMSO-*d₆*) δ -112.9 (s). HRMS (EI-MS) m/z calcd for C₂₁H₂₁FN₅OS [M+H]⁺: 410.1445, found: 410.1440.

### Example 86: N-(3-Chlorophenyl)-4-(5,7,8,9-tetrahydrothiopyrano [3',4':4,5] pyrrolo[2,3-d]pyrimidin-4-yl)-3,6-dihydropyridine-1(2H)-carboxamide (155)

The reaction was carried out as described in general procedure **G** using amine **151** (70 mg, 0.26 mmol) in anhydrous CH₂Cl₂ (3 mL). The crude reaction mixture was purified by silica gel column chromatography using a gradient solvent system of CH₂Cl₂/acetone from 80/20 up to 10/90 to afford compound **155** (89 mg, 81%) as a brown solid. R*_{f}* (CH₂Cl₂/acetone 40/60) 0.28. Mp: degradation 206°C. ¹H NMR (250 MHz, MeOD-*d₄*) δ 8.59 (s, 1H, CH), 7.74 - 7.67 (m, 1H, CH), 7.42 - 7.33 (m, 1H, CH), 7.23 - 7.12 (m, 2H, 2 x CH), 6.06 (s, 2H, CH), 4.33 - 4.24 (m, 2H, CH₂), 3.82 (t, *J* = 5.6 Hz, 2H, CH₂), 3.77 (s, 2H, CH₂), 3.11 -2.96 (m, 4H, 2 x CH₂), 2.73 (s, 2H, CH₂). HRMS (EI-MS) m/z calcd for C₂₁H₂₁ClN₅OS [M+H]⁺: 426.1150, found: 426.1147.

### Example 87: N-(3-Bromophenyl)-4-(5,7,8,9-tetrahydrothiopyrano [3',4':4,5]pyrrolo[2,3-d]pyrimidin-4-yl)-3,6-dihydropyridine-1(2H)-carboxamide (156)

The reaction was carried out as described in general procedure **G** using amine **151** (70 mg, 0.26 mmol) in anhydrous CH₂Cl₂ (3 mL). The crude reaction mixture was purified by silica gel column chromatography using a gradient solvent system of CH₂Cl₂/acetone from 80/20 up to 10/90 to afford compound **156** (99 mg, 82%) as a brown solid. R*_{f}* (CH₂Cl₂/acetone 40/60) 0.28. Mp: degradation 220°C. ¹H NMR (250 MHz, MeOD-*d₄*) δ 8.61 (s, 1H, CH), 7.56 (d, *J* = 2.2 Hz, 1H, CH), 7.32 (d, *J* = 8.4 Hz, 1H, CH), 7.24 (t, *J* = 8.0 Hz, 1H, CH), 7.01 (d, *J* = 7.7 Hz, 1H, CH), 6.12 - 6.04 (m, 1H, CH), 4.34 - 4.24 (m, 2H, CH₂), 3.83 (t, *J* = 5.6 Hz, 2H, CH₂), 3.77 (s, 2H, CH₂), 3.12 - 2.93 (m, 4H, 2 x CH₂), 2.79 - 2.67 (m, 2H, CH₂). HRMS (EI-MS) m/z calcd for C₂₁H₂₁BrN₅OS [M+H]⁺: 470.0645, found: 470.0639.

### Example 88: N-(3-Fluorophenyl)-4-(5,7,8,9-tetrahydropyrano[3',4':4,5] pyrrolo[2,3-d]pyrimidin-4-yl)-3,6-dihydropyridine-1(2H)-carboxamide (157)

The reaction was carried out as described in general procedure **G** using amine **152** (60 mg, 0.16 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound **157** (42 mg, 67%) as a beige solid. R*_{f}* (CH₂Cl₂/acetone 50/50) 0.25. Mp: 223 - 225°C. ¹H NMR (400 MHz, DMSO-*d₆*) δ 12.02 (br s, 1H, NH), 8.78 (br s, 1H, NH), 8.62 (s, 1H, CH), 7.48 (dt, *J* = 12.3 Hz, *J* = 2.2 Hz, 1H, CH), 7.32 - 7.21 (m, 2H, 2 x CH), 6.79 - 6.71 (m, 1H, CH), 6.11 - 6.06 (m, 1H, CH), 4.69 (s, 2H, CH₂), 4.30 - 4.14 (m, 2H, CH₂), 3.96 (t, *J* = 5.6 Hz, 2H, CH₂), 3.71 (t, *J* = 5.6 Hz, 2H, CH₂), 2.86 - 2.78 (m, 2H, CH₂), 2.71 - 2.62 (m, 2H, CH₂). ¹⁹F NMR (376 MHz, DMSO-*d₆*) δ -112.9 (s). HRMS (EI-MS) m/z calcd for C₂₁H₂₁FN₅O₂ [M+H]⁺: 394.1674, found: 394.1673.

### Example 89: N-(3-Chlorophenyl)-4-(5,7,8,9-tetrahydropyrano[3',4':4,5] pyrrolo[2,3-d]pyrimidin-4-yl)-3,6-dihydropyridine-1(2H)-carboxamide (158)

The reaction was carried out as described in general procedure **G** using amine **152** (60 mg, 0.16 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound 158 (47 mg, 72%) as a beige solid. R*_{f}* (CH₂Cl₂/acetone 50/50) 0.28. Mp: 224 - 226°C. ¹H NMR (400 MHz, DMSO-*d₆*) δ 12.03 (br s, 1H, NH), 8.76 (br s, 1H, NH), 8.62 (s, 1H, CH), 7.69 (t, *J* = 2.2 Hz, 1H, CH), 7.44 (dd, *J* = 8.2 Hz, *J* = 2.1 Hz, 1H, CH), 7.26 (t, *J=* 8.1 Hz, 1H, CH), 6.98 (dd, *J* = 7.8 Hz, *J* = 2.1 Hz, 1H, CH), 6.13 - 6.06 (m, 1H, CH), 4.69 (s, 2H, CH₂), 4.26 - 4.17 (m, 2H, CH₂), 3.96 (t, *J* = 5.6 Hz, 2H, CH₂), 3.71 (t, *J* = 5.6 Hz, 2H, CH₂), 2.86 - 2.79 (m, 2H, CH₂), 2.73 - 2.62 (m, 2H, CH₂). HRMS (EI-MS) m/z calcd for C₂₁H₂₁ClN₅O₂[M+H]⁺: 410.1378, found: 410.1377.

### Example 90: N-(3-Bromophenyl)-4-(5,7,8,9-tetrahydropyrano[3',4':4,5] pyrrolo[2,3-d]pyrimidin-4-yl)-3,6-dihydropyridine-1(2H)-carboxamide (159)

The reaction was carried out as described in general procedure **G** using amine **152** (60 mg, 0.16 mmol) in anhydrous CH₂Cl₂ (3 mL). The crude reaction mixture was purified by silica gel column chromatography using a gradient solvent system of CH₂Cl₂/acetone from 80/20 up to 20/80 to afford compound **159** (52 mg, 71%) as a beige solid. R*_{f}* (CH₂Cl₂/acetone 50/50) 0.27. Mp: 219 - 221°C. ¹H NMR (400 MHz, DMSO-*d₆*) δ 12.03 (br s, 1H, NH), 8.74 (br s, 1H, NH), 8.62 (s, 1H, CH), 7.83 (t, *J* = 2.1 Hz, 1H, CH), 7.49 (dd, *J* = 8.1 Hz, *J=* 2.0 Hz, 1H, CH), 7.20 (t, *J=* 8.0 Hz, 1H, CH), 7.11 (dd, *J* = 7.9 Hz, *J* = 1.9 Hz, 1H, CH), 6.13 - 6.04 (m, 1H, CH), 4.69 (s, 2H, CH₂), 4.25 - 4.16 (m, 2H, CH₂), 3.96 (t, *J=* 5.5 Hz, 2H, CH₂), 3.71 (t, *J* = 5.6 Hz, 2H, CH₂), 2.83 (t, *J* = 5.5 Hz, 2H, CH₂), 2.71 - 2.63 (m, 2H, CH₂). HRMS (EI-MS) m/z calcd for C₂₁H₂₁BrN₅O₂ [M+H]⁺: 454.0873, found: 454.0870.

### Example 91: N-(3-Fluorophenyl)-4-(6-methyl-6,7,8,9-tetrahydro-5H-pyrido[3',4':4,5]pyrrolo[2,3-d]pyrimidin-4-yl)-3,6-dihydropyridine-1(2H-carboxamide (160)

The reaction was carried out as described in general procedure **G** using amine **153** (40 mg, 0.15 mmol) in anhydrous CH₂Cl₂ (3 mL). The crude reaction mixture was purified by silica gel column chromatography using a gradient solvent system of CH₂Cl₂/MeOH from 98/2 up to 82/18 to afford compound **160** (41 mg, 68%) as a beige solid. R*_{f}* (CH₂Cl₂/MeOH 85/15) 0.16. Mp: degradation 191°C¹H NMR (400 MHz, DMSO-*d₆*) δ 12.25 (br s, 1H, NH), 8.90 (br s, 1H, NH), 8.67 (s, 1H, CH), 7.51 (d, *J* = 12.3 Hz, 1H, CH), 7.39 - 7.16 (m, 2H, 2 x CH), 6.75 (t, *J* = 8.6 Hz, 1H, CH), 6.19 - 6.00 (m, 1H, CH), 4.26 (s, 2H, CH₂), 4.11 (s, 2H, CH₂), 3.75 (s, 2H, CH₂), 3.40 - 3.28 (m, 2H, CH₂), 3.07 (s, 2H, CH₂), 2.80 (s, 3H, NCH₃), 2.71 (s, 2H, CH₂). ¹⁹F NMR (376 MHz, DMSO-*d₆*) δ -112.9 (s). HRMS (EI-MS) m/z calcd for C₂₂H₂₄FN₆O [M+H]⁺: 407.1990, found: 407.1988.

### Example 92: N-(3-Chlorophenyl)-4-(6-methyl-6,7,8,9-tetrahydro-5H-pyrido[3',4':4,5]pyrrolo[2,3-d]pyrimidin-4-yl)-3,6-dihydropyridine-1(2H)-carboxamide (161)

The reaction was carried out as described in general procedure **G** using amine **153** (70 mg, 0.26 mmol) in anhydrous CH₂Cl₂ (3 mL). The crude reaction mixture was purified by silica gel column chromatography using a gradient solvent system of CH₂Cl₂/MeOH from 98/2 up to 82/18 to afford compound **161** (58 mg, 53%) as a beige solid. R*_{f}* (CH₂Cl₂/MeOH 85/15) 0.16. Mp: degradation 193°C. ¹H NMR (400 MHz, DMSO-*d₆*) δ 12.21 (br s, 1H, NH), 8.93 (br s, 1H, NH), 8.65 (s, 1H, CH), 7.73 (s, 1H, CH), 7.49 (d, *J* = 8.4 Hz, 1H, CH), 7.26 (t, *J* = 8.1 Hz, 1H, CH), 6.98 (d, *J* = 8.1 Hz, 1H, CH), 6.13 - 6.05 (m, 1H, CH), 4.26 (s, 2H, CH₂), 3.99 (s, 2H, CH₂), 3.79 - 3.70 (m, 2H, CH₂), 3.28 - 3.16 (m, 2H, CH₂), 3.04 (s, 2H, CH₂), 2.78 - 2.64 (m, 5H, NCH₃ + CH₂). HRMS (EI-MS) m/z calcd for C₂₂H₂₄ClN₆O [M+H]⁺: 423.1695, found: 423.1694.

### Example 93: N-(3-Bromophenyl)-4-(6-methyl-6,7,8,9-tetrahydro-5H-pyrido [3',4':4,5]pyrrolo[2,3-d]pyrimidin-4-yl)-3,6-dihydropyridine-1(2H)-carboxamide (162)

The reaction was carried out as described in general procedure **G** using amine **153** (70 mg, 0.26 mmol) in anhydrous CH₂Cl₂ (3 mL). The crude reaction mixture was purified by silica gel column chromatography using a gradient solvent system of CH₂Cl₂/MeOH from 98/2 up to 82/18 to afford compound **162** (61 mg, 50%) as a beige solid. R*_{f}* (CH₂Cl₂/MeOH 85/15) 0.17. Mp: degradation 191°C. ¹H NMR (400 MHz, DMSO-*d₆*) δ 12.05 (br s, 1H, NH), 8.90 (br s, 1H, NH), 8.61 (s, 1H, CH), 7.87 (s, 1H, CH), 7.54 (d, *J* = 8.5 Hz, 1H, CH), 7.20 (t, *J* = 8.0 Hz, 1H, CH), 7.11 (d, *J* = 8.0 Hz, 1H, CH), 6.14 - 6.05 (m, 1H, CH), 4.23 (s, 2H, CH₂), 3.79 - 3.58 (m, 4H, 2 x CH₂), 2.90 (s, 4H, 2 x CH₂), 2.72 - 2.62 (m, 2H, CH₂), 2.50 (s, 3H, NCH₃). HRMS (EI-MS) m/z calcd for C₂₂H₂₄BrN₆O [M+H]⁺: 467.1189, found: 467.1183.

### FAMILY 8.

### Fluorescent probes synthesized from LIMK/Rock inhibitors for cellular tests

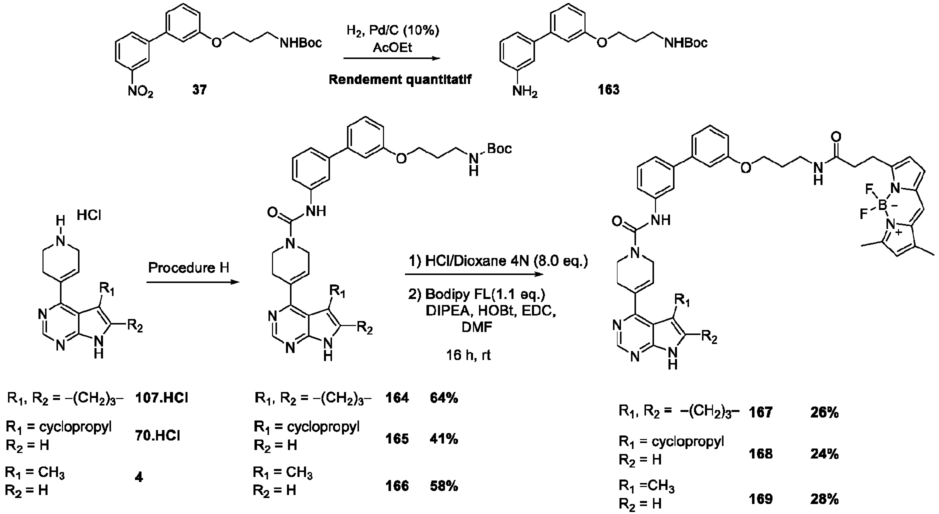

### tert-Butyl N-[3-[3-(3-aminophenyl)phenoxy]propyl]carbamate (163)

Compound **37** (377 mg, 1.01 mmol) was suspended in 30 mL EtOAc in a hydrogenation reactor. Pd/C (11 mg, 0.10 mmol, 0.1 equiv.) was then added and the reaction was stirred under 10 bar of hydrogen for 48 hrs. The reaction mixture was then filtered over Celite and concentrated under reduced pressure to give compound **163** as a yellow oil (0.345 g, quant.) which was used in the next step without further purification. R*_{f}* (EP/Et₂O 80/20) 0.11. ¹H NMR (400 MHz, MeOD-*d₄*) δ 7.29 (t, *J=* 7.9 Hz, 1H, CH), 7.18 - 7.07 (m, 3H, CH), 6.98 (t, *J* = 1.8 Hz, 1H, CH), 6.95 - 6.90 (m, 1H, CH), 6.89 - 6.85 (m, 1H, CH), 6.73 - 6.67 (m, 1H, CH), 4.03 (t, *J* = 6.3 Hz, 2H, CH₂), 3.23 (q, *J* = 6.3 Hz 2H, CH₂), 1.93 (p, *J* = 6.3 Hz, 2H, CH₂), 1.44 (s, 9H, 3 x CH₃). HRMS (EI-MS) m/z calcd for C₂₀H₂₇N₂O₃ [M+H]⁺: 343.2016, found: 343.2011.

### tert-butyl (3-((3'-(4-(5,6,7,8-tetrahydrocyclopenta[4,5]pyrrolo[2,3-d] pyrimidin-4-yl)-1,2,3,6-tetrahydropyridine-1-carboxamido)-[1,1'-biphenyl]-3-yl)oxy)propyl)carbamate (164)

The reaction was carried out as described in general procedure **H** using compound **163** (309 mg, 0.90 mmol) and amine **107.HCl** (300 mg, 1.08 mmol, 1.2 equiv.) in THF (13 mL). The crude reaction mixture was purified by silica gel column chromatography using a gradient solvent system of CH₂Cl₂/acetone from 90/10 up to 50/50 to afford compound **164** (350 mg, 64%) as a beige solid. R*_{f}* (CH₂Cl₂/acetone 50/50) 0.41. Mp: degradation 120°C. ¹H NMR (400 MHz, DMSO-*d₆*) δ 11.96 (s, 1H, NH), 8.64 (s, 1H, NH), 8.58 (s, 1H, CH), 7.80 (t, J = 1.88 Hz, 1H, CH), 7.59 - 7.54 (m, 1H, CH), 7.40 - 7.29 (m, 2H, CH), 7.27 - 7.21 (m, 1H, CH), 7.20 - 7.16 (m, 1H, CH), 7.14 - 7.10 (m, 1H, CH), 6.96 - 6.88 (m, 2H, CH + NH), 6.66 - 6.56 (m, 1H, CH), 4.28 (d, J = 3.0 Hz, 2H, CH₂), 4.04 (t, J = 6.3 Hz, 2H, CH₂), 3.73 (t, J = 5.6 Hz, 2H, CH₂), 3.11 (q, J = 6.6 Hz, 2H, CH₂), 2.95 - 2.83 (m, 4H, 2 x CH₂), 2.79 - 2.72 (m, 2H, CH₂), 2.43 (p, J = 7.28 Hz, 2H, CH₂), 1.86 (p, J = 6.6 Hz, 2H, CH₂), 1.37 (s, 9H, 3 x CH₃). HRMS (EI-MS) m/z calcd for C₃₅H₄₁N₆O₄ [M+H]+: 609.3184, found: 609.3180.

### tert-butyl N-[3-[3-[3-[[4-(5-cyclopropyl-7H-pyrrolo[2,3-d]pyrimidin-4-yl)-3,6-dihydro-2H-pyridine-1-carbonyl]amino]phenyl]phenoxy]propyl]carbamate (165)

The reaction was carried out as described in general procedure **H** using compound **163** (210 mg, 0.61 mmol) and amine **70.HCl** (205 mg, 0.74 mmol, 1.2 equiv.) in THF (13 mL). The crude reaction mixture was purified by silica gel column chromatography using a gradient solvent system of CH₂Cl₂/acetone from 90/10 up to 50/50 to afford compound **165** (152 mg, 41%) as a beige solid. R*_{f}* (CH₂Cl₂ /acetone 50/50) 0.36. Mp: degradation 113°C. ¹H NMR (400 MHz, DMSO-*d₆*) δ 11.84 (s, 1H, NH), 8.65 (s, 2H, NH + CH), 7.80 (s, 1H, CH), 7.55 (d, *J* = 8.0 Hz, 1H, CH), 7.40 - 7.29 (m, 2H, CH), 7.26 - 7.22 (m, 2H, 2 x CH), 7.18 (d, *J* = 7.8 Hz, 1H, CH), 7.12 (s, 1H, CH), 6.96 - 6.86 (m, 2H, NH + CH), 6.32 - 6.26 (m, 1H, CH), 4.30 - 4.20 (m, 2H, CH₂), 4.04 (t, J = 6.3 Hz, 2H, CH₂), 3.77 (t, J = 5.6 Hz, 2H, CH₂), 3.11 (q, J = 6.5 Hz, 2H, CH₂), 2.81 - 2.66 (m, 2H, CH₂), 1.93 - 1.80 (m, 3H, CH + CH₂), 1.37 (s, 9H, 3 x CH₃), 0.86 - 0.76 (m, 2H, CH₂), 0.66 - 0.57 (m, 2H, CH₂). HRMS (EI-MS) m/z calcd for C₃₅H₄₁N₆O₄[M+H]⁺: 609.3184, found: 609.3180.

### tert-butyl N-[3-[3-[3-[[4-(5-methyl-7H-pyrrolo[2,3-d]pyrimidin-4-yl)-3,6-di hydro-2H-pyridine-1-carbonyl]amino]phenyl]phenoxy]propyl]carbamate (166)

The reaction was carried out as described in general procedure **H** using compound **163** (300 mg, 0.88 mmol) and amine **4** (263 mg, 1.05 mmol, 1.2 equiv.) in THF (13 mL). The crude reaction mixture was purified by silica gel column chromatography using a gradient solvent system of CH₂Cl₂/acetone from 90/10 up to 50/50 to afford compound **166** (294 mg, 58%) as a beige solid. R*_{f}* (CH₂Cl₂/acetone 50/50) 0.32. Mp: degradation 105°C. ¹H NMR (250 MHz, DMSO-*d₆*) δ 11.83 (s, 1H, NH), 8.67 (s, 1H, NH), 8.65 (s, 1H, CH), 7.81 (s, 1H, CH), 7.57 (d, *J* = 7.9 Hz, 1H, CH), 7.42 - 7.08 (m, 6H, 6 x CH), 6.97 - 6.84 (m, 2H, CH + NH), 6.06 (s, 1H, CH), 4.35 - 4.20 (m, 2H, CH₂), 4.04 (t, *J* = 6.0 Hz, 2H, CH₂), 3.76 (t, *J* = 5.5 Hz, 2H, CH₂), 3.21 - 3.02 (m, 2H, CH₂), 2.77 - 2.63 (m, 2H, CH₂), 2.25 (s, 3H, CH₃), 1.87 (p, *J* = 6.6 Hz, 2H, CH₂), 1.37 (s, 9H, 3 x CH₃). HRMS (EI-MS) m/z calcd for C₃₃H₃₉N₆O₄ [M+H]⁺: 583.3027, found: 583.3018.

### N-(3'-(3-(3-(5,5-Difluoro-7,9-dimethyl-5H-dipyrrolo[1,2-c:2',1'-f][1,3,2] diaza borinin-3-yl)propanamido)propoxy)-[1,1'-biphenyl]-3-yl)-4-(5,6,7,8-tetrahydro cyclopenta[4,5]pyrrolo[2,3-d]pyrimidin-4-yl)-3,6-dihydropyridine-1(2H)-carboxamide (167)

A solution of HCl (4N in dioxane, 0.164 mL, 8 equiv.) was slowly added to a solution of urea **164** (50 mg, 0.08 mmol) in CH₂Cl₂ (3 mL) at 0°C and stirred at r.t. until the reaction was complete by TLC. The solvent was then evaporated under reduced pressure and the crude amine salt was used in the next step without further purification. Anhydrous DMF (10 mL) was added and the reaction placed under Ar. DIPEA (0.086 mL, 0.49 mmol, 6.0 equiv.), HOBt (33 mg, 0.25 mmol, 3.0 equiv.), and 3-(5,5-difluoro-7,9-dimethyl-5*H*-dipyrrolo[1,2-c:2',1'-*f*][1,3,2]diazaborinin-3-yl)propanoic acid (26 mg, 0.09 mmol, 1.1 equiv.) were then added followed by EDC (38 mg, 43 µL, 0.25 mmol, 3.0 equiv.). The reaction was stirred at r.t. overnight. The reaction mixture was diluted with water (3 mL) and the aqueous layer extracted with AcOEt (3 x 10 mL). The combined organic layers were washed with brine, dried over MgSO₄, and the solvent removed under reduced pressure. The crude reaction mixture was purified by silica gel column chromatography using a gradient solvent system of CH₂Cl₂/ acetone from 100/0 up to 70/30 to afford compound **167** (16 mg, 25%) as a red solid. R*_{f}* (CH₂Cl₂/acetone 50/50) 0.18. Mp: degradation 103°C. ¹H NMR (400MHz, CDCl₃) δ (ppm) 9.63 (s, 1H, NH), 8.71 (s, 1H, CH), 7.57 (s, 1H, CH), 7.53 - 7.47 (m, 1H, CH), 7.34 (t, *J* = 7.9 Hz, 1H, CH), 7.29 - 7.23 (m, 2H, 2 x CH), 7.19 - 7.14 (m, 1H, CH), 7.06 - 7.03 (m, 1H, CH), 6.94 (s, 1H, CH), 6.82 - 6.78 (m, 2H, NH + CH), 6.76 (d, *J* = 3.9 Hz, 1H, CH), 6.54 - 6.50 (m, 1H, CH), 6.24 (d, *J* = 4.0 Hz, 1H, CH), 6.14 (t, *J* = 5.7 Hz, 1H, NH), 6.06 (s, 1H, CH), 4.30 (d, *J =* 3.0 Hz, 2H, CH₂), 3.93 (t, *J* = 6.1 Hz, 2H, CH₂), 3.79 (t, *J=* 5.6 Hz, 2H, CH₂), 3.42 (q, *J* = 6.2 Hz, 2H, CH₂), 3.27 (t, *J* = 7.4 Hz, 2H, CH₂), 2.95 (t, *J* = 7.2 Hz, 2H, CH₂), 2.92 - 2.83 (m, 4H, 2 x CH₂), 2.66 (t, *J* = 7.3 Hz, 2H, CH₂), 2.58 - 2.43 (m, 5H, CH₂ + CH₃), 2.17 (s, 3H, CH₃), 1.92 (p, *J* = 6.3 Hz, 2H, CH₂). HRMS (EI-MS) m/z calcd for C₄₄H₄₆BF₂N₈O₃ [M+H]⁺: 783.3749, found: 783.3754.

### N-(3'-(3-(3-(5,5-Difluoro-7,9-dimethyl-5H-dipyrrolo[1,2-c:2',1'-f][1,3,2]diaza borinin-3-yl)propanamido)propoxy)-[1,1'-biphenyl]-3-yl)-4-(5-isopropyl-7H-pyrrolo[2,3-d]pyrimidin-4-yl)-3,6-dihydropyridine-1(2H)-carboxamide (168)

A solution of HCl (4N in dioxane, 0.26 mL, 8 equiv.) was slowly added to a solution of urea **165** (81 mg, 0.13 mmol) in CH₂Cl₂ (3 mL) at 0°C and stirred at r.t. until the reaction was complete by TLC. The solvent was then evaporated under reduced pressure and the crude amine salt was used in the next step without further purification. Anhydrous DMF (10 mL) was added and the reaction placed under Ar. DIPEA (0.140 mL, 0.80 mmol, 6.0 equiv.), HOBt (54 mg, 0.40 mmol, 3.0 equiv.), and 3-(5,5-difluoro-7,9-dimethyl-5*H*-dipyrrolo[1,2-c:2',1'-*f*][1,3,2]diazaborinin-3-yl)propanoic acid (43 mg, 0.15 mmol, 1.1 equiv.) were then added followed by EDC (71,2 µL, 0.40 mmol, 3.0 equiv.). The reaction was stirred at r.t. overnight. The reaction mixture was diluted with water (3 mL) and the aqueous layer extracted with AcOEt (3 x 10 mL). The combined organic layers were washed with brine, dried over MgSO₄, and the solvent removed under reduced pressure. The crude reaction mixture was purified by silica gel column chromatography using a gradient solvent system of CH₂Cl₂/MeOH from 100/0 up to 90/10 to afford compound **168** (25 mg, 24%) as a red solid. R*_{f}* (CH₂Cl₂ /acetone 90/10) 0.52. Mp: degradation 138°C. ¹H NMR (250 MHz, CDCl₃) δ 10.18 (brs, 1H, NH), 8.80 (s, 1H, CH), 7.60 - 7.54 (m, 1H, CH), 7.50 (d, *J* = 8.0 Hz, 1H, CH), 7.33 (t, *J* = 7.8 Hz, 1H, CH), 7.26 (t, *J* = 7.9 Hz, 2H, 2 x CH), 7.15 (d, *J* = 7.8 Hz, 1H, CH), 7.04 - 7.01 (m, 1H, CH), 7.01 - 6.95 (m, 2H, CH + NH), 6.94 (s, 1H, CH), 6.82 - 6.73 (m, 2H, 2 x CH), 6.28 - 6.15 (m, 3H, 2 x CH, NH), 6.05 (s, 1H, CH), 4.32 - 4.18 (m, 2H, CH₂), 3.91 (t, *J* = 6.1 Hz, 2H, CH₂), 3.82 (t, *J* = 5.5 Hz, 2H, CH₂), 3.41 (q, *J* = 6.3 Hz, 2H, CH₂), 3.26 (t, *J=* 7.3 Hz, 2H, CH₂), 2.84 (s, 2H, CH₂), 2.65 (t, *J* = 7.3 Hz, 2H, CH₂), 2.48 (s, 3H, CH₃), 2.16 (s, 3H, CH₃), 1.91 (p, *J* = 6.0 Hz, 2H, CH₂), 1.85 - 1.74 (m, 1H, CH), 0.91 - 0.78 (m, 2H, CH₂), 0.62 - 0.51 (m, 2H, CH₂). HRMS (EI-MS) m/z calcd for C₄₄H₄₆BF₂N₈O₃ [M+H]⁺: 783.3749, found: 783.3755.

### N-(3'-(3-(3-(5,5-difluoro-7,9-dimethyl-5H-dipyrrolo[1,2-c:2',1'-f][[1,3,2] diaza borinin-3-yl)propanamido)propoxy)-[1,1'-biphenyl]-3-yl)-4-(5-methyl-7H-pyrrolo [2,3-d|pyrimidin-4-yl)-3,6-dihydropyridine-1(2H)-carboxamide (169)

A solution of HCl (4N in dioxane, 0.34 mL, 8 equiv.) was slowly added to a solution of urea **166** (100 mg, 0.17 mmol) in CH₂Cl₂ (4 mL) at 0°C and stirred at r.t. until the reaction was complete by TLC. The solvent was then evaporated under reduced pressure and the crude amine salt was used in the next step without further purification. Anhydrous DMF (12 mL) was added and the reaction placed under Ar. DIPEA (0.18 mL, 1.03 mmol, 6.0 equiv.), HOBt (70 mg, 0.52 mmol, 3.0 equiv.), and 3-(5,5-difluoro-7,9-dimethyl-5*H*-dipyrrolo[1,2-c:2',1'-*f*][1,3,2]diazaborinin-3-yl)propanoic acid (55 mg, 0.19 mmol, 1.1 equiv.) were then added followed by EDC (91 µL, 0.52 mmol, 3.0 equiv.). The reaction was stirred at r.t. overnight. The reaction mixture was diluted with water (4 mL) and the aqueous layer extracted with AcOEt (3 x 15 mL). The combined organic layers were washed with brine, dried over MgSO_{4,} and the solvent removed under reduced pressure. The crude reaction mixture was purified by silica gel column chromatography using a gradient solvent system of CH₂Cl₂/ MeOH from 100/0 up to 90/10 to afford compound **169** (37 mg, 28%) as a red solid. R*_{f}* (CH₂Cl₂/acetone 95/5) 0.25. Mp: 130 °C. ¹H NMR (400 MHz, CDCl₃) δ 9.70 (s, 1H, NH), 8.80 (s, 1H, CH), 7.60 - 7.55 (m, 1H, CH), 7.52 - 7.48 (m, 1H, CH), 7.34 (t, *J=* 7.9 Hz, 1H, CH), 7.30 - 7.23 (m, 2H, CH), 7.19 - 7.14 (m, 1H, CH), 7.08 (brs, 1H, CH), 7.04 (t, *J=* 2.1 Hz, 1H, CH), 6.95 (brs, 1H, CH), 6.86 (brs, 1H, NH), 6.80 (dd, *J* = 8.2, 2.5 Hz, 1H, CH), 6.75 (d, *J=* 4.0 Hz, 1H, CH), 6.23 (d, *J* = 4.0 Hz, 1H, CH), 6.16 (t, *J* = 5.9 Hz, 1H, NH), 6.06 (s, 1H, CH), 5.99 (s, 1H, CH), 4.27 (d, *J* = 2.9 Hz, 2H, CH₂), 3.93 (t, *J* = 6.2 Hz, 2H, CH₂), 3.82 (t, *J=* 5.6 Hz, 2H, CH₂), 3.42 (q, *J* = 6.3 Hz, 2H, CH₂), 3.26 (t, *J* = 7.4 Hz, 2H, CH₂), 2.87 - 2.77 (m, 2H, CH₂), 2.65 (t, *J* = 7.4 Hz, 2H, CH₂), 2.48 (s, 3H, CH₃), 2.26 (s, 3H, CH₃), 2.17 (s, 3H, CH₃), 1.92 (p, *J* = 6.3 Hz, 2H, CH₂). HRMS (EI-MS): m/z calcd for C₄₂H₄₄BF₂N₈O₃ [M+H]+ 757.3593; found: 757.3592.

### BIOLOGICAL STUDIES

### 1. In vitro assays

### 1.1. Inhibition of LIMK1 and LIMK2

Inhibition of LIMK1 and LIMK2 was measured with « Lanthascreen ^{®} Eu binding assay » (Life Technologies). This TR-FRET assay is based on the competition between a fluorescent tracer and the inhibitor to be tested, allowing the determination of an inhibition constant, Ki.

133 compounds have been tested. A dozen of these compounds have their Ki below 10 nM, and about fifty below 50 nM. These results are very promising, as Ki values of the reference compounds LIMKi3 and LX7101 are 8 and 3.9 nM, respectively, and the Pyr1 compound tested on breast cancer tumors has an IC₅₀ of 50 nM on LIMK1 and 75 nM on LIMK2.

### 1.2. Inhibition of ROCK1 and ROCK2

ROCK1 and ROCK2 inhibition has also been assessed, but at a single concentration of 1 µM. These tests have been performed by using the « Z'Lyte ^{®} » assay based on the inhibition of the phosphorylation of a reference measured by FRET (Life Technologies).

Based on this assay, the compounds were classified in two groups: (i) compounds selectively inhibiting LIMKs, (ii) potential "dual" compounds inhibiting both LIMKs and ROCKs.

### 1.3. Selectivity on a panel of 100 kinases

The selectivity of the compounds towards LIMK1 and LIMK2 has been tested by Eurofins Kinase Screening & Profiling Services against 100 representative kinases at the concentration of 1 µM.

18 compounds have been tested. 6 of them are highly selective and inhibit exclusively LIMK1 and LIMK2 at more than 80%. These compounds are good candidates for future *in vivo* assays. Selective compounds avoid off-targets and secondary effects that might be deleterious. Especially, we want to develop compounds that do not target ROCK1 and ROCK2 the kinase upstream of LIMK1/2 that has many physiological targets. LIMK1 and LIMK2 are really downstream of their signalling pathway, the only downstream effector is their quasi-unique substrate cofiline. By targeting selectively LIMK1 and LIMK2, we should avoid side effects.

### 2. In cell assays

Six *in cell* assays have been used to characterize the activity of our compounds:
1. Determination of cytotoxicity on several cell lines
2. Actin cytoskeleton remodelling, by measuring the level of phospho-cofilin, cofilin is the canonical substrate of LlMKs;
3. Actin cytoskeleton dynamics, by visualising stress fibers induced by LIMK2 overexpression
4. Migration properties measured by a wound-healing assay
5. Microtubule dynamics by visualizing mitotic spindles
6. Dendrite plasticity by measuring their size

### 2.1. Cytotoxicity

The cytotoxicity of our compounds was assessed with the « CellTiter-Glo^{®} Luminescent Cell Viability » assay from Promega. This assay is based on the production of ATP by live cells. Two cell lines were studied; HeLa, and MPNST (Malignant Peripheral Nerve Sheath tumor, tumors associated with Neurofibromatose type I genetic disease).

### Day 1: Cell preparation in 96 well plates

Seed HeLa cells at 10,000 cells per well in 100 µl of DMEM.

Seed MPNST cells at 30,000 cells per well in 100 µl of DMEM.

### Day 2: Incubation with the inhibitor

For each inhibitor, a 1 mM solution in DMEM is prepared from the 10 mM stock solution. Serial dilutions are made from this solution with DMEM, to reach the following concentrations: 10, 20, 30, 40, 50, 60, 70, 80, 90, 100 µM. Cell medium is removed, and 100 µL of the solution of the inhibitors at the different concentrations are added.
Control conditions: (i) 100µl of 1 :100 DMSO, (ii) 100 µL of DMEM.

Incubation for 48 hours at 37 °C under CO₂ and humid atmosphere.

**Day 4:** Cytotoxicity determination with the "CellTiter-Glo^{®} Luminescent Cell Viability" assay from Promega, readout of the plates with Mithras apparatus.

The plate is removed from the incubator. 100 µL of reagent is added to each well. The plate is then incubated at 22 °C for 30 minutes. A standard with ATP diluted in DMEM is prepared: 100 µL of 1µM, 100 nM and 10 nM in triplicate to load on the same plate. Readout luminescence on Mithras apparatus.

### Data interpretation.

Luminescent values are directly linked to the amount of ATP present in the cells, this amount of ATP is linked to the number of live cells.

Data are normalized, 100% corresponds to the control condition 1:100 DMSO.

50 compounds have been tested on HeLa cell lines. EC₅₀ (inhibitor concentration corresponding to half of live cells) of most of the compounds of the invention are between 15 and 35 µM as compared to those of LIMKi3 and Pyr1 on HeLa cells which is 9 and 1 µM, respectively. 17 compounds have been tested on MPNST cell line. EC₅₀ of the compounds of the invention are between 35 and 90 µM (Table 1). Compared to published data, T56-LIMKi, the compound developed by Y. Kloog, has an EC₅₀ of about 20 µM on the MPNST cell line.

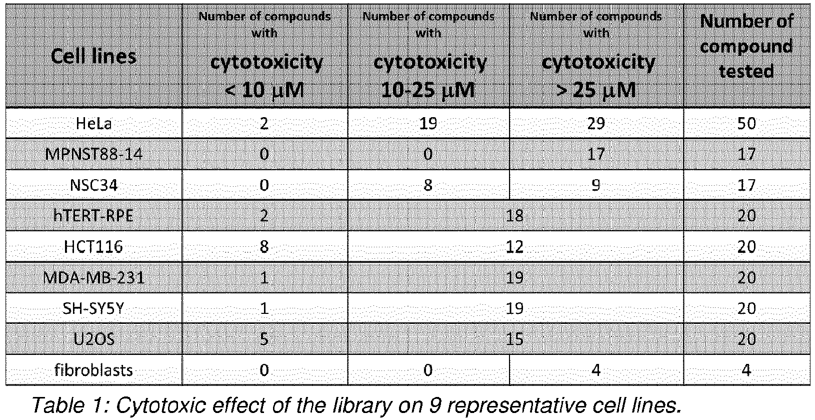

### 2.2. Actin cytoskeleton remodelling by measuring the level of phospho-cofilin

Cofilin is the canonical substrate of LIMKs. LIMKs inhibit cofilin by phosphorylation of its Serine 3. As cofilin is an Actin Depolymerization Factor (ADF), its inactivation blocks actin cytoskeleton remodelling.

The effect of the compounds on the level of phosphorylation of cofilin was measured by Western Blot, using a specific antibody: anti-phospho-Ser3-cofilin.

HeLa and MPNST cell lines were studied.

### Day 1: Cell preparation in 6 well plates

Seed HeLa cells at 150,000 cells per well in 2 mL of DMEM.

Seed MPNST cells at 500,000 cells per well in 2 mL of DMEM.

### Day 3: Incubation with the inhibitor and lysis

5 µl of the 10 mM stock solution or 5 µL of DMSO (control) are added to the media of the cultured cells => final concentration: 25 µM.

Incubate for 2 hours at 37 °C under CO₂ and humid atmosphere.

### Lysis:

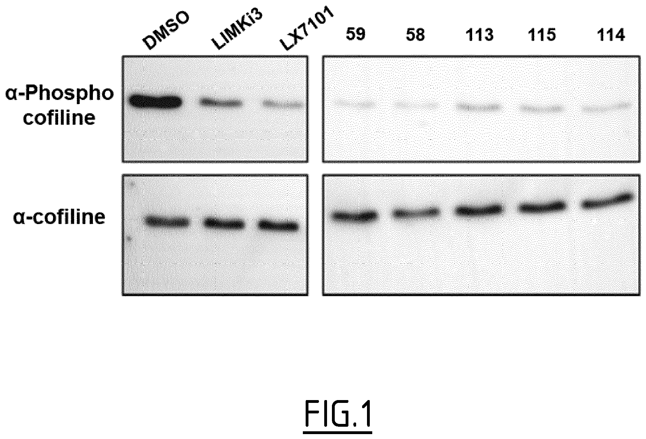

Remove the media. Wash the cells twice with 1 ml PBS, and incubate with 150 µl of lysis buffer (50 mM Tris/HCl, pH 7.5, 100 mM NaCl, 5 mM EDTA, 0.1% Triton X-100, 50 mM NaF, 10 mM sodium pyrophosphate, 1 mM Na₃VO₄,20 mM p-nitrophenyl phosphate, 20 mM β-glycerophosphate, 10 mg/mL aprotinin, 0.05 mg/mL okaidic acid, 1 mg/mL leupeptin, 1 mM PMSF) for 10 minutes on ice. Cell are collected (with a cell scraper), transferred in a tube and centrifuged at 10,000xg for 10 minutes at 4°C. Take 60 µL of supernatant and add 20 µL of Laemmli buffer 4x. Heat the samples at 95 °C for 5 minutes, spin at 10,000xg for 5 minutes, and analyse by Western Blot. Antibodies are anti-phosphoSer3-cofilin (#3313) and anti-cofilin (#3312) from Cell Signalling Technology (Figure 1 - Example of a Western Blot after treatment of HeLa cells with several inhibitors. Antibodies used in this experiment: upper part anti-phospho-Ser3-cofilin, lower part anti-cofilin).

The control condition correspond to cells incubated with DMSO (solvent in which inhibitors are diluted).

The rate of phospho-cofilin is quantified, divided by the rate of total cofilin, and normalized with the control condition DMSO (100% of phosphorylation). Each sample is analyzed twice to increase reproducibility, and the best molecules are tested twice in two independent experiments.

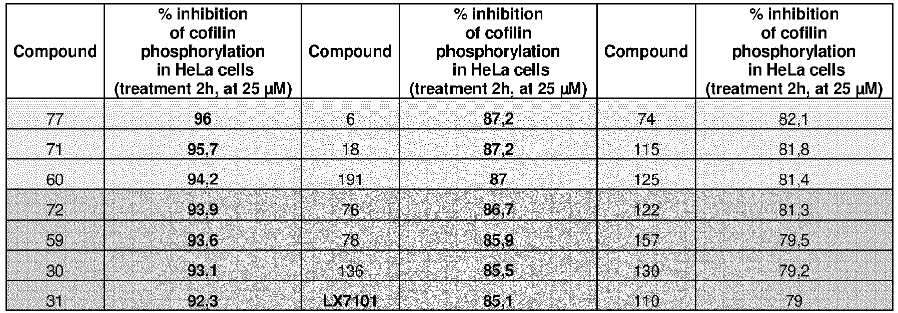

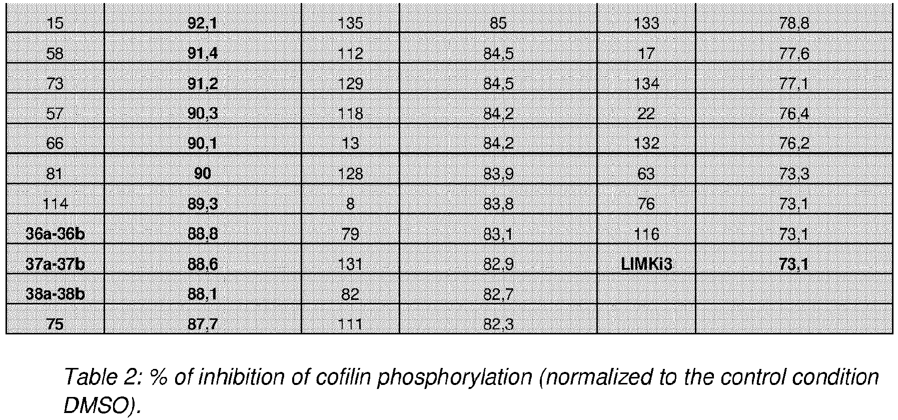

70 compounds have been tested on HeLa cell line. More than 80% of inhibition of the phosphorylation of the cofilin is observed. 50 compounds are more efficient than LlMKi3, 24 of these compounds are more efficient than LX7101.

20 compounds have been tested on MPNST cell line. Cofilin phosphorylation is inhibited between 60 and 80%. 9 compounds are more efficient than LIMKi3 and LX7101. Furthermore, 3 compounds which are not very good on the HeLa cell line (60 % of inhibition of phospho-cofilin) are really efficient on the MPNST cell line (80 % of inhibition of phospho-cofilin). These data suggest that some compounds selectively inhibit specific cell lines, and so they may target specific diseases.

To conclude, the compounds of the invention are highly active in cell on the direct phosphorylation of cofilin by LIMKs.

Cofilin phosphorylation was also tested by using another technic: LUMIT^{™} technology from Promega. LUMIT^{™} technology is an immunoassay based on luminescence detection. It requires the use of two different secondary antibodies coupled to a small and a big subunit of nanoluciferase respectively. When the two antibodies are fixed on their analyte, full nanoluciferase is reconstituted and active, it generates a bright luminescent signal in presence of its substrate. We have set up the optimale conditions to measure phospho-cofilin with this assay. And we are now testing the compounds of the invention with this new technic to reinforce our results obtained by western-blot.

### 2.3. Actin cytoskeleton dynamics by visualising stress fibers induced by LIMK2 overexpression

Another approach was then developed to test the efficiency of the compounds of the invention directly in live cells by imaging. In HeLa cells, stress fibers induced by LIMK2 overexpression are visualized by immunofluorescence. Indeed, when LIMK2 is overproduced, it phosphorylates cofilin, resulting in its inhibition. Cofilin can no longer depolymerise actin filaments, stress fibers are then observed.

### Day 1: Cell preparation in 6 well plates

Seed HeLa cells at 30,000 cells per well in 2 mL of DMEM.

### Day 2: Transfection

Remove the media from the cells. Add 1.5 mL of DMEM.

In Eppendorf tubes, add 150µL of OptiMEM.

Dilute 2.5 µg of DNA (Plasmidic preparation) in 150 µL of OptiMEM.

Mix the + Reagent (Invitrogen), add 7.5 µL of + Reagent to the previous mixture. Homogenize by inversion. Incubate 5min at room temperature.

Mix Lipofectamin LTX (Invitrogen). Add 7.5 µL of Lipofectamin LTX to 150 µL of OptiMEM in a new tube. Homogenize by inversion. Incubate 5 min at RT.

Add the OptiMEM-ADN mix to the OptiMEM-Lipofectamin mix. Homogenize by inversion. Incubate 30 min at RT.

Add on the top of the cells the OptiMEM-ADN-Lipofectamine mix without agitating to prevent complex disruption, drop by drop everywhere in the well.

Incubate for 4 to 5 hours.

Change the media.

### Day 3: Seed the cells in 4 well plates

Treat the cells with trypsin to detach them from the plate. Seed cells at 20,000 cellules per wells in 500µl of DMEM.

### Day 4: Incubation with the inhibitors and labelling

Cells are incubated for 2 hours with 25 µM of inhibitors (i.e. 1.25 µl of 10 mM stock solution) or 1.25 µL of DMSO (control) at 37°C under CO₂ and humid atmosphere.

Labelling: Remove the media. Wash three times with 500 µL of PBS 1x.

Add 250 µL of PBS/4% PFA. Incubate 20 min at RT.

Remove PBS/PFA. Wash 3 times with 500 µL of PBS 1x.

Add 250µL of PBS/0.5% Triton. Incubate 15 min at RT.

Remove PBS/Triton. Wash 3 times with 500 µL of PBS 1x.

Add 250 µL of primary antibody (anti-HA from Roche Applied Science) diluted in PBS/1% BSA. Incubate 1h at RT.

Remove primary antibody. Wash 3 times with 500 µL of PBS/0.5% BSA.

Add 250 µL of secondary antibody (FITC anti-rat) or 250 µL de phalloidin coupled to AlexaFluor568 diluted in PBS/1% BSA. Incubate 1h at RT with no light.

Remove the media. Wash 3 times with 500 µL of PBS/0.5% BSA.

### Mounting

Mount coverslip with a drop of Vectashield (5µL).

Seal coverslip with nail polish to prevent drying and movement under microscope.

Store in dark at - +4 °C for 2-3 days, and then at -20 °C.

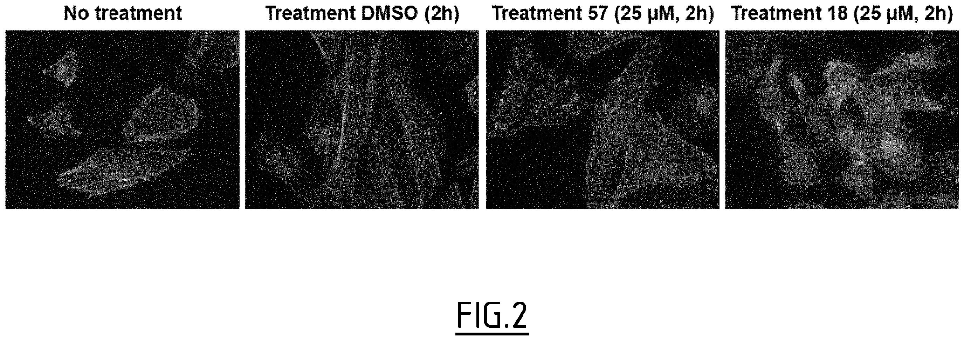

LIMK2 overexpression does induce stress fiber formation, which are not sensitive to DMSO treatment. When cells are treated with our compounds, stress fibers are much less numerous (Figure 2 - Confocal imaging of actin cytoskeleton. HeLa cells are transfected with a plasmid allowing the overproduction of LIMK2, treated for 2 h with 25 µM of inhibitors or DMSO (control). Stress fibers are visualized by phalloidin labelling, which binds to filamentous actin, by immunofluorescence (stress fibers appear as white thick lines inside the cells)). Therefore, the compounds of the invention inhibit stress fiber formation induced by LIMK2 overexpression.

This second test further shows that our compounds are efficient on the dynamics of actin filament polymerisation in live cells.

### 2.4. Migration properties measured by a wound-healing assay

Effects of the compounds of the invention on cell migration were measured by a wound-healing assay. A scratch was made on a cell monolayer, and the speed of cells to migrate to close the gap was measured.

U2OS cells were inoculated at the density of 40000 by well (96 well plate), grown overnight. 5 h before wound-healing, cells were starved in 0.5% fetal calf serum. Wound-healing was performed by a WoundMaker^{™} (Essen BioScience). Inhibitors were added in the medium at a final concentration of 2.5 µM. Data are acquired and analysed every hours during 24h by an IncuCyte^{®} S3 (Sartorius, Essen BioScience, MI UA).

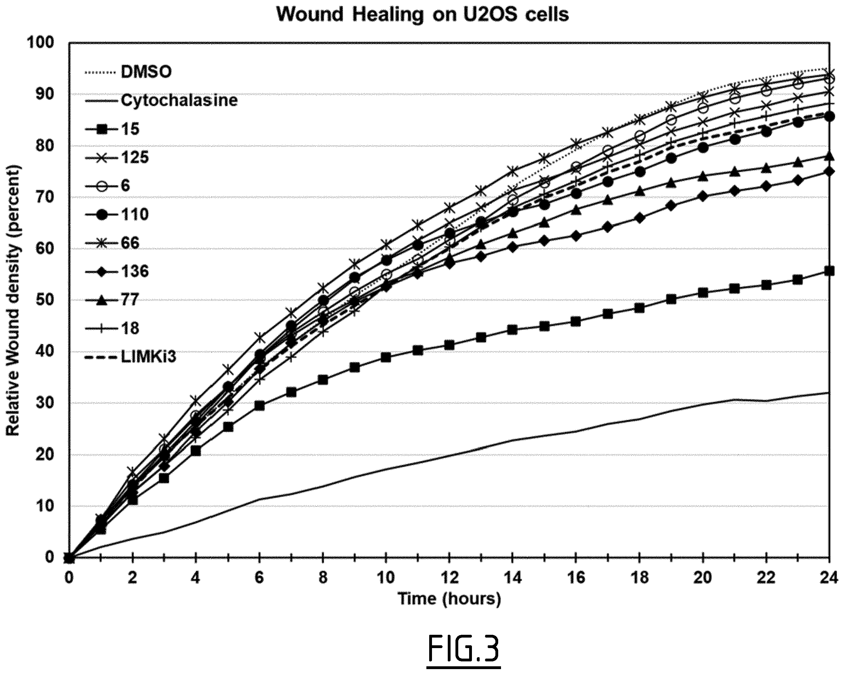

The results are shown in Figure 3. 8 compounds have been tested. 4 of them have stronger propensity to inhibit U2OS cell migration upon scratch compared to the reference LlMKi3. 1 compound is particularly efficient (compound 15). These data show that the tested compounds are efficient on cells to prevent their migration, a property playing a major role in the formation of metastases.

### 2.5. Microtubule dynamics by visualizing mitotic spindles

LIMK1 and LIMK2 are also affecting microtubule dynamics, but the molecular actors involved in this process are still unknown. To observe microtubule dynamics, microtubules have been labelled with an anti-tubulin antiboby, and mitotic spindles have been observed.

U2OS cells were plated on coverslips and grown for 24 hours. Inhibitors were added in the medium at a final concentration of 2.5 µM for 24 hours. Cells were then fixed in 4% formaldehyde, permeabilized in TBS-0.5% Triton-X100, and microtubules were labelled with anti-tubulin antibody.

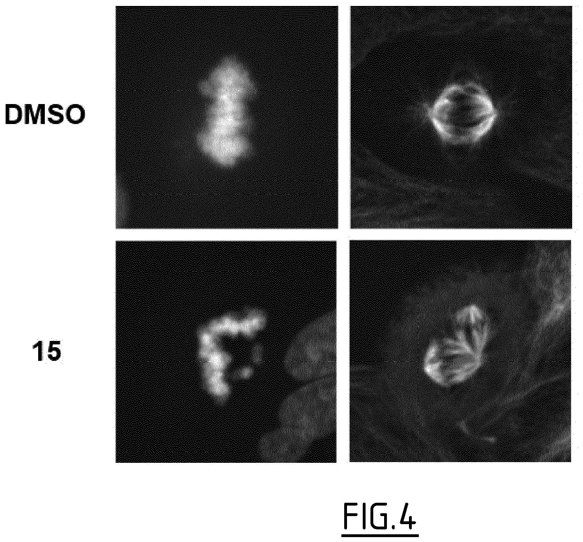

3 compounds have been tested. Compound 15 appeared to have strong effects on microtubule dynamics: astral microtubules disappeared, spindle poles were fractionated, and microtubules depolymerized as free tubulin was diffusing in the cytoplasm and polymerized microtubule structures were lost (see Figure 4).

### 2.6. Dendrite plasticity by measuring their size

Effect of the compounds of the invention on cytoskeleton dynamics was also assessed on specialized cells (NSC34, Mouse Motor Neuron-Like Hybrid cell line) to evaluate their effect on dendrite plasticity for neurological apllications.

40,000 NSC34 cells were seeded in 1 ml of DMEM per well (12 well plates) for 24 hours. Cells were then grown without serum to induce their differentiation in presence of 25µM of inhibitor for 24 hours. Dendrites were visualized by microscopy.

10 compounds have been tested. 4 of them strongly affected the size of the neurites showing an effect on their plasticity.

### 3. In vivo assay on a mouse model of ALS (Amyotrophic Lateral Sclerosis)

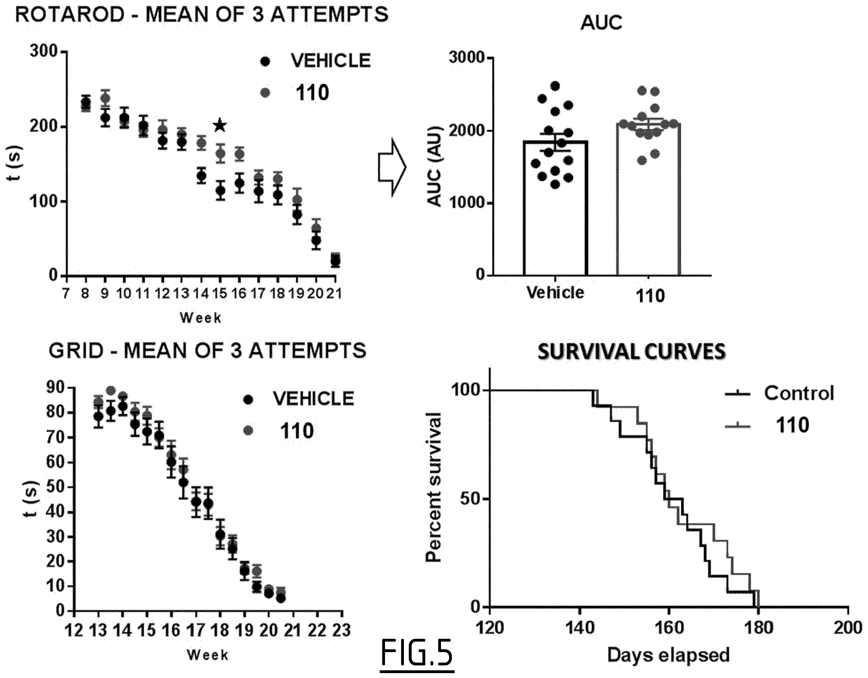

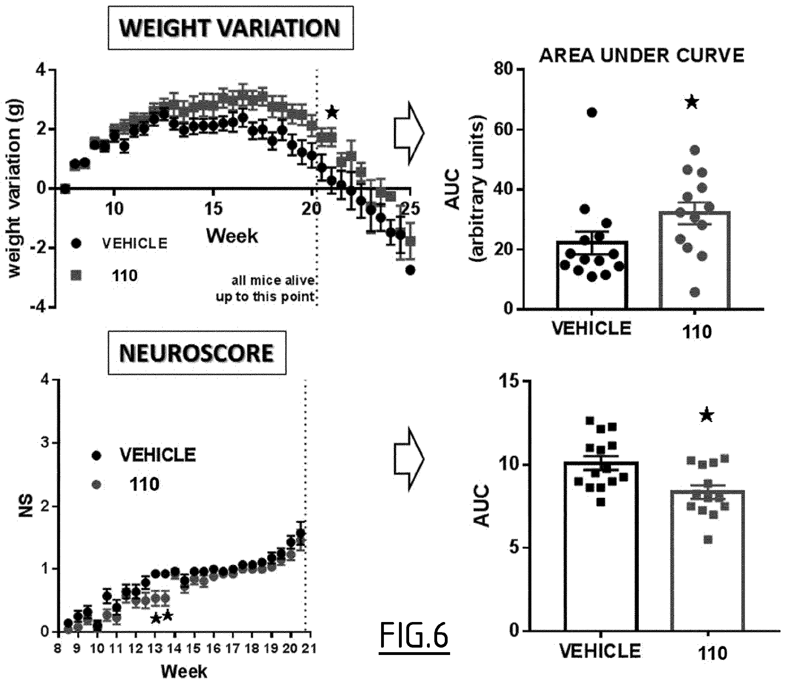

The inventors used the SOD1-G93A transgenic mice (Jackson Laboratory, B6.Cg-Tg (SOD1*G93A)1Gur/J), consisting in a commonly used pre-clinical model for ALS, to study the effect of compound 110 *in vivo.* These mice express copies of the human SOD1 transgene carrying the G93A mutation and recapitulate many of the pathological features of ALS in humans, such as progressive paralysis and premature death. Mice were given Compound 110 at 10 mg/Kg (n=13) or a vehicule solution (control, n=14) by intraperitoneal injection, 2 times a week from week 8 of age until the endpoint. Body weight was monitored twice a week from week 8 until reaching the endpoint. The endpoint (survival) was reached when mice were not able to right themselves once placed on their back within 30 seconds (three times). Several tests were perfomed to evaluate the motor behaviour. The neuroscore, a scoring system (1 slighly abnormal to 4 paralysis), was used to assess hindlimb function twice a week. It was performed by observing the mouse under 3 conditions performed sequentially: the mouse was suspended by the tail, was allowed to walk, and was placed on its side. The rotarod test, evaluating the balance and motor coordination, was performed by placing mice on a rotating cylinder (5 minutes, 3 attemps). The grid test was done by placing mice on a metal grid that is later inverted to assess muscle strength (Cut-off time 90 s). The results are shown in Figures 5 and 6

The inventors showed that Compound 110 has a significant effect on delaying the loss of weight and the onset of motor symptoms (early Neuroscore stages and first time decay in the rotarod test). We do not observed improvement in later development of the disease phenotype and endpoint of the disease (survival).

| Compound | Ki LIMK1 (nM) | Ki LIMK2 (nM) | %inhibition (ROCK1) 1uM | %inhibition (ROCK2) 1uM | % inhibition Cofilin phosphorylation HeLa cells treated 2h with 25 uM of inhibitor | % inhibition Cofilin phosphorylation MPNST88-14 cells treated 2h with 25 uM of inhibitor | % inhibition Cofilin phosphorylation MPNST90-8 cells treated 2h with 25 uM of inhibitor |
|---|---|---|---|---|---|---|---|
| **5** | 82.3 | 234 | 81 | 59 | | | |
| **6** | 124 | 319 | 86 | 69 | 87.20 | | |
| **7** | 51.4 | 105 | 72 | 70 | | | |
| **8** | 52.2 | 106 | 94 | 78 | 83.80 | | |
| **9** | 128 | 264 | 60 | 45 | | | |
| **10** | 244 | 268 | 46 | 30 | | | |
| **11** | 142 | 161 | 71 | 59 | | | |
| **12** | 114 | 116 | 79 | 69 | | | |
| **13** | 75 | 91 | 73 | 56 | 84.20 | | |
| **14** | 481 | 693 | 79 | 59 | | | |
| **15** | 19.4 | 14.4 | 44 | 24 | 92.10 | 78.60 | 83.20 |
| **16** | 80.1 | 341 | 15 | 14 | | | |
| **17** | 13,2 | 24,7 | 23 | 21 | | | |
| **18** | 54 | 128 | 17 | 18 | 87.20 | 83.30 | |
| **19** | 41 | 101 | 74 | 65 | 72.60 | | |
| **20** | 162 | 193 | 55 | 28 | | | |
| **21** | 84.6 | 150 | 54 | 39 | | | |
| **22** | 46.2 | 119 | 66 | 31 | 76.40 | | 74.20 |
| **24** | 190 | 180 | 40 | 25 | | | |
| **26** | 278 | 90.7 | 85 | 64 | | | |
| **28** | 34.5 | 43.6 | 76 | 35 | | | |
| **29** | 27.1 | 13.7 | 30 | 17 | 73.10 | | |
| **30** | 15.2 | 8.15 | 63 | 22 | 93.10 | | |
| **31** | 12.4 | 25.1 | 48 | 46 | 92.30 | | |
| **32** | 1420 | 2850 | 55 | 39 | | | |
| **33** | 50.1 | 186 | 96 | 87 | | | |
| **34-rac** | 610 | 742 | 79 | 52 | | | |
| **35-R** | 851 | 2540 | 24 | 12 | | | |
| **40** | 10.6 | 13.4 | 33 | 22 | | | |
| **42** | 30.7 | 53.3 | 37 | 31 | | | |

| Compound | Ki LIMK1 (nM) | Ki LIMK2 (nM) | %inhibition (ROCK1) 1uM | %inhibition (ROCK2) 1uM | % inhibition cofilin phosphorylation HeLa cells treated 2h with 25 uM of inhibitor | % inhibition cofilin phosphorylation MPNST88-14 cells treated 2h with 25 uM of inhibitor | % inhibition cofilin phosphorylation MPNST90-8 cells treated 2h with 25 uM of inhibitor |
|---|---|---|---|---|---|---|---|
| **47a-47b** | 15.9 | 27.7 | 98 | 94 | 88.8 | | |
| **48a-48b** | 13.9 | 14.1 | 95 | 95 | 88.6 | | |
| **49a-49b** | 10.8 | 8.89 | 96 | 98 | 88.1 | | |
| **57** | 46.6 | 103 | 59 | 36 | 90.3 | 81 | 93.3 |
| **58** | 49.7 | 72.5 | 34 | 32 | 91.4 | 77.3 | |
| **59** | 24 | 62.7 | 34 | 40 | 93.6 | 74.7 | |
| **60** | 186 | 54.3 | 11 | 7 | 94.2 | 74.5 | 81.1 |
| **63** | 58 | 8.49 | 77 | 61 | 90.1 | | |
| **66** | 58 | 8.49 | 77 | 61 | 90.1 | | |
| **71** | 109 | 86.1 | 37 | 10 | 95.7 | 80.4 | 92.7 |
| **72** | 60.6 | 32 | 54 | 31 | 93.9 | 74.6 | 94.4 |
| **73** | 63.1 | 31.7 | 73 | 37 | 91.2 | 77.7 | 94 |
| **74** | 27.7 | 9.67 | 9 | 8 | 82.1 | | |
| **75** | 32.1 | 2.73 | 28 | 19 | 87.7 | | |
| **76** | 20.5 | 6.95 | 28 | 19 | 86.7 | | |
| **77** | 22.2 | 4.64 | 19 | 12 | 96 | | |
| **78** | 19.8 | 21.1 | 20 | 11 | 85.9 | | |
| **79** | 42.5 | 10.9 | 6 | 8 | 83.1 | | |
| **81** | 13.3 | 9.98 | 36 | 35 | 90 | | |
| **82** | 47.4 | 20.3 | 16 | 11 | 82.7 | | |
| **83** | 90.9 | 203 | 6 | -4 | | | |

| Compound | Ki LIMK1 (nM) | Ki LIMK2 (nM) | %inhibition (ROCK1) 1uM | %inhibition (ROCK2) 1uM | % inhibition Cofilin phosphorylation HeLa cells treated 2h with 25 uM of inhibitor | % inhibition Cofilin phosphorylation MPNST88-14 cells treated 2h with 25 uM of inhibitor | % inhibition Cofilin phosphorylation MPNST90-8 cells treated 2h with 25 uM of inhibitor |
|---|---|---|---|---|---|---|---|
| **89** | 6.6 | 23.3 | 0 | 7 | | | |
| **90** | 16.9 | 108 | 4 | -5 | | | |
| **91** | 14.7 | 78.5 | 3 | -1 | | | |
| **92** | 8.99 | 16.5 | 2 | -6 | | | |
| **93** | 3.71 | 27.4 | 1 | 0 | | | |
| **94** | 31 | 18.4 | 3 | -4 | | | |
| **110** | 5 | 41 | 3 | 9 | 79 | | |
| **111** | 5.78 | 25.5 | 10 | 14 | 82.3 | | |
| **112** | 7 | 24 | 8 | 13 | 84.5 | | |
| **113** | 7.13 | 25.6 | 7 | 4 | 87 | | |
| **114** | 9.98 | 25.5 | 4 | 8 | 89.3 | 74.7 | |
| **115** | 5.37 | 16.2 | 7 | 11 | 81.8 | | |
| **116** | 16.5 | 64 | -8 | -7 | 73.1 | | |
| **117** | 22 | 61.3 | 4 | 7 | 69.2 | | |
| **118** | 14.7 | 40.4 | 10 | -4 | 84.2 | | |
| **119** | 7.98 | 30.4 | 0 | 11 | 64.1 | | |
| **120** | 14.9 | 29.6 | 0 | -9 | | | |
| **121** | 6.1 | 25.2 | 3 | 18 | 64.5 | | |
| **122** | 7.11 | 4.02 | 4 | 14 | 81.3 | | 65.6 |
| **123** | 11.5 | 16.2 | 10 | 1 | | | |
| **124** | 6.49 | 19.6 | 7 | 2 | | | |
| **125** | 29 | 28.5 | 10 | -2 | 81.4 | 80.8 | 76.1 |

| Compound | Ki LIMK1 (nM) | Ki LIMK2 (nM) | %inhibition (ROCK1) 1uM | %inhibition (ROCK2) 1uM | % inhibition Cofilin phosphorylation HeLa cells treated 2h with 25 uM of inhibitor | |
|---|---|---|---|---|---|---|
| **126** | 30.2 | 20.5 | 7 | -5 | | |
| **127** | 8.19 | 39.3 | 3 | 20 | 68.5 | |
| **128** | 27.1 | 68.3 | ok | -14 | 83.9 | |
| **129** | 16.6 | 54.9 | 18 | 11 | 84.5 | |
| **130** | 26.7 | 87.5 | 26 | 0 | 79.2 | |
| **131** | 46.9 | 8.73 | 14 | 1 | 82.9 | |
| **132** | 389 | 415 | -6 | 2 | 76.2 | |
| **133** | 49.1 | 40.5 | -19 | 1 | 78.8 | |
| **134** | 36.1 | 33.4 | -20 | -5 | 77.1 | |
| **135** | 22.1 | 36.2 | -14 | -3 | 85 | |
| **136** | 2.75 | 21.6 | 6 | 11 | 85.5 | |
| **137** | 4.97 | 8.59 | -1 | 4 | | |

| Compound | Ki LlMK1 (nM) | Ki LIMK2 (nM) | %inhibition (ROCK1) | %inhibition (ROCK2) | % inhibition Cofilin phosphorylation HeLa cells treated 2h with 25 uM of inhibitor | % inhibition Cofilin phosphorylation MPNST88-14 cells treated 2h with 25 uM of inhibitor |
|---|---|---|---|---|---|---|
| **154** | 52.7 | 78.6 | 5 | 7 | 66.9 | 57.7 |
| **155** | 94.1 | 94.2 | 10 | 14 | 72.8 | 57.7 |
| **156** | 41.4 | 31.5 | 3 | 15 | 68.2 | 61.6 |
| **157** | 172 | 319 | 7 | -1 | 79.5 | 56.5 |
| **158** | 65.6 | 93 | 2 | 1 | 65.3 | 59.8 |
| **159** | 51 | 51.5 | 11 | 3 | 70.3 | 64.3 |
| **160** | 1170 | 2440 | 1 | -9 | | |
| **161** | 1170 | 2440 | 1 | -9 | | |
| **162** | 722 | 1030 | 4 | -8 | | |
| **175** | 12 | 9.66 | 43 | 36 | 98.12 | |

| Compound | Ki LIMK1 (nM) | Ki LIMK2 (nM) | %inhibition (ROCK1) 1uM | %inhibition (ROCK2) 1uM | | |
|---|---|---|---|---|---|---|
| **164** | 48.4 | 41.2 | -2 | 4 | | |
| **165** | 169 | 58.1 | -2 | 7 | | |
| **167** | 112 | 113 | -4 | 6 | | |
| **168** | 244 | 116 | 2 | 20 | | |
| **166** | 94.6 | 122 | 12 | 2 | | |
| **169** | 160 | 148 | 10 | 8 | | |
| **172** | 1080 | 2540 | 88 | 78 | | |
| **177** | 33.6 | 150 | 49 | 30 | | |
| **184** | 710 | 1470 | 87 | 78 | | |
| **185** | 669 | 863 | 99 | 97 | | |
| **188** | 65 | 50.8 | 48 | 33 | | |
| **191** | 85.5 | 217 | 72 | 57 | | |
| **195** | 174 | 189 | 99 | 93 | | |
| **196** | 192 | 49.3 | 21 | 12 | | |
| **198** | 3.08 | 2.89 | 79 | 65 | | |
| **200** | 46.2 | 9.83 | 35 | 13 | | |
| **202** | 70.4 | 68.7 | 26 | 15 | | |
| **204** | 50.5 | 15.6 | 71 | 46 | | |
| **206** | 418 | 676 | 13 | 3 | | |
| **208** | 37.3 | 7.57 | 91 | 72 | | |
| **211** | 103 | 466 | 5 | 3 | | |
| **213** | 12.9 | 16.3 | 55 | 27 | | |
| **216** | 4.88 | 14.6 | 71 | 56 | | |
| **219** | 4.93 | 5.46 | 60 | 37 | | |
| **222** | 4.59 | 2.96 | 66 | 48 | | |

## Claims

1. A compound having the following formula (I): 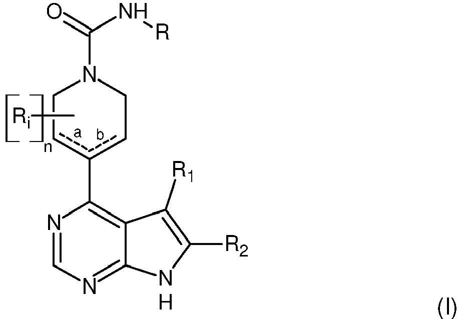 wherein:
- "a" is a single bond and "b" is a double bond, or "a" is a double bond and "b" is a single bond;
- R₁ is selected from the group consisting of: H, (C₁-C₆)alkyl group, halogen, and (C₃-C₇)cycloalkyl group;
- R₂ is selected from the group consisting of: H, and (C₁-C₆)alkyl group;
- or R₁ and R₂ may form together with the carbon atoms carrying them a (C₅-C₇)cycloalkyl group or a (C₅-C₇)heterocycloalkyl group, said heterocycloalkyl group being optionally substituted with a (C₁-C₆)alkyl group;
- n is 0, 1 or 2;
- each Rᵢ, identical or different, is selected from the (C₁-C₆)alkyl groups;
- R is selected from the group consisting of: (C₃-C₇)cycloalkyl group, optionally fused with an aryl group, aryl group, heteroaryl group, heterocycloalkyl group fused with an aryl group, and aryl group fused with a heterocycloalkyl group, said aryl and heteroaryl groups being optionally substituted with at least one substituent R₃ selected from the group consisting of:
. H,
. (C₁-C₆)alkyl, said alkyl being optionally interrupted with at least one heteroatom,
. heterocycloalkyl,
. aryl, said aryl being optionally substituted with at least one substituent such as:
. (C₁-C₆)alkoxy;
. NRₐR_{b}, Rₐ and R_{b}, independently from each other, being H or a (C₁-C₆)alkyl group;
. -X-R_{c}, X being a bond, a (C₁-C₆)alkylene group, preferably -CH₂-, or -C(=O)-, and R_{c} being a heterocycloalkyl group, optionally substituted with at least one (C₁-C₆)alkyl group, SO₂CH₃ or (C₁-C₆)alkylamino group;
. halogen;
. -C(=O)-NRₐR_{b}, Rₐ and R_{b}, independently from each other, being H or a (C₁-C₆)alkyl group;
. -C(=O)-N(R_{d})-X₁-NRₐR_{b}, R_{d}, Rₐ and R_{b}, independently from each other, being H or a (C₁-C₆)alkyl group, and X₁ being a (C₁-C₆)alkylene group, preferably CH₂; or
. -O-X₂-NRₑR_{f}, X₂ being a (C₁-C₆)alkylene group, Rₑ and R_{f}, independently from each other, being H or a (C₁-C₆)alkyl group, or at least one of Rₑ and R_{f} is an amino-protecting group such as a carbamate or comprises one fluorophore or is SO₂CH₃; or
. heteroaryl, said heteroaryl being optionally substituted with at least one substituent such as:
. (C₁-C₆)alkoxy;
. NRₐR_{b}, Rₐ and R_{b}, independently from each other, being H or a (C₁-C₆)alkyl group;
. -C(=O)-NRₐR_{b}, Rₐ and R_{b}, independently from each other, being H or a (C₁-C₆)alkyl group; or
. -NH-C(=O)-Rₐ, Rₐ being H or a (C₁-C₆)alkyl group;
. halogen,
. halo(C₁-C₆)alkyl,
. aryloxy, said aryloxy being optionally substituted with at least one substituent such as:
. (C₁-C₆)alkoxy;
. OH;
. NRₐR_{b}, Rₐ and R_{b}, independently from each other, being H or a (C₁-C₆)alkyl group;
. -X-R_{c}, X being a bond, a (C₁-C₆)alkylene group, preferably -CH₂-, or -C(=O)-, and R_{c} being a heterocycloalkyl group, optionally substituted with at least one (C₁-C₆)alkyl group, SO₂CH₃ or (C₁-C₆)alkylamino group;
. halogen;
. -O-benzyl,
. -O-C(=O)-NRₐR_{b}, Rₐ and R_{b}, independently from each other, being H or a (C₁-C₆)alkyl group;
. -NH-C(=O)-Rₐ, Rₐ being H or a (C₁-C₆)alkyl group;
. -C(=O)-NRₐR_{b}, Rₐ and R_{b}, independently from each other, being H or a (C₁-C₆)alkyl group; or
. -C(=O)-N(R_{d})-X₁-NRₐR_{b}, R_{d}, Rₐ and R_{b}, independently from each other, being H or a (C₁-C₆)alkyl group, and X₁ being a (C₁-C₆)alkylene group, preferably CH₂;
. heteroaryloxy, said heteroaryloxy being optionally substituted with at least one substituent such as:
-C(=O)-NRₐR_{b}, Rₐ and R_{b}, independently from each other, being H or a (C₁-C₆)alkyl group;
. -O-halo(C₁-C₆)alkyl,
. cyano,
. (C₁-C₆)alkoxy,
. -X-NRₐR_{b}, X being selected in the group consisting of: (C₁-C₆)alkylene group, -O-C(=O)-, -C(=O)-, and -NH-C(=O)-, and Rₐ and R_{b}, independently from each other, being H or a (C₁-C₆)alkyl group;
. -X'-R_{c}, X' being -C(=O)- or a -O-(C₁-C₆)alkylene radical, R_{c} being a cycloalkyl group, optionally substituted with at least one halogen, or R_{c} being a heterocycloalkyl group, optionally substituted with SO₂CH₃ or at least one (C₁-C₆)alkyl group; and
. -O-X₂-NRₑR_{f}, X₂ being a (C₁-C₆)alkylene group, Rₑ and Rf, independently from each other, being H or a (C₁-C₆)alkyl group, or at least one of Rₑ and R_{f} is an amino-protecting group such as a carbamate (Boc);
or its pharmaceutically acceptable salts, racemates, diastereomers or enantiomers.

2. The compound of claim 1, wherein R is a (C₃-C₇)cycloalkyl group, preferably an unsubstituted cyclohexyl group.

3. The compound of claim 1, wherein R is an aryl group, optionally substituted with at least one substituent R₃ as defined in claim 1, preferably an optionally substituted phenyl group.

4. The compound of claim 1 or 3, having the following formula (II): 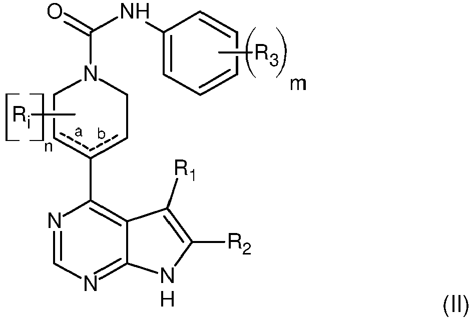 wherein:
- a, b, n, Rᵢ, R₁, and R₂ are as defined in claim 1, and
- m is 1 or 2, and each R₃, identical or different, is as defined in claim 1.

5. The compound of any one of claims 1, 3, or 4, having the following formula (III): 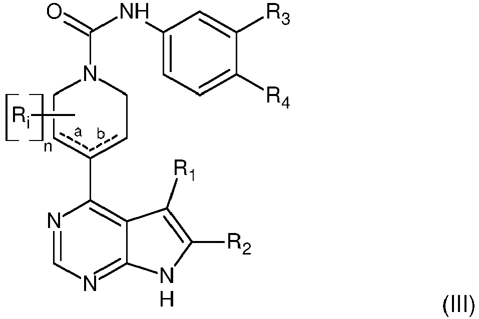 wherein:
- a, b, n, Rᵢ, R₁, R₂, and R₃ are as defined in claim 1, and
- R₄ is H, halogen or aryloxy.

6. The compound of any one of claims 1, and 3 to 5, having the following formula (IV): 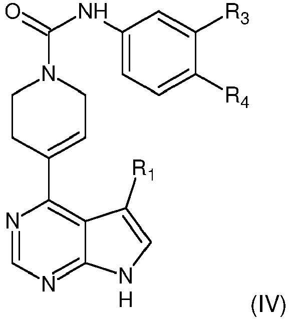 wherein:
- R₁ is H or (C₁-C₆)alkyl group;
- R₃ is selected from the group consisting of:
. H,
. (C₁-C₆)alkyl,
. aryl, preferably phenyl, said aryl being optionally substituted with at least one substituent such as (C₁-C₆)alkoxy, in particular OMe, or halogen such as F,
. heteroaryl, such as pyridinyl, imidazolyl, pyrrolyl or triazolyl,
. halogen, such as F, Cl, or Br,
. halo(C₁-C₆)alkyl, such as CF₃,
. aryloxy, in particular phenyloxy, said aryloxy being optionally substituted with at least one substituent such as (C₁-C₆)alkoxy, preferably OMe,
. -O-halo(C₁-C₆)alkyl, such as OCF₃,
. cyano,
. (C₁-C₆)alkoxy, such as OMe, and
. -O-C(=O)-NRₐR_{b}, Rₐ and R_{b}, independently from each other, being H or a (C₁-C₆)alkyl group; and
- R₄ is H, halogen or aryloxy, such as phenyloxy,
or R₃ and R₄ form together with the carbon atoms carrying them a phenyl group or a heterocycloalkyl group comprising preferably 2 oxygen atoms and 3 carbon atoms.

7. A compound of any one of claims 1, 3, and 4, having the following formula (V): 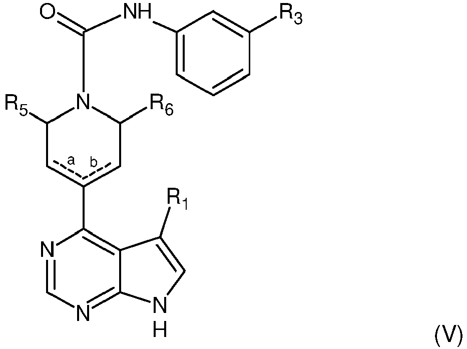 wherein:
- "a" is a single bond and "b" is a double bond, or "a" is a double bond and "b" is a single bond;
- R₁ is (C₁-C₆)alkyl group, preferably methyl;
- R₅ is (C₁-C₆)alkyl group, preferably methyl;
- R₆ is H or (C₁-C₆)alkyl group, preferably methyl;
- R₃ is selected from the group consisting of:
. halogen, such as F, Cl, or Br, and
. aryloxy, in particular phenyloxy.

8. The compound of any one of claims 1, 3 and 4, having the following formula (VI): 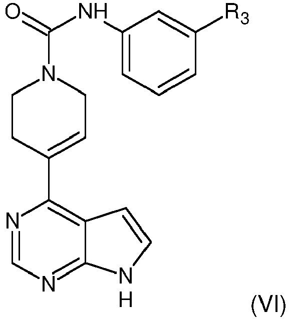 wherein R₃ is halogen.

9. The compound of any one of claims 1, 3 and 4, having the following formula (VII): 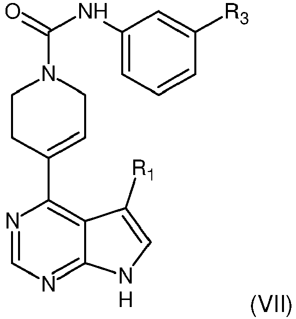 wherein:
- R₁ is halogen, preferably Cl; and
- R₃ is -O-C(=O)-NRₐR_{b}, Rₐ and R_{b}, independently from each other, being H or a (C₁-C₆)alkyl group.

10. The compound of any one of claims 1, 3 and 4, having the following formula (VIII): 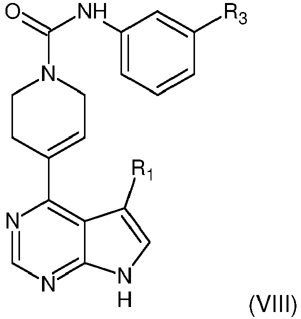 wherein:
- R₁ is (C₃-C₇)cycloalkyl group, preferably cyclopropyl;
- R₃ is selected from the group consisting of:
. aryl, preferably phenyl, said aryl being optionally substituted with at least one substituent such as (C₁-C₆)alkoxy, in particular OMe,
. heteroaryl, such as pyrrolyl,
. halogen, such as F, Cl, or Br,
. aryloxy, in particular phenyloxy, said aryloxy being optionally substituted with at least one substituent such as (C₁-C₆)alkoxy, preferably OMe, and
. -O-C(=O)-NRₐR_{b}, Rₐ and R_{b}, independently from each other, being H or a (C₁-C₆)alkyl group.

11. The compound of any one of claims 1, 3 and 4, having the following formula (IX): 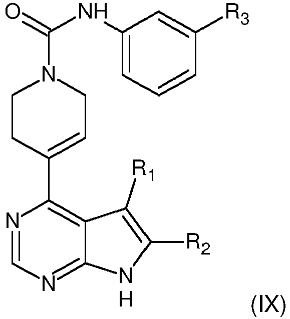 wherein:
- R₁ is (C₁-C₆)alkyl group, preferably methyl;
- R₂ is (C₁-C₆)alkyl group, preferably methyl;
- R₃ is selected from the group consisting of:
. H,
. aryl, preferably phenyl, said aryl being optionally substituted with at least one substituent such as (C₁-C₆)alkoxy, in particular OMe,
. halogen, such as F, Cl, or Br,
. (C₁-C₆)alkoxy, such as OMe, and
. -O-C(=O)-NRₐR_{b}, Rₐ and R_{b}, independently from each other, being H or a (C₁-C₆)alkyl group.

12. The compound of any one of claims 1, 3 and 4, having the following formula (X): 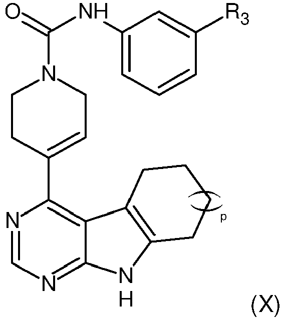 wherein:
- p is 0, 1, or 2;
- R₃ is selected from the group consisting of:
. H,
. (C₁-C₆)alkyl, said alkyl being optionally interrupted with at least one heteroatom,
. heterocycloalkyl, such as morpholinyl,
. aryl, preferably phenyl, said aryl being optionally substituted with at least one substituent such as (C₁-C₆)alkoxy,
. heteroaryl, such as pyridinyl,
. halogen,
. aryloxy,
. halo(C₁-C₆)alkyl,
. cyano,
. (C₁-C₆)alkoxy, and
. -X-NRₐR_{b}, X being selected in the group consisting of: (C₁-C₆)alkylene group, -O-C(=O)-, -C(=O)-, and -NH-C(=O)-, and Rₐ and R_{b}, independently from each other, being H or a (C₁-C₆)alkyl group.

13. The compound of any one of claims 1, 3 and 4, having the following formula (XI): 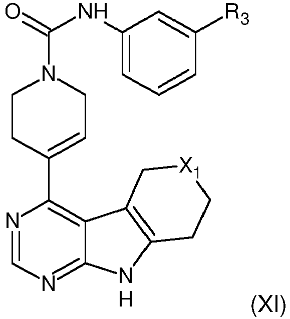 wherein:
- X₁ is -O-, -S-, or -N((C₁-C₆)alkyl)-, such as -N(Me)-; and
- R₃ is halogen.

14. A medicament comprising a compound according to any one of claims 1 to 13, or a pharmaceutically acceptable salt thereof.

15. A compound according to any one of claims 1 to 13, for use in a method of treating a condition selected in the group consisting of: cancers, virion infections, ocular hypertension and glaucoma formation, Neurofibromatosis type 1 and 2, psoriatic lesions, inflammatory diseases and hyperalgesia, central sensitization and chronic pain, reproduction erectile dysfunction, and neuronal diseases.

## Patentansprüche

1. Eine Verbindung mit der folgenden Formel (I): 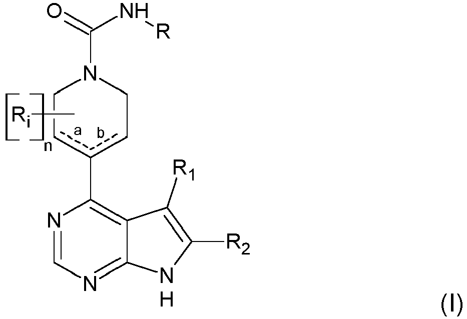 worin:
- "a" eine Einfachbindung und "b" eine Doppelbindung ist, oder "a" eine Doppelbindung und "b" eine Einfachbindung ist;
- R₁ ausgewählt ist aus der Gruppe bestehend aus: H, (C₁-C₆)Alkylgruppe, Halogen und (C₃-C₇)Cycloalkylgruppe;
- R₂ ausgewählt ist aus der Gruppe, bestehend aus: H und (C₁-C₆)-Alkylgruppe;
- oder R₁ und R₂ können zusammen mit den Kohlenstoffatomen, die sie tragen, eine (C₅-C₇)-Cycloalkylgruppe oder eine (C₅-C₇)-Heterocycloalkylgruppe bilden, wobei die Heterocycloalkylgruppe gegebenenfalls mit einer (C₁-C₆)-Alkylgruppe substituiert ist;
- n 0, 1 oder 2 ist;
- jedes Ri, gleich oder verschieden, ausgewählt ist aus den (C₁-C₆)-Alkylgruppen;
- R ausgewählt ist aus der Gruppe bestehend aus: einer (C₃-C₇)-Cycloalkylgruppe, gegebenenfalls kondensiert mit einer Arylgruppe, einer Arylgruppe, einer Heteroarylgruppe, einer Heterocycloalkylgruppe, kondensiert mit einer Arylgruppe, und einer Arylgruppe, kondensiert mit einer Heterocycloalkylgruppe,
wobei die Aryl- und Heteroarylgruppen gegebenenfalls mit mindestens einem Substituenten R₃, ausgewählt aus der Gruppe bestehend aus, substituiert sind:
. H,
. (C₁-C₆)-Alkyl, wobei das Alkyl gegebenenfalls durch mindestens ein Heteroatom unterbrochen ist,
. Heterocycloalkyl,
. Aryl, wobei das Aryl gegebenenfalls mit mindestens einem Substituenten substituiert ist, wie z.B.:
. (C₁-C₆)-Alkoxy;
. NRaRb, wobei Ra und Rb unabhängig voneinander H oder eine (C₁-C₆)-Alkylgruppe sind;
. -X-Rc, wobei X eine Bindung, eine (C₁-C₆)-Alkylengruppe, vorzugsweise -CH₂-, oder -C(=O)- ist, und Rc eine Heterocycloalkylgruppe ist, die gegebenenfalls mit mindestens einer (C₁-C₆)-Alkylgruppe, SO₂CH₃ oder (C₁-C₆)-Alkylaminogruppe substituiert ist;
. Halogen;
. -C(=O)-NRaRb, wobei Ra und Rb unabhängig voneinander H oder eine (C₁-C₆)-Alkylgruppe sind;
. -C(=O)-N(Rd)-X1-NRaRb, wobei Rd, Ra und Rb unabhängig voneinander H oder eine (C₁-C₆)-Alkylgruppe sind und X1 eine (C₁-C₆)-Alkylengruppe, vorzugsweise CH₂, ist; oder
. -O-X₂-NRₑR_{f}, wobei X₂ eine (C₁-C₆)-Alkylengruppe ist, Re und Rf unabhängig voneinander H oder eine (C₁-C₆)-Alkylgruppe sind, oder mindestens eines von Re und Rf eine Aminoschutzgruppe wie ein Carbamat ist oder ein Fluorophor enthält oder SO₂CH₃ ist; oder
. Heteroaryl, wobei das Heteroaryl gegebenenfalls mit mindestens einem Substituenten substituiert ist, wie z. B.:
. (C₁-C₆)-Alkoxy;
. NRaRb, wobei Ra und Rb unabhängig voneinander H oder eine (C₁-C₆)-Alkylgruppe sind;
. -C(=O)-NRaRb, wobei Ra und Rb unabhängig voneinander für H oder eine (C₁-C₆)-Alkylgruppe stehen; oder
. -NH-C(=O)-Ra, wobei Ra für H oder eine (C₁-C₆)-Alkylgruppe steht;
. halogen,
. Halo(C₁-C₆)alkyl,
. Aryloxy, wobei das Aryloxy gegebenenfalls mit mindestens einem Substituenten substituiert ist, wie z.B.:
. (C₁-C₆)-Alkoxy;
. OH;
. NRₐR_{b}, wobei Rₐ und R_{b} unabhängig voneinander H oder eine (C₁-C₆)-Alkylgruppe sind;
. -X-R_{c}, wobei X eine Bindung, eine (C₁-C₆)-Alkylengruppe, vorzugsweise -CH₂-, oder -C(=O)- ist, und R_{c} eine Heterocycloalkylgruppe ist, die gegebenenfalls mit mindestens einer (C₁-C₆)-Alkylgruppe, SO₂CH₃ oder (C₁-C₆)-Alkylaminogruppe substituiert ist;
. Halogen;
. -O-Benzyl,
. -O-C(=O)-NRₐR_{b}, wobei Rₐ und R_{b} unabhängig voneinander H oder eine (C1-C6)-Alkylgruppe sind;
. -NH-C(=O)-Rₐ, wobei Rₐ für H oder eine (C₁-C₆)-Alkylgruppe steht;
. -C(=O)-NRₐR_{b}, wobei Rₐ und R_{b} unabhängig voneinander für H oder eine (C1-C6)-Alkylgruppe stehen; oder
. -C(=O)-N(R_{d})-X1-NRₐR_{b}, wobei R_{d}, Rₐ und R_{b} unabhängig voneinander H oder eine (C₁-C₆)-Alkylgruppe sind, und X1 eine (C₁-C₆)-Alkylengruppe, vorzugsweise CH₂ ist;
. Heteroaryloxy, wobei das Heteroaryloxy gegebenenfalls mit mindestens einem Substituenten, wie z.B.:
-C(=O)-NRₐR_{b}, wobei Rₐ und R_{b}, unabhängig voneinander, H oder eine (C₁-C₆)-Alkylgruppe sind;
-O-Halogen-(C₁-C₆)-Alkyl,
. Cyano,
. (C₁-C₆)-Alkoxy,
. -X-NRₐR_{b}, wobei X ausgewählt ist aus der Gruppe bestehend aus: (C₁-C₆)Alkylengruppe, -O-C(=O)-, -C(=O)- und -NH-C(=O)-, und Rₐ und R_{b} unabhängig voneinander H oder eine (C₁-C₆)Alkylgruppe sind;
. -X'-Rₑ, wobei X' -C(=O)- oder ein -O-(C₁-C₆)-Alkylenrest ist, Rc eine Cycloalkylgruppe, gegebenenfalls substituiert mit mindestens einem Halogen, ist oder Rc eine Heterocycloalkylgruppe, gegebenenfalls substituiert mit SO₂CH₃ oder mindestens einer (C₁-C₆)-Alkylgruppe, ist; und
.. -O-X₂-NRₑR_{f}, wobei X₂ eine (C₁-C₆)-Alkylengruppe ist, Rₑ und Rf unabhängig voneinander H oder eine (C₁-C₆)-Alkylgruppe sind, oder mindestens eines von Re und Rf eine Amino-Schutzgruppe wie ein Carbamat (Boc) ist;
oder seine pharmazeutisch akzeptablen Salze, Racemate, Diastereomere oder Enantiomere.

2. Verbindung nach Anspruch 1, wobei R eine (C₃-C₇)-Cycloalkylgruppe, vorzugsweise eine unsubstituierte Cyclohexylgruppe, ist.

3. Verbindung nach Anspruch 1, wobei R eine Arylgruppe ist, die gegebenenfalls mit mindestens einem Substituenten R₃, wie in Anspruch 1 definiert, substituiert ist, vorzugsweise eine gegebenenfalls substituierte Phenylgruppe.

4. Die Verbindung nach Anspruch 1 oder 3 mit der folgenden Formel 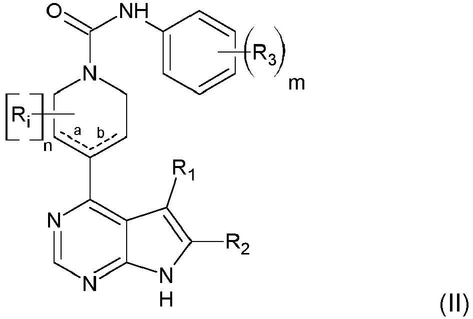 worin:
- a, b, n, Ri, R₁ und R₂ wie in Anspruch 1 definiert sind und
- m 1 oder 2 ist und jedes R₃, gleich oder verschieden, die in Anspruch 1 angegebene Bedeutung hat.

5. Die Verbindung nach einem der Ansprüche 1, 3 oder 4 mit der folgenden Formel (III): 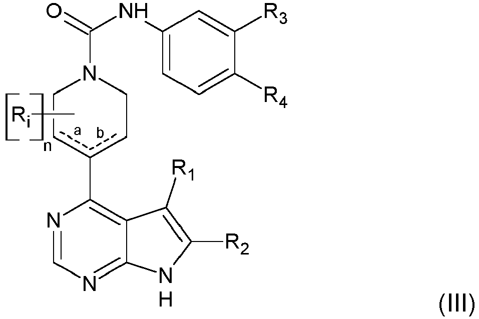 worin:
- a, b, n, Ri, R₁, R₂ und R₃ wie in Anspruch 1 definiert sind, und
- R₄ H, Halogen oder Aryloxy ist.

6. Die Verbindung nach einem der Ansprüche 1 und 3 bis 5 mit der folgenden Formel (IV): 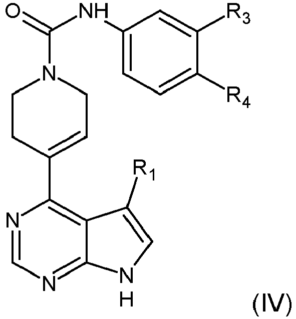 worin:
- R₁ H oder eine (C₁-C₆)-Alkylgruppe ist;
- R₃ ausgewählt ist aus der Gruppe bestehend aus:
. H,
. (C₁-C₆)-Alkyl,
. Aryl, vorzugsweise Phenyl, wobei das Aryl gegebenenfalls mit mindestens einem Substituenten wie (C₁-C₆)-Alkoxy, insbesondere OMe, oder Halogen, wie F, substituiert ist
. Heteroaryl, wie Pyridinyl, Imidazolyl, Pyrrolyl oder Triazolyl,
. Halogen, wie F, CI oder Br,
. Halo(C₁-C₆)alkyl, wie CF₃,
. Aryloxy, insbesondere Phenyloxy, wobei das Aryloxy gegebenenfalls mit mindestens einem Substituenten wie (C₁-C₆)-Alkoxy, vorzugsweise OMe, substituiert ist
. -O-Halogen-( C₁-C₆)-Alkyl, wie OCF₃,
. Cyano,
. (C₁-C₆)-Alkoxy, wie OMe, und
. -O-C(=O)-NRaRb, wobei Rₐ und R_{b} unabhängig voneinander H oder eine (C₁-C₆)-Alkylgruppe sind; und
- R₄ H, Halogen oder Aryloxy, wie z.B. Phenyloxy, ist,
oder R₃ und R₄ bilden zusammen mit den sie tragenden Kohlenstoffatomen eine Phenylgruppe oder eine Heterocycloalkylgruppe mit vorzugsweise 2 Sauerstoffatomen und 3 Kohlenstoffatomen.

7. Verbindung nach einem der Ansprüche 1, 3 und 4, die die folgende Formel (V) aufweist: 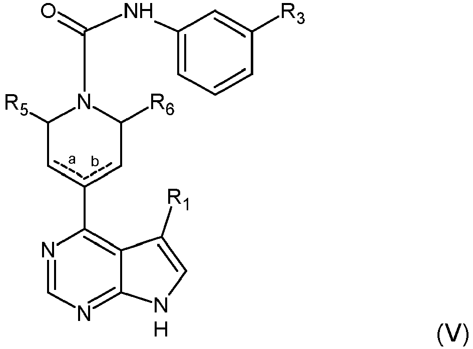 worin:
- "a" ist eine Einfachbindung und "b" ist eine Doppelbindung, oder "a" ist eine Doppelbindung und "b" ist eine Einfachbindung;
- R₁ ist eine (C₁-C₆)-Alkylgruppe, vorzugsweise Methyl;
- R₅ ist eine (C₁-C₆)-Alkylgruppe, vorzugsweise Methyl;
- R₆ ist H oder eine (C₁-C₆)-Alkylgruppe, vorzugsweise Methyl;
- R₃ ausgewählt ist aus der Gruppe bestehend aus:
. Halogen, wie F, Cl oder Br, und
. Aryloxy, insbesondere Phenyloxy.

8. Die Verbindung nach einem der Ansprüche 1, 3 und 4 mit der folgenden Formel (VI): 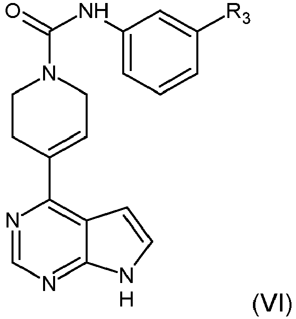 worin R₃ Halogen ist.

9. Die Verbindung nach einem der Ansprüche 1, 3 und 4 mit der folgenden Formel (VII): 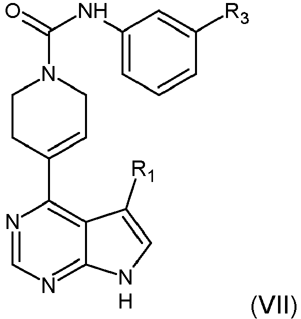 worin:
- R₁ Halogen, vorzugsweise CI, ist; und
- R₃ -O-C(=O)-NRₐR_{b} ist, wobei Rₐ und R_{b} unabhängig voneinander H oder eine (C₁-C₆)-Alkylgruppe sind.

10. Die Verbindung nach einem der Ansprüche 1, 3 und 4 mit der folgenden Formel (VIII): 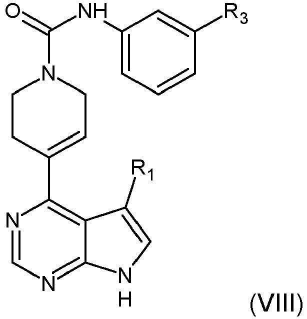 worin:
- R₁ ist eine (C₃-C₇)-Cycloalkylgruppe, vorzugsweise Cyclopropyl;
- R₃ ausgewählt ist aus der Gruppe bestehend aus:
. Aryl, vorzugsweise Phenyl, wobei das Aryl gegebenenfalls mit mindestens einem Substituenten wie (C₁-C₆)-Alkoxy, insbesondere OMe, substituiert ist
. Heteroaryl, wie z.B. Pyrrolyl,
. Halogen, wie F, CI oder Br,
. Aryloxy, insbesondere Phenyloxy, wobei das Aryloxy gegebenenfalls mit mindestens einem Substituenten wie (C₁-C₆)-Alkoxy, vorzugsweise OMe, substituiert ist, und
. -O-C(=O)-NRₐR_{b}, wobei Rₐ und R_{b} unabhängig voneinander H oder eine (C₁-C₆)-Alkylgruppe sind.

11. Die Verbindung nach einem der Ansprüche 1, 3 und 4 mit der folgenden Formel (IX): 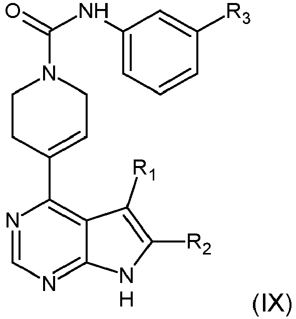 worin:
- R₁ eine (C₁-C₆)-Alkylgruppe, vorzugsweise Methyl ist;
- R₂ ist eine (C₁-C₆)-Alkylgruppe, vorzugsweise Methyl;
- R₃ ausgewählt ist aus der Gruppe bestehend aus:
. H,
. Aryl, vorzugsweise Phenyl, wobei das Aryl gegebenenfalls mit mindestens einem Substituenten wie (C₁-C₆)-Alkoxy, insbesondere OMe, substituiert ist
. Halogen, wie F, CI oder Br,
. (C₁-C₆)-Alkoxy, wie OMe, und
. -O-C(=O)-NRaRb, wobei Ra und Rb unabhängig voneinander H oder eine (C₁-C₆)-Alkylgruppe sind.

12. Die Verbindung nach einem der Ansprüche 1, 3 und 4 mit der folgenden Formel (X): 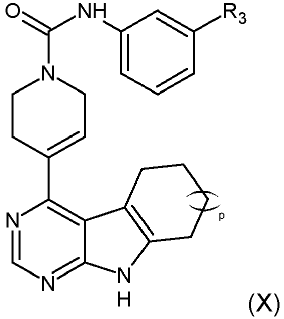 wobei:
- p 0, 1 oder 2 ist;
- R₃ ausgewählt ist aus der Gruppe bestehend aus:
. H,
. (C₁-C₆)-Alkyl, wobei das Alkyl gegebenenfalls durch mindestens ein Heteroatom unterbrochen ist,
. Heterocycloalkyl, wie z.B. Morpholinyl,
. Aryl, vorzugsweise Phenyl, wobei das Aryl gegebenenfalls mit mindestens einem Substituenten, wie (C₁-C₆)-Alkoxy, substituiert ist
. Heteroaryl, wie Pyridinyl,
. Halogen,
. Aryloxy,
. Halo(C₁-C₆)alkyl, ,
. Cyano,
. (C₁-C₆)-Alkoxy, und
. -X-NRₐR_{b}, wobei X ausgewählt ist aus der Gruppe bestehend aus: (C₁-C₆)Alkylengruppe, -O-C(=O)-, -C(=O)- und -NH-C(=O)-, und Ra und R_{b} unabhängig voneinander H oder eine (C₁-C₆)Alkylgruppe sind.

13. Die Verbindung nach einem der Ansprüche 1, 3 und 4 mit der folgenden Formel (XI): 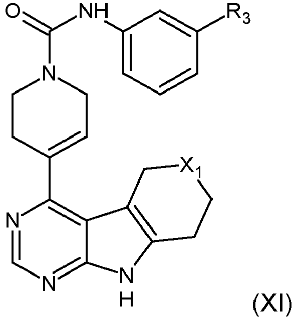 worin:
- X₁ -O-, -S- oder -N((C₁-C₆)Alkyl)-, wie -N(Me)-, ist; und
- R₃ Halogen ist.

14. Medikament, umfassend eine Verbindung nach einem der Ansprüche 1 bis 13 oder ein pharmazeutisch akzeptables Salz davon.

15. Verbindung nach einem der Ansprüche 1 bis 13 zur Verwendung in einem Verfahren zur Behandlung eines Zustands, ausgewählt aus der Gruppe bestehend aus: Krebserkrankungen, Virusinfektionen, okulare Hypertension und Glaukombildung, Neurofibromatose Typ 1 und 2, Psoriasisläsionen, entzündliche Erkrankungen und Hyperalgesie, zentrale Sensibilisierung und chronische Schmerzen, reproduktive erektile Dysfunktion und neuronale Erkrankungen.

## Revendications

1. Composé ayant la formule suivante (I) : 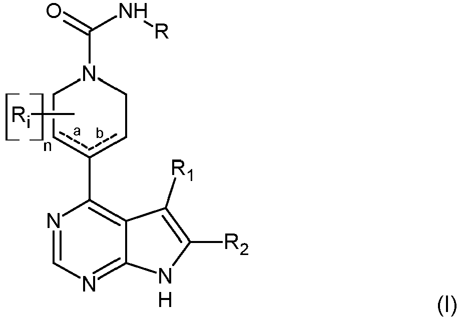 dans laquelle :
- "a" est une liaison simple et "b" est une liaison double, ou "a" est une liaison double et "b" est une liaison simple
- R₁ est choisi dans le groupe constitué de : H, groupe (C₁-C₆)alkyle, halogène et groupe (C₃ -C₇ )cycloalkyle ;
- R₂ est choisi dans le groupe constitué de : H, et groupe (C₁-C₆)alkyle ;
- ou R₁ et R₂ peuvent former avec les atomes de carbone qui les portent un groupe cycloalkyle (C₅-C₇) ou un groupe hétérocycloalkyle (C₅-C₇), ledit groupe hétérocycloalkyle étant éventuellement substitué par un groupe alkyle (C₁-C₆) ;
- n est 0, 1 ou 2
- chaque Rᵢ , identique ou différent, est choisi parmi les groupes (C₁-C₆)alkyle ;
- R est choisi dans le groupe consistant en : (C₃-C₇)groupe cycloalkyle, éventuellement fusionné avec un groupe aryle, groupe aryle, groupe hétéroaryle, groupe hétérocycloalkyle fusionné avec un groupe aryle, et groupe aryle fusionné avec un groupe hétérocycloalkyle,
lesdits groupes aryles et hétéroaryles étant éventuellement substitués par au moins un substituant R₃ choisi dans le groupe constitué par :
. H,
. (C₁-C₆)alkyle, ledit alkyle étant éventuellement interrompu par au moins un hétéroatome,
. hétérocycloalkyle,
. aryle, ledit aryle étant éventuellement substitué par au moins un substituanttel que :
. (C₁-C₆)alkoxy ;
. NRₐR_{b}, Rₐ et R_{b}, indépendamment l'un de l'autre, étant H ou un groupe (C₁ -C₆ )alkyle ;
. -X-R_{c}, X étant un lien, un groupe (C₁-C₆)alkylène, de préférence -CH₂, ou C(=O)-, et R_{c} étant un groupe hétérocycloalkyle, éventuellement substitué par au moins un groupe (C₁-C₆)alkyle, SO₂CH₃ ou (C₁-C₆)alkylamino ;
. halogène ;
. -C(=O)-NRₐR_{b} , Rₐ et R_{b}, indépendamment l'un de l'autre, étant H ou un groupe (C₁-C₆)alkyle ;
. -C(=O)-N(R_{d})-X₁-NRₐR_{b}, R_{d}, Rₐ et R_{b} , indépendamment les uns des autres, étant H ou un groupe (C₁-C₆ )alkyle, et X₁ étant un groupe (C₁-C₆)alkylène, de préférence CH₂ ; ou
. -O-X₂-NRₑR_{f}, X₂ étant un groupe (C₁-C₆)alkylène, Rₑ et Rf , indépendamment l'un de l'autre, étant H ou un groupe (C₁ -C₆ )alkyle, ou au moins un des Rₑ et R_{f} est un groupe protecteur aminé tel qu'un carbamate ou comprend un fluorophore ou est SO₂CH₃ ; ou
. hétéroaryle, ledit hétéroaryle étant éventuellement substitué par au moins un substituant tel que
. (C₁-C₆)alkoxy ;
. NRₐR_{b}, Rₐ et R_{b} , indépendamment l'un de l'autre, étant H ou un groupe (C₁-C₆)alkyle ;
. -C(=O)-NRₐR_{b}, Rₐ et R_{b}, indépendamment l'un de l'autre, étant H ou un groupe (C₁ -C₆ )alkyle ; ou
. -NH-C(=O)-Rₐ, Rₐ étant H ou un groupe alkyle (C₁-C₆) ;
. halogène
. halo(C₁-C₆)alkyle,
. aryloxy, ledit aryloxy étant éventuellement substitué parau moins un substituant tel que :
. (C₁-C₆)alkoxy ;
. OH ;
. NRₐR_{b}, Rₐ et R_{b}, indépendamment l'un de l'autre, étant H ou un groupe (C₁-C₆)alkyle ;
. -X-R_{c}, X étant un lien, un groupe (C₁-C₆)alkylène, de préférence -CH₂, ou -C(=O)-, et R_{c} étant un groupe hétérocycloalkyle, éventuellement substitué par au moins un groupe (C₁-C₆)alkyle, SO₂CH₃ ou (C₁-C₆)alkylamino ;
. halogène,
. -O-benzyl,
. -O-C(=O)-NRₐR_{b}, Rₐ et R_{b} , indépendamment l'un de l'autre, étant H ou un groupe (C₁-C₆)alkyle ;
. -NH-C(=O)-Rₐ, Rₐ étant H ou un groupe alkyle (C₁ -C₆) ;
. -C(=O)-NRₐR_{b}, Rₐ et R_{b}, indépendamment l'un de l'autre, étant H ou un groupe (C₁ -C₆)alkyle ; ou
. -C(=O)-N(R_{d})-X₁-NRₐR_{b}, R_{d}, Rₐ et R_{b}, indépendamment les uns des autres, étant H ou un groupe (C₁-C₆)alkyle, et X₁ étant un groupe (C₁-C₆)alkylène, de préférence CH₂ ;
. hétéroaryloxy, ledit hétéroaryloxy étant éventuellement substitué par au moins un substituant tel que
-C(=O)-NRₐR_{b}, Rₐ et R_{b}, indépendamment l'un de l'autre, étant H ou un groupe (C₁-C₆)alkyle ;
. -O-halo(C₁-C₆)alkyl,
. cyano,
. (C₁ -C₆)alkoxy,
. -X-NRₐR_{b}, X étant choisi dans le groupe constitué par : (C₁-C₆)groupe alkylène, -O-C(=O)-, -C(=O)-, et -NH-C(=O)-, et Rₐ et R_{b}, indépendamment l'un de l'autre, étant H ou un groupe (C₁ -C₆)alkyle ;
. -X'-R_{c}, X' étant -C(=O)- ou un radical -O-(C₁-C₆)alkylène, R_{c} étant un groupe cycloalkyle, éventuellement substitué par au moins un halogène, ou R_{c} étant un groupe hétérocycloalkyle, éventuellement substitué par SO₂CH₃ ou au moins un groupe (C₁ -C₆)alkyle ; et
. -O-X₂-NRₑR_{f}, X₂ étant un groupe (C₁-C₆)alkylène, Rₑ et R_{f} , indépendamment l'un de l'autre, étant H ou un groupe (C₁ -C₆ )alkyle, ou au moins un des Rₑ et R_{f} est un groupe protecteur aminé tel qu'un carbamate (Boc) ;
ou ses sels, racémates, diastéréoisomères ou énantiomères pharmaceutiquement acceptables.

2. Composé de la revendication 1, dans lequel R est un groupe cycloalkyle (C₃ -C₇), de préférence un groupe cyclohexyle non substitué.

3. Composé de la revendication 1, dans lequel R est un groupe aryle, éventuellement substitué par au moins un substituant R₃ tel que défini dans la revendication 1, de préférence un groupe phényle éventuellement substitué.

4. Le composé de la revendication 1 ou 3, ayant la formule suivante (II) : 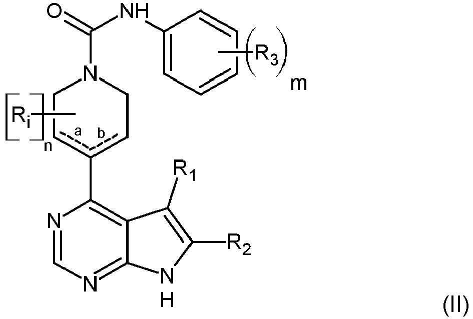 dans laquelle :
- a, b, n, Rᵢ, R₁, et R₂ sont tels que définis dans la revendication 1, et
- m is 1 or 2, et chaque R₃ , identique ou différent, est tel que défini dans la revendication 1.

5. Composé de l'une des revendications 1, 3 ou 4, ayant la formule suivante (III) : 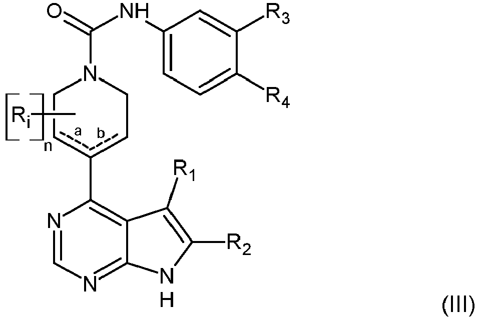 dans laquelle :
- a, b, n, Rᵢ, R₁, R₂, et R₃ sont tels que définis dans la revendication 1, et
- R₄ est H, halogène ou aryloxy.

6. Composé de l'une quelconque des revendications 1 et 3 à 5, ayant la formule suivante (IV) : 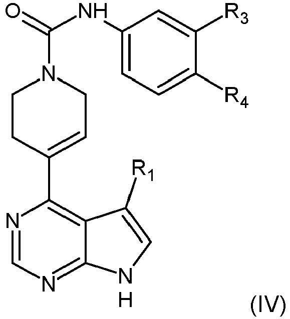 dans laquelle :
- R₁ est H ou un groupe alkyle (C₁-C₆) ;
- R₃ est choisi dans le groupe constitué par :
. H,
. (C₁ -C₆)alkyle,
. aryle, de préférence phényle, ledit aryle étant éventuellement substitué par au moins un substituant tel que (C₁-C₆)alkoxy, en particulier OMe, ou halogène tel que F,
. hétéroaryle, tel que pyridinyle, imidazolyle, pyrrolyle ou triazolyle
. halogène, tel que F, CI ou Br,
. halo(C₁ -C₆)alkyle, tel que CF₃,
. aryloxy, en particulier phényloxy, ledit aryloxy étant éventuellement substitué par au moins un substituant tel que (C₁-C₆)alkoxy, de préférence OMe,
.. -O-halo(C₁-C₆)alkyl, tel que OCF₃,
. cyano,
. (C₁-C₆)alkoxy, tel que OMe, et
. -O-C(=O)-NRₐR_{b}, Rₐ et R_{b}, indépendamment l'un de l'autre, étant H ou un groupe (C₁-C₆)alkyle ; et
- R₄ est H, halogène ou aryloxy, tel que phényloxy,
soit R₃ et R₄ forment avec les atomes de carbone qui les portent un groupe phényle ou un groupe hétérocycloalkyle comprenant de préférence 2 atomes d'oxygène et 3 atomes de carbone.

7. Composé de l'une quelconque des revendications 1, 3 et 4, ayant la formule suivante (V) : 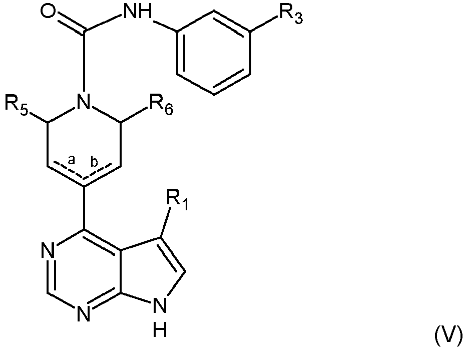 dans laquelle :
- "a" est une liaison simple et "b" est une liaison double, ou "a" est une liaison double et "b" est une liaison simple
- R₁ est un groupe alkyle (C₁-C₆), de préférence un groupe méthyle ;
- R₅ est un groupe alkyle (C₁-C₆), de préférence un groupe méthyle ;
- R₆ est H ou un groupe alkyle (C₁-C₆), de préférence méthyle ;
- R₃ est choisi dans le groupe constitué par :
. halogène, tel que F, CI ou Br, et
. aryloxy, en particulier phenyloxy.

8. Composé de l'une quelconque des revendications 1, 3 et 4, ayant la formule suivante (VI) : 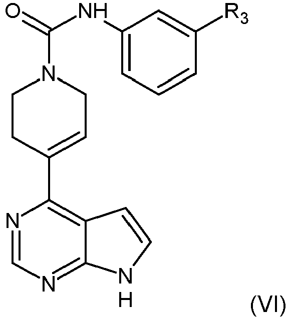 dans laquelle R₃ est un halogène.

9. Composé de l'une quelconque des revendications 1, 3 et 4, ayant la formule suivante (VII) : 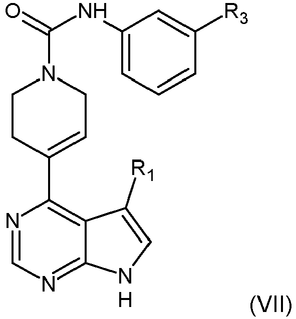 dans laquelle :
- R₁ est un halogène, de préférence CI ; et
- R₃ is -O-C(=O)-NRₐR_{b}, Rₐ et R_{b}, indépendamment l'un de l'autre, étant H ou un groupe (C₁-C₆)alkyle.

10. Composé de l'une quelconque des revendications 1, 3 et 4, ayant la formule suivante (VIII) : 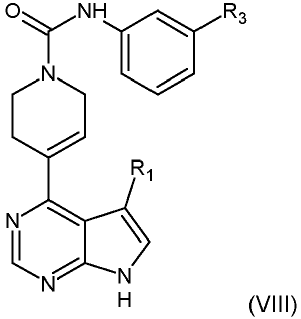 dans laquelle :
- R₁ est un groupe cycloalkyle (C₃-C₇), de préférence cyclopropyle ;
- R₃ est choisi dans le groupe constitué par :
. aryle, de préférence phényle, ledit aryle étant éventuellement substitué par au moins un substituant tel que (C₁-C₆)alkoxy, en particulier OMe,
. hétéroaryle, tel que le pyrrolyle
. halogène, tel que F, CI ou Br,
. aryloxy, en particulier phényloxy, ledit aryloxy étant éventuellement substitué par au moins un substituant tel que (C₁-C₆)alkoxy, de préférence OMe, et
. -O-C(=O)-NRₐR_{b}, Rₐ et R_{b}, indépendamment l'un de l'autre, étant H ou un groupe (C₁-C₆)alkyle.

11. Composé de l'une quelconque des revendications 1, 3 et 4, ayant la formule suivante (IX) : 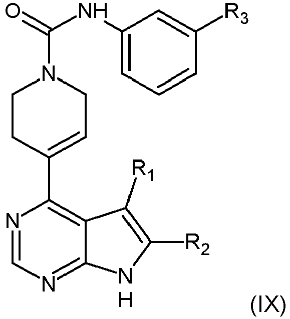 dans laquelle :
- R₁ est un groupe alkyle (C₁-C₆), de préférence un groupe méthyle ;
- R₂ est un groupe alkyle (C₁-C₆), de préférence un groupe méthyle ;
- R₃ est choisi dans le groupe constitué par :
. H,
. aryle, de préférence phényle, ledit aryle étant éventuellement substitué par au moins un substituant tel que (C₁-C₆)alkoxy, en particulier OMe,
. halogène, tel que F, CI ou Br,
. (C₁-C₆)alkoxy, tel que OMe, et
. -O-C(=O)-NRₐR_{b}, Rₐ et R_{b}, indépendamment l'un de l'autre, étant H ou un groupe (C₁ -C₆ )alkyle.

12. Composé de l'une quelconque des revendications 1, 3 et 4, ayant la formule suivante (X) : 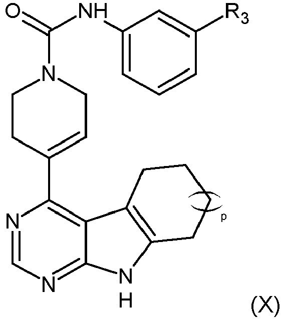 dans laquelle :
- p est 0, 1 ou 2 ;
- R₃ est choisi dans le groupe constitué par :
. H,
. (C₁-C₆)alkyle, ledit alkyle étant éventuellement interrompu par au moins un hétéroatome,
hétérocycloalkyle, tel que le morpholinyle,
. aryle, de préférence phényle, ledit aryle étant éventuellement substitué par au moins un substituant tel que (C₁-C₆)alkoxy,
. hétéroaryle, tel que le pyridinyle
. halogène,
. aryloxy
. halo(C₁-C₆)alkyle,
. cyano,
. (C₁-C₆)alkoxy, et
. -X-NRₐR_{b}, X étant choisi dans le groupe constitué par : (C₁-C₆)groupe alkylène, -O-C(=O)-, -C(=O)-, et -NH-C(=O)-, et Rₐ et R_{b} , indépendamment l'un de l'autre, étant H ou un groupe (C₁-C₆)alkyle.

13. Composé de l'une quelconque des revendications 1, 3 et 4, ayant la formule suivante (XI) : 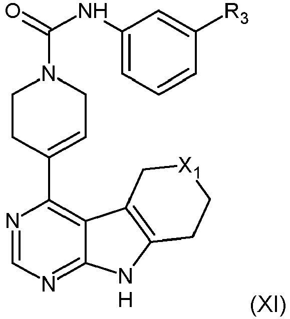 dans laquelle :
- X₁ est -O-, -S-, ou -N((Ci -C₆ )alkyl)-, tel que -N(Me)- ; et
- R₃ est un halogène.

14. Médicament comprenant un composé selon l'une des revendications 1 à 13, ou un sel pharmaceutiquement acceptable de celui-ci.

15. Composé selon l'une des revendications 1 à 13, pour utilisation dans une méthode de traitement d'une condition sélectionnée dans le groupe consistant en : cancers, infections virales, hypertension oculaire et formation de glaucome, neurofibromatose de type 1 et 2, lésions psoriasiques, maladies inflammatoires et hyperalgésie, sensibilisation centrale et douleur chronique, reproduction, dysfonctionnement érectile, et maladies neuronales.
